(19) Europäisches Patentamt
European Patent Office
Office européen des brevets

(11) EP 1 655 364 A2

(12) EUROPEAN PATENT APPLICATION

(43) Date of publication:
**10.05.2006 Bulletin 2006/19**

(51) Int Cl.:
***C12N 15/00*** *(2006.01)*

(21) Application number: **05023477.2**

(22) Date of filing: **27.10.2005**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **05.11.2004 EP 04026295**
**08.12.2004 EP 04029024**
**03.02.2005 EP 05002262**
**11.02.2005 EP 05002850**

(71) Applicant: **BASF Plant Science GmbH**
**67056 Ludwigshafen (DE)**

(72) Inventors:
- **Keetmann, Ulrich, Dr.**
  **06484 Quedlinburg (DE)**
- **Duwenig, Elke, Dr.**
  **67061 Ludwigshafen (DE)**
- **Loyall, Linda Patricia, Dr.**
  **68229 Mannheim (DE)**
- **Herbers, Karin, Dr.**
  **67434 Neustadt (DE)**
- **Hillebrand, Helke, Dr.**
  **68159 Mannheim (DE)**

(74) Representative: **Bieberbach, Andreas et al**
**BASF Aktiengesellschaft**
**67056 Ludwigshafen (DE)**

# (54) Expression cassettes for seed-preferential expression in plants

(57) The present invention relates to expression cassettes comprising transcription regulating sequences with seed-preferential or seed-specific expression profiles in plants obtainable from *Arabidopsis thaliana* genes At4g12910, At1g66250, At4g00820, At2g36640, At2g34200, At3g11180, At4g00360, At2g48030, At2g38590, At1g23000, At3g15510, At3g50870, At4g00220, or At4g26320.

EP 1 655 364 A2

## Description

### FIELD OF THE INVENTION

The present invention relates to expression cassettes comprising transcription regulating nucleotide sequences with seed-preferential or seed-specific expression profiles in plants obtainable from *Arabidopsis thaliana* genes At4g12910, At1g66250, At4g00820, At2g36640, At2g34200, At3g11180, At4g00360, At2g48030, At2g38590, Atlg23000, At3g 15510, At3g50870, At4gGO220, or At4g26320.

### BACKGROUND OF THE INVENTION

Manipulation of plants to alter and/or improve phenotypic characteristics (such as productivity or quality) requires the expression of heterologous genes in plant tissues. Such genetic manipulation relies on the availability of a means to drive and to control gene expression as required. For example, genetic manipulation relies on the availability and use of suitable promoters which are effective in plants and which regulate gene expression so as to give the desired effect (s) in the transgenic plant.

The seed-preferential or seed-specific promoters are useful for expressing genes as well as for producing large quantities of protein, for expressing oils or proteins of interest, e.g., antibodies, genes for increasing the nutritional value of the seed and the like. It is advantageous to have the choice of a variety of different promoters so that the most suitable promoter may be selected for a particular gene, construct, cell, tissue, plant or environment. Moreover, the increasing interest in cotransforming plants with multiple plant transcription units (PTU) and the potential problems associated with using common regulatory sequences for these purposes merit having a variety of promoter sequences available.

There is, therefore, a great need in the art for the identification of novel sequences that can be used for expression of selected transgenes in economically important plants. It is thus an objective of the present invention to provide new and altemative expression cassettes for seed-preferential or seed-specific expression of transgenes in plants. The objective is solved by the present invention.

### SUMMARY OF THE INVENTION

Accordingly, a first embodiment of the invention relates to an expression cassette for seed-specific or seed-preferential transcription (e.g., of an operatively linked nucleic acid sequence) in plants comprising

i) at least one transcription regulating nucleotide sequence of a plant gene, said plant gene selected from the group of genes described by the GenBank *Arabidopsis thaliana* genome loci At4g12910, At1g66250, At4g00820, At2g36640, At2g34200, At3g11180, At4g00360, At2g48030, At2g38590, At1g23000, At3g15510, At3g50870, At4g00220, or At4g26320, or a functional equivalent thereof, and functionally linked thereto

ii) at least one nucleic acid sequence which is heterologous in relation to said transcription regulating nucleotide sequence.

Preferably, the transcription regulating nucleotide sequence (or the functional equivalent thereof) is selected from the group of sequences consisting of

i) the sequences described by SEQ ID NOs: 1, 2, 3, 4, 5, 6, 7, 8, 9, 10, 11, 12, 15, 16, 17, 18, 19, 20, 23, 24, 25, 26, 27, 28, 31, 32, 33, 36, 37, 38, 39, 40, 41, 42, 43, 44, 45, 46, 47, 50, 51, 52, 53, 54, 55, 56, 57, 60, 61, 62, 63, 64, 65, 66, 67, 68, 69, 70, 71, 74, 75, 76, 77, 80, 81, 84, 85, 86, 87, 148, 149, 150, 151, 152, 153, 154, 155, 158, 159, 160, 161, 162, 163, 164, 165, 168, 169, 170, 171, 172, 173, 174, 175, 178, 179, 180, 181, 182, 183, 184, and 185,

ii) a fragment of at least 50 consecutive bases of a sequence under i) which has substantially the same promoter activity as the corresponding transcription regulating nucleotide sequence described by SEQ ID NO: 1, 2, 3, 4, 5, 6, 7, 8, 9, 10, 11, 12, 15, 16, 17, 18, 19, 20, 23, 24, 25, 26, 27, 28, 31, 32, 33, 36, 37, 38, 39, 40, 41, 42, 43, 44, 45, 46, 47, 50, 51, 52, 53, 54, 55, 56, 57, 60, 61, 62, 63, 64, 65, 66, 67, 68, 69, 70, 71, 74, 75, 76, 77, 80, 81, 84, 85, 86, 87, 148, 149, 150, 151, 152, 153, 154, 155, 158, 159, 160, 161, 162, 163, 164, 165, 168, 169, 170, 171, 172, 173, 174, 175, 178, 179, 180, 181, 182, 183, 184, or 185 ;

iii) a nucleotide sequence having substantial similarity (e.g., with a sequence identity of at least 40, 50, 60, to 70%, e.g., preferably 71%, 72%, 73%, 74%, 75%, 76%, 77%, 78%, to 79%, generally at least 80%, e.g., 81% to 84%, at least 85%, e.g., 86%, 87%, 88%, 89%, 90%, 91%, 92%, 93%, 94%, 95%, 96%, 97%, to 98% and 99%) to a transcription regulating nucleotide sequence described by SEQ ID NO: 1, 2, 3, 4, 5, 6, 7, 8, 9, 10, 11, 12, 15, 16, 17, 18, 19, 20, 23, 24, 25, 26, 27, 28, 31, 32, 33, 36, 37, 38, 39, 40, 41, 42, 43, 44, 45, 46, 47, 50, 51, 52, 53, 54, 55, 56, 57, 60, 61, 62, 63, 64, 65, 66, 67, 68, 69, 70, 71, 74, 75, 76, 77, 80, 81, 84, 85, 86, 87, 148, 149, 150, 151, 152, 153, 154, 155, 158, 159, 160, 161, 162, 163, 164, 165, 168, 169, 170, 171, 172, 173, 174, 175, 178, 179, 180, 181, 182, 183, 184, or 185

iv) a nucleotide sequence capable of hybridizing under conditions equivalent to hybridization in 7% sodium dodecyl sulfate (SDS), 0.5 M $NaPO_4$, 1 mM EDTA at 50°C with washing in 2 X SSC, 0. 1% SDS at 50°C (more desirably in 7% sodium dodecyl sulfate (SDS), 0.5 M $NaPO_4$, 1 mM EDTA at 50°C with washing in 1 X SSC, 0.1% SDS at 50°C, more desirably still in 7% sodium dodecyl sulfate (SDS), 0.5 M $NaPO_4$, 1 mM EDTA at 50°C with washing in 0.5 X SSC, 0. 1% SDS at 50°C, preferably in 7% sodium dodecyl sulfate (SDS), 0.5 M $NaPO_4$, 1 mM EDTA at 50°C with washing in 0.1 X SSC, 0.1 % SDS at 50°C, more preferably in 7% sodium dodecyl sulfate (SDS), 0.5 M $NaPO_4$, 1 mM EDTA at 50°C with washing in 0.1 X SSC, 0.1% SDS at 65°C) to a transcription regulating nucleotide sequence described by SEQ ID NO: 1, 2, 3, 4, 5, 6, 7, 8, 9, 10, 11, 12, 15, 16, 17, 18, 19, 20, 23, 24, 25, 26, 27, 28, 31, 32, 33, 36, 37, 38, 39, 40, 41, 42, 43, 44, 45, 46, 47, 50, 51, 52, 53, 54, 55, 56, 57, 60, 61, 62, 63, 64, 65, 66, 67, 68, 69, 70, 71, 74, 75, 76, 77, 80, 81, 84, 85, 86, 87, 148, 149, 150, 151, 152, 153, 154, 155, 158, 159, 160, 161, 162, 163, 164, 165, 168, 169, 170, 171, 172, 173, 174, 175, 178, 179, 180, 181, 182, 183, 184, or 185, or the complement thereof;

v) a nucleotide sequence capable of hybridizing under conditions equivalent to hybridization in 7% sodium dodecyl sulfate (SDS), 0.5 M $NaPO_4$, 1 mM EDTA at 50°C with washing in 2 X SSC, 0. 1% SDS at 50°C (more desirably in 7% sodium dodecyl sulfate (SDS), 0.5 M $NaPO_4$, 1 mM EDTA at 50°C with washing in 1 X SSC, 0.1% SDS at 50°C, more desirably still in 7% sodium dodecyl sulfate (SDS), 0.5 M $NaPO_4$, 1 mM EDTA at 50°C with washing in 0.5 X SSC, 0. 1% SDS at 50°C, preferably in 7% sodium dodecyl sulfate (SDS), 0.5 M $NaPO_4$, 1 mM EDTA at 50°C with washing in 0.1 X SSC, 0.1% SDS at 50°C, more preferably in 7% sodium dodecyl sulfate (SDS), 0.5 M $NaPO_4$, 1 mM EDTA at 50°C with washing in 0.1 X SSC, 0.1% SDS at 65°C) to a nucleic acid comprising 50 to 200 or more consecutive nucleotides of a transcription regulating nucleotide sequence described by SEQ ID NO: 1, 2, 3, 4, 5, 6, 7, 8, 9, 10, 11, 12, 15, 16, 17, 18, 19, 20, 23, 24, 25, 26, 27, 28, 31, 32, 33, 36, 37, 38, 39, 40, 41, 42, 43, 44, 45, 46, 47, 50, 51, 52, 53, 54, 55, 56, 57, 60, 61, 62, 63, 64, 65, 66, 67, 68, 69, 70, 71, 74, 75, 76, 77, 80, 81, 84, 85, 86, 87, 148, 149, 150, 151, 152, 153, 154, 155, 158, 159, 160, 161, 162, 163, 164, 165, 168, 169, 170, 171, 172, 173, 174, 175, 178, 179, 180, 181, 182, 183, 184, and 185, or the complement thereof;

vi) a nucleotide sequence which is the complement or reverse complement of any of the previously mentioned nucleotide sequences under i) to v).

The functional equivalent of the transcription regulating nucleotide sequence is obtained or obtainable from plant genomic DNA from a gene encoding a polypeptide which has at least 70% amino acid sequence identity to a polypeptide selected from the group described by SEQ ID NO: 14, 22, 30, 35, 49, 59, 73, 79, 83, 89, 157, 167, 177, and 187, respectively.

The expression cassette may be employed for numerous expression purposes such as for example expression of a protein, or expression of a antisense RNA, sense or double-stranded RNA. Preferably, expression of the nucleic acid sequence confers to the plant an agronomically valuable trait.

Other embodiments of the invention relate to vectors comprising an expression cassette of the invention, and transgenic host cell or non-human organism comprising an expression cassette or a vector of the invention. Preferably the organism is a plant.

Another embodiment of the invention relates to a method for identifying and/or isolating a sequence with seed-specific or seed-preferential transcription regulating activity characterized that said identification and/or isolation utilizes a nucleic acid sequence encoding a amino acid sequence as described by SEQ ID NO: 14, 22, 30, 35, 49, 59, 73, 79, 83, 89, 157, 167, 177, or 187 or a part of at least 15 bases thereof. Preferably the nucleic acid sequences is described by SEQ ID NO: 13, 21, 29, 34, 48, 58, 72, 78, 82, 88, 156, 166, 176, or 186 or a part of at least 15 bases thereof. More preferably, identification and/or isolation is realized by a method selected from polymerase chain reaction, hybridization, and database screening.

Another embodiment of the invention relates to a method for providing a transgenic expression cassette for seed-specific or seed-preferential expression comprising the steps of:

I. isolating of a seed-preferential or seed-specific transcription regulating nucleotide sequence u6lizing at least one nucleic acid sequence or a part thereof, wherein said sequence is encoding a polypeptide described by SEQ ID NO: 14, 22, 30, 35, 49, 59, 73, 79, 83, 89, 157, 167, 177, or 187, or a part of at least 15 bases thereof, and

II. functionally linking said seed-preferential or seed-specific transcription regulating nucleotide sequence to another nudeotide sequence of interest, which is heterologous in relation to said seed-preferentlat or seed-specific transcription regulating nucleotide sequence.

## DEFINITIONS

It is to be understood that this invention is not limited to the particular methodology, protocols, cell lines, plant species or genera, constructs, and reagents described as such. It is also to be understood that the terminology used herein is for the purpose of describing particular embodiments only, and is not intended to limit the scope of the present invention which will be limited only by the appended claims. It must be noted that as used herein and in the appended claims, the singular forms "a," "and," and "the" include plural reference unless the context clearly dictates otherwise. Thus, for example, reference to "a vector" is a reference to one or more vectors and includes equivalents thereof known to those skilled in the art, and so forth.

The term "about" is used herein to mean approximately, roughly, around, or in the region of. When the term "about" is used in conjunction with a numerical range, it modifies that range by extending the boundaries above and below the numerical values set forth. In general, the term "about" is used herein to modify a numerical value above and below the stated value by a variance of 20 per-cent up or down (higher or lower). As used herein, the word "or" means any one member of a particular list and also includes any combination of members of that list.

The term "gene" is used broadly to refer to any segment of nucleic acid associated with a biological function. Thus, genes include coding sequences and/or the regulatory sequences required for their expression. For example, gene refers to a nucleic acid fragment that expresses mRNA or functional RNA, or encodes a specific protein, and which includes regulatory sequences. Genes also include non-expressed DNA segments that, for example, form recognition sequences for other proteins. Genes can be obtained from a variety of sources, including cloning from a source of interest or synthesizing from known or predicted sequence information, and may include sequences designed to have desired parameters.

The term "native" or "wild type" gene refers to a gene that is present in the genome of an untransformed cell, i.e., a cell not having a known mutation.

A "marker gene" encodes a selectable or screenable trait.

The term "chimeric gene" refers to any gene that contains

1) DNA sequences, including regulatory and coding sequences, that are not found together in nature, or

2) sequences encoding parts of proteins not naturally adjoined, or

3) parts of promoters that are not naturally adjoined.

Accordingly, a chimeric gene may comprise regulatory sequences and coding sequences that are derived from different sources, or comprise regulatory sequences. and coding sequences derived from the same source, but arranged in a manner different from that found in nature.

A "transgene" refers to a gene that has been introduced into the genome by transformation and is stably maintained. Transgenes may include, for example, genes that are either heterologous or homologous to the genes of a particular plant to be transformed. Additionally, transgenes may comprise native genes inserted into a non-native organism, or chimeric genes. The term "endogenous gene" refers to a native gene in its natural location in the genome of an organism. A "foreign" gene refers to a gene not normally found in the host organism but that is introduced by gene transfer.

An "oligonucleotide" corresponding to a nucleotide sequence of the invention, e.g., for use in probing or amplification reactions, may be about 30 or fewer nucleotides in length (e.g., 9, 12, 15, 18, 20, 21 or 24, or any number between 9 and 30). Generally specific primers are upwards of 14 nucleotides in length. For optimum specificity and cost effectiveness, primers of 16 to 24 nucleotides in length may be preferred. Those skilled in the art are well versed in the design of primers for use processes such as PCR. If required, probing can be done with entire restriction fragments of the gene disclosed herein which may be 100’s or even 1000’s of nucleotides in length.

The terms "protein," "peptide" and "polypeptide" are used interchangeably herein. The nucleotide sequences of the invention can be introduced into any plant. The genes to be introduced can be conveniently used in expression cassettes for introduction and expression in any plant of interest. Such expression cassettes will comprise the transcriptional initiation region of the invention linked to a nucleotide sequence of interest. Preferred promoters include constitutive, tissue-specific, developmental-specific, inducible and/or viral promoters, most preferred are the seed-specific or seed-preferential promoters of the invention. Such an expression cassette is provided with a plurality of restriction sites for insertion of the gene of interest to be under the transcriptional regulation of the regulatory regions. The expression cassette may additionally contain selectable marker genes. The cassette will include in the 5'-3' direction of transcription, a transcriptional and translational initiation region, a DNA sequence of interest, and a transcriptional and translational termination region functional in plants. The termination region may be native with the transcriptional initiation region, may be native with the DNA sequence of interest, or may be derived from another source. Convenient termination regions are available from the Tr-plasmid of *A. tumefaciens*, such, as the octopine synthase and nopaline synthase termination regions (see also, Guerineau 1991; Proudfoot 1991; Sanfacon 1991; Mogen 1990; Munroe 1990; Ballas 1989; Joshi 1987).

"Coding sequence" refers to a DNA or RNA sequence that codes for a specific amino acid sequence and excludes the non-coding sequences. It may constitute an "uninterrupted coding sequence", i.e., lacking an intron, such as in a cDNA or it may include one or more introns bounded by appropriate splice junctions. An "intron" is a sequence of RNA which is contained in the primary transcript but which is removed through cleavage and re-ligation of the RNA within the cell to create the mature mRNA that can be translated into a protein.

The terms "open reading frame" and "ORF" refer to the amino acid sequence encoded between translation initiation and termination codons of a coding sequence. The terms "initiation codon" and "termination codon" refer to a unit of three adjacent nucleotides ('codon') in a coding sequence that specifies initiation and chain termination, respectively, of protein synthesis (mRNA translation).

A "functional RNA" refers to an antisense RNA, ribozyme, or other RNA that is not translated.

The term "RNA transcript" refers to the product resulting from RNA polymerase catalyzed transcription of a DNA sequence. When the RNA transcript is a perfect complementary copy of the DNA sequence, it is referred to as the primary transcript or it may be a RNA sequence derived from posttranscriptional processing of the primary transcript and is referred to as the mature RNA. "Messenger RNA" (mRNA) refers to the RNA that is without introns and that can be translated into protein by the cell. "cDNA" refers to a single- or a double-stranded DNA that is complementary to and derived from mRNA.

"Transcription regulating nucleotide sequence", "regulatory sequences", and "suitable regulatory sequences", each refer to nucleotide sequences influencing the transcription, RNA processing or stability, or translation of the associated (or functionally linked) nucleotide sequence to be transcribed. The transcription regulating nudeotide sequence may have various localizations with the respect to the nucleotide sequences to be transcribed. The transcription regulating nucleotide sequence may be located upstream (5' non-coding sequences), within, or downstream (3' non-coding sequences) of the sequence to be transcribed (e.g., a coding sequence). The transcription regulating nucleotide sequences may be selected from the group comprising enhancers, promoters, translation leader sequences, introns, 5'-untranslated sequences, 3'-untranslated sequences, and polyadenylation signal sequences. They include natural and synthetic sequences as well as sequences, which may be a combination of synthetic and natural sequences. As is noted above, the term "transcription regulating nucleotide sequence" is not limited to promoters. However, preferably a transcription regulating nucleotide sequence of the invention comprises at least one promoter sequence (e.g., a sequence localized upstream of the transcription start of a gene capable to induce transcription of the downstream sequences). In one preferred embodiment the transcription regulating nucleotide sequence of the invention comprises the promoter sequence of the corresponding gene and - optionally and preferably - the native 5'-untranslated region of said gene. Furthermore, the 3'-untranslated region and/or the polyadenylation region of said gene may also be employed.

"5' non-coding sequence" refers to a nucleotide sequence located 5' (upstream) to the coding sequence. It is present in the fully processed mRNA upstream of the initiation codon and may affect processing of the primary transcript to mRNA, mRNA stability or translation efficiency (Turner 1995).

"3' non-coding sequence" refers to nucleotide sequences located 3' (downstream) to a coding sequence and include polyadenylation signal sequences and other sequences encoding regulatory signals capable of affecting mRNA processing or gene expression. The polyadenylation signal is usually characterized by affecting the addition of polyadenylic acid tracts to the 3' end of the mRNA precursor. The use of different 3' non-coding sequences is exemplified by Ingelbrecht et al., 1989.

The term "translation leader sequence" refers to that DNA sequence portion of a gene between the promoter and coding sequence that is transcribed into RNA and is present in the fully processed mRNA upstream (5') of the translation start codon. The translation leader sequence may affect processing of the primary transcript to mRNA, mRNA stability or translation efficiency.

"Signal peptide" refers to the amino terminal extension of a polypeptide, which is translated in conjunction with the polypeptide forming a precursor peptide and which is required for its entrance into the secretory pathway. The term "signal sequence" refers to a nucleotide sequence that encodes the signal peptide. The term "transit peptide" as used herein refers part of a expressed polypeptide (preferably to the amino terminal extension of a polypeptide), which is translated in conjunction with the polypeptide forming a precursor peptide and which is required for its entrance into a cell organelle (such as the plastids (e.g., chloroplasts) or mitochondria). The term "transit sequence" refers to a nucleotide sequence that encodes the transit peptide.

"Promoter" refers to a nucleotide sequence, usually upstream (5') to its coding sequence, which controls the expression of the coding sequence by providing the recognition for RNA polymerase and other factors required for proper transcription. "Promoter" includes a minimal promoter that is a short DNA sequence comprised of a TATA box and other sequences that serve to specify the site of transcription initiation, to which regulatory elements are added for control of expression. "Promoter" also refers to a nucleotide sequence that includes a minimal promoter plus regulatory elements that is capable of controlling the expression of a coding sequence or functional RNA. This type of promoter sequence consists of proximal and more distal upstream elements, the latter elements often referred to as enhancers. Accordingly, an "enhancer" is a DNA sequence which can stimulate promoter activity and may be an innate element of the promoter or a heterologous element inserted to enhance the level or tissue specificity of a promoter. It is capable of operating in both orientations (normal or flipped), and is capable of functioning even when moved either upstream or downstream from the promoter. Both enhancers and other upstream promoter elements bind sequence-specific DNA-binding proteins that mediate their effects. Promoters may be derived in their entirety from a native gene, or be composed of different elements, derived from different promoters found in nature, or even be comprised of synthetic DNA segments. A promoter may also contain DNA sequences that are involved in the binding of protein factors which control the effectiveness of transcription initiation in response to physiological or developmental conditions.

The "initiation site" is the position surrounding the first nucleotide that is part of the transcribed sequence, which is also defined as position +1. With respect to this site all other sequences of the gene and its controlling regions are numbered. Downstream sequences (i.e., further protein encoding sequences in the 3' direction) are denominated positive, while upstream sequences (mostly of the controlling regions in the 5' direction) are denominated negative.

Promoter elements, particularly a TATA element, that are inactive or that have greatly reduced promoter activity in the absence of upstream activation are referred to as "minimal or core promoters." In the presence of a suitable transcription factor, the minimal promoter functions to permit transcription. A "minimal or core promoter" thus consists only of all basal elements needed for transcription initiation, e.g., a TATA box and/or an initiator.

"Constitutive expression" refers to expression using a constitutive or regulated promoter. "Conditional" and "regulated expression" refer to expression controlled by a regulated promoter.

"Constitutive promoter" refers to a promoter that is able to express the open reading frame (ORF) that it controls in all or nearly all of the plant tissues during all or nearly all developmental stages of the plant. Each of the transcription-activating elements do not exhibit an absolute tissue-specificity, but mediate transcriptional activation in most plant parts at a level of at least 1 % of the level reached in the part of the plant in which transcription is most active.

"Regulated promoter" refers to promoters that direct gene expression not constitutively, but in a temporally- and/or spatially-regulated manner, and includes both tissue-specific and inducible promoters. It includes natural and synthetic sequences as well as sequences which may be a combination of synthetic and natural sequences. Different promoters may direct the expression of a gene in different tissues or cell types, or at different stages of development, or in response to different environmental conditions. New promoters of various types useful in plant cells are constantly being discovered, numerous examples may be found in the compilation by Okamuro et al. (1989). Typical regulated promoters useful in plants include but are not limited to safener-inducible promoters, promoters derived from the tetracycline-inducible system, promoters derived from salicylate-inducible systems, promoters derived from alcohol-inducible systems, promoters derived from glucocorticoid-inducible system, promoters derived from pathogen-inducible systems, and promoters derived from ecdysone-inducible systems.

"Tissue-specific promoter" refers to regulated promoters that are not expressed in all plant cells but only in one or more cell types in specific organs (such as leaves or seeds), specific tissues (such as embryo or cotyledon), or specific cell types (such as leaf parenchyma or seed storage cells). These also include promoters that are temporally regulated, such as in early or late embryogenesis, during fruit ripening in developing seeds or fruit, in fully differentiated leaf, or at the onset of senescence.

"Inducible promoter" refers to those regulated promoters that can be turned on in one or more cell types by an external stimulus, such as a chemical, light, hormone, stress, or a pathogen.

"Operably-linked" or "functionally linked" refers preferably to the association of nucleic acid sequences on single nucleic acid fragment so that the function of one is affected by the other. For example, a regulatory DNA sequence is said to be "operably linked to" or "associated with" a DNA sequence that codes for an RNA or a polypeptide if the two sequences are situated such that the regulatory DNA sequence affects expression of the coding DNA sequence (i.e., that the coding sequence or functional RNA is under the transcriptional control of the promoter). Coding sequences can be operably-linked to regulatory sequences in sense or antisense orientation.

"Expression" refers to the transcription and/or translation of an endogenous gene, ORF or portion thereof, or a transgene in plants. For example, in the case of antisense constructs, expression may refer to the transcription of the antisense DNA only. In addition, expression refers to the transcription and stable accumulation of sense (mRNA) or functional RNA. Expression may also refer to the production of protein.

"Specific expression" is the expression of gene products which is limited to one or a few plant tissues (spatial limitation) and/or to one or a few plant developmental stages (temporal limitation). It is acknowledged that hardly a true specificity exists: promoters seem to be preferably switch on in some tissues, while in other tissues there can be no or only little activity. This phenomenon is known as leaky expression. However, with specific expression in this invention is meant preferable expression in one or a few plant tissues.

The "expression pattern" of a promoter (with or without enhancer) is the pattern of expression levels which shows where in the plant and in what developmental stage transcription is initiated by said promoter. Expression patterns of a set of promoters are said to be complementary when the expression pattern of one promoter shows little overlap with the expression pattern of the other promoter. The level of expression of a promoter can be determined by measuring the 'steady state' concentration of a standard transcribed reporter mRNA. This measurement is indirect since the concentration of the reporter mRNA is dependent not only on its synthesis rate, but also on the rate with which the mRNA is degraded. Therefore, the steady state level is the product of synthesis rates and degradation rates.

The rate of degradation can however be considered to proceed at a fixed rate when the transcribed sequences are identical, and thus this value can serve as a measure of synthesis rates. When promoters are compared in this way techniques available to those skilled in the art are hybridization S1-RNAse analysis, northern blots and competitive RT-PCR. This list of techniques in no way represents all available techniques, but rather describes commonly used procedures used to analyze transcription activity and expression levels of mRNA.

The analysis of transcription start points in practically all promoters has revealed that there is usually no single base at which transcription starts, but rather a more or less clustered set of initiation sites, each of which accounts for some start points of the mRNA. Since this distribution varies from promoter to promoter the sequences of the reporter mRNA in each of the populations would differ from each other. Since each mRNA species is more or less prone to degradation, no single degradation rate can be expected for different reporter mRNAs. It has been shown for various eukaryotic promoter sequences that the sequence surrounding the initiation site ('initiator') plays an important role in determining the level of RNA expression directed by that specific promoter. This includes also part of the transcribed sequences. The direct fusion of promoter to reporter sequences would therefore lead to suboptimal levels of transcription.

A commonly used procedure to analyze expression patterns and levels is through determination of the 'steady state' level of protein accumulation in a cell. Commonly used candidates for the reporter gene, known to those skilled in the art are beta-glucuronidase (GUS), chloramphenicol acetyl transferase (CAT) and proteins with fluorescent properties, such as green fluorescent protein (GFP) from Aequora victoria. In principle, however, many more proteins are suitable for this purpose, provided the protein does not interfere with essential plant functions. For quantification and determination of localization a number of tools are suited. Detection systems can readily be created or are available which are based on, e.g., immunochemical, enzymatic, fluorescent detection and quantification. Protein levels can be determined in plant tissue extracts or in intact tissue using in situ analysis of protein expression.

Generally, individual transformed lines with one chimeric promoter reporter construct will vary in their levels of expression of the reporter gene. Also frequently observed is the phenomenon that such transformants do not express any detectable product (RNA or protein). The variability in expression is commonly ascribed to 'position effects', although the molecular mechanisms underlying this inactivity are usually not clear.

"Overexpression" refers to the level of expression in transgenic cells or organisms that exceeds levels of expression in normal or untransformed (non-transgenic) cells or organisms.

"Antisense inhibition" refers to the production of antisense RNA transcripts capable of suppressing the expression of protein from an endogenous gene or a transgene.

"Gene silencing" refers to homology-dependent suppression of viral genes, transgenes, or endogenous nuclear genes. Gene silencing may be transcriptional, when the suppression is due to decreased transcription of the affected genes, or post-transcriptional, when the suppression is due to increased tumover (degradation) of RNA species homologous to the affected genes (English 1996). Gene silencing includes virus-induced gene silencing (Ruiz et al. 1998).

The terms "heterologous DNA sequence," "exogenous DNA segment" or "heterologous nucleic acid," as used herein, each refer to a sequence that originates from a source foreign to the particular host cell or, if from the same source, is modified from its original form. Thus, a heterologous gene in a host cell includes a gene that is endogenous to the particular host cell but has been modified through, for example, the use of DNA shuffling. The terms also include non-naturally occurring multiple copies of a naturally occurring DNA sequence. Thus, the terms refer to a DNA segment that is foreign or heterologous to the cell, or homologous to the cell but in a position within the host cell

nucleic acid in which the element is not ordinarily found. Exogenous DNA segments are expressed to yield exogenous polypeptides. A "homologous" DNA sequence is a DNA sequence that is naturally associated with a host cell into which it is introduced.

"Homologous to" in the context of nucleotide sequence identity refers to the similarity between the nucleotide sequence of two nucleic acid molecules or between the amino acid sequences of two protein molecules. Estimates of such homology are provided by either DNA-DNA or DNA-RNA hybridization under conditions of stringency as is well understood by those skilled in the art (as described in Haines and Higgins (eds.), Nucleic Acid Hybridization, IRL Press, Oxford, U.K.), or by the comparison of sequence similarity between two nucleic acids or proteins.

The term "substantially similar" refers to nucleotide and amino acid sequences that represent functional and/or structural equivalents of *Arabidopsis* sequences disclosed herein.

In its broadest sense, the term "substantially similar" when used herein with respect to a nucleotide sequence means that the nucleotide sequence is part of a gene which encodes a polypeptide having substantially the same structure and function as a polypeptide encoded by a gene for the reference nucleotide sequence, e.g., the nucleotide sequence comprises a promoter from a gene that is the ortholog of the gene corresponding to the reference nucleotide sequence, as well as promoter sequences that are structurally related the promoter sequences particularly exemplified herein, i.e., the substantially similar promoter sequences hybridize to the complement of the promoter sequences exemplified herein under high or very high stringency conditions. For example, altered nucleotide sequences which simply reflect the degeneracy of the genetic code but nonetheless encode amino acid sequences that are identical to a particular amino acid sequence are substantially similar to the particular sequences. The term "substantially similar" also includes nucleotide sequences wherein the sequence has been modified, for example, to optimize expression in particular cells, as well as nucleotide sequences encoding a variant polypeptide having one or more amino acid substitutions relative to the (unmodified) polypeptide encoded by the reference sequence, which substitution(s) does not after the activity of the variant polypeptide relative to the unmodified polypeptide.

In its broadest sense, the term "substantially similar" when used herein with respect to polypeptide means that the polypeptide has substantially the same structure and function as the reference polypeptide. In addition, amino acid sequences that are substantially similar to a particular sequence are those wherein overall amino acid identity is at least 65% or greater to the instant sequences. Modifications that result in equivalent nucleotide or amino acid sequences are well within the routine skill in the art. The percentage of amino acid sequence identity between the substantially similar and the reference polypeptide is at least 65%, 66%, 67%, 68%, 69%, 70%, e.g., 71%, 72%, 73%,

74%, 75%, 76%, 77%, 78%, 79%, 80%, 81%, 82%, 83%, 84%, 85%, 86%, 87%, 88%, 89%, and even 90% or more, e.g., 91%, 92%, 93%, 94%, 95%, 96%, 97%, 98%, up to at least 99%, wherein the reference polypeptide is an *Arabidopsis* polypeptide encoded by a gene with a promoter having any one of SEQ ID NOs: 1, 2, 3, 4, 5, 6, 7, 8, 9, 10, 11, 12, 15, 16, 17, 18, 19, 20, 23, 24, 25, 26, 27, 28, 31, 32, 33, 36, 37, 38, 39, 40, 41, 42, 43, 44, 45, 46, 47, 50, 51, 52, 53, 54, 55, 56, 57, 60, 61, 62, 63, 64, 65, 66, 67, 68, 69, 70, 71, 74, 75, 76, 77, 80, 81, 84, 85, 86, 87, 148, 149, 150, 151, 152, 153, 154, 155, 158, 159, 160, 161, 162, 163, 164, 165, 168, 169, 170, 171, 172, 173, 174, 175, 178, 179, 180, 181, 182, 183, 184, or 185, a nucleotide sequence comprising an open reading frame having any one of SEQ ID NOs: 13, 21, 29, 34, 48, 58, 72, 78, 82, 88, 156, 166, 176, or 186, which encodes one of SEQ ID NOs: 14, 22, 30, 35, 49, 59, 73, 79, 83, 89, 157, 167, 177, or 187. One indication that two polypeptides are substantially similar to each other, besides having substantially the same function, is that an agent, e.g., an antibody, which specifically binds to one of the polypeptides, also specifically binds to the other.

Sequence comparisons maybe carried out using a Smith-Waterman sequence alignment algorithm (see e.g., Waterman (1995)). The locals program, version 1.16, is preferably used with following parameters: match: 1, mismatch penalty: 0.33, open-gap penalty: 2, extended-gap penalty: 2.

Moreover, a nucleotide sequence that is "substantially similar" to a reference nucleotide sequence is said to be "equivalent" to the reference nucleotide sequence. The skilled artisan recognizes that equivalent nucleotide sequences encompassed by this invention can also be defined by their ability to hybridize, under low, moderate and/or stringent conditions (e.g., 0.1 X SSC, 0.1% SDS, 65°C), with the nucleotide sequences that are within the literal scope of the instant claims.

What is meant by "substantially the same activity" when used in reference to a polynucleotide or polypeptide fragment is that the fragment has at least 65%, 66%, 67%, 68%, 69%, 70%, e.g., 71%, 72%, 73%, 74%, 75%, 76%, 77%, 78%, 79%, 80%, 81%, 82%, 83%, 84%, 85%, 86%, 87%, 88%, 89%, and even 90% or more, e.g., 91%, 92%, 93%, 94%, 95%, 96%, 97%, 98%, up to at least 99% of the activity of the full length polynucleotide or full length polypeptide.

'Target gene" refers to a gene on the replicon that expresses the desired target coding sequence, functional RNA, or protein. The target gene is not essential for replicon replication. Additionally, target genes may comprise native non-viral genes inserted into a non-native organism, or chimeric genes, and will be under the control of suitable regulatory sequences. Thus, the regulatory sequences in the target gene may come from any source, including the virus. Target genes may include coding sequences that are either heterologous or homologous to the genes of a particular plant to be transformed. However, target genes do not include native viral genes. Typical target genes include, but are not limited to genes encoding a structural protein, a seed storage protein, a protein that conveys herbicide resistance, and a protein that conveys insect resistance. Proteins encoded by target genes are known as "foreign proteins". The expression of a target gene in a plant will typically produce an altered plant trait.

The term "altered plant trait" means any phenotypic or genotypic change in a transgenic plant relative to the wild-type or non-trarisgenic plant host.

"Replication gene" refers to a gene encoding a viral replication protein. In addition to the ORF of the replication protein, the replication gene may also contain other overlapping or non-overlapping ORF(s), as are found in viral sequences in nature. While not essential for replication, these additional ORFs may enhance replication and/or viral DNA accumulation. Examples of such additional ORFs are AC3 and AL3 in ACMV and TGMV geminiviruses, respectively.

"Chimeric trans-acting replication gene" refers either to a replication gene in which the coding sequence of a replication protein is under the control of a regulated plant promoter other than that in the native viral replication gene, or a modified native viral replication gene, for example, in which a site specific sequence(s) is inserted in the 5' transcribed but untranslated region. Such chimeric genes also include insertion of the known sites of replication protein binding between the promoter and the transcription start site that attenuate transcription of viral replication protein gene.

"Chromosomally-integrated" refers to the integration of a foreign gene or DNA construct into the host DNA by covalent bonds. Where genes are not "chromosomally integrated" they may be "transiently expressed." Transient expression of a gene refers to the expression of a gene that is not integrated into the host chromosome but functions independently, either as part of an autonomously replicating plasmid or expression cassette, for example, or as part of another biological system such as a virus. The term "transformation" refers to the transfer of a nucleic acid fragment into the genome of a host cell, resulting in genetically stable inheritance. Host cells containing the transformed nucleic acid fragments are referred to as "transgenic" cells, and organisms comprising transgenic cells are referred to as "transgenic organisms". Examples of methods of transformation of plants and plant cells include Agrobacterium-mediated transformation (De Blaere 1987) and particle bombardment technology (US 4,945,050). Whole plants may be regenerated from transgenic cells by methods well known to the skilled artisan (see, for example, Fromm 1990).

"Transformed," "transgenic," and "recombinant" refer to a host organism such as a bacterium or a plant into which a heterologous nucleic acid molecule has been introduced. The nucleic acid molecule can be stably integrated into the genome generally known in the art and are disclosed (Sambrook 1989; Innis 1995; Gelfand 1995; Innis & Gelfand 1999. Known methods of PCR include, but are not limited to, methods using paired primers, nested primers, single specific primers, degenerate primers, gene-specific primers, vector-specific primers, partially mismatched primers, and the like. For example, "transformed," "transformant," and "transgenic" plants or calli have been through the transformation process and contain a foreign gene integrated into their chromosome. The term "untransformed" refers to normal plants that have not been through the transformation process.

"Transiently transformed" refers to cells in which transgenes and foreign DNA have been introduced (for example, by such methods as Agrobacterium-mediated transformation or biolistic bombardment), but not selected for stable maintenance.

"Stably transformed" refers to cells that have been selected and regenerated on a selection media following transformation.

"Transient expression" refers to expression in cells in which a virus or a transgene is introduced by viral infection or by such methods as Agrobacterium-mediated transformation, electroporation, or biolistic bombardment, but not selected for its stable maintenance.

"Genetically stable" and "heritable" refer to chromosomally-integrated genetic elements that are stably maintained in the plant and stably inherited by progeny through successive generations.

"Primary transformant" and "T0 generation" refer to transgenic plants that are of the same genetic generation as the tissue which was initially transformed (i.e., not having gone through meiosis and fertilization since transformation).

"Secondary transformants" and the "T1, T2, T3, etc. generations" refer to transgenic plants derived from primary transformants through one or more meiotic and fertilization cycles. They may be derived by self-fertilization of primary or secondary transformants or crosses of primary or secondary transformants with other transformed or untransformed plants.

"Wild-type" refers to a virus or organism found in nature without any known mutation.

"Genome" refers to the complete genetic material of an organism.

The term "nucleic acid" refers to deoxyribonucleotides or ribonucleotides and polymers thereof in either single- or double-stranded form, composed of monomers (nucleotides) containing a sugar, phosphate and a base which is either a purine or pyrimidine. Unless specifically limited, the term encompasses nucleic acids containing known analogs of natural nucleotides which have similar binding properties as the reference nucleic acid and are metabolized in a manner similar to naturally occurring nudeotides. Unless otherwise indicated, a particular nucleic acid sequence also implicitly encompasses consenratively modified variants thereof (e.g., degenerate codon substitutions) and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer 1991; Ohtsuka 1985; Rossolini 1994). A "nucleic acid fragment" is a fraction of a given nucleic acid molecule. In higher plants, deoxyribonucleic acid (DNA) is the genetic material while ribonucleic acid (RNA) is involved in the transfer of information contained within DNA into proteins. The term "nucleotide sequence" refers to a polymer of DNA or RNA which can be single- or double-stranded, optionally containing synthetic, non-natural or altered nucleotide bases capable of incorporation into DNA or RNA polymers. The terms "nucleic acid" or "nucleic acid sequence" may also be used interchangeably with gene, cDNA, DNA and RNA encoded by a gene.

The invention encompasses isolated or substantially purified nucleic acid or protein compositions. In the context of the present invention, an "isolated" or "purified" DNA molecule or an "isolated" or "purified" polypeptide is a DNA molecule or polypeptide that, by the hand of man, exists apart from its native environment and is therefore not a product of nature. An isolated DNA molecule or polypeptide may exist in a purified form or may exist in a non-native environment such as, for example, a transgenic host cell. For example, an "isolated" or "purified" nucleic acid molecule or protein, or biologically active portion thereof, is substantially free of other cellular material, or culture medium when produced by recombinant techniques, or substantially free of chemical precursors or other chemicals when chemically synthesized. Preferably, an "isolated" nucleic acid is free of sequences (preferably protein encoding sequences) that naturally flank the nucleic acid (i.e., sequences located at the 5' and 3' ends of the nucleic acid) in the genomic DNA of the organism from which the nucleic acid is derived. For example, in various embodiments, the isolated nucleic acid molecule can contain less than about 5 kb, 4 kb, 3 kb, 2 kb, 1 kb, 0.5 kb, or 0.1 kb of nucleotide sequences that naturally flank the nucleic acid molecule in genomic DNA of the cell from which the nucleic acid is derived. A protein that is substantially free of cellular material includes preparations of protein or polypeptide having less than about 30%, 20%, 10%, 5%, (by dry weight) of contaminating protein. When the protein of the invention, or biologically active portion thereof, is recombinantly produced, preferably culture medium represents less than about 30%, 20%, 10%, or 5% (by dry weight) of chemical precursors or non-protein of interest chemicals.

The nucleotide sequences of the invention include both the naturally occurring sequences as well as mutant (variant) forms. Such variants will continue to possess the desired activity, i.e., either promoter activity or the activity of the product encoded by the open reading frame of the non-variant nucleotide sequence.

The term "variant" with respect to a sequence (e.g., a polypeptide or nucleic acid sequence such as - for example - a transcription regulating nucleotide sequence of the invention) is intended to mean substantially similar sequences. For nucleotide sequences comprising an open reading frame, variants include those sequences that, because of the degeneracy of the genetic code, encode the identical amino acid sequence of the native protein. Naturally occurring allelic variants such as these can be identified with the use of well-known molecular biology techniques, as, for example, with polymerase chain reaction (PCR) and hybridization techniques. Variant nucleotide sequences also include synthetically derived nucleotide sequences, such as those generated, for example, by using site-directed mutagenesis and for open reading frames, encode the native protein, as well as those that encode a polypeptide having amino acid substitutions relative to the native protein. Generally, nucleotide sequence variants of the invention will have at least 40, 50, 60, to 70%, e.g., preferably 71%, 72%, 73%, 74%, 75%, 76%, 77%, 78%, to 79%, generally at least 80%, e.g., 81%-84%, at least 85%, e.g., 86%, 87%, 88%, 89%, 90%, 91%, 92%, 93%, 94%, 95%, 96%, 97%, to 98% and 99% nucleotide sequence identity to the native (wild type or endogenous) nucleotide sequence.

"Conservatively modified variations" of a particular nucleic acid sequence refers to those nucleic acid sequences that encode identical or essentially identical amino acid sequences, or where the nucleic acid sequence does not encode an amino acid sequence, to essentially identical sequences. Because of the degeneracy of the genetic code, a large number of functionally identical nucleic acids encode any given polypeptide. For instance the codons CGT, CGC, CGA, CGG, AGA, and AGG all encode the amino acid arginine. Thus, at every position where an arginine is specified by a codon, the codon can be altered to any of the corresponding codons described without altering the encoded protein. Such nucleic acid variations are "silent variations" which are one species of "conservatively modified variations." Every nucleic acid sequence described herein which encodes a polypeptide also describes every possible silent variation, except where otherwise noted. One of skill will recognize that each codon in a nucleic acid (except ATG, which is ordinarily the only codon for methionine) can be modified to yield a functionally identical molecule by standard techniques. Accordingly, each "silent variation" of a nucleic acid which encodes a polypeptide is implicit in each described sequence.

The nucleic acid molecules of the invention can be "optimized" for enhanced expression in plants of interest (see, for example, WO 91/16432; Perlak 1991; Murray 1989). In this manner, the open reading frames in genes or gene fragments can be synthesized utilizing plant-preferred codons (see, for example, Campbell & Gowri, 1990 for a discussion of host-preferred codon usage). Thus, the nucleotide sequences can be optimized for expression in any plant. It is recognized that all or any part of the gene sequence may be optimized or synthetic. That is, synthetic or partially optimized sequences may also be used. Variant nucleotide sequences and proteins also encompass, sequences and protein derived from a mutagenic and recombinogenic procedure such as DNA shuffling. With such a procedure, one or more different coding sequences can be manipulated to create a new polypeptide possessing the desired properties. In this manner, libraries of recombinant polynucleotides are generated from a population of related sequence polynucleotides comprising sequence regions that have substantial sequence identity and can be homologously recombined in vitro or in vivo. Strategies for such DNA shuffling are known in the art (see, for example, Stemmer 1994; Stemmer 1994; Crameri 1997; Moore 1997; Zhang 1997; Crameri 1998; and US 5,605,793 and 5,837,458).

By "variant" polypeptide is intended a polypeptide derived from the native protein by deletion (so-called truncation) or addition of one or more amino acids to the N-terminal and/or C-terminal end of the native protein; deletion or addition of one or more amino acids at one or more sites in the native protein; or substitution of one or more amino acids at one or more sites in the native protein. Such variants may result from, for example, genetic polymorphism or from human manipulation. Methods for such manipulations are generally known in the art.

Thus, the polypeptides may be altered in various ways including amino acid substitutions, deletions, truncations, and insertions. Methods for such manipulations are generally known in the art. For example, amino acid sequence variants of the polypeptides can be prepared by mutations in the DNA. Methods for mutagenesis and nucleotide sequence alterations are well known in the art (see, for example, Kunkel 1985; Kunkel 1987; US 4,873,192; Walker & Gaastra, 1983 and the references cited therein). Guidance as to appropriate amino acid substitutions that do not affect biological activity of the protein of interest may be found in the model of Dayhoff et al. (1978). Conservative substitutions, such as exchanging one amino acid with another having similar properties, are preferred. Individual substitutions deletions or additions that alter, add or delete a single amino acid or a small percentage of amino acids (typically less than 5%, more typically less than 1%) in an encoded sequence are "conservatively modified variations," where the alterations result in the substitution of an amino acid with a chemically similar amino acid. Conservative substitution tables providing functionally similar amino acids are well known in the art. The following five groups each contain amino acids that are conservative substitutions for one another. Aliphatic: Glycine (G), Alanine (A), Valine (V), Leucine (L), Isoleucine (I); Aromatic: Phenylalanine (F), Tyrosine (Y), Tryptophan (W); Sulfur-containing: Methionine (M), Cysteine (C); Basic: Arginine (R), Lysine (K), Histidine (H); Acidic: Aspartic acid (D), Glutamic acid (E), Asparagine (N), Glutamine (Q). See also, Creighton, 1984. In addition, individual substitutions, deletions or additions which alter, add or delete a single amino acid or a small percentage of amino acids in an encoded sequence are also "conservatively modified variations."

"Expression cassette" as used herein means a DNA sequence capable of directing expression of a particular nucleotide sequence in an appropriate host cell, comprising a promoter operably linked to a nucleotide sequence of interest, which is — optionally - operably linked to termination signals and/or other regulatory elements. An expression cassette may also comprise sequences required for proper translation of the nucleotide sequence. The coding region usually codes for a protein of interest but may also code for a functional RNA of interest, for example antisense RNA or a nontranslated RNA, in the sense or antisense direction. The expression cassette comprising the nucleotide sequence of interest may be chimeric, meaning that at least one of its components is heterologous with respect to at least one of its other components. The expression cassette may also be one, which is naturally occurring but has been obtained in a recombinant form useful for heterologous expression. An expression cassette may be assembled entirely extracellularly (e.g., by recombinant cloning techniques). However, an expression cassette may also be assembled using in part endogenous components. For example, an expression cassette may be obtained by placing (or inserting) a promoter sequence upstream of an endogenous sequence, which thereby becomes functionally linked and controlled by said promoter sequences. Likewise, a nucleic acid sequence to be expressed may be placed (or inserted) downstream of an endogenous promoter sequence thereby forming an expression cassette. The expression of the nucleotide sequence in the expression cassette may be under the control of a constitutive promoter or of an inducible promoter which initiates transcription only when the host cell is exposed to some particular external stimulus. In the case of a multicellular organism, the promoter can also be specific to a particular tissue or organ or stage of development (e.g., the seed-specific or seed-preferential promoters of the invention).

"Vector" is defined to include, inter alia, any plasmid, cosmid, phage or Agrobacterium binary vector in double or single stranded linear or circular form which may or may not be self transmissible or mobilizable, and which can transform prokaryotic or eukaryotic host either by integration into the cellular genome or exist extrachromosomally (e.g. autonomous replicating plasmid with an origin of replication).

Specifically included are shuttle vectors by which is meant a DNA vehicle capable, naturally or by design, of replication in two different host organisms, which may be selected from actinomycetes and related species, bacteria and eukaryotic (e.g. higher plant, mammalian, yeast or fungal cells).

Preferably the nucleic acid in the vector is under the control of, and operably linked to, an appropriate promoter or other regulatory elements for transcription in a host cell such as a microbial, e.g. bacterial, or plant cell. The vector may be a bi-functional expression vector which functions in multiple hosts. In the case of genomic DNA, this may contain its own promoter or other regulatory elements and in the case of cDNA this may be under the control of an appropriate promoter or other regulatory elements for expression in the host cell. "Cloning vectors" typically contain one or a small number of restriction endonuclease recognition sites at which foreign DNA sequences can be inserted in a determinable fashion without loss of essential biological function of the vector, as well as a marker gene that is suitable for use in the identification and selection of cells transformed with the cloning vector. Marker genes typically include genes that provide tetracycline resistance, hygromycin resistance or ampicillin resistance.

A "transgenic plant" is a plant having one or more plant cells that contain an expression vector.

"Plant tissue" includes differentiated and undifferentiated tissues or plants, induding but not limited to roots, stems, shoots, leaves, pollen, seeds, tumor tissue and various forms of cells and culture such as single cells, protoplast, embryos, and callus tissue. The plant tissue may be in plants or in organ, tissue or cell culture.

The following terms are used to describe the sequence relationships between two or more nucleic acids or polynucleotides: (a) "reference sequence", (b) "comparison window", (c) "sequence identity", (d) "percentage of sequence identity", and (e) "substantial identity".

(a) As used herein, "reference sequence" is a defined sequence used as a basis for sequence comparison. A reference sequence may be a subset or the entirety of a specified sequence; for example, as a segment of a full length cDNA or gene sequence, or the complete cDNA or gene sequence.

(b) As used herein, "comparison window" makes reference to a contiguous and specified segment of a polynucleotide sequence, wherein the polynucleotide sequence in the comparison window may comprise additions or deletions (i.e., gaps) compared to the reference sequence (which does not comprise additions or deletions) for optimal alignment of the two sequences. Generally, the comparison window is at least 20 contiguous nucleotides in length, and optionally can be 30, 40, 50, 100, or longer. Those of skill in the art understand that to avoid a high similarity to a reference sequence due to inclusion of gaps in the polynucleotide sequence a gap penalty is typically introduced and is subtracted from the number of matches.
Methods of alignment of sequences for comparison are well known in the art. Thus, the determination of percent identity between any two sequences can be accomplished using a mathematical algorithm. Preferred, non-limiting examples of such mathematical algorithms are the algorithm of Myers and Miller, 1988; the local homology algorithm of Smith et al. 1981; the homology alignment algorithm of Needleman and Wunsch 1970; the search-for-similarity-method of Pearson and Lipman 1988; the algorithm of Karlin and Altschul, 1990, modified as in Karlin and Altschul, 1993.
Computer implementations of these mathematical algorithms can be utilized for comparison of sequences to determine sequence identity. Such implementations include, but are not limited to: CLUSTAL in the PC/Gene program (available from Intelligenetics, Mountain Vew, Calif.); the ALIGN program (Version 2.0) and GAP, BESTFIT, BLAST, FASTA, and TFASTA in the Wisconsin Genetics Software Package, Version 8 (available from Genetics Computer Group (GCG), 575 Science Drive, Madison, Wis., USA). Alignments using these programs can be performed using the default parameters. The CLUSTAL program is well described (Higgins 1988, 1989; Corpet 1988; Huang 1992; Pearson 1994). The ALIGN program is based on the algorithm of Myers and Miller, supra. The BLAST programs of Altschul et al., 1990, are based on the algorithm of Karlin and Altschul, supra.
Software for performing BLAST analyses is publicly available through the National Center for Biotechnology Information (http://www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Attschul 1990). These initial neighborhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always >0) and N (penalty score for mismatching residues; always <0). For amino acid sequences, a scoring matrix is used to calculate the cumulative score. Extension of the word hits in each direction are halted when the cumulative alignment score falls off by the quantity X from its maximum achieved value, the cumulative score goes to zero or below due to the accumulation of one or more negative-scoring residue alignments, or the end of either sequence is reached.
In addition to calculating percent sequence identity, the BLAST algorithm also performs a statistical analysis of the similarity between two sequences (see, e.g., Karlin & Altschul (1993). One measure of similarity provided by the BLAST algorithm is the smallest sum probability (P(N)), which provides an indication of the probability by which a match between two nucleotide or amino acid sequences would occur by chance. For example, a test nucleic acid sequence is considered similar to a reference sequence if the smallest sum probability in a comparison of the test nucleic acid sequence to the reference nucleic acid sequence is less than about 0.1, more preferably less than about 0.01, and most preferably less than about 0.001.
To obtain gapped alignments for comparison purposes, Gapped BLAST (in BLAST 2.0) can be utilized as described in Altschul et al. 1997. Alternatively, PSI-BLAST (in BLAST 2.0) can be used to perform an iterated search that detects distant relationships between molecules. See Altschul et al., supra. When utilizing BLAST, Gapped BLAST, PSI-BLAST, the default parameters of the respective programs (e.g. BLASTN for nucleotide sequences, BLASTX for proteins) can be used. The BLASTN program (for nudeotide sequences) uses as defaults a wordlength (W) of 11, an expectation (E) of 10, a cutoff of 100, M=5, N=-4, and a comparison of both strands. For amino acid sequences, the BLASTP program uses as defaults a wordlength (W) of 3, an expectation (E) of 10, and the BLOSUM62 scoring matrix (see Henikoff & Henikoff, 1989). See hftp://www.ncbi.nim.nih.gov. Alignment may also be performed manually by inspection.
For purposes of the present invention, comparison of nucleotide sequences for determination of percent sequence identity to the promoter sequences disclosed herein is preferably made using the BlastN program (version 1.4.7 or later) with its default parameters or any equivalent program. By "equivalent program" is intended any sequence comparison program that, for any two sequences in question, generates an alignment having identical nucleotide or amino acid residue matches and an identical percent sequence identity when compared to the corresponding alignment generated by the preferred program.

(c) As used herein, "sequence identity" or "identity" in the context of two nucleic acid or polypeptide sequences makes reference to the residues in the two sequences that are the same when aligned for maximum correspondence over a specified comparison window. When percentage of sequence identity is used in reference to proteins it is recognized that residue positions which are not identical often differ by conservative amino acid substitutions, where amino acid residues are substituted for other amino acid residues with similar chemical properties (e.g., charge or hydrophobicity) and therefore do not change the functional properties of the molecule. When sequences differ in conservative substitutions, the percent sequence identity may be adjusted upwards to correct for the conservative nature of the substitution. Sequences that differ by such conservative substitutions are said to have "sequence similarity" or "similarity." Means for making this adjustment are well known to those of skill in the art. Typically this involves scoring a conservative substitution as a partial rather than a full mismatch, thereby increasing the percentage sequence identity. Thus, for example, where an identical amino acid is given a score of 1 and a non-conservative substitution is given a score of zero, a conservative substitution is given a score between zero and 1. The scoring of conservative substitutions is calculated, e.g., as implemented in the program PC/GENE (Intelligenetics, Mountain View, Calif.).

(d) As used herein, "percentage of sequence identity" means the value determined by comparing two optimally aligned sequences over a comparison window, wherein the portion of the polynucleotide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison, and multiplying the result by 100 to yield the percentage of sequence identity.

(e) (i) The term "substantial identity" or "substantial similarity" of polynucleotide sequences for a protein encoding sequence means that a polynucleotide comprises a sequence that has at least 70%, 71%, 72%, 73%, 74%, 75%, 76%, 77%, 78%, or 79%, preferably at least 80%, 81%, 82%, 83%, 84%, 85%, 86%, 87%, 88%, or 89%, more preferably at least 90%, 91%, 92%, 93%, or 94%, and most preferably at least 95%, 96%, 97%, 98%, or 99% sequence identity, compared to a reference sequence using one of the alignment programs described using standard parameters. The term "substantial identity" or "substantial similarity" of polynucleotide sequences for promoter sequence means (as described above for variants) that a polynucleotide comprises a sequence that has at least 40, 50, 60, to 70%, e.g., preferably 71%, 72%, 73%, 74%, 75%, 76%, 77%, 78%, to 79%, generally at least 80%, e.g., 81%-84%, at least 85%, e.g., 86%, 87%, 88%, 89%, 90%, 91%, 92%, 93%, 94%, 95%, 96%, 97%, to 98% and 99% nucleotide sequence identity compared to a reference sequence using one of the alignment programs described using standard parameters. One of skill in the art will recognize that these values can be appropriately adjusted to determine corresponding identity of proteins encoded by two nucleotide sequences by taking into account codon degeneracy, amino acid similarity, reading frame positioning, and the like. Substantial identity of amino acid sequences for these purposes normally means sequence identity of at least 70%, more preferably at least 80%, 90%, and most preferably at least 95%.
Another indication that nucleotide sequences are substantially identical is if two molecules hybridize to each other under stringent conditions (see below). Generally, stringent conditions are selected to be about 5°C lower than the thermal melting point ($T_m$) for the specific sequence at a defined ionic strength and pH. However, stringent conditions encompass temperatures in the range of about 1 °C to about 20°C, depending upon the desired degree of stringency as otherwise qualified herein. Nucleic adds that do not hybridize to each other under stringent conditions are still substantially identical if the polypeptides they encode are substantially identical. This may occur, e.g., when a copy of a nucleic acid is created using the maximum codon degeneracy permitted by the genetic code. One indication that two nucleic acid sequences are substantially identical is when the polypeptide encoded by the first nucleic acid is immunologically cross reactive with the polypeptide encoded by the second nucleic acid.

(ii) The term "substantial identity" in the context of a peptide indicates that a peptide comprises a sequence with at least 70%, 71%, 72%, 73%, 74%, 75%, 76%, 77%, 78%, or 79%, preferably 80%, 81%. 82%, 83%, 84%, 85%, 86%, 87%, 88%, or 89%, more preferably at least 90%, 91 %, 92%, 93%, or 94%, or even more preferably, 95%, 96%, 97%, 98% or 99%, sequence identity to the reference sequence over a specified comparison window. Preferably, optimal alignment is conducted using the homology alignment algorithm of Needleman and Wunsch (1970). An indication that two peptide sequences are substantially identical is that one peptide is immunologically reactive with antibodies raised against the second peptide. Thus, a peptide is substantially identical to a second peptide, for example, where the two peptides differ only by a conservative substitution.

For sequence comparison, typically one sequence acts as a reference sequence to which test sequences are compared. When using a sequence comparison algorithm, test and reference sequences are input into a computer, subsequence coordinates are designated if necessary, and sequence algorithm program parameters are designated.

The sequence comparison algorithm then calculates the percent sequence identity for the test sequence(s) relative to the reference sequence, based on the designated program parameters.

As noted above, another indication that two nucleic acid sequences are substantially identical is that the two molecules hybridize to each other under stringent conditions. The phrase "hybridizing specfically to" refers to the binding, duplexing, or hybridizing of a molecule only to a particular nucleotide sequence under stringent conditions when that sequence is present in a complex mixture (e.g., total cellular) DNA or RNA. "Bind(s) substantially" refers to complementary hybridization between a probe nucleic acid and a target nucleic acid and embraces minor mismatches that can be accommodated by reducing the stringency of the hybridization media to achieve the desired detection of the target nucleic acid sequence.

"Stringent hybridization conditions" and "stringent hybridization wash conditions" in the context of nucleic acid hybridization experiments such as Southern and Northern hybridization are sequence dependent, and are different under different environmental parameters. The $T_m$ is the temperature (under defined ionic strength and pH) at which 50% of the target sequence hybridizes to a perfectly matched probe. Specificity is typically the function of post-hybridization washes, the critical factors being the ionic strength and temperature of the final wash solution. For DNA-DNA hybrids, the $T_m$ can be approximated from the equation of Meinkoth and Wahl, 1984:

$$T_m = 81.5°C + 16.6 (\log_{10} M) + 0.41 (\%GC) - 0.61 (\% \text{form}) - 500 / L$$

where M is the molarity of monovalent cations, %GC is the percentage of guanosine and cytosine nucleotides in the DNA, % form is the percentage of formamide in the hybridization solution, and L is the length of the hybrid in base pairs. $T_m$ is reduced by about 1°C for each 1% of mismatching; thus, $T_m$, hybridization, and/or wash conditions can be adjusted to hybridize to sequences of the desired identity. For example, if sequences with >90% identity are sought, the $T_m$ can be decreased 10°C. Generally, stringent conditions are selected to be about 5°C lower than the thermal melting point I for the specific sequence and its complement at a defined ionic strength and pH. However, severely stringent conditions can utilize a hybridization and/or wash at 1, 2, 3, or 4°C lower than the thermal melting point I; moderately stringent conditions can utilize a hybridization and/or wash at 6, 7, 8, 9, or 10°C lower than the thermal melting point I; low stringency conditions can utilize a hybridization and/or wash at 11, 12, 13, 14, 15, or 20°C lower than the thermal melting point I. Using the equation, hybridization and wash compositions, and desired T, those of ordinary skill will understand that variations in the stringency of hybridization and/or wash solutions are inherently described. If the desired degree of mismatching results in a T of less than 45°C (aqueous solution) or 32°C (formamide solution), it is preferred to increase the SSC concentration so that a higher temperature can be used. An extensive guide to the hybridization of nucleic acids is found in Tijssen, 1993. Generally, highly stringent hybridization and wash conditions are selected to be about 5°C lower than the thermal melting point $T_m$ for the specific sequence at a defined ionic strength and pH.

An example of highly stringent wash conditions is 0.15 M NaCl at 72°C for about 15 minutes. An example of stringent wash conditions is a 0.2 X SSC wash at 65°C for 15 minutes (see, Sambrook, infra, for a description of SSC buffer). Often, a high stringency wash is preceded by a low stringency wash to remove background probe signal. An example medium stringency wash for a duplex of, e.g., more than 100 nucleotides, is 1 X SSC at 45°C for 15 minutes. An example low stringency wash for a duplex of, e.g., more than 100 nucleotides, is 4 to 6 X SSC at 40°C for 15 minutes. For short probes (e.g., about 10 to 50 nucleotides), stringent conditions typically involve salt concentrations of less than about 1.5 M, more preferably about 0.01 to 1.0 M, Na ion concentration (or other salts) at pH 7.0 to 8.3, and the temperature is typically at least about 30°C and at least about 60°C for long robes (e.g., >50 nucleotides). Stringent conditions may also be achieved with the addition of destabilizing agents such as formamide. In general, a signal to noise ratio of 2 X (or higher) than that observed for an unrelated probe in the particular hybridization assay indicates detection of a specific hybridization. Nucleic acids that do not hybridize to each other under stringent conditions are still substantially identical if the proteins that they encode are substantially identical. This occurs, e.g., when a copy of a nucleic acid is created using the maximum codon degeneracy permitted by the genetic code.

Very stringent conditions are selected to be equal to the $T_m$ for a particular probe. An example of stringent conditions for hybridization of complementary nucleic acids which have more than 100 complementary residues on a filter in a Southern or Northern blot is 50% formamide, e.g., hybridization in 50% formamide, 1 M NaCl, 1% SDS at 37°C, and a wash in 0.1 x SSC at 60 to 65°C. Exemplary low stringency conditions include hybridization with a buffer solution of 30 to 35% formamide, 1 M NaCl, 1% SDS (sodium dodecyl sulphate) at 37°C, and a wash in 1 X to 2 X SSC (20 X SSC=3.0 M NaCl/0.3 M trisodium citrate) at 50 to 55°C. Exemplary moderate stringency conditions include hybridization in 40 to 45% formamide, 1.0 M NaCl, 1% SDS at 37°C, and a wash in 0.5 X to 1 X SSC at 55 to 60°C.

The following are examples of sets of hybridization/wash conditions that may be used to clone orthologous nucleotide sequences that are substantially identical to reference nucleotide sequences of the present invention: a reference nucleotide sequence preferably hybridizes to the reference nucleotide sequence in 7% sodium dodecyl sulfate (SDS), 0.5 M $NaPO_4$, 1 mM EDTA at 50°C with washing in 2 X SSC, 0. 1% SDS at 50°C, more desirably in 7% sodium dodecyl sulfate (SDS), 0.5 M $NaPO_4$, 1 mM EDTA at 50°C with washing in 1 X SSC, 0.1 % SDS at 50°C, more desirably still in 7% sodium dodecyl sulfate (SDS), 0.5 M $NaPO_4$, 1 mM EDTA at 50°C with washing in 0.5 X SSC, 0. 1% SDS at 50°C, preferably in 7% sodium dodecyl sulfate (SDS), 0.5 M $NaPO_4$, 1 mM EDTA at 50°C with washing in 0.1 X SSC, 0.1% SDS at 50°C, more preferably in 7% sodium dodecyl sulfate (SDS), 0.5 M $NaPO_4$, 1 mM EDTA at 50°C with washing in 0.1 X SSC, 0.1% SDS at 65°C.

"DNA shuffling" is a method to introduce mutations or rearrangements, preferably randomly, in a DNA molecule or to generate exchanges of DNA sequences between two or more DNA molecules, preferably randomly. The DNA molecule resulting from DNA shuffling is a shuffled DNA molecule that is a non-naturally occurring DNA molecule derived from at least one template DNA molecule. The shuffled DNA preferably encodes a variant polypeptide modified with respect to the polypeptide encoded by the template DNA, and may have an altered biological activity with respect to the polypeptide encoded by the template DNA.

"Recombinant DNA molecule' is a combination of DNA sequences that are joined together using recombinant DNA technology and procedures used to join together DNA sequences as described, for example, in Sambrook et al., 1989.

The word "plant" refers to any plant, particularly to agronomically useful plants (e.g., seed plants), and "plant cell" is a structural and physiological unit of the plant, which comprises a cell wall but may also refer to a protoplast. The plant cell may be in form of an isolated single cell or a cultured cell, or as a part of higher organized unit such as, for example, a plant tissue, or a plant organ differentiated into a structure that is present at any stage of a plant's development. Such structures include one or more plant organs including, but are not limited to, fruit, shoot, stem, leaf, flower petal, etc. Preferably, the term "plant" includes whole plants, shoot vegetative organs/structures (*e.g.* leaves, stems and tubers), roots, flowers and floral organs/structures (e.g. bracts, sepals, petals, stamens, carpels, anthers and ovules), seeds (including embryo, endosperm, and seed coat) and fruits (the mature ovary), plant tissues (e.g. vascular tissue, ground tissue, and the like) and cells (e.g. guard cells, egg cells, trichomes and the like), and progeny of same.

The class of plants that can be used in the method of the invention is generally as broad as the class of higher and lower plants amenable to transformation techniques, including angiosperms (monocotyledonous and dicotyledonous plants), gymnosperms, ferns, and multicellular algae. It includes plants of a variety of ploidy levels, including aneuploid, polyploid, diploid, haploid and hemizygous. Included within the scope of the invention are all genera and species of higher and lower plants of the plant kingdom. Included are furthermore the mature plants, seed, shoots and seedlings, and parts, propagation material (for example seeds and fruit) and cultures, for example cell cultures, derived therefrom. Preferred are plants and plant materials of the following plant families: *Amaranthaceae, Brassicaceae, Carophyllaceae, Chenopodiaceae, Compositae, Cucurbitaceae, Labiatae, Leguminosae, Papilionoideae, Liliaceae, Linaceae, Malvaceae, Rosaceae, Saxifragaceae, Scrophulariaceae, Solanaceae, Tetragoniaceae.*

Annual, perennial, monocotyledonous and dicotyledonous plants are preferred host organisms for the generation of transgenic plants. The use of the recombination system, or method according to the invention is furthermore advantageous in all ornamental plants, forestry, fruit, or ornamental trees, flowers, cut flowers, shrubs or turf. Said plant may include - but shall not be limited to - bryophytes such as, for example, Hepaticae (hepaticas) and Musci (mosses); pteridophytes such as ferns, horsetail and clubmosses; gymnosperms such as conifers, cycads, ginkgo and *Gnetaeae;* algae such as *Chlorophyceae, Phaeophpyceae, Rhodophyceae, Myxophyceae, Xanthophyceae, Bacillariophyceae* (diatoms) and *Euglenophyceae*.

Plants for the purposes of the invention may comprise the families of the Rosaceae such as rose, Ericaceae such as rhododendrons and azaleas, *Euphorbiaceae* such as poinsettias and croton, *Caryophyllaceae* such as pinks, Solanaceae such as petunias, *Gesneriaceae* such as African violet, *Balsaminaceae* such as touch-me-not, Orchidaceae such as orchids, *Iridaceae* such as gladioli, iris, freesia and crocus, *Compositae* such as marigold, *Geraniaceae* such as geraniums, *Liliaceae* such as *Drachaena, Moraceae* such as ficus, *Araceae* such as philodendron and many others.

The transgenic plants according to the invention are furthermore selected in particular from among dicotyledonous crop plants such as, for example, from the families of the Leguminosae such as pea, alfalfa and soybean; the family of the *Umbelliferae*, particularly the genus *Daucus* (very particularly the species *carota* (carrot)) and *Apium* (very particularly the species *graveolens* var. dulce (celery)) and many others; the family of the Solanaceae, particularly the genus *Lycopersicon*, very particularly the species *esculentum* (tomato) and the genus *Solanum*, very particularly the species *tuberosum* (potato) and *melongena* (aubergine), tobacco and many others; and the genus *Capsicum,* very particularly the species *annum* (pepper) and many others; the family of the *Leguminosae,* particularly the genus *Glycine,* very particularly the species max (soybean) and many others; and the family of the *Cruciferae*, particularly the genus *Bras*sica, very particularly the species *napus* (oilseed rape), *campestris* (beet), *oleracea* cv Tastie (cabbage), *oleracea* cv Snowball Y (cauliflower) and *oleracea* cv Emperor (broccoli); and the genus *Arabidopsis,* very particularly the species *thaliana* and many others; the family of the Compositae, particularly the genus *Lactuca,* very particularly the species *sativa* (lettuce) and many others.

The transgenic plants according to the invention may be selected among monocotyledonous crop plants, such as, for example, cereals such as wheat, barley, sorghum and millet, rye, triticale, maize, rice or oats, and sugarcane.

Further preferred are trees such as apple, pear, quince, plum, cherry, peach, nectarine, apricot, papaya, mango, and other woody species including coniferous and deciduous trees such as poplar, pine, sequoia, cedar, oak, *etc*. Especially preferred are *Arabidopsis thaliana, Nicotiana tabacum,* oilseed rape, soybean, corn (maize), wheat, Linum usitatissimum (linseed and fax), Camelina sativa, Brassica juncea, potato and tagetes.

"Significant increase" is an increase that is larger than the margin of error inherent in the measurement technique, preferably an increase by about 2-fold or greater.

"Significantly less" means that the decrease is larger than the margin of error inherent in the measurement technique, preferably a decrease by about 2-fold or greater.

## DETAILED DESCRIPTION OF THE INVENTION

The present invention thus provides for isolated nucleic acid molecules comprising a plant nucleotide sequence that directs seed-preferential or seed-specific transcription of an operably linked nucleic acid fragment in a plant cell.

Specifically, the present invention provides transgenic expression cassettes for regulating seed-preferential or seed-specific expression (or transcription) in plants comprising

i) at least one transcription regulating nucleotide sequence of a plant gene, said plant gene selected from the group of genes described by the GenBank *Arabidopsis thaliana* genome locii At4g12910, At1g66250, At4g00820, At2g36640, At2g34200, At3g11180, At4g00360, At2g48030, At2g38590, At1g23000, At3g15510, At3g50870, At4g00220, or At4g26320, or a functional equivalent thereof, and functionally linked thereto

ii) at least one nucleic acid sequence which is heterologous in relation to said transcription regulating nucleotide sequence.

The seed-preferential or seed-specific promoters may be useful for expressing genes as well as for producing large quantities of protein, for expressing oils or proteins of interest, e.g., antibodies, genes for increasing the nutritional value of the seed and the like.

The term "seed" in the context of the inventions means a seed of a plant in any stage of its development i.e. starting from the fusion of pollen and oocyte, continuing over the embryo stage and the stage of the dormant seed, until the germinating seed, ending with early seedling organs, as e.g. cotyledons and hypocotyl.

"Seed-specific transcription" in the context of this invention means the transcription of a nucleic acid sequence by a transcription regulating element in a way that transcription of said nucleic acid sequence in seeds contribute to more than 90%, preferably more than 95%, more preferably more than 99% of the entire quantity of the RNA transcribed from said nucleic acid sequence in the entire plant during any of its developmental stage. The transcription regulating nucleotide sequences designated pSUK19, pSUK29, pSUK298, pSUK352 and their respective shorter and longer variants are considered to be seed-specific transcription regulating nucleotide sequences.

"Seed-preferential transcription" in the context of this invention means the transcription of a nucleic acid sequence by a transcription regulating element in a way that transcription of said nucleic acid sequence in seeds contribute to more than 50%, preferably more than 70%, more preferably more than 80% of the entire quantity of the RNA transcribed from said nucleic acid sequence in the entire plant during any of its developmental stage. The transcription regulating nucleotide sequences designated (pSUK222, pSUK224, pSUK226), (pSUK322, pSUK324), (pSUK250, pSUK252), (pSUK28, pSUK30), pSUK300, (pSUK292, pSUK294, pSUK296), pSUK164, (pSUK40, pSUK42), (pSUK48, pSUK50), (pSUK96, pSUK98), (pSUK152, pSUK154) and their respective shorter and longer variants are considered to be seed-preferential transcription regulating nucleotide sequences (transcription regulating nucleotide sequences in brackets are variants from one specific gene locus).

Preferably a transcription regulating nucleotide sequence of the invention comprises at least one promoter sequence of the respective gene (e.g., a sequence localized upstream of the transcription start of the respective gene capable to induce transcription of the downstream sequences). Said transcription regulating nudeotide sequence may comprise the promoter sequence of said genes but may further comprise other elements such as the 5'-untranslated sequence, enhancer, introns etc. Preferably, said promoter sequence directs seed-preferential or seed-specific transcription of an operably linked nucleic acid segment in a plant or plant cell e.g., a linked plant DNA comprising an open reading frame for a structural or regulatory gene.

The following Table 1 illustrates the genes from which the promoters of the invention are preferably isolated, the function of said genes, the cDNA encoded by said genes, and the protein (ORF) encoded by said genes.

**Table 1**: Genes from which the promoters of the invention are preferably isolated, putative function of said genes, cDNA and the protein encoded by said genes.

| Gene Locus | Putative function | Promoter SEQ ID | mRNA locus ID cDNA SEQ ID | Proteine ID Protein SEQ ID |
|---|---|---|---|---|
| At4g12910 | serine carboxypeptidase I precursor-like protein | SEQ ID NO: 1, 2, 3, 4, 5, 6, 7, 8, 9, 10, 11, 12 | NM_117360 SEQ ID NO: 13 | NP_193027.2 SEQ ID NO: 14 |
| At1g66250 | glycosyl hydrolase family 17 protein | SEQ ID NO: 15,16,17,18,19, 20 | NM_105296 SEQ ID NO: 21 | NP_176799.2 SEQ ID NO: 22 |
| At4g00820 | calmodulin-binding protein-related protein | SEQ ID NO: 23,24,25,26,27, 28 | NM_116308 SEQ ID NO: 29 | NP_567191.2 SEQ ID NO: 30 |
| At2g36640 | late embryogenesis abundant protein (ECP63)/ LEA protein | SEQ ID NO: 31,32,33 | NM_129219 SEQ ID NO: 34 | NP_181202.1 SEQ ID NO: 35 |
| At2g34200 | Zinc finger (C3HC4-type RING finger) family protein | SEQ ID NO: 36,37,38,39,40, 41,42,43,44,45, 46,47 | NM_128971 SEQ ID NO: 48 | NP_180967.2 SEQ ID NO: 49 |
| At3g11180 | oxidoreductase, 20G-Fe(II) oxygenase family protein | SEQ ID NO: 50, 51, 52,53,54, 55,56,57 | NM_111954 SEQ ID NO: 58 | NP_187728.1 SEQ ID NO: 59 |
| At4g00360 | putative cytochrome P450 | SEQ ID NO: 60,61,62,63,64, 65,66,67,68,69 70,71 | NM_116260 SEQ ID NO: 72 | NP_191946.1 SEQ ID NO: 73 |
| At2g48030 | endonuclease/ exonuclease/ phosphatase family protein | SEQ ID NO: 74,75,76,77 | NM_130370 SEQ ID NO: 78 | NP_566121.1 SEQ ID NO: 79 |
| At2g38590 | F-box family protein | SEQ ID NO: 80,81 | NM_129416 SEQ ID NO: 82 | NP_181393.1 SEQ ID NO: 83 |
| At1g23000 | heavy-metal-associated domain-containing protein | SEQ ID NO: 84,85,86,87 | NM_102148 SEQ ID NO: 88 | NP_173713.1 SEQ ID NO: 89 |
| At3g15510 | encoding Arabidopsis thaliana no apical meristem (NAM) family protein (NAC2) | SEQ ID NO: 148,149,150,151, 152,153,154,155 | NM_112419 SEQ ID NO: 156 | NP_188170.1 SEQ ID NO: 157 |
| At3g50870 | encoding Arabidopsis thaliana zinc finger (GATA type) family protein | SEQ ID NO: 158,159,160,161, 162,163,164,165 | NM_114947 SEQ ID NO: 166 | NP_566939.1 SEQ ID NO: 167 |
| At4g00220 | encoding Arabidopsis thaliana LOB domain protein 30 / lateral organ boundaries domain protein 30 (LBD30) | SEQ ID NO: 168,169,170,171, 172,173,174,175 | NM_116239 SEQ ID NO: 176 | NP_191933.1 SEQ ID NO: 177 |
| At4g26320 | encoding Arabidopsis thaliana arabinogalactanprotein (AGP13) | SEQ ID NO: 178,179,180,181, 182,183,184,185 | NM_118765 SEQ ID NO: 186 | NP_194362.1 SEQ ID NO: 187 |

Preferably the transcription regulating nucleotide sequence (or the functional equivalent thereof) is selected from the group of sequences consisting of

i) the sequences described by SEQ ID NOs: 1, 2, 3, 4, 5, 6, 7, 8, 9, 10, 11, 12, 15, 16, 17, 18, 19, 20, 23, 24, 25, 26, 27, 28, 31, 32, 33, 36, 37, 38, 39, 40, 41, 42, 43, 44, 45, 46, 47, 50, 51, 52, 53, 54, 55, 56, 57, 60, 61, 62, 63, 64, 65, 66, 67, 68, 69, 70, 71, 74, 75, 76, 77, 80, 81, 84, 85, 86, 87, 148, 149, 150, 151, 152, 153, 154, 155, 158, 159, 160, 161, 162, 163, 164, 165, 168, 169, 170, 171, 172, 173, 174, 175, 178, 179, 180, 181, 182, 183, 184, and 185,

ii) a fragment of at least 50 consecutive bases of a sequence under i) which has substantially the same promoter activity as the corresponding transcription regulating nucleotide sequence described by 1, 2, 3, 4, 5, 6, 7, 8, 9, 10, 11, 12, 15, 16, 17, 18, 19, 20, 23, 24, 25, 26, 27, 28, 31, 32, 33, 36, 37, 38, 39, 40, 41, 42, 43, 44, 45, 46, 47, 50, 51, 52, 53, 54, 55, 56, 57, 60, 61, 62, 63, 64, 65, 66, 67, 68, 69, 70, 71, 74, 75, 76, 77, 80, 81, 84, 85, 86, 87, 148, 149, 150, 151, 152, 153, 154, 155, 158, 159, 160, 161, 162, 163, 164, 165, 168, 169, 170, 171, 172, 173, 174, 175, 178, 179, 180, 181, 182, 183, 184, or 185 ;

iii) a nucleotide sequence having substantial similarity (e.g., with a sequence identity of at least 40, 50, 60, to 70%, e.g., preferably 71%, 72%, 73%, 74%, 75%, 76%, 77%, 78%, to 79%, generally at least 80%, e.g., 81% to 84%, at least 85%, e.g., 86%, 87%, 88%, 89%, 90%, 91 %, 92%, 93%, 94%, 95%, 96%, 97%, to 98% and 99%) to a transcription regulating nucleotide sequence described by SEQ ID NO: 1, 2, 3, 4, 5, 6, 7, 8, 9, 10, 11, 12, 15, 16, 17, 18, 19, 20, 23, 24, 25, 26, 27, 28, 31, 32, 33, 36, 37, 38, 39, 40, 41, 42, 43, 44, 45, 46, 47, 50, 51, 52, 53, 54, 55, 56, 57, 60, 61, 62, 63, 64, 65, 66, 67, 68, 69, 70, 71, 74, 75, 76, 77, 80, 81, 84, 85, 86, 87, 148, 149, 150, 151, 152, 153, 154, 155, 158, 159, 160, 161, 162, 163, 164, 165, 168, 169, 170, 171, 172, 173, 174, 175, 178, 179, 180, 181, 182, 183, 184, or 185;

iv) a nucleotide sequence capable of hybridizing under conditions equivalent to hybridization in 7% sodium dodecyl sulfate (SDS), 0.5 M $NaPO_4$, 1 mM EDTA at 50°C with washing in 2 X SSC, 0.1% SDS at 50°C (more desirably in 7% sodium dodecyl sulfate (SDS), 0.5 M $NaPO_4$, 1 mM EDTA at 50°C with washing in 1 X SSC, 0.1% SDS at 50°C, more desirably still in 7% sodium dodecyl sulfate (SDS), 0.5 M $NaPO_4$, 1 mM EDTA at 50°C with washing in 0.5 X SSC, 0. 1% SDS at 50°C, preferably in 7% sodium dodecyl sulfate (SDS), 0.5 M $NaPO_4$, 1 mM EDTA at 50°C with washing in 0.1 X SSC, 0.1 % SDS at 50°C, more preferably in 7% sodium dodecyl sulfate (SDS), 0.5 M $NaPO_4$, 1 mM EDTA at 50°C with washing in 0.1 X SSC, 0.1% SDS at 65°C) to a transcription regulating nucleotide sequence described by SEQ ID NO: 1, 2, 3, 4, 5, 6, 7, 8, 9, 10, 11, 12, 15, 16, 17, 18, 19, 20, 23, 24, 25, 26, 27, 28, 31, 32, 33, 36, 37, 38, 39, 40, 41, 42, 43, 44, 45, 46, 47, 50, 51, 52, 53, 54, 55, 56, 57, 60, 61, 62, 63, 64, 65, 66, 67, 68, 69, 70, 71, 74, 75, 76, 77, 80, 81, 84, 85, 86, 87, 148, 149, 150, 151, 152, 153, 154, 155, 158, 159, 160, 161, 162, 163, 164, 165, 168, 169, 170, 171, 172, 173, 174, 175, 178, 179, 180, 181, 182, 183, 184, or 185, or the complement thereof;

v) a nucleotide sequence capable of hybridizing under conditions equivalent to hybridization in 7% sodium dodecyl sulfate (SDS), 0.5 M $NaPO_4$, 1 mM EDTA at 50°C with washing in 2 X SSC, 0.1% SDS at 50°C (more desirably in 7% sodium dodecyl sulfate (SDS), 0.5 M $NaPO_4$, 1 mM EDTA at 50°C with washing in 1 X SSC, 0.1% SDS at 50°C, more desirably still in 7% sodium dodecyl sulfate (SDS), 0.5 M $NaPO_4$, 1 mM EDTA at 50°C with washing in 0.5 X SSC, 0. 1% SDS at 50°C, preferably in 7% sodium dodecyl sulfate (SDS), 0.5 M $NaPO_4$, 1 mM EDTA at 50°C with washing in 0.1 X SSC, 0.1% SDS at 50°C, more preferably in 7% sodium dodecyl sulfate (SDS), 0.5 M $NaPO_4$, 1 mM EDTA at 50°C with washing in 0.1 X SSC, 0.1% SDS at 65°C) to a nucleic acid comprising 50 to 200 or more consecutive nucleotides (preferably at least 100, 200, or 300, more preferably 400, 500, or 600, most preferably at least 700, 800, or 900 consecutive nucleotides) of a transcription regulating nucleotide sequence described by SEQ ID NO: 1, 2, 3, 4, 5, 6, 7, 8, 9, 10, 11, 12, 15, 16, 17, 18, 19, 20, 23, 24, 25, 26, 27, 28, 31, 32, 33, 36, 37, 38, 39, 40, 41, 42, 43, 44, 45, 46, 47, 50, 51, 52, 53, 54, 55, 56, 57, 60, 61, 62, 63, 64, 65, 66, 67, 68, 69, 70, 71, 74, 75, 76, 77, 80, 81, 84, 85, 86, 87, 148, 149, 150, 151, 152, 153, 154, 155, 158, 159, 160, 161, 162, 163, 164, 165, 168, 169, 170, 171, 172, 173, 174, 175, 178, 179, 180, 181, 182, 183, 184, or 185, or the complement thereof;

vi) a nucleotide sequence which is the complement or reverse complement of any of the previously mentioned nucleotide sequences under i) to v).

A functional equivalent of the transcription regulating nucleotide sequence can also be obtained or is obtainable from plant genomic DNA from a gene encoding a polypeptide which is substantially similar and preferably has at least 70%, preferably 80%, more preferably 90%, most preferably 95% amino acid sequence identity to a polypeptide encoded by an *Arabidopsis thaliana* gene comprising any one of SEQ ID NOs: 14, 22, 30, 35, 49, 59, 73, 79, 83, 89, 157, 167, 177, or 187, respectively, or a fragment of said transcription regulating nucleotide sequence which exhibits promoter activity in a seed-preferential or seed-specific fashion.

The activity of a transcription regulating nucleotide sequence is considered equivalent if transcription is initiated in a seed-preferential or seed-specific fashion (as defined above). Such expression profile is preferably demonstrated using reporter genes operably linked to said transcription regulating nucleotide sequence. Preferred reporter genes (Schenbom 1999) in this context are green fluorescence protein (GFP) (Chui 1996; Leffel 1997), chloramphenicol transferase, luciferase (Millar 1992), β-glucuronidase or β-galactosidase. Especially preferred is β-glucuronidase (Jefferson 1987).

Beside this the transcription regulating activity of a function equivalent may vary from the activity of its parent sequence, especially with respect to expression level. The expression level may be higher or lower than the expression level of the parent sequence. Both derivations may be advantageous depending on the nucleic acid sequence of interest to be expressed. Preferred are such functional equivalent sequences, which - in comparison with its parent sequence - does, not derivate from the expression level of said parent sequence by more than 50%, preferably 25%, more preferably 10% (as to be preferably judged by either mRNA expression or protein (e.g., reporter gene) expression). Furthermore preferred are equivalent sequences which demonstrate an increased expression in comparison to its parent sequence, preferably an increase my at least 50%, more preferably by at least 100%, most preferably by at least 500%.

Preferably functional equivalent of the transcription regulating nucleotide sequence can be obtained or is obtainable from plant genomic DNA from a gene expressing a mRNA described by a cDNA which is substantially similar and preferably has at least 70%, preferably 80%, more preferably 90%, most preferably 95% sequence identity to a sequence described by any one of SEQ ID NOs: 13, 21, 29, 34, 48, 58, 72, 78, 82, 88, 156, 166, 176, or 186, respectively, or a fragment of said transcription regulating nucleotide sequence which exhibits promoter activity in a seed-preferential or seed-specific fashion.

Such functional equivalent of the transcription regulating nucleotide sequence may be obtained from other plant species by using the seed-preferential or seed-specific *Arabidopsis* promoter sequences described herein as probes to screen for homologous structural genes in other plants by hybridization under low, moderate or stringent hybridization conditions. Regions of the seed-preferential or seed-specific promoter sequences of the present invention which are conserved among species could also be used as PCR primers to amplify a segment from a species other than *Arabidopsis,* and that segment used as a hybridization probe (the latter approach permitting higher stringency screening) or in a transcription assay to determine promoter activity. Moreover, the seed-preferential or seed-specific promoter sequences could be employed to identify structurally related sequences in a database using computer algorithms.

More specifically, based on the *Arabidopsis* nucleic acid sequences of the present invention, orthologs may be identified or isolated from the genome of any desired organism, preferably from another plant, according to well known techniques based on their sequence similarity to the *Arabidopsis* nucleic acid sequences, e.g., hybridization, PCR or computer generated sequence comparisons. For example, all or a portion of a particular *Arabidopsis* nucleic acid sequence is used as a probe that selectively hybridizes to other gene sequences present in a population of cloned genomic DNA fragments or cDNA fragments (i.e., genomic or cDNA libraries) from a chosen source organism. Further, suitable genomic and cDNA libraries may be prepared from any cell or tissue of an organism. Such techniques include hybridization screening of plated DNA libraries (either plaques or colonies; see, e.g., Sambrook 1989) and amplification by PCR using oligonucleotide primers preferably corresponding to sequence domains conserved among related polypeptide or subsequences of the nucleotide sequences provided herein (see, e.g., Innis 1990). These methods are particularly well suited to the isolation of gene sequences from organisms closely related to the organism from which the probe sequence is derived. The application of these methods using the *Arabidopsis* sequences as probes is well suited for the isolation of gene sequences from any source organism, preferably other plant species. In a PCR approach, oligonucleotide primers can be designed for use in PCR reactions to amplify corresponding DNA sequences from cDNA or genomic DNA extracted from any plant of interest. Methods for designing PCR primers and PCR cloning are generally known in the art.

In hybridization techniques, all or part of a known nucleotide sequence is used as a probe that selectively hybridizes to other corresponding nucleotide sequences present in a population of cloned genomic DNA fragments or cDNA fragments (i.e., genomic or cDNA libraries) from a chosen organism. The hybridization probes may be genomic DNA fragments, cDNA fragments, RNA fragments, or other oligonucleotides, and may be labeled with a detedable group such as $^{32}P$, or any other detectable marker. Thus, for example, probes for hybridization can be made by labeling synthetic oligonucleotides based on the sequence of the invention. Methods for preparation of probes for hybridization and for construction of cDNA and genomic libraries are generally known in the art and are disclosed in Sambrook et al. (1989). In general, sequences that hybridize to the sequences disclosed herein will have at least 40% to 50%, about 60% to 70% and even about 80% 85%, 90%, 95% to 98% or more identity with the disclosed sequences. That is, the sequence similarity of sequences may range, sharing at least about 40% to 50%, about 60% to 70%, and even about 80%, 85%, 90%, 95% to 98% sequence similarity.

The nucleic acid molecules of the invention can also be identified by, for example, a search of known databases for genes encoding polypeptides having a specified amino acid sequence identity or DNA having a specified nucleotide sequence identity. Methods of alignment of sequences for comparison are well known in the art and are described hereinabove.

Hence, the isolated nucleic acid molecules of the invention include the orthologs of the *Arabidopsis* sequences disclosed herein, i.e., the corresponding nucleotide sequences in organisms other than *Arabidopsis*, including, but not limited to, plants other than *Arabidopsis,* preferably dicotyledonous plants, e.g., Brassica napus, alfalfa, sunflower, soybean, cotton, peanut, tobacco or sugar beet, but also cereal plants such as corn, wheat, rye, turfgrass, sorghum, millet, sugarcane, barley and banana. An orthologous gene is a gene from a different species that encodes a product having the same or similar function, e.g., catalyzing the same reaction as a product encoded by a gene from a reference organism. Thus, an ortholog includes polypeptides having less than, e.g., 65% amino acid sequence identity, but which ortholog encodes a polypeptide having the same or similar function. Databases such GenBank may be employed to identify sequences related to the *Arabidopsis* sequences, e.g., orthologs in other dicotyledonous plants such as Brassica napus and others. Alternatively, recombinant DNA techniques such as hybridization or PCR may be employed to identify sequences related to the *Arabidopsis* sequences or to clone the equivalent sequences from different *Arabidopsis* DNAs.

The transcription regulating nucleotide sequences of the invention or their functional equivalents can be obtained or isolated from any plant or non-plant source, or produced synthetically by purely chemical means. Preferred sources include, but are not limited to the plants defined in the DEFINITION section above.

Thus, another embodiment of the invention relates to a method for identifying and/or isolating a sequence with seed-preferential or seed-specific transcription regulating activity utilizing a nucleic acid sequence encoding a amino acid sequence as described by SEQ ID NO: 14, 22, 30, 35, 49, 59, 73, 79, 83, 89, 157, 167, 177, or 187 or a part thereof. Preferred are nucleic acid sequences described by SEQ ID NO: 13, 21, 29, 34, 48, 58, 72, 78, 82, 88, 156, 166, 176, or 186 or parts thereof. "Part" in this context means a nucleic acid sequence of at least 15 bases preferably at least 25 bases, more preferably at least 50 bases. The method can be based on (but is not limited to) the methods described above such as polymerase chain reaction, hybridization or database screening. Preferably, this method of the invention is based on a polymerase chain reaction, wherein said nucleic acid sequence or its part is utilized as oligonucleotide primer. The person skilled in the art is aware of several methods to amplify and isolate the promoter of a gene starting from part of its coding sequence (such as, for example, part of a cDNA). Such methods may include but are not limited to method such as inverse PCR ("iPCR") or "thermal asymmetric interlaced PCR" ("TAIL PCR").

Another embodiment of the invention is related to a method for providing a transgenic expression cassette for seed-preferential or seed-specific expression comprising the steps of:

I. isolating of a seed-preferential or seed-specific transcription regulating nucleotide sequence utilizing at least one nucleic acid sequence or a part thereof, wherein said sequence is encoding a polypeptide described by SEQ ID NO: 14, 22, 30, 35, 49, 59, 73, 79, 83, 89, 157, 167, 177, or 187, or a part of at least 15 bases thereof, and

II. functionally linking said seed-preferential or seed-specific transcription regulating nucleotide sequence to another nucleotide sequence of interest, which is heterologous in relation to said seed-preferential or seed-specific transcription regulating nucleotide sequence.

Preferably, the nucleic acid sequence employed for the isolation comprises at least 15 base, preferably at least 25 bases, more preferably at least 50 bases of a sequence described by SEQ ID NO: 13, 21, 29, 34, 48, 58, 72, 78, 82, 88, 156, 166, 176, or 186. Preferably, the isolation of the seed-preferential or seed-specific transcription regulating nucleotide sequence is realized by a polymerase chain reaction utilizing said nucleic acid sequence as a primer. The operable linkage can be realized by standard cloning method known in the art such as ligation-mediated cloning or recombination-mediated cloning.

Preferably, the transcription regulating nucleotide sequences and promoters of the invention include a consecutive stretch of about 25 to 2000, including 50 to 500 or 100 to 250, and up to 1000 or 1500, contiguous nucleotides, e.g., 40 to about 743, 60 to about 743, 125 to about 743, 250 to about 743, 400 to about 743, 600 to about 743, of any one of SEQ ID NOs: 1, 2, 3, 4, 5, 6, 7, 8, 9, 10, 11, 12, 15, 16, 17, 18, 19, 20, 23, 24, 25, 26, 27, 28, 31, 32, 33, 36, 37, 38, 39, 40, 41, 42, 43, 44, 45, 46, 47, 50, 51, 52, 53, 54, 55, 56, 57, 60, 61, 62, 63, 64, 65, 66, 67, 68, 69, 70, 71, 74, 75, 76, 77, 80, 81, 84, 85, 86, 87, 148, 149, 150, 151, 152, 153, 154, 155, 158, 159, 160, 161, 162, 163, 164, 165, 168, 169, 170, 171, 172, 173, 174, 175, 178, 179, 180, 181, 182, 183, 184, and 185, or the promoter orthologs thereof, which include the minimal promoter region.

In a particular embodiment of the invention said consecutive stretch of about 25 to 2000, including 50 to 500 or 100 to 250, and up to 1000 or 1500, contiguous nucleotides, e.g., 40 to about 743, 60 to about 743, 125 to about 743, 250 to about 743, 400 to about 743, 600 to about 743, has at least 75%, preferably 80%, more preferably 90% and most preferably 95%, nucleic acid sequence identity with a corresponding consecutive stretch of about 25 to 2000, including 50 to 500 or 100 to 250, and up to 1000 or 1500, contiguous nucleotides, e.g., 40 to about 743, 60 to about 743, 125 to about 743, 250 to about 743, 400 to about 743, 600 to about 743, of any one of SEQ ID NOs: 1, 2, 3, 4, 5, 6, 7, 8, 9, 10, 11, 12, 15, 16, 17, 18, 19, 20, 23, 24, 25, 26, 27, 28, 31, 32, 33, 36, 37, 38, 39, 40, 41, 42, 43, 44, 45, 46, 47, 50, 51, 52, 53, 54, 55, 56, 57, 60, 61, 62, 63, 64, 65, 66, 67, 68, 69, 70, 71, 74, 75, 76, 77, 80, 81, 84, 85, 86, 87, 148, 149, 150, 151, 152, 153, 154, 155, 158, 159, 160, 161, 162, 163, 164, 165, 168, 169, 170, 171, 172, 173, 174, 175, 178, 179, 180, 181, 182, 183, 184, and 185, or the promoter orthologs thereof, which include the minimal promoter region. The above-defined stretch of contiguous nucleotides preferably comprises one or more promoter motifs selected from the group consisting of TATA box, GC-box, CAAT-box and a transcription start site.

The transcription regulating nucleotide sequences of the invention or their functional equivalents are capable of driving seed-preferential or seed-specific expression of a coding sequence in a target cell, particularly in a plant cell. The promoter sequences and methods disclosed herein are useful in regulating seed-preferential or seed-specific expression, respectively, of any heterologous nudeotide sequence in a host plant in order to vary the phenotype of that plant. These promoters can be used with combinations of enhancer, upstream elements, and/or activating sequences from the 5' flanking regions of plant expressible structural genes. Similarly the upstream element can be used in combination with various plant promoter sequences.

The transcription regulating nucleotide sequences and promoters of the invention are useful to modify the phenotype of a plant. Various changes in the phenotype of a transgenic plant are desirable, i.e., modifying the fatty acid composition in a plant, altering the amino acid content of a plant, altering a plant's pathogen defense mechanism, and the like. These results can be achieved by providing expression of heterologous products or increased expression of endogenous products in plants. Alternatively, the results can be achieved by providing for a reduction of expression of one or more endogenous products, particularly enzymes or cofactors in the plant. These changes result in an alteration in the phenotype of the transformed plant.

Generally, the transcription regulating nucleotide sequences and promoters of the invention may be employed to express a nucleic acid segment that is operably linked to said promoter such as, for example, an open reading frame, or a portion thereof, an anti-sense sequence, a sequence encoding for a double-stranded RNA sequence, or a transgene in plants.

An operable linkage may - for example - comprise an sequential arrangement of the transcription regulating nucleotide sequence of the invention (for example a sequence as described by SEQ ID NO: 1, 2, 3, 4, 5, 6, 7, 8, 9, 10, 11, 12, 15, 16, 17, 18, 19, 20, 23, 24, 25, 26, 27, 28, 31, 32, 33, 36, 37, 38, 39, 40, 41, 42, 43, 44, 45, 46, 47, 50, 51, 52, 53, 54, 55, 56, 57, 60, 61, 62, 63, 64, 65, 66, 67, 68, 69, 70, 71, 74, 75, 76, 77, 80, 81, 84, 85, 86, 87, 148, 149, 150, 151, 152, 153, 154, 155, 158, 159, 160, 161, 162, 163, 164, 165, 168, 169, 170, 171, 172, 173, 174, 175, 178, 179, 180, 181, 182, 183, 184, or 185) with a nucleic acid sequence to be expressed, and - optionally - additional regulatory elements such as for example polyadenylation or transcription termination elements, enhancers, introns etc, in a way that the transcription regulating nucleotide sequence can fulfill its function in the process of expression the nucleic acid sequence of interest under the appropriate conditions. The term "appropriate conditions" mean preferably the presence of the expression cassette in a plant cell. Preferred are arrangements, in which the nucleic acid sequence of interest to be expressed is placed down-stream (i.e., in 3'-direction) of the transcription regulating nucleotide sequence of the invention in a way, that both sequences are covalently linked. Optionally additional sequences may be inserted in-between the two sequences. Such sequences may be for example linker or multiple cloning sites. Furthermore, sequences can be inserted coding for parts of fusion proteins (in case a fusion protein of the protein encoded by the nucleic acid of interest is intended to be expressed). Preferably, the distance between the nucleic add sequence of interest to be expressed and the transcription regulating nucleotide sequence of the invention is not more than 200 base pairs, preferably not more than 100 base pairs, more preferably no more than 50 base pairs.

An operable linkage in relation to any expression cassette or of the invention may be realized by various methods known in the art, comprising both *in vitro* and *in vivo* procedure. Thus, an expression cassette of the invention or an vector comprising such expression cassette may by realized using standard recombination and cloning techniques well known in the art (see e.g., Maniatis 1989; Silhavy 1984; Ausubel 1987).

An expression cassette may also be assembled by inserting a transcription regulating nucleotide sequence of the invention (for example a sequence as described by SEQ ID NO: 1, 2, 3, 4, 5, 6, 7, 8, 9, 10, 11, 12, 15, 16, 17, 18, 19, 20, 23, 24, 25, 26, 27, 28, 31, 32, 33, 36, 37, 38, 39, 40, 41, 42, 43, 44, 45, 46, 47, 50, 51, 52, 53, 54, 55, 56, 57, 60, 61, 62, 63, 64, 65, 66, 67, 68, 69, 70, 71, 74, 75, 76, 77, 80, 81, 84, 85, 86, 87, 148, 149, 150, 151, 152, 153, 154, 155, 158, 159, 160, 161, 162, 163, 164, 165, 168, 169, 170, 171, 172, 173, 174, 175, 178, 179, 180, 181, 182, 183, 184, or 185) into the plant genome. Such insertion will result in an operable linkage to a nucleic acid sequence of interest which as such already existed in the genome. By the insertion the nucleic acid of interest is expressed in a seed-preferential or seed-specific way due to the transcription regulating properties of the transcription regulating nucleotide sequence. The insertion may be directed or by chance. Preferably the insertion is directed and realized by for example homologous recombination. By this procedure a natural promoter may be exchanged against the transcription regulating nucleotide sequence of the invention, thereby modifying the expression profile of an endogenous gene. The transcription regulating nucleotide sequence may also be inserted in a way, that antisense mRNA of an endogenous gene is expressed, thereby inducing gene silencing.

Similar, a nucleic acid sequence of interest to be expressed may by inserted into a plant genome comprising the transcription regulating nucleotide sequence in its natural genomic environment (i.e. linked to its natural gene) in a way that the inserted sequence becomes operably linked to the transcription regulating nucleotide sequence, thereby forming an expression cassette of the invention.

The open reading frame to be linked to the transcription regulating nucleotide sequence of the invention may be obtained from an insect resistance gene, a disease resistance gene such as, for example, a bacterial disease resistance gene, a fungal disease resistance gene, a viral disease resistance gene, a nematode disease resistance gene, a herbicide resistance gene, a gene affecting grain composition or quality, a nutrient utilization gene, a mycotoxin reduction gene, a male sterility gene, a selectable marker gene, a screenable marker gene, a negative selectable marker, a positive selectable marker, a gene affecting plant agronomic characteristics, i.e., yield, standability, and the like, or an environment or stress resistance gene, i.e., one or more genes that confer herbicide resistance or tolerance, insect resistance or tolerance, disease resistance or tolerance (viral, bacterial, fungal, oomycete, or nematode), stress tolerance or resistance (as exemplified by resistance or tolerance to drought, heat, chilling, freezing, excessive moisture, salt stress, or oxidative stress), increased yields, food content and makeup, physical appearance, male sterility, drydown, standability, prolificacy, starch properties or quantity, oil quantity and quality, amino acid or protein composition, and the like. By "resistant" is meant a plant, which exhibits substantially no phenotypic changes as a consequence of agent administration, infection with a pathogen, or exposure to stress. By "tolerant" is meant a plant, which, although it may exhibit some phenotypic changes as a consequence of infection, does not have a substantially decreased reproductive capacity or substantially altered metabolism.

Seed-preferential or seed-specific transcription regulating nucleotide sequences (e.g., promoters) are useful for expressing a wide variety of genes including those which alter metabolic pathways, confer disease resistance, for protein production, e.g., antibody production, or to improve nutrient uptake and the like. Seed-preferential or seed-specific transcription regulating nucleotide sequences (e.g., promoters) may be modified so as to be regulatable, e.g., inducible. The genes and transcription regulating nucleotide sequences (e.g., promoters) described hereinabove can be used to identify orthologous genes and their transcription regulating nucleotide sequences (e.g., promoters) which are also likely expressed in a particular tissue and/or development manner. Moreover, the orthologous transcription regulating nucleotide sequences (e.g., promoters) are useful to express linked open reading frames. In addition, by aligning the transcription regulating nucleotide sequences (e.g., promoters) of these orthologs, novel cis elements can be identified that are useful to generate synthetic transcription regulating nucleotide sequences (e.g., promoters).

The expression regulating nucleotide sequences specified above may be optionally operably linked to other suitable regulatory sequences, e.g., a transcription terminator sequence, operator, repressor-binding site, transcription factor binding site and/or an enhancer.

The present invention further provides a recombinant vector containing the expression cassette of the invention, and host cells comprising the expression cassette or vector, e.g., comprising a plasmid. The expression cassette or vector may augment the genome of a transformed plant or may be maintained extra chromosomally. The expression cassette or vector of the invention may be present in the nucleus, chloroplast, mitochondria and/or plastid of the cells of the plant Preferably, the expression cassette or vector of the invention is comprised in the chromosomal DNA of the plant nucleus. The present invention also provides a transgenic plant prepared by this method, a seed from such a plant and progeny plants from such a plant including hybrids and inbreds. The expression cassette may be operatively linked to a structural gene, the open reading frame thereof, or a portion thereof. The expression cassette may further comprise a Ti plasmid and be contained in an Agrobacterium tumefaciens cell; it may be carried on a microparticle, wherein the microparticle is suitable for ballistic transformation of a plant cell; or it may be contained in a plant cell or protoplast. Further, the expression cassette or vector can be contained in a transformed plant or cells thereof, and the plant may be a dicot or a monocot. In particular, the plant may be a dicotyledonous plant. Preferred transgenic plants are transgenic maize, soybean, barley, alfalfa, sunflower, canola, soybean, cotton, peanut, sorghum, tobacco, sugarbeet, rice, wheat, rye, turfgrass, millet, sugarcane, tomato, or potato.

The invention also provides a method of plant breeding, e.g., to prepare a crossed fertile transgenic plant. The method comprises crossing a fertile transgenic plant comprising a particular expression cassette of the invention with itself or with a second plant, e.g., one lacking the particular expression cassette, to prepare the seed of a crossed fertile transgenic plant comprising the particular expression cassette. The seed is then planted to obtain a crossed fertile transgenic plant. The plant may be a monocot or a dicot. In a particular embodiment, the plant is a dicotyledonous plant. The crossed fertile transgenic plant may have the particular expression cassette inherited through a female parent or through a male parent. The second plant may be an inbred plant. The crossed fertile transgenic may be a hybrid. Also included within the present invention are seeds of any of these crossed fertile transgenic plants.

The transcription regulating nucleotide sequences of the invention further comprise sequences which are complementary to one (hereinafter "test" sequence) which hybridizes under stringent conditions with a nucleic acid molecule as described by SEQ ID NO: 1, 2, 3, 4, 5, 6, 7, 8, 9, 10, 11, 12, 15, 16, 17, 18, 19, 20, 23, 24, 25, 26, 27, 28, 31, 32, 33, 36, 37, 38, 39, 40, 41, 42, 43, 44, 45, -46, 47, 50, 51, 52, 53, 54, 55, 56, 57, 60, 61, 62, 63, 64, 65, 66, 67, 68, 69, 70, 71, 74, 75, 76, 77, 80, 81, 84, 85, 86, 87, 148, 149, 150, 151, 152, 153, 154, 155, 158, 159, 160, 161, 162, 163, 164, 165, 168, 169, 170, 171, 172, 173, 174, 175, 178, 179, 180, 181, 182, 183, 184, or 185 as well as RNA which is transcribed from the nucleic acid molecule. When the hybridization is performed under stringent conditions, either the test or nucleic acid molecule of invention is preferably supported, e.g., on a membrane or DNA chip. Thus, either a denatured test or nucleic acid molecule of the invention is preferably first bound to a support and hybridization is effected for a specified period of time at a temperature of, e.g., between 55 and 70°C, in double strength citrate buffered saline (SC) containing 0.1% SDS followed by rinsing of the support at the same temperature but with a buffer having a reduced SC concentration. Depending upon the degree of stringency required such reduced concentration buffers are typically single strength SC containing 0.1% SDS, half strength SC containing 0.1% SDS and one-tenth strength SC containing 0.1% SDS. More preferably hybridization is carried out under high stringency conditions (as defined above).

Virtually any DNA composition may be used for delivery to recipient plant cells, e.g., dicotyledonous cells, to ultimately produce fertile transgenic plants in accordance with the present invention. For example, DNA segments or fragments in the form of vectors and plasmids, or linear DNA segments or fragments, in some instances containing only the DNA element to be expressed in the plant, and the like, may be employed. The construction of vectors, which may be employed in conjunction with the present invention, will be known to those of skill of the art in light of the present disclosure (see, e.g., Sambrook 1989; Gelvin 1990).

Vectors, plasmids, cosmids, YACs (yeast artificial chromosomes), BACs (bacterial artificial chromosomes) and DNA segments for use in transforming such cells will, of course, generally comprise the cDNA, gene or genes which one desires to introduce into the cells. These DNA constructs can further include structures such as promoters, enhancers, polylinkers, or even regulatory genes as desired. The DNA segment, fragment or gene chosen for cellular introduction will often encode a protein which will be expressed in the resultant recombinant cells, such as will result in a screenable or selectable trait and/or which will impart an improved phenotype to the regenerated plant. However, this may not always be the case, and the present invention also encompasses transgenic plants incorporating non-expressed transgenes.

In certain embodiments, it is contemplated that one may wish to employ replication-competent viral vectors in monocot transformation. Such vectors include, for example, wheat dwarf virus (WDV) "shuttle" vectors, such as pW1-11 and PW1-GUS (Ugaki 1991). These vectors are capable of autonomous replication in maize cells as well as E. coli, and as such may provide increased sensitivity for detecting DNA delivered to transgenic cells. A replicating vector may also be useful for delivery of genes flanked by DNA sequences from transposable elements such as Ac, Ds, or Mu. It has been proposed (Laufs 1990) that transposition of these elements within the maize genome requires DNA replication. It is also contemplated that transposable elements would be useful for introducing DNA segments or fragments lacking elements necessary for selection and maintenance of the plasmid vector in bacteria, e.g., antibiotic resistance genes and origins of DNA replication. It is also proposed that use of a transposable element such as Ac, Ds, or Mu would actively promote integration of the desired DNA and hence increase the frequency of stably transformed cells. The use of a transposable element such as Ac, Ds, or Mu may actively promote integration of the DNA of interest and hence increase the frequency of stably transformed cells. Transposable elements may be useful to allow separation of genes of interest from elements necessary for selection and maintenance of a plasmid vector in bacteria or selection of a transformant. By use of a transposable element, desirable and undesirable DNA sequences may be transposed apart from each other in the genome, such that through genetic segregation in progeny, one may identify plants with either the desirable undesirable DNA sequences.

The nucleotide sequence of interest linked to one or more of the transcription regulating nucleotide sequences of the invention can, for example, code for a ribosomal RNA, an antisense RNA or any other type of RNA that is not translated into protein. In another preferred embodiment of the invention, said nucleotide sequence of interest is translated into a protein product. The transcription regulating nucleotide sequence and/or nucleotide sequence of interest linked thereto may be of homologous or heterologous origin with respect to the plant to be transformed. A recombinant DNA molecule useful for introduction into plant cells includes that which has been derived or isolated from any source, that may be subsequently characterized as to structure, size and/or function, chemically altered, and later introduced into plants. An example of a nucleotide sequence or segment of interest "derived" from a source, would be a nucleotide sequence or segment that is identified as a useful fragment within a given organism, and which is then chemically synthesized in essentially pure form. An example of such a nucleotide sequence or segment of interest "isolated" from a source, would be nucleotide sequence or segment that is excised or removed from said source by chemical means, e.g., by the use of restriction endonucleases, so that it can be further manipulated, e.g., amplified, for use in the invention, by the methodology of genetic engineering. Such a nucleotide sequence or segment is commonly referred to as "recombinant"

Therefore a useful nucleotide sequence, segment or fragment of interest includes completely synthetic DNA, semi-synthetic DNA, DNA isolated from biological sources, and DNA derived from introduced RNA. Generally, the introduced DNA is not originally resident in the plant genotype which is the recipient of the DNA, but it is within the scope of the invention to isolate a gene from a given plant genotype, and to subsequently introduce multiple copies of the gene into the same genotype, e.g., to enhance production of a given gene product such as a storage protein or a protein that confers tolerance or resistance to water deficit.

The introduced recombinant DNA molecule includes but is not limited to, DNA from plant genes, and non-plant genes such as those from bacteria, yeasts, animals or viruses. The introduced DNA can include modified genes, portions of genes, or chimeric genes, including genes from the same or different genotype. The term "chimeric gene" or "chimeric DNA" is defined as a gene or DNA sequence or segment comprising at least two DNA sequences or segments from species which do not combine DNA under natural conditions, or which DNA sequences or segments are positioned or linked in a manner which does not normally occur in the native genome of untransformed plant.

The introduced recombinant DNA molecule used for transformation herein may be circular or linear, double-stranded or single-stranded. Generally, the DNA is in the form of chimeric DNA, such as plasmid DNA, that can also contain coding regions flanked by regulatory sequences, which promote the expression of the recombinant DNA present in the resultant plant. Generally, the introduced recombinant DNA molecule will be relatively small, i.e., less than about 30 kb to minimize any susceptibility to physical, chemical, or enzymatic degradation which is known to increase as the size of the nucleotide molecule increases. As noted above, the number of proteins, RNA transcripts or mixtures thereof, which is introduced into the plant genome, is preferably preselected and defined, e.g., from one to about 5-10 such products of the introduced DNA may be formed.

Two principal methods for the control of expression are known, viz.: overexpression and underexpression. Overexpression can be achieved by insertion of one or more than one extra copy of the selected gene. It is, however, not unknown for plants or their progeny, originally transformed with one or more than one extra copy of a nucleotide sequence, to exhibit the effects of underexpression as well as overexpression. For underexpression there are two principle methods, which are commonly referred to in the art as "antisense downregulation" and "sense downregulation" (sense downregulation is also referred to as "cosuppression"). Generically these processes are referred to as "gene silencing". Both of these methods lead to an inhibition of expression of the target gene.

Obtaining sufficient levels of transgene expression in the appropriate plant tissues is an important aspect in the production of genetically engineered crops. Expression of heterologous DNA sequences in a plant host is dependent upon the presence of an operably linked promoter that is functional within the plant host. Choice of the promoter sequence will determine when and where within the organism the heterologous DNA sequence is expressed.

It is specifically contemplated by the inventors that one could mutagenize a promoter to potentially improve the utility of the elements for the expression of transgenes in plants. The mutagenesis of these elements can be carried out at random and the mutagenized promoter sequences screened for activity in a trial-by-error procedure. Alternatively, particular sequences which provide the promoter with desirable expression characteristics, or the promoter with expression enhancement activity, could be identified and these or similar sequences introduced into the sequences via mutation. It is further contemplated that one could mutagenize these sequences in order to enhance their expression of transgenes in a particular species.

The means for mutagenizing a DNA segment encoding a promoter sequence of the current invention are well known to those of skill in the art. As indicated, modifications to promoter or other regulatory element may be made by random, or site-specific mutagenesis procedures. The promoter and other regulatory element may be modified by altering their structure through the addition or deletion of one or more nucleotides from the sequence which encodes the corresponding unmodified sequences.

Mutagenesis may be performed in accordance with any of the techniques known in the art, such as, and not limited to, synthesizing an oligonucleotide having one or more mutations within the sequence of a particular regulatory region. In particular, site-specific mutagenesis is a technique useful in the preparation of promoter mutants, through specific mutagenesis of the underlying DNA. The technique further provides a ready ability to prepare and test sequence variants, for example, incorporating one or more of the foregoing considerations, by introducing one or more nucleotide sequence changes into the DNA. Site-specific mutagenesis allows the production of mutants through the use of specific oligonucleotide sequences which encode the DNA sequence of the desired mutation, as well as a sufficient number of adjacent nucleotides, to provide a primer sequence of sufficient size and sequence complexity to form a stable duplex on both sides of the deletion junction being traversed. Typically, a primer of about 17 to about 75 nucleotides or more in length is preferred, with about 10 to about 25 or more residues on both sides of the junction of the sequence being altered.

In general, the technique of site-specific mutagenesis is well known in the art, as exemplified by various publications. As wilt be appreciated, the technique typically employs a phage vector, which exists in both a single stranded and double stranded form. Typical vectors useful in site-directed mutagenesis include vectors such as the M13 phage. These phages are readily commercially available and their use is generalty well known to those skilled in the art. Double stranded plasmids also are routinely employed in site directed mutagenesis, which eliminates the step of transferring the gene of interest from a plasmid to a phage.

In general, site-directed mutagenesis in accordance herewith is performed by first obtaining a single-stranded vector or melting apart of two strands of a double stranded vector which includes within its sequence a DNA sequence which encodes the promoter. An oligonucleotide primer bearing the desired mutated sequence is prepared, generally synthetically. This primer is then annealed with the single-stranded vector, and subjected to DNA polymerizing enzymes such as E. coli polymerase I Klenow fragment, in order to complete the synthesis of the mutation-bearing strand. Thus, a heteroduplex is formed wherein one strand encodes the original non-mutated sequence and the second strand bears the desired mutation. This heteroduplex vector is then used to transform or transfect appropriate cells, such as E. coli cells, and cells are selected which include recombinant vectors bearing the mutated sequence arrangement. Vector DNA can then be isolated from these cells and used for plant transformation. A genetic selection scheme was devised by Kunkel et al. (1987) to enrich for clones incorporating mutagenic oligonucleotides. Alternatively, the use of PCR with commercially available thermostable enzymes such as Taq polymerase may be used to incorporate a mutagenic oligonucleotide primer into an amplified DNA fragment that can then be cloned into an appropriate cloning or expression vector. The PCR-mediated mutagenesis procedures of Tomic et al. (1990) and Upender et al. (1995) provide two examples of such protocols. A PCR employing a thermostable ligase in addition to a thermostable polymerase also may be used to incorporate a phosphorylated mutagenic oligonucleotide into an amplified DNA fragment that may then be cloned into an appropriate cloning or expression vector. The mutagenesis procedure described by Michael (1994) provides an example of one such protocol.

The preparation of sequence variants of the selected promoter-encoding DNA segments using site-directed mutagenesis is provided as a means of producing potentially useful species and is not meant to be limiting, as there are other ways in which sequence variants of DNA sequences may be obtained. For example, recombinant vectors encoding the desired promoter sequence may be treated with mutagenic agents, such as hydroxylamine, to obtain sequence variants.

As used herein; the term "oligonucleotide directed mutagenesis procedure" refers to template-dependent processes and vector-mediated propagation which result in an increase in the concentration of a specific nucleic acid molecule relative to its initial concentration, or in an increase in the concentration of a detectable signal, such as amplification. As used herein, the term "oligonucleotide directed mutagenesis procedure" also is intended to refer to a process that involves the template-dependent extension of a primer molecule. The term template-dependent process refers to nucleic acid synthesis of an RNA or a DNA molecule wherein the sequence of the newly synthesized strand of nucleic acid is dictated by the well-known rules of complementary base pairing (see, for example, Watson and Ramstad, 1987). Typically, vector mediated methodologies involve the introduction of the nucleic acid fragment into a DNA or RNA vector, the clonal amplification of the vector, and the recovery of the amplified nucleic acid fragment. Examples of such methodologies are provided by U.S. Pat. No. 4,237,224. A number of template-dependent processes are available to amplify the target sequences of interest present in a sample, such methods being well known in the art and specifically disclosed herein below.

Where a clone comprising a promoter has been isolated in accordance with the instant invention, one may wish to delimit the essential promoter regions within the clone. One efficient, targeted means for preparing mutagenizing promoters relies upon the identification of putative regulatory elements within the promoter sequence. This can be initiated by comparison with promoter sequences known to be expressed in similar tissue-specific or developmentally unique manner. Sequences, which are shared among promoters with similar expression patterns, are likely candidates for the binding of transcription factors and are thus likely elements that confer expression patterns. Confirmation of these putative regulatory elements can be achieved by deletion analysis of each putative regulatory region followed by functional analysis of each deletion construct by assay of a reporter gene, which is functionally attached to each construct. As such, once a starting promoter sequence is provided, any of a number of different deletion mutants of the starting promoter could be readily prepared.

Functionally equivalent fragments of a transcription regulating nucleotide sequence of the invention can also be obtained by removing or deleting non-essential sequences without deleting the essential one. Narrowing the transcription regulating nucleotide sequence to its essential, transcription mediating elements can be realized in vitro by trial-and-arrow deletion mutations, or in sitico using promoter element search routines. Regions essential for promoter activity often demonstrate clusters of certain, known promoter elements. Such analysis can be performed using available computer algorithms such as PLACE ("Plant Cis-acting Regulatory DNA Elements"; Higo 1999), the BIOBASE database "Transfac" (Biologische Datenbanken GmbH, Braunschweig; Wingender 2001) or the database PlantCARE (Lescot 2002).

Preferably, functional equivalent fragments of one of the transcription regulating nucleotide sequences of the invention comprises at least 100 base pairs, preferably, at least 200 base pairs, more preferably at least 500 base pairs of a transcription regulating nucleotide sequence as described by SEQ ID NO: 1, 2, 3, 4, 5, 6, 7, 8, 9, 10, 11, 12, 15, 16, 17, 18, 19, 20, 23, 24, 25, 26, 27, 28, 31, 32, 33, 36, 37, 38, 39, 40, 41, 42, 43, 44, 45, 46, 47, 50, 51, 52, 53, 54, 55, 56, 57, 60, 61, 62, 63, 64, 65, 66, 67, 68, 69, 70, 71, 74, 75, 76, 77, 80, 81, 84, 85, 86, 87, 148, 149, 150, 151, 152, 153, 154, 155, 158, 159, 160, 161, 162, 163, 164, 165, 168, 169, 170, 171, 172, 173, 174, 175, 178, 179, 180, 181, 182, 183, 184, or 185. More preferably this fragment is starting from the 3'-end of the indicated sequences.

Especially preferred are equivalent fragments of transcription regulating nucleotide sequences, which are obtained by deleting the region encoding the 5'-untranslated region of the mRNA, thus only providing the (untranscribed) promoter region. The 5'-untranslated region can be easily determined by methods known in the art (such as 5'-RACE analysis). Accordingly, some of the transcription regulating nucleotide sequences of the invention are equivalent fragments of other sequences (see Table 2 below).

**Table 2**: Relationship of transcription regulating nucleotide sequences of the invention

| Transcription regulating sequence | Equivalent sequence | Equivalent fragment |
|---|---|---|
| SEQ ID NO: 9 (3954 bp) | SEQ ID NO: 10 (3962 bp) | SEQ ID NO: 1 (980 bp)<br>SEQ ID NO: 2 (994 bp)<br>SEQ ID NO: 3 (686 bp)<br>SEQ ID NO: 4 (698 bp)<br>SEQ ID NO: 5 (2051 bp)<br>SEQ ID NO: 6 (2066 bp)<br>SEQ ID NO: 7 (1757 bp)<br>SEQ ID NO: 8 (1770 bp)<br>SEQ ID NO: 11 (3660 bp)<br>SEQ ID NO: 12 (3666 bp) |
| SEQ ID NO: 19 (3892 bp) | SEQ ID NO: 20 (3083 bp) | SEQ ID NO: 15 (2254 bp)<br>SEQ ID NO: 16 (2259 bp)<br>SEQ ID NO: 17 (1446 bp)<br>SEQ ID NO: 18 (1450 bp) |
| SEQ ID NO: 27 (2352 bp) | SEQ ID NO: 28 (2168 bp) | SEQ ID NO: 23 (1026 bp)<br>SEQ ID NO: 24 (1030 bp)<br>SEQ ID NO: 25 (844 bp)<br>SEQ ID NO: 26 (846 bp) |
| SEQ ID NO: 33 (1441 bp) | - | SEQ ID NO: 31 (1201 bp)<br>SEQ ID NO: 32 (1216 bp) |
| SEQ ID NO: 36 (1587 bp) | SEQ ID NO: 37 (1602 bp) | SEQ ID NO: 38 (1130 bp)<br>SEQ ID NO: 39 (1145 bp)<br>SEQ ID NO: 40 (1169 bp)<br>SEQ ID NO: 41 (1191 bp)<br>SEQ ID NO: 42 (1130 bp)<br>SEQ ID NO: 43 (1145 bp)<br>SEQ ID NO: 44 (1120 bp)<br>SEQ ID NO: 45 (1135 bp)<br>SEQ ID NO: 46 (663 bp)<br>SEQ ID NO: 47 (678 bp) |
| SEQ ID NO: 54 (2012 bp) | SEQ ID NO: 55 (2033 bp) | SEQ ID NO: 50 (1069 bp)<br>SEQ ID NO: 51 (1088 bp)<br>SEQ ID NO: 52 (1034 bp)<br>SEQ ID NO: 53 (1053 bp)<br>SEQ ID NO: 56 (1977 bp)<br>SEQ ID NO: 57 (1998 bp) |
| SEQ ID NO: 68 (2996 bp) | SEQ ID NO: 69 (3008 bp) | SEQ ID NO: 60 (949 bp)<br>SEQ ID NO: 61 (962 bp)<br>SEQ ID NO: 62 (768 bp)<br>SEQ ID NO: 63 (779 bp)<br>SEQ ID NO: 64 (2040 bp)<br>SEQ ID NO: 65 (2053 bp)<br>SEQ ID NO: 66 (1859 bp) |

Table continued

| Transcription regulating sequence | Equivalent sequence | Equivalent fragment |
|---|---|---|
| | | SEQ ID NO: 67 (1870 bp)<br>SEQ ID NO: 70 (2815 bp)<br>SEQ ID NO: 71 (2825 bp) |
| SEQ ID NO: 74 (1564 bp) | SEQ ID NO: 75 (1578 bp) | SEQ ID NO: 76 (1404 bp)<br>SEQ ID NO: 77 (1418 bp) |
| SEQ ID NO: 80 (524 bp) | SEQ ID NO: 81 (539 bp) | - |
| SEQ ID NO: 84 (1988 bp) | SEQ ID NO: 85 (2010 bp) | SEQ ID NO: 86 (1776 bp)<br>SEQ ID NO: 87 (1800 bp) |
| SEQ ID NO: 152 (1845 bp) | SEQ ID NO: 153 (1854 bp) | SEQ ID NO: 148 (1106 bp)<br>SEQ ID NO: 149 (1115 bp)<br>SEQ ID NO: 150 (923 bp)<br>SEQ ID NO: 151 (930 bp)<br>SEQ ID NO: 154 (1662 bp)<br>SEQ ID NO: 155 (1669 bp) |
| SEQ ID NO: 162 (2017 bp) | SEQ ID NO: 163 (2004 bp) | SEQ ID NO: 158 (1017 bp)<br>SEQ ID NO: 159 (1008 bp)<br>SEQ ID NO: 160 (894 bp)<br>SEQ ID NO: 161 (886 bp)<br>SEQ ID NO: 164 (1894 bp)<br>SEQ ID NO: 165 (1882 bp) |
| SEQ ID NO: 172 (3232 bp) | SEQ ID NO: 173 (3269 bp) | SEQ ID NO: 168 (1300 bp)<br>SEQ ID NO: 169 (1337 bp)<br>SEQ ID NO: 170 (1190 bp)<br>SEQ ID NO: 171 (1226 bp)<br>SEQ ID NO: 174 (3123 bp)<br>SEQ ID NO: 175 (3158 bp) |
| SEQ ID NO: 182 (3019 bp) | SEQ ID NO: 183 (3006 bp) | SEQ ID NO: 178 (1504 bp)<br>SEQ ID NO: 179 (1492 bp)<br>SEQ ID NO: 180 (1450 bp)<br>SEQ ID NO: 181 (1436 bp)<br>SEQ ID NO: 184 (2965 bp)<br>SEQ ID NO: 185 (2950 bp) |

As indicated above, deletion mutants, deletion mutants of the promoter of the invention also could be randomly prepared and then assayed. With this strategy, a series of constructs are prepared, each containing a different portion of the clone (a subclone), and these constructs are then screened for activity. A suitable means for screening for activity is to attach a deleted promoter or intron construct, which contains a deleted segment to a selectable or screenable marker, and to isolate only those cells expressing the marker gene. In this way, a number of different, deleted promoter constructs are identified which still retain the desired, or even enhanced, activity. The smallest segment, which is required for activity, is thereby identified through comparison of the selected constructs. This segment may then be used for the construction of vectors for the expression of exogenous genes.

An expression cassette of the invention may comprise further regulatory elements. The term in this context is to be understood in the a broad meaning comprising all sequences which may influence construction or function of the expression cassette. Regulatory elements may for example modify transcription and/or translation in prokaryotic or eukaryotic organism. In an preferred embodiment the expression cassette of the invention comprised downstream (in 3'-direction) of the nucleic acid sequence to be expressed a transcription termination sequence and - optionally additional regulatory elements - each operably liked to the nucleic acid sequence to be expressed (or the transcription regulating nucleotide sequence).

Additional regulatory elements may comprise additional promoter, minimal promoters, or promoter elements, which may modify the expression regulating properties. For example the expression may be made depending on certain stress factors such water stress, abscisin (Lam 1991) or heat stress (Schoffl 1989). Furthermore additional promoters or promoter elements may be employed, which may realize expression in other organisms (such as E.coli or Agrobacterium). Such regulatory elements can be found in the promoter sequences or bacteria such as amy and SPO2 or in the promoter sequences of yeast or fungal promoters (such as ADC1, MFa, AC, P-60, CYC1, GAPDH, TEF, rp28, and ADH).

Furthermore, it is contemplated that promoters combining elements from more than one promoter may be useful. For example, US 5,491,288 discloses combining a Cauliflower Mosaic Virus promoter with a histone promoter. Thus, the elements from the promoters disclosed herein may be combined with elements from other promoters. Promoters, which are useful for plant transgene expression include those that are inducible, viral, synthetic, constitutive (Odell 1985), temporally regulated, spatially regulated, tissue-specific, and spatial-temporally regulated.

Where expression in specific tissues or organs is desired, tissue-specific promoters may be used. In contrast, where gene expression in response to a stimulus is desired, inducible promoters are the regulatory elements of choice. Where continuous expression is desired throughout the cells of a plant, constitutive promoters are utilized. Additional regulatory sequences upstream and/or downstream from the core promoter sequence may be included in expression constructs of transformation vectors to bring about varying levels of expression of heterologous nucleotide sequences in a transgenic plant.

A variety of 5' and 3' transcriptional regulatory sequences are available for use in the present invention. Transcriptional terminators are responsible for the termination of transcription and correct mRNA polyadenylation. The 3' nontranslated regulatory DNA sequence preferably includes from about 50 to about 1,000, more preferably about 100 to about 1,000, nucleotide base pairs and contains plant transcriptional and translational termination sequences. Appropriate transcriptional terminators and those which are known to function in plants include the CaMV 35S terminator, the tml terminator, the nopaline synthase terminator, the pea rbcS E9 terminator, the terminator for the T7 transcript from the octopine synthase gene of Agrobacterium tumefaciens, and the 3' end of the protease inhibitor I or II genes from potato or tomato, although other 3' elements known to those of skill in the art can also be employed. Alternatively, one also could use a gamma coixin, oleosin 3 or other terminator from the genus Coix.

Preferred 3' elements include those from the nopaline synthase gene of Agrobacterium tumefaciens (Bevan 1983), the terminator for the T7 transcript from the octopine synthase gene of Agrobacterium tumefaciens, and the 3' end of the protease inhibitor I or II genes from potato or tomato.

As the DNA sequence between the transcription initiation site and the start of the coding sequence, i.e., the untranslated leader sequence, can influence gene expression, one may also wish to employ a particular leader sequence. Preferred leader sequences are contemplated to include those, which include sequences, predicted to direct optimum expression of the attached gene, i.e., to include a preferred consensus leader sequence, which may increase or maintain mRNA stability and prevent inappropriate initiation of translation. The choice of such sequences will be known to those of skill in the art in light of the present disclosure. Sequences that are derived from genes that are highly expressed in plants will be most preferred.

Preferred regulatory elements also include the 5'-untranslated region, introns and the 3'-untranslated region of genes. Such sequences that have been found to enhance gene expression in transgenic plants include intron sequences (e.g., from Adh1, bronze1, actin1, actin 2 (WO 00/760067), or the sucrose synthase intron; see: The Maize Handbook, Chapter 116, Freeling and Walbot, Eds., Springer, New York (1994)) and viral leader sequences (e.g., from TMV, MCMV and AMV; Gallie 1987). For example, a number of non-translated leader sequences derived from viruses are known to enhance expression. Specifically, leader sequences from Tobacco Mosaic Virus (TMV), Maize Chlorotic Mottle Virus (MCMV), and Alfalfa Mosaic Virus (AMV) have been shown to be effective in enhancing expression (e.g., Gallie 1987; Skuzeski 1990). Other leaders known in the art include but are not limited to: Picomavirus leaders, for example, EMCV leader (Encephalomyocarditis 5' noncoding region) (Elroy-Stein 1989); Potyvirus leaders, for example, TEV leader (Tobacco Etch Virus); MDMV leader (Maize Dwarf Mosaic Virus); Human immunoglobulin heavy-chain binding protein (BiP) leader, (Macejak 1991); Untranslated leader from the coat protein mRNA of alfalfa mosaic virus (AMV RNA 4), (Jobling 1987; Tobacco mosaic virus leader (TMV), (Gallie 1989; and Maize Chlorotic Mottle Virus leader (MCMV) (Lommel 1991. See also, Della-Cioppa 1987. Regulatory elements such as Adh intron 1 (Callis 1987), sucrose synthase intron (Vasil 1989) or TMV omega element (Gallie 1989), may further be included where desired. Especially preferred are the 5'-untranslated region, introns and the 3'-untranslated region from the genes described by the GenBank *Arabidopsis thaliana* genome locii At4g12910, At1g66250, At4g00820, At2g36640, At2g34200, At3g11180, At4g00360, At2g48030, At2g38590, At1g23000, At3g15510, At3g50870, At4g00220, or At4g26320, or of functional equivalent thereof.

Additional preferred regulatory elements are enhancer sequences or polyadenylation sequences. Preferred polyadenylation sequences are those from plant genes or Agrobacterium T-DNA genes (such as for example the terminator sequences of the OCS (octopine synthase) or NOS (nopaline synthase) genes).

Examples of enhancers include elements from the CaMV 35S promoter, octopine synthase genes (Ellis el al., 1987), the rice actin I gene, the maize alcohol dehydrogenase gene (Callis 1987), the maize shrunken I gene (Vasil 1989), TMV Omega element (Gallie 1989) and promoters from non-plant eukaryotes (e.g. yeast; Ma 1988). Vectors for use in accordance with the present invention may be constructed to include the ocs enhancer element. This element was first identified as a 16 bp palindromic enhancer from the octopine synthase (ocs) gene of ultilane (Ellis 1987), and is present in at least 10 other promoters (Bouchez 1989). The use of an enhancer element, such as the ocs elements and particularly multiple copies of the element, will act to increase the level of transcription from adjacent promoters when applied in the context of plant transformation.

An expression cassette of the invention (or a vector derived therefrom) may comprise additional functional elements, which are to be understood in the broad sense as all elements which influence construction, propagation, or function of an expression cassette or a vector or a transgenic organism comprising them. Such functional elements may include origin of replications (to allow replication in bacteria; for the ORI of pBR322 or the P15A ori; Sambrook 1989), or elements required for Agrobacterium T-DNA transfer (such as for example the left and/or rights border of the T-DNA).

Ultimately, the most desirable DNA segments for introduction into, for example, a dicot genome, may be homologous genes or gene families which encode a desired trait (e.g., increased yield per acre) and which are introduced under the control of novel promoters or enhancers, etc., or perhaps even homologous or tissue specific (e.g., root-, collar/sheath-, whorl-, stalk-, earshank-, kemel- or leaf-specific) promoters or control elements. Indeed, it is envisioned that a particular use of the present invention will be the expression of a gene in a seed-preferential or seed-specific manner. Additionally, vectors may be constructed and employed in the intracellular targeting of a specific gene product within the cells of a transgenic plant or in directing a protein to the extracellular environment. This will generally be achieved by joining a DNA sequence encoding a transit or signal peptide sequence to the coding sequence of a particular gene. The resultant transit or signal peptide will transport the protein to a particular intracellular or extracellular destination, respectively, and will then be post-translationally removed. Transit or signal peptides act by facilitating the transport of proteins through intracellular membranes, e.g., vacuole, vesicle, plastid and mitochondrial membranes, whereas signal peptides direct proteins through the extracellular membrane.

A particular example of such a use concerns the direction of a herbicide resistance gene, such as the EPSPS gene, to a particular organelle such as the chloroplast rather than to the cytoplasm. This is exemplified by the use of the rbcs transit peptide which confers plastid-specific targeting of proteins. In addition, it is proposed that it may be desirable to target certain genes responsible for male sterility to the mitochondria, or to target certain genes for resistance to phytopathogenic organisms to the extracellular spaces, or to target proteins to the vacuole.

By facilitating the transport of the protein into compartments inside and outside the cell, these sequences may increase the accumulation of gene product protecting them from proteolytic degradation. These sequences also allow for additional mRNA sequences from highly expressed genes to be attached to the coding sequence of the genes. Since mRNA being translated by ribosomes is more stable than naked mRNA, the presence of translatable mRNA in front of the gene may increase the overall stability of the mRNA transcript from the gene and thereby increase synthesis of the gene product. Since transit and signal sequences are usually post-translationally removed from the initial translation product, the use of these sequences allows for the addition of extra translated sequences that may not appear on the final polypeptide. Targeting of certain proteins may be desirable in order to enhance the stability of the protein (US 5,545,818).

It may be useful to target DNA itself within a cell. For example, it may be useful to target introduced DNA to the nucleus as this may increase the frequency of transformation. Within the nucleus itself it would be useful to target a gene in order to achieve site-specific integration. For example, it would be useful to have a gene introduced through transformation replace an existing gene in the cell. Other elements include those that can be regulated by endogenous or exogenous agents, e.g., by zinc finger proteins, including naturally occurring zinc finger proteins or chimeric zinc finger proteins (see, e.g., US 5,789,538, WO 99/48909; WO 99/45132; WO 98/53060; WO 98/53057; WO 98/53058; WO 00/23464; WO 95119431; and WO 98/54311) or myb-like transcription factors. For example, a chimeric zinc finger protein may include amino acid sequences, which bind to a specific DNA sequence (the zinc finger) and amino acid sequences that activate (e.g., GAL 4 sequences) or repress the transcription of the sequences linked to the specific DNA sequence.

It is one of the objects of the present invention to provide recombinant DNA molecules comprising a nucleotide sequence according to the invention operably linked to a nucleotide segment of interest.

A nucleotide segment of interest is reflective of the commercial markets and interests of those involved in the development of the crop. Crops and markets of interest changes, and as developing nations open up world markets, new crops and technologies will also emerge. In addition, as the understanding of agronomic traits and characteristics such as yield and heterosis increase, the choice of genes for transformation will change accordingly. General categories of nucleotides of interest include, for example, genes involved in information, such as zinc fingers, those involved in communication, such as kinases, and those involved in housekeeping, such as heat shock proteins. More specific categories of transgenes, for example, include genes encoding important traits for agronomics, insect resistance, disease resistance, herbicide resistance, sterility, grain characteristics, and commercial products. Genes of interest include, generally, those involved in starch, oil, carbohydrate, or nutrient metabolism, as well as those affecting kernel size, sucrose loading, zinc finger proteins, see, e.g., US 5,789,538, WO 99/48909; WO 99/45132; WO 98/53060; WO 98/53057; WO 98/53058: WO 00/23464; WO 95/19431; and WO 98/54311, and the like.

One skilled in the art recognizes that the expression level and regulation of a transgene in a plant can vary significantly from line to line. Thus, one has to test several lines to find one with the desired expression level and regulation. Once a line is identified with the desired regulation specificity of a chimeric Cre transgene, it can be crossed with lines carrying different inactive replicons or inactive transgene for activation.

Other sequences which may be linked to the gene of interest, which encodes a polypeptide, are those which can target to a specific organelle, e.g., to the mitochondria, nucleus, or plastid, within the plant cell. Targeting can be achieved by providing the polypeptide with an appropriate targeting peptide sequence, such as a secretory signal peptide (for secretion or cell wall or membrane targeting, a plastid transit peptide, a chloroplast transit peptide, e.g., the chlorophyll a/b binding protein, a mitochondrial target peptide, a vacuole targeting peptide, or a nuclear targeting peptide, and the like. For example, the small subunit of ribulose bisphosphate carboxylase transit peptide, the EPSPS transit peptide or the dihydrodipicolinic acid synthase transit peptide may be used, for examples of plastid organelle targeting sequences (see WO 00/12732). Plastids are a class of plant organelles derived from proplastids and include chloroplasts, leucoplasts, amyloplasts, and chromoplasts. The plastids are major sites of biosynthesis in plants. In addition to photosynthesis in the chloroplast, plastids are also sites of lipid biosynthesis, nitrate reduction to ammonium, and starch storage. And while plastids contain their own circular, genome, most of the proteins localized to the plastids are encoded by the nuclear genome and are imported into the organelle from the cytoplasm.

Transgenes used with the present invention will often be genes that direct the expression of a particular protein or polypeptide product, but they may also be non-expressible DNA segments, e.g., transposons such as Ds that do no direct their own transposition. As used herein, an "expressible gene" is any gene that is capable of being transcribed into RNA (e.g., mRNA, antisense RNA, etc.) or translated into a protein, expressed as a trait of interest, or the like, etc., and is not limited to selectable, screenable or non-selectable marker genes. The invention also contemplates that, where both an expressible gene that is not necessarily a marker gene is employed in combination with a marker gene, one may employ the separate genes on either the same or different DNA segments for transformation. In the latter case, the different vectors are delivered concurrently to recipient cells to maximize cotransformation.

The choice of the particular DNA segments to be delivered to the recipient cells will often depend on the purpose of the transformation. One of the major purposes of transformation of crop plants is to add some commercially desirable, agronomically important traits to the plant. Such traits include, but are not limited to, herbicide resistance or tolerance; insect resistance or tolerance; disease resistance or tolerance (viral, bacterial, fungal, nematode); stress tolerance and/or resistance, as exemplified by resistance or tolerance to drought, heat, chilling, freezing, excessive moisture, salt stress; oxidative stress; increased yields; food content and makeup; physical appearance; male sterility; drydown; standability; prolificacy; starch properties; oil quantity and quality; and the like. One may desire to incorporate one or more genes conferring any such desirable trait or traits, such as, for example, a gene or genes encoding pathogen resistance.

In certain embodiments, the present invention contemplates the transformation of a recipient cell with more than one advantageous transgene. Two or more transgenes can be supplied in a single transformation event using either distinct transgene-encoding vectors, or using a single vector incorporating two or more gene coding sequences. For example, plasmids bearing the bar and aroA expression units in either convergent, divergent, or colinear orientation, are considered to be particularly useful. Further preferred combinations are those of an insect resistance gene, such as a Bt gene, along with a protease inhibitor gene such as pinll, or the use of bar in combination with either of the above genes. Of course, any two or more transgenes of any description, such as those conferring herbicide, insect, disease (viral, bacterial, fungal, nematode) or drought resistance, male sterility, drydown, standability, prolificacy, starch properties, oil quantity and quality, or those increasing yield or nutritional quality may be employed as desired.

## 1. Exemplary Transgenes

### 1.1. Herbicide Resistance

The genes encoding phosphinothricin acetyltransferase (bar and pat), glyphosate tolerant EPSP synthase genes, the glyphosate degradative enzyme gene gox encoding glyphosate oxidoreductase, deh (encoding a dehalogenase enzyme that inactivates dalapon), herbicide resistant (e.g., sulfonylurea and imidazolinone) acetolactate synthase, and bxn genes (encoding a nitrilase enzyme that degrades bromoxynil) are good examples of herbicide resistant genes for use in transformation. The bar and pat genes code for an enzyme, phosphinothricin acetyltransferase (PAT), which inactivates the herbicide phosphinothricin and prevents this compound from inhibiting glutamine synthetase enzymes. The enzyme 5-enolpyruvylshikimate 3-phosphate synthase (EPSP Synthase), is normally inhibited by the herbicide N-(phosphonomethyl)glycine (glyphosate). However, genes are known that encode glyphosate-resistant EPSP Synthase enzymes. The deh gene encodes the enzyme dalapon dehalogenase and confers resistance to the herbicide dalapon. The bxn gene codes for a specific nitrilase enzyme that converts bromoxynil to a non-herbicidal degradation product.

**1.2 Insect Resistance**

An important aspect of the present invention concerns the introduction of insect resistance-conferring genes into plants. Potential insect resistance genes, which can be introduced, include Bacillus thuringiensis crystal toxin genes or Bt genes (Watrud 1985). Bt genes may provide resistance to lepidopteran or coleopteran pests such as European Corn Borer (ECB) and corn rootworm (CRW). Preferred Bt toxin genes for use in such embodiments include the CrylA (b) and CrylA(c) genes. Endotoxin genes from other species of B. thuringiensis, which affect insect growth or development, may also be employed in this regard. Protease inhibitors may also provide insect resistance (Johnson 1989), and will thus have utility in plant transformation. The use of a protease inhibitor It gene, pinII, from tomato or potato is envisioned to be particularly useful. Even more advantageous is the use of a pinII gene in combination with a Bt toxin gene, the combined effect of which has been discovered by the present inventors to produce synergistic insecticidal activity. Other genes, which encode inhibitors of the insects' digestive system, or those that encode enzymes or co-factors that facilitate the production of inhibitors, may also be useful. Cystatin and amylase inhibitors, such as those from wheat and barley, may exemplify this group.

Also, genes encoding lectins may confer additional or attemative insecticide properties. Lectins (originally termed phytohemagglutinins) are multivalent carbohydrate-binding proteins, which have the ability to agglutinate red blood cells from a range of species. Lectins have been identified recently as insecticidal agents with activity against weevils, ECB and rootworm (Murdock 1990; Czapla & Lang, 1990). Lectin genes contemplated to be useful include, for example, barley and wheat germ agglutinin (WGA) and rice lectins (Gatehouse 1984), with WGA being preferred.

Genes controlling the production of large or small polypeptides active against insects when introduced into the insect pests, such as, e.g., lytic peptides, peptide hormones and toxins and venoms, form another aspect of the invention. For example, it is contemplated, that the expression of juvenile hormone esterase, directed towards specific insect pests, may also result in insecticidal activity, or perhaps cause cessation of metamorphosis (Hammock 1990).

Transgenic plants expressing genes, which encode enzymes that affect the integrity of the insect cuticle form yet another aspect of the invention. Such genes include those encoding, e.g., chitinase, proteases, lipases and also genes for the production of nikkomycin, a compound that inhibits chitin synthesis, the introduction of any of which is contemplated to produce insect resistant maize plants. Genes that code for activities that affect insect molting, such those affecting the production of ecdysteroid UDP-glucosyl transferase, also fall within the scope of the useful transgenes of the present invention.

Genes that code for enzymes that facilitate the production of compounds that reduce the nutritional quality of the host plant to insect pests are also encompassed by the present invention. It may be possible, for instance, to confer insecticidal activity on a plant by altering its sterol composition. Sterols are obtained by insects from their diet and are used for hormone synthesis and membrane stability. Therefore alterations in plant sterol composition by expression of novel genes, e.g., those that directly promote the production of undesirable sterols or those that convert desirable sterols into undesirable forms, could have a negative effect on insect growth and/or development and hence endow the plant with insecticidal activity. Lipoxygenases are naturally occurring plant enzymes that have been shown to exhibit anti-nutritional effects on insects and to reduce the nutritional quality of their diet. Therefore, further embodiments of the invention concern transgenic plants with enhanced lipoxygenase activity which may be resistant to insect feeding.

The present invention also provides methods and compositions by which to achieve qualitative or quantitative changes in plant secondary metabolites. One example concems transforming plants to produce DIMBOA which, it is contemplated, will confer resistance to European corn borer, rootworm and several other maize insect pests. Candidate genes that are particularly considered for use in this regard include those genes at the bx locus known to be involved in the synthetic DIMBOA pathway (Dunn 1981). The introduction of genes that can regulate the production of maysin, and genes involved in the production of dhurrin in sorghum, is also contemplated to be of use in facilitating resistance to earworm and rootworm, respectively.

Tripsacum dactyloides is a species of grass that is resistant to certain insects, including corn rootworm. It is anticipated that genes encoding proteins that are toxic to insects or are involved in the biosynthesis of compounds toxic to insects will be isolated from Tripsacum and that these novel genes will be useful in conferring resistance to insects. It is known that the basis of insect resistance in Tripsacum is genetic, because said resistance has been transferred to Zea mays via sexual crosses (Branson & Guss, 1972).

Further genes encoding proteins characterized as having potential insecticidal activity may also be used as transgenes in accordance herewith. Such genes include, for example, the cowpea trypsin inhibitor (CpTI; Hilder 1987) which may be used as a rootworm deterrent; genes encoding avermectin (Campbell 1989; Ikeda 1987) which may prove particularly useful as a corn rootworm deterrent; ribosome inactivating protein genes; and even genes that regulate plant structures. Transgenic maize including antiinsect antibody genes and genes that code for enzymes that can covert a non-toxic insecticide (pro-insecticide) applied to the outside of the plant into an insecticide inside the plant are also contemplated.

### 1.3 Environment or Stress Resistance

Improvement of a plant's ability to tolerate various environmental stresses such as, but not limited to, drought, excess moisture, chilling, freezing, high temperature, salt, and oxidative stress, can also be effected through expression of heterologous, or overexpression of homologous genes. Benefits may be realized in terms of increased resistance to freezing temperatures through the introduction of an "antifreeze" protein such as that of the Winter Flounder (Cutler 1989) or synthetic gene derivatives thereof. Improved chilling tolerance may also be conferred through increased expression of glycerol-3-phosphate acetyltransferase in chloroplasts (Murata 1992; Wolter 1992). Resistance to oxidative stress (often exacerbated by conditions such as chilling temperatures in combination with high light intensities) can be conferred by expression of superoxide dismutase (Gupta 1993), and may be improved by glutathione reductase (Bowler 1992). Such strategies may allow for tolerance to freezing in newly emerged fields as well as extending later maturity higher yielding varieties to earlier relative maturity zones.

Expression of novel genes that favorably effect plant water content, total water potential, osmotic potential, and turgor can enhance the ability of the plant to tolerate drought. As used herein, the terms "drought resistance" and "drought tolerance" are used to refer to a plants increased resistance or tolerance to stress induced by a reduction in water availability, as compared to normal circumstances, and the ability of the plant to function and survive in lower-water environments, and perform in a relatively superior manner. In this aspect of the invention it is proposed, for example, that the expression of a gene encoding the biosynthesis of osmotically active solutes can impart protection against drought. Within this class of genes are DNAs encoding mannitol dehydrogenase (Lee and Saier, 1982) and trehalose-6-phosphate synthase (Kaasen 1992). Through the subsequent action of native phosphatases in the cell or by the introduction and coexpression of a specific phosphatase, these introduced genes will result in the accumulation of either mannitol or trehalose, respectively, both of which have been well documented as protective compounds able to mitigate the effects of stress. Mannitol accumulation in transgenic tobacco has been verified and preliminary results indicate that plants expressing high levels of this metabolite are able to tolerate an applied osmotic stress (Tarczynski 1992).

Similarly, the efficacy of other metabolites in protecting either enzyme function (e.g. alanopine or propionic acid) or membrane integrity (e.g., alanopine) has been documented (Loomis 1989), and therefore expression of gene encoding the biosynthesis of these compounds can confer drought resistance in a manner similar to or complimentary to mannitol. Other examples of naturally occurring metabolites that are osmotically active and/or provide some direct protective effect during drought and/or desiccation include sugars and sugar derivatives such as fructose, erythritol (Coxson 1992), sorbitol, dulcitol (Karsten 1992), glucosylglycerol (Reed 1984; Erdmann 1992), sucrose, stachyose (Koster & Leopold 1988; Blackman 1992), ononitol and pinitol (Vernon & Bohnert 1992), and raffinose (Bernal-Lugo & Leopold 1992). Other osmotically active solutes, which are not sugars, include, but are not limited to, proline and glycinebetaine (Wyn-Jones and Storey, 1981). Continued canopy growth and increased reproductive fitness during times of stress can be augmented by introduction and expression of genes such as those controlling the osmotically active compounds discussed above and other such compounds, as represented in one exemplary embodiment by the enzyme myoinositol O-methyltransferase.

It is contemplated that the expression of specific proteins may also increase drought tolerance. Three classes of Late Embryogenic Proteins have been assigned based on structural similarities (see Dure 1989). All three classes of these proteins have been demonstrated in maturing (i.e., desiccating) seeds. Within these 3 types of proteins, the Type-II (dehydrin-type) have generally been implicated in drought and/or desiccation tolerance in vegetative plant parts (e.g.. Mundy and Chua, 1988; Piattcowski 1990; Yamaguchi-Shinozaki 1992). Recently, expression of a Type-III LEA (HVA-1) in tobacco was found to influence plant height, maturity and drought tolerance (Fitzpatrick, 1993). Expression of structural genes from all three groups may therefore confer drought tolerance. Other types of proteins induced during water stress include thiol proteases, aldolases and transmembrane transporters (Guerrero 1990), which may confer various protective and/or repair-type functions during drought stress. The expression of a gene that effects lipid biosynthesis and hence membrane composition can also be useful in conferring drought resistance on the plant.

Many genes that improve drought resistance have complementary modes of action. Thus, combinations of these genes might have additive and/or synergistic effects in improving drought resistance in maize. Many of these genes also improve freezing tolerance (or resistance); the physical stresses incurred during freezing and drought are similar in nature and may be mitigated in similar fashion. Benefit may be conferred via constitutive expression or tissue-specific of these genes, but the preferred means of expressing these novel genes may be through the use of a turgor-induced promoter (such as the promoters for the turgor-induced genes described in Guerrero et al. 1990 and Shagan 1993). Spatial and temporal expression patterns of these genes may enable maize to better withstand stress.

Expression of genes that are involved with specific morphological traits that allow for increased water extractions from drying soil would be of benefit. For example, introduction and expression of genes that alter root characteristics may enhance water uptake. Expression of genes that enhance reproductive fitness during times of stress would be of significant value. For example, expression of DNAs that improve the synchrony of pollen shed and receptiveness of the female flower parts, i.e., silks, would be of benefit. In addition, expression of genes that minimize kernel abortion during times of stress would increase the amount of grain to be harvested and hence be of value. Regulation of cytokinin levels in monocots, such as maize, by introduction and expression of an isopentenyl transferase gene with appropriate regulatory sequences can improve monocot stress resistance and yield (Gan 1995).

Given the overall role of water in determining yield, it is contemplated that enabling plants to utilize water more efficiently, through the introduction and expression of novel genes, will improve overall performance even when soil water availability is not limiting. By introducing genes that improve the ability of plants to maximize water usage across a full range of stresses relating to water availability, yield stability or consistency of yield performance may be realized.

Improved protection of the plant to abiotic stress factors such as drought, heat or chill, can also be achieved - for example - by overexpressing antifreeze polypeptides from Myoxocephalus Scorpius (WO 00/00512), Myoxocephalus octodecemspinosus, the Arabidopsis thaliana transcription activator CBF1, glutamate dehydrogenases (WO 97/12983, WO 98/11240), calcium-dependent protein kinase genes (WO 98/26045), calcineurins (WO 99/05902), casein kinase from yeast (WO 02/052012), famesyltransferases (WO 99/06580; Pei ZM et al. (1998) Science 282:287-290), ferritin (Deak M et al. (1999) Nature Biotechnology 17:192-196), oxalate oxidase (WO 99/04013; Dunwell JM (1998) Biotechn Genet Eng Rev 15:1-32), DREB1A factor ("dehydration response element B 1A"; Kasuga M et al. (1999) Nature Biotech 17:276-286), genes of mannitol or trehalose synthesis such as trehalose-phosphate synthase or trehalose-phosphate phosphatase (WO 97/42326) or by inhibiting genes such as trehalase (WO 97/50561).

1.4 Disease Resistance

It is proposed that increased resistance to diseases may be realized through introduction of genes into plants period. It is possible to produce resistance to diseases caused, by viruses, bacteria, fungi, root pathogens, insects and nematodes. It is also contemplated that control of mycotoxin producing organisms may be realized through expression of introduced genes.

Resistance to viruses may be produced through expression of novel genes. For example, it has been demonstrated that expression of a viral coat protein in a transgenic plant can impart resistance to infection of the plant by that virus and perhaps other closely related viruses (Cuozzo 1988, Hemenway 1988, Abel 1986). It is contemplated that expression of antisense genes targeted at essential viral functions may impart resistance to said virus. For example, an antisense gene targeted at the gene responsible for replication of viral nucleic acid may inhibit said replication and lead to resistance to the virus. It is believed that interference with other viral functions through the use of antisense genes may also increase resistance to viruses. Further it is proposed that it may be possible to achieve resistance to viruses through other approaches, including, but not limited to the use of satellite viruses.

It is proposed that increased resistance to diseases caused by bacteria and fungi may be realized through introduction of novel genes. It is contemplated that genes encoding so-called "peptide antibiotics," pathogenesis related (PR) proteins, toxin resistance, and proteins affecting host-pathogen interactions such as morphological characteristics will be useful. Peptide antibiotics are polypeptide sequences, which are inhibitory to growth of bacteria and other microorganisms. For example, the classes of peptides referred to as cecropins and magainins inhibit growth of many species of bacteria and fungi. It is proposed that expression of PR proteins in plants may be useful in conferring resistance to bacterial disease. These genes are induced following pathogen attack on a host plant and have been divided into at least five classes of proteins (Bol 1990). Included amongst the PR proteins are beta-1,3-glucanases, chitinases, and osmotin and other proteins that are believed to function in plant resistance to disease organisms. Other genes have been identified that have antifungal properties, e.g., UDA (stinging nettle lectin) and hevein (Broakgert 1989; Barkai-Golan 1978). It is known that certain plant diseases are caused by the production of phytotoxins. Resistance to these diseases could be achieved through expression of a novel gene that encodes an enzyme capable of degrading or otherwise inactivating the phytotoxin. Expression novel genes that alter the interactions between the host plant and pathogen may be useful in reducing the ability the disease organism to invade the tissues of the host plant, e.g., an increase in the waxiness of the leaf cuticle or other morphological characteristics.

Plant parasitic nematodes are a cause of disease in many plants. It is proposed that it would be possible to make the plant resistant to these organisms through the expression of novel genes. It is anticipated that control of nematode infestations would be accomplished by altering the ability of the nematode to recognize or attach to a host plant and/or enabling the plant to produce nematicidal compounds, including but not limited to proteins.

Furthermore, a resistance to fungi, insects, nematodes and diseases, can be achieved by by targeted accumulation of certain metabolites or proteins. Such proteins include but are not limited to glucosinolates (defense against herbivores), chitinases or glucanases and other enzymes which destroy the cell wall of parasites, ribosome-inactivating proteins (RIPs) and other proteins of the plant resistance and stress reaction as are induced when plants are wounded or attacked by microbes, or chemically, by, for example, salicylic acid, jasmonic acid or ethylene, or lysorymes from nonplant sources such as, for example, T4-lysozyme or lysozyme from a variety of mammals, insecticidal proteins such as *Bacillus thuringiensis* endotoxin, a-amylase inhibitor or protease inhibitors (cowpea trypsin inhibitor), lectins such as wheatgerm agglutinin, RNAses or ribozymes. Further examples are nucleic acids which encode the Trichoderma harzianum chit42 endochitinase (GenBank Acc. No.: S78423) or the N-hydroxylating, multi-fundional cytochrome P-450 (CYP79) protein from Sorghum bicolor (GenBank Acc. No.: U32624), or functional equivalents of these. The accumulation of glucosinolates as protection from pests (Rask L et al. (2000) Plant Mol Biol 42:93-113; Menard R et al. (1999) Phytochemistry 52:29-35), the expression of *Bacillus thuringiensis* endotoxins (Vaeck et al. (1987) Nature 328:33-37) or the protection against attack by fungi, by expression of chitinases, for example from beans (Broglie et al. (1991) Science 254:1194-1197), is advantageous. Resistance to pests such as, for example, the rice pest *Nilaparvata lugens* in rice plants can be achieved by expressing the snowdrop *(Galanthus nivalis*) lectin agglutinin (Rao et al. (1998) Plant J 15(4):469-77).The expression of synthetic crylA(b) and crylA(c) genes, which encode lepidopteraspecific *Bacillus thuringiensis* D-endotoxins can bring about a resistance to insect pests in various plants (Goyal RK et al. (2000) Crop Protection 19(5):307-312). Further target genes which are suitable for pathogen defense comprise "polygalacturonase-inhibiting protein" (PGIP), thaumatine, invertase and antimicrobial peptides such as lactoferrin (Lee TJ et al. (2002) J Amer Soc Horticult Sci 127(2):158-164).

**1.5 Mycotoxin Reduction/Elimination**

Production of mycotoxins, including aflatoxin and fumonisin, by fungi associated with plants is a significant factor in rendering the grain not useful. These fungal organisms do not cause disease symptoms and/or interfere with the growth of the plant, but they produce chemicals (mycotoxins) that are toxic to animals. Inhibition of the growth of these fungi would reduce the synthesis of these toxic substances and, therefore, reduce grain losses due to mycotoxin contamination. Novel genes may be introduced into plants that would inhibit synthesis of the mycotoxin without interfering with fungal growth. Expression of a novel gene, which encodes an enzyme capable of rendering the mycotoxin nontoxic, would be useful in order to achieve reduced mycotoxin contamination of grain. The result of any of the above mechanisms would be a reduced presence of mycotoxins on grain.

**1.6 Grain Composition or Quality**

Genes may be introduced into plants, particularly commercially important cereals such as maize, wheat or rice, to improve the grain for which the cereal is primarily grown. A wide range of novel transgenic plants produced in this manner may be envisioned depending on the particular end use of the grain.

For example, the largest use of maize grain is for feed or food. Introduction of genes that alter the composition of the grain may greatly enhance the feed or food value. The primary components of maize grain are starch, protein, and oil. Each of these primary components of maize grain may be improved by altering its level or composition. Several examples may be mentioned for illustrative purposes but in no way provide an exhaustive list of possibilities.

The protein of many cereal grains is suboptimal for feed and food purposes especially when fed to pigs, poultry, and humans. The protein is deficient in several amino acids that are essential in the diet of these species, requiring the addition of supplements to the grain. Limiting essential amino acids may include lysine, methionine, tryptophan, threonine, valine, arginine, and histidine. Some amino acids become limiting only after the grain is supplemented with other inputs for feed formulations. For example, when the grain is supplemented with soybean meal to meet lysine requirements, methionine becomes limiting. The levels of these essential amino acids in seeds and grain may be elevated by mechanisms which include, but are not limited to, the introduction of genes to increase the biosynthesis of the amino acids, decrease the degradation of the amino acids, increase the storage of the amino acids in proteins, or increase transport of the amino acids to the seeds or grain.

One mechanism for increasing the biosynthesis of the amino acids is to introduce genes that deregulate the amino acid biosynthetic pathways such that the plant can no longer adequately control the levels that are produced. This may be done by deregulating or bypassing steps in the amino acid biosynthetic pathway that are normally regulated by levels of the amino acid end product of the pathway. Examples include the introduction of genes that encode deregulated versions of the enzymes aspartokinase or dihydrodipicolinic acid (DHDP)-synthase for increasing lysine and threonine production, and anthranilate synthase for increasing tryptophan production. Reduction of the catabolism of the amino acids may be accomplished by introduction of DNA sequences that reduce or eliminate the expression of genes encoding enzymes that catalyse steps in the catabolic pathways such as the enzyme lysine-ketoglutarate reductase.

The protein composition of the grain may be altered to improve the balance of amino acids in a variety of ways including elevating expression of native proteins, decreasing expression of those with poor composition, changing the composition of native proteins, or introducing genes encoding entirely new proteins possessing superior composition. DNA may be introduced that decreases the expression of members of the zein family of storage proteins. This DNA may encode ribozymes or antisense sequences directed to impairing expression of zein proteins or expression of regulators of zein expression such as the opaque-2 gene product. The protein composition of the grain may be modified through the phenomenon of oosuppression, i.e., inhibition of expression of an endogenous gene through the expression of an identical structural gene or gene fragment introduced through transformation (Goring 1991). Additionally, the introduced DNA may encode enzymes, which degrade zeines. The decreases in zein expression that are achieved may be accompanied by increases in proteins with more desirable amino acid composition or increases in other major seed constituents such as starch. Alternatively, a chimeric gene may be introduced that comprises a coding sequence for a native protein of adequate amino acid composition such as for one of the globulin proteins or 10 kD zein of maize and a promoter or other regulatory sequence designed to elevate expression of said protein. The coding sequence of said gene may include additional or replacement codons for essential amino acids. Further, a coding sequence obtained from another species, or, a partially or completely synthetic sequence encoding a completely unique peptide sequence designed to enhance the amino acid composition of the seed may be employed.

The introduction of genes that alter the oil content of the grain may be of value. Increases in oil content may result in increases in metabolizable energy content and density of the seeds for uses in feed and food. The introduced genes may encode enzymes that remove or reduce rate-limitations or regulated steps in fatty acid or lipid biosynthesis. Such genes may include, but are not limited to, those that encode acetyl-CoA carboxylase, ACP-acyltransferase, beta-ketoacyl-ACP synthase, plus other well-known fatty acid biosynthetic activities. Other possibilities are genes that encode proteins that do not possess enzymatic activity such as acyl carrier protein. Additional examples include 2-acetyttransferase, oleosin pyruvate dehydrogenase complex, acetyl CoA synthetase, ATP citrate lyase, ADP-glucose pyrophosphorylase and genes of the camitine-CoA-acetyl-CoA shuttles. It is anticipated that expression of genes related to oil biosynthesis will be targeted to the plastid, using a plastid transit peptide sequence and preferably expressed in the seed embryo. Genes may be introduced that alter the balance of fatty acids present in the oil providing a more healthful or nutritive feedstuff. The introduced DNA may also encode sequences that block expression of enzymes involved in fatty acid biosynthesis, altering the proportions of fatty acids present in the grain such as described below.

Genes may be introduced that enhance the nutritive value of the starch component of the grain, for example by increasing the degree of branching, resulting in improved utilization of the starch in cows by delaying its metabolism.

Besides affecting the major constituents of the grain, genes may be introduced that affect a variety of other nutritive, processing, or other quality aspects of the grain as used for feed or food. For example, pigmentation of the grain may be increased or decreased. Enhancement and stability of yellow pigmentation is desirable in some animal feeds and may be achieved by introduction of genes that result in enhanced production of xanthophylls and carotenes by eliminating rate-limiting steps in their production. Such genes may encode altered forms of the enzymes phytoene synthase, phytoene desaturase, or lycopene synthase. Alternatively, unpigmented white corn is desirable for production of many food products and may be produced by the introduction of DNA, which blocks or eliminates steps in pigment production pathways.

Feed or food comprising some cereal grains possesses insufficient quantities of vitamins and must be supplemented to provide adequate nutritive value. Introduction of genes that enhance vitamin biosynthesis in seeds may be envisioned including, for example, vitamins A, E, $B_{12}$, choline, and the like. For example, maize grain also does not possess sufficient mineral content for optimal nutritive value. Genes that affect the accumulation or availability of compounds containing phosphorus, sulfur, calcium, manganese, zinc, and iron among others would be valuable. An example may be the introduction of a gene that reduced phytic acid production or encoded the enzyme phytase, which enhances phytic acid breakdown. These genes would increase levels of available phosphate in the diet, reducing the need for supplementation with mineral phosphate.

Numerous other examples of improvement of cereals for feed and food purposes might be described. The improvements may not even necessarily involve the grain, but may, for example, improve the value of the grain for silage. Introduction of DNA to accomplish this might include sequences that alter lignin production such as those that result in the "brown midrib" phenotype associated with superior feed value for cattle.

In addition to direct improvements in feed or food value, genes may also be introduced which improve the processing of grain and improve the value of the products resulting from the processing. The primary method of processing certain grains such as maize is via wetmilling. Maize may be improved though the expression of novel genes that increase the efficiency and reduce the cost of processing such as by decreasing steeping time.

Improving the value of wetmilling products may include altering the quantity or quality of starch, oil, corn gluten meal, or the components of corn gluten feed. Elevation of starch may be achieved through the identification and elimination of rate limiting steps in starch biosynthesis or by decreasing levels of the other components of the grain resulting in proportional increases in starch. An example of the former may be the introduction of genes encoding ADP-glucose pyrophosphorylase enzymes with altered regulatory activity or which are expressed at higher level. Examples of the latter may include selective inhibitors of, for example, protein or oil biosynthesis expressed during later stages of kernel development.

The properties of starch may be beneficially altered by changing the ratio of amylose to amylopectin, the size of the starch molecules, or their branching pattern. Through these changes a broad range of properties may be modified which include, but are not limited to, changes in gelatinization temperature, heat of gelatinization, clarity of films and pastes, Theological properties, and the like. To accomplish these changes in properties, genes that encode granule-bound or soluble starch synthase activity or branching enzyme activity may be introduced alone or combination. DNA such as antisense constructs may also be used to decrease levels of endogenous activity of these enzymes. The introduced genes or constructs may possess regulatory sequences that time their expression to specific intervals in starch biosynthesis and starch granule development. Furthermore, it may be advisable to introduce and express genes that result in the in vivo derivatization, or other modification, of the glucose moieties of the starch molecule. The covalent attachment of any molecule may be envisioned, limited only by the existence of enzymes that catalyze the derivatizations and the accessibility of appropriate substrates in the starch granule. Examples of important derivations may include the addition of functional groups such as amines, carboxyls, or phosphate groups, which provide sites for subsequent in vitro derivatizations or affect starch properties through the introduction of ionic charges. Examples of other modifications may include direct changes of the glucose units such as loss of hydroxyl groups or their oxidation to aldehyde or carboxyl groups.

Oil is another product of wetmilling of corn and other grains, the value of which may be improved by introduction and expression of genes. The quantity of oil that can be extracted by wetmilling may be elevated by approaches as described for feed and food above. Oil properties may also be altered to improve its performance in the production and use of cooking oil, shortenings, lubricants or other oil-derived products or improvement of its health attributes when used in the food-related applications. Novel fatty acids may also be synthesized which upon extraction can serve as starting materials for chemical syntheses. The changes in oil properties may be achieved by altering the type, level, or lipid arrangement of the fatty acids present in the oil. This in turn may be accomplished by the addition of genes that encode enzymes that catalyze the synthesis of novel fatty acids and the lipids possessing them or by increasing levels of native fatty acids while possibly reducing levels of precursors. Alternatively DNA sequences may be introduced which slow or block steps in fatty acid biosynthesis resulting in the increase in precursor fatty acid intermediates. Genes that might be added include desaturases, epoxidases, hydratases, dehydratases, and other enzymes that catalyze reactions involving fatty acid intermediates. Representative examples of catalytic steps that might be blocked include the desaturations from stearic to oleic acid and oleic to linolenic acid resulting in the respective accumulations of stearic and oleic acids.

Improvements in the other major cereal wetmilling products, gluten meal and gluten feed, may also be achieved by the introduction of genes to obtain novel plants. Representative possibilities include but are not limited to those described above for improvement of food and feed value.

In addition it may further be considered that the plant be used for the production or manufacturing of useful biological compounds that were either not produced at all, or not produced at the same level, in the plant previously. The novel plants producing these compounds are made possible by the introduction and expression of genes by transformation methods. The possibilities include, but are not limited to, any biological compound which is presently produced by any organism such as proteins, nucleic acids, primary and intermediary metabolites, carbohydrate polymers, etc. The compounds may be produced by the plant, extracted upon harvest and/or processing, and used for any presently recognized useful purpose such as pharmaceuticals, fragrances, industrial enzymes to name a few.

Further possibilities to exemplify the range of grain traits or properties potentially encoded by introduced genes in transgenic plants include grain with less breakage susceptibility for export purposes or larger grit size when processed by dry milling through introduction of genes that enhance gamma-zein synthesis, popcorn with improved popping, quality and expansion volume through genes that increase pericarp thickness, corn with whiter grain for food uses though introduction of genes that effectively block expression of enzymes involved in pigment production pathways, and improved quality of alcoholic beverages or sweet corn through introduction of genes which affect flavor such as the shrunken gene (encoding sucrose synthase) for sweet corn.

1.7 Tuber or Seed Composition or Quality

Various traits can be advantegously expressed especially in seeds or tubers to improve composition or quality. Such traits include but are not liited to:

- Expression of metabolic enzymes for use in the food-and-feed sector, for example of phytases and cellulases. Especially preferred are nucleic acids such as the artificial cDNA which encodes a microbial phytase (GenBank Acc. No.: A19451) or functional equivalents thereof.

- Expression of genes which bring about an accumulation of fine chemicals such as of tocopherols, tocotrienols or carotenoids. An example which may be mentioned is phytoene desaturase. Preferred are nucleic acids which encode the Narcissus pseudonarcissus photoene desaturase (GenBank Acc. No.: X78815) or functional equivalents thereof.

- Production of nutraceuticals such as, for example, polyunsaturated fatty acids (for example arachidonic acid, eicosapentaenoic acid or docosahexaenoic acid) by expression of fatty acid elongases and/or desaturases, or production of proteins with improved nutritional value such as, for example, with a high content of essential amino acids (for example the high-methionine 2S albumin gene of the brazil nut). Preferred are nucleic acids which encode the Bertholletia excelsa high-methionine 2S albumin (GenBank Acc. No.: AB044391), the Physcomitrella patens Δ6-acyl-lipid desaturase (GenBank Acc. No.: AJ222980; Girke et al. (1998) Plant J 15:39-48), the Mortierella alpina Δ6-desaturase (Sakuradani et al. 1999 Gene 238:445-453), the Caenorhabditis elegans Δ5-desaturase (Michaelson et al. 1998, FEBS Letters 439:215-218), the Caenorhabditis elegans Δ5-fatty acid desaturase (des-5) (GenBank Acc. No.: AF078796), the Mortierella alpina Δ5-desaturase (Michaelson et al. JBC 273:19055-19059), the Caenorhabditis elegans Δ6-eiongase (Beaudoin et al. 2000, PNAS 97:6421-6426), the Physcomitrella patens Δ6-elongase (Zank et al. 2000, Biochemical Society Transactions 28:654-657), or functional equivalents of these.

- Production of high-quality proteins and enzymes for industrial purposes (for example enzymes, such as lipases) or as pharmaceuticals (such as, for example, antibodies, blood clotting factors, interferons, lymphokins, colony stimulation factor, plasminogen activators, hormones or vaccines, as described by Hood EE, Jilka JM (1999) Curr Opin Biotechnol 10(4):382-6; Ma JK, Vine ND (1999) Curr Top Microbiol Immunol 236:275-92). For example, it has been possible to produce recombinant avidin from chicken albumen and bacterial b-glucuronidase (GUS) on a large scale in transgenic maize plants (Hood et al. (1999) Adv Exp Med Biol 464:127-47. Review).

- Obtaining an increased storability in cells which normally comprise fewer storage proteins or storage lipids, with the purpose of increasing the yield of these substances, for example by expression of acetyl-CoA carboxylase. Preferred nucleic acids are those which encode the Medicago sativa acetyl-CoA carboxylase (ACCase) (GenBank Acc. No.: L25042), or functional equivalents thereof.

- Reducing levels of α-glucan L-type tuber phosphorylase (GLTP) or .α-glucan H-type tuber phosphorylase (GHTP) enzyme activity preferably within the potato tuber (see US 5,998,701). The conversion of starches to sugars in potato tubers, particularly when stored at temperatures below 7°C., is reduced in tubers exhibiting reduced GLTP or GHTP enzyme activity. Reducing cold-sweetening in potatoes allows for potato storage at cooler temperatures, resulting in prolonged dormancy, reduced incidence of disease, and increased storage life. Reduction of GLTP or GHTP activity within the potato tuber may be accomplished by such techniques as suppression of gene expression using homologous antisense or double-stranded RNA, the use of co-suppression, regulatory silencing sequences. A potato plant having improved cold-storage characteristics, comprising a potato plant transformed with an expression cassette having a TPT promoter sequence operably linked to a DNA sequence comprising at least 20 nucleotides of a gene encoding an α-glucan phosphorylase selected from the group consisting of α-glucan L-type tuber phosphorylase (GLTP) and α-glucan H-type phosphorylase (GHTP).
Further examples of advantageous genes are mentioned for example in Dunwell JM, Transgenic approaches to crop improvement, J Exp Bot. 2000; 51 Spec No; pages 487-96.

### 1.8 Plant Agronomic Characteristics

Two of the factors determining where plants can be grown are the average daily temperature during the growing season and the length of time between frosts. Within the areas where it is possible to grow a particular plant, there are varying limitations on the maximal time it is allowed to grow to maturity and be harvested. The plant to be grown in a particular area is selected for its ability to mature and dry down to harvestable moisture content within the required period of time with maximum possible yield. Therefore, plants of varying maturities are developed for different growing locations. Apart from the need to dry down sufficiently to permit harvest is the desirability of having maximal drying take place in the field to minimize the amount of energy required for additional drying post-harvest. Also the more readily the grain can dry down, the more time there is available for growth and kernel fill. Genes that influence maturity and/or dry down can be identified and introduced into plant lines using transformation techniques to create new varieties adapted to different growing locations or the same growing location but having improved yield to moisture ratio at harvest. Expression of genes that are involved in regulation of plant development may be especially useful, e.g., the liguleless and rough sheath genes that have been identified in plants.
Genes may be introduced into plants that would improve standability and other plant growth characteristics. For example, expression of novel genes, which confer stronger stalks, improved root systems, or prevent or reduce ear droppage would be of great value to the corn farmer. Introduction and expression of genes that increase the total amount of photoassimilate available by, for example, increasing light distribution and/or interception would be advantageous. In addition the expression of genes that increase the efficiency of photosynthesis and/or the leaf canopy would further increase gains in productivity. Such approaches would allow for increased plant populations in the field.
Delay of late season vegetative senescence would increase the flow of assimilates into the grain and thus increase yield. Overexpression of genes within plants that are associated with "stay green" or the expression of any gene that delays senescence would be advantageous. For example, a non-yellowing mutant has been identified in Festuca pratensis (Davies 1990). Expression of this gene as well as others may prevent premature breakdown of chlorophyll and thus maintain canopy function.

### 1.9 Nutrient Utilization

The ability to utilize available nutrients and minerals may be a limiting factor in growth of many plants. It is proposed that it would be possible to after nutrient uptake, tolerate pH extremes, mobilization through the plant, storage pools, and availability for metabolic activities by the introduction of novel genes. These modifications would allow a plant to more efficiently utilize available nutrients. It is contemplated that an increase in the activity of, for example, an enzyme that is normally present in the plant and involved in nutrient utilization would increase the availability of a nutrient. An example of such an enzyme would be phytase. It is also contemplated that expression of a novel gene may make a nutrient source available that was previously not accessible, e.g., an enzyme that releases a component of nutrient value from a more complex molecule, perhaps a macromolecule.

### 1.10 Male Sterility

Male sterility is useful in the production of hybrid seed. It is proposed that male sterility may be produced through expression of novel genes. For example, it has been shown that expression of genes that encode proteins that interfere with development of the male inflorescence and/or gametophyte result in male sterility. Chimeric ribonuclease genes that express in the anthers of transgenic tobacco and oilseed rape have been demonstrated to lead to male sterility (Mariani 1990). For example, a number of mutations were discovered in maize that confer cytoplasmic male sterility. One mutation in particular, referred to as T cytoplasm, also correlates with sensitivity to Southern corn leaf blight. A DNA sequence, designated TURF-13 (Levings 1990), was identified that correlates with T cytoplasm. It would be possible through the introduction of TURF-13 via transformation to separate male sterility from disease sensitivity. As it is necessary to be able to restore male fertility for breeding purposes and for grain production, it is proposed that genes encoding restoration of male fertility may also be introduced.

### 1.11. Non-Protein-Expressing Sequences

#### 1.11.1 RNA-Expressing

DNA may be introduced into plants for the purpose of expressing RNA transcripts that function to affect plant phenotype yet are not translated into protein. Two examples are antisense RNA and RNA with ribozyme activity. Both may serve possible functions in reducing or eliminating expression of native or introduced plant genes.

Genes may be constructed or isolated, which when transcribed, produce antisense RNA or double-stranded RNA that is complementary to all or part(s) of a targeted messenger RNA(s). The antisense RNA reduces production of the polypeptide product of the messenger RNA. The polypeptide product may be any protein encoded by the plant genome. The aforementioned genes will be referred to as antisense genes. An antisense gene may thus be introduced into a plant by transformation methods to produce a novel transgenic plant with reduced expression of a selected protein of interest. For example, the protein may be an enzyme that catalyzes a reaction in the plant. Reduction of the enzyme activity may reduce or eliminate products of the reaction which include any enzymatically synthesized compound in the plant such as fatty acids, amino acids, carbohydrates, nucleic acids and the like. Alternatively, the protein may be a storage protein, such as a zein, or a structural protein, the decreased expression of which may lead to changes in seed amino acid composition or plant morphological changes respectively. The possibilities cited above are provided only by way of example and do not represent the full range of applications.

Expression of antisense-RNA or double-stranded RNA by one of the expression cassettes of the invention is especially preferred. Also expression of sense RNA can be employed for gene silencing (co-suppression). This RNA is preferably a non-translatable RNA. Gene regulation by double-stranded RNA ("double-stranded RNA interference"; dsRNAi) is well known in the arte and described for various organism including plants (e.g., Matzke 2000; Fire A et al 1998; WO 99/32619; WO 99/53050; WO 00/68374; WO 00/44914; WO 00/44895; WO 00/49035; WO 00/63364).

Genes may also be constructed or isolated, which when transcribed produce RNA enzymes, or ribozymes, which can act as endoribonucleases and catalyze the cleavage of RNA molecules with selected sequences. The cleavage of selected messenger RNA's can result in the reduced production of their encoded polypeptide products. These genes may be used to prepare novel transgenic plants, which possess them. The transgenic plants may possess reduced levels of polypeptides including but not limited to the polypeptides cited above that may be affected by antisense RNA.

It is also possible that genes may be introduced to produce novel transgenic plants, which have reduced expression of a native gene product, by a mechanism of cosuppression. It has been demonstrated in tobacco, tomato, and petunia (Goring 1991; Smith 1990; Napoli 1990; van der Krol 1990) that expression of the sense transcript of a native gene will reduce or eliminate expression of the native gene in a manner similar to that observed for antisense genes. The introduced gene may encode all or part of the targeted native protein but its translation may not be required for reduction of levels of that native protein.

### 1.11.2 Non-RNA-Expressing

For example, DNA elements including those of transposable elements such as Ds, Ac, or Mu, may be, inserted into a gene and cause mutations. These DNA elements may be inserted in order to inactivate (or activate) a gene and thereby "tag" a particular trait. In this instance the transposable element does not cause instability of the tagged mutation, because the utility of the element does not depend on its ability to move in the genome. Once a desired trait is tagged, the introduced DNA sequence may be used to clone the corresponding gene, e.g., using the introduced DNA sequence as a PCR primer together with PCR gene cloning techniques (Shapiro, 1983; Dellaporta 1988). Once identified, the entire gene(s) for the particular trait, including control or regulatory regions where desired may be isolated, cloned and manipulated as desired. The utility of DNA elements introduced into an organism for purposed of gene tagging is independent of the DNA sequence and does not depend on any biological activity of the DNA sequence, i.e., transcription into RNA or translation into protein. The sole function of the DNA element is to disrupt the DNA sequence of a gene.

It is contemplated that unexpressed DNA sequences, including novel synthetic sequences could be introduced into cells as proprietary "labels" of those cells and plants and seeds thereof. It would not be necessary for a label DNA element to disrupt the function of a gene endogenous to the host organism, as the sole function of this DNA would be to identify the origin of the organism. For example, one could introduce a unique DNA sequence into a plant and this DNA element would identify all cells, plants, and progeny of these cells as having arisen from that labeled source. It is proposed that inclusion of label DNAs would enable one to distinguish proprietary germplasm or germplasm derived from such, from unlabelled germplasm.

Another possible element, which may be introduced, is a matrix attachment region element (MAR), such as the chicken lysozyme A element (Stief 1989), which can be positioned around an expressible gene of interest to effect an increase in overall expression of the gene and diminish position dependant effects upon incorporation into the plant genome (Stief 1989; Phi-Van 1990).

Further nucleotide sequences of interest that may be contemplated for use within the scope of the present invention in operable linkage with the promoter sequences according to the invention are isolated nucleic acid molecules, e.g., DNA or RNA, comprising a plant nucleotide sequence according to the invention comprising an open reading frame that is preferentially expressed in a specific tissue, i.e., seed-, root, green tissue (leaf and stem), panicle-, or pollen, or is expressed constitutively.

## 2. Marker Genes

In order to improve the ability to identify transformants, one may desire to employ a selectable or screenable marker gene as, or in addition to, the expressible gene of interest. "Marker genes" are genes that impart a distinct phenotype to cells expressing the marker gene and thus allow such transformed cells to be distinguished from cells that do not have the marker. Such genes may encode either a selectable or screenable marker, depending on whether the marker confers a trait which one can 'select' for by chemical means, i.e., through the use of a selective agent (e.g., a herbicide, antibiotic, or the like), or whether it is simply a trait that one can identify through observation or testing, i.e., by 'screening' (e.g., the R-locus trait, the green fluorescent protein (GFP)). Of course, many examples of suitable marker genes are known to the art and can be employed in the practice of the invention.

Included within the terms selectable or screenable marker genes are also genes which encode a "secretable marker" whose secretion can be detected as a means of identifying or selecting for transformed cells. Examples include markers, which encode a secretable antigen that can be identified by antibody interaction, or even secretable enzymes, which can be detected by their catalytic activity. Secretable proteins fall into a number of classes, including small, diffusible proteins detectable, e.g., by ELISA; small active enzymes detectable in extracellular solution (e.g., alpha-amylase, beta-lactamase, phosphinothricin acetyltransferase); and proteins that are inserted or trapped in the cell wall (e.g., proteins that include a leader sequence such as that found in the expression unit of extensin or tobacco PR-S).

With regard to selectable secretable markers, the use of a gene that encodes a protein that becomes sequestered in the cell wall, and which protein includes a unique epitope is considered to be particularly advantageous. Such a secreted antigen marker would ideally employ an epitope sequence that would provide low background in plant tissue, a promoter-leader sequence that would impart efficient expression and targeting across the plasma membrane, and would produce protein that is bound in the cell wall and yet accessible to antibodies. A normally secreted wall protein modified to include a unique epitope would satisfy all such requirements.

One example of a protein suitable for modification in this manner is extensin, or hydroxyproline rich glycoprotein (HPRG). For example, the maize HPRG (Steifel 1990) molecule is well characterized in terms of molecular biology, expression and protein structure. However, any one of a variety of ultilane and/or glycine-rich wall proteins (Keller 1989) could be modified by the addition of an antigenic site to create a screenable marker.

One exemplary embodiment of a secretable saeenabte marker concerns the use of a maize sequence encoding the wall protein HPRG, modified to include a 15 residue epitope from the pro-region of murine interleukin, however, virtually any detectable epitope may be employed in such embodiments, as selected from the extremely wide variety of antigen-antibody combinations known to those of skill in the art. The unique extracellular epitope can then be straightforwardly detected using antibody labeling in conjunction with chromogenic or fluorescent adjuncts.

Elements of the present disclosure may be exemplified in detail through the use of the bar and/or GUS genes, and also through the use of various other markers. Of course, in light of this disclosure, numerous other possible selectable and/or screenable marker genes will be apparent to those of skill in the art in addition to the one set forth herein below. Therefore, it will be understood that the following discussion is exemplary rather than exhaustive. In light of the techniques disclosed herein and the general recombinant techniques which are known in the art, the present invention renders possible the introduction of any gene, including marker genes, into a recipient cell to generate a transformed plant.

### 2.1 Selectable Markers

Various selectable markers are known in the art suitable for plant transformation. Such markers may include but are not limited to:

#### 2.1.1 Negative selection markers

Negative selection markers confer a resistance to a biocidal compound such as a metabolic inhibitor (e.g., 2-deoxyglucose-6-phosphate, WO 98/45456), antibiotics (e.g., kanamycin, G 418, bleomycin or hygromycin) or herbicides (e.g., phosphinothricin or glyphosate). Transformed plant material (e.g., cells, tissues or plantlets), which express marker genes, are capable of developing in the presence of concentrations of a corresponding selection compound (e.g., antibiotic or herbicide), which suppresses growth of an untransformed wild type tissue. Especially preferred negative selection markers are those, which confer resistance to herbicides. Examples, which may be mentioned, are:

- Phosphinothricin acetyltransferases (PAT; also named Bialophos ® resistance; bar; de Block 1987; Vasil 1992, 1993; Weeks 1993; Becker 1994; Nehra 1994; Wan & Lemaux 1994; EP 0 333 033; US 4,975,374). Preferred are the bar gene from Streptomyces hygroscopicus or the pat gene from Streptomyces viridochromogenes. PAT inactivates the active ingredient in the herbicide bialaphos, phosphinothricin (PPT). PPT inhibits glutamine synthetase, (Murakami 1986; Twell 1989) causing rapid accumulation of ammonia and cell death.
- altered 5-enolpyruvylshikimate-3-phosphate synthase (EPSPS) conferring resistance to Glyphosate® (N-(phosphonomethyl)glycine) (Hinchee 1988; Shah 1986; Della-Cioppa 1987). Where a mutant EPSP synthase gene is employed, additional benefit may be realized through the incorporation of a suitable chloroplast transit peptide, CTP (EP-A1 0 218 571).
- Glyphosate® degrading enzymes (Glyphosate® oxidoreductase; gox),
- Dalapon® inactivating dehalogenases (deh)
- sulfonylurea- and/or imidazolinone-inactivating acetolactate synthases (*ahas* or ALS; for example mutated ahas/ALS variants with, for example, the S4, XI12, XA17, and/or Hra mutation (EP-A1 154 204)
- Bromoxynil® degrading nitrilases (bxn; Stalker 1988)
- Kanamycin- or geneticin (G418) resistance genes (NPTII; NPT or neo; Potrykus 1985) coding e.g., for neomycin phosphotransferases (Fraley 1983; Nehra 1994)
- 2-Desoxyglucose-6-phosphate phosphatase ($DOG^R1$-Gene product; WO 98/45456; EP 0 807 836) conferring resistance against 2-desoxyglucose (Randez-Gil 1995).
- hygromycin phosphotransferase (HPT), which mediates resistance to hygromycin (Vanden Elzen 1985).
- altered dihydrofolate reductase (Eichholtz 1987) conferring resistance against methotrexat (Thillet 1988);
- mutated anthranilate synthase genes that confers resistance to 5-methyl tryptophan.

Additional negative selectable marker genes of bacterial origin that confer resistance to antibiotics include the aadA gene, which confers resistance to the antibiotic spectinomycin, gentamycin acetyl transferase, streptomycin phosphotransferase (SPT), aminoglycoside-3-adenyl transferase and the bleomycin resistance determinant (Hayford 1988; Jones 1987; Svab 1990; Hille 1986).

Especially preferred are negative selection markers that confer resistance against the toxic effects imposed by D-amino acids like e.g., D-alanine and D-serine (WO 03/060133; Erikson 2004). Espedally preferred as negative selection marker in this contest are the daol gene (EC: 1.4. 3.3: GenBank Acc.-No.: U60066) from the yeast *Rhodotorula gracilis (Rhodosporidium toruloides)* and the *E. coli* gene *dsd*A (D-serine dehydratase (D-serine deaminase) [EC: 4.3.

1.18; GenBank Acc.-No.: J01603).

Transformed plant material (e.g., cells, embryos, tissues or plantlets) which express such marker genes are capable of developing in the presence of concentrations of a corresponding selection compound (e.g., antibiotic or herbicide) which suppresses growth of an untransformed wild type tissue. The resulting plants can be bred and hybridized in the customary fashion. Two or more generations should be grown in order to ensure that the genomic integration is stable and hereditary. Corresponding methods are described (Jenes 1993; Potrykus 1991).

Furthermore, reporter genes can be employed to allow visual screening, which may or may not (depending on the type of reporter gene) require supplementation with a substrate as a selection compound.

Various time schemes can be employed for the various negative selection marker genes. In case of resistance genes (e.g., against herbicides or D-amino acids) selection is preferably applied throughout callus induction phase for about 4 weeks and beyond at least 4 weeks into regeneration. Such a selection scheme can be applied for all selection regimes. It is furthermore possible (although not explicitly preferred) to remain the selection also throughout the entire regeneration scheme including rooting.

For example, with the phosphinotricin resistance gene (bar) as the selective marker, phosphinotricin at a concentration of from about 1 to 50 mg/l may be included in the medium. For example, with the *dao1* gene as the selective marker, D-serine or D-alanine at a concentration of from about 3 to 100 mg/l may be included in the medium. Typical concentrations for selection are 20 to 40 mg/l. For example, with the mutated ahas genes as the selective marker, PURSUIT™ at a concentration of from about 3 to 100 mg/l may be included in the medium. Typical concentrations for selection are 20 to 40 mg/l.

### 2.1.2 Positive selection marker

Furthermore, positive selection marker can be employed. Genes like isopentenyltransferase from *Agrobacterium* tumefaciens (strain:PO22; Genbank Acc.-No.: AB025109) may — as a key enzyme of the cytokinin biosynthesis — facilitate regeneration of transformed plants (e.g., by selection on cytokinin-free medium). Corresponding selection methods are described (Ebinuma 2000a,b). Additional positive selection markers, which confer a growth advantage to a transformed plant in comparison with a non-transformed one, are described e.g., in EP-A 0 601 092. Growth stimulation selection markers may include (but shall not be limited to) β-Glucuronidase (in combination with e.g., a cytokinin glucuronide), mannose-6-phosphate isomerase (in combination with mannose), UDP-galactose-4-epimerase (in combination with e.g., galactose), wherein mannose-6-phosphate isomerase in combination with mannose is especially preferred.

2.1.3 Counter-selection marker

Counter-selection markers are especially suitable to select organisms with defined deleted sequences comprising said marker (Koprek 1999). Examples for counter- selection marker comprise thymidin kinases (TK), cytosine deaminases (Gleave 1999; Perera 1993; Stougaard 1993), cytochrom P450 proteins (Koprek 1999), haloalkan dehalogenases (Naested 1999), iaaH gene products (Sundaresan 1995), cytosine deaminase codA (Schlaman & Hooykaas 1997), tms2 gene products (Fedoroff & Smith 1993), or α-naphthalene acetamide (NAM; Depicker 1988). Counter selection markers may be useful in the construction of transposon tagging lines. For example, by marking an autonomous transposable element such as Ac, Master Mu, or En/Spn with a counter selection marker, one could select for transformants in which the autonomous element is not stably integrated into the genome. This would be desirable, for example, when transient expression of the autonomous element is desired to activate in trans the transposition of a defective transposable element, such as Ds, but stable integration of the autonomous element is not desired. The presence of the autonomous element may not be desired in order to stabilize the defective element, i.e., prevent it from further transposing. However, it is proposed that if stable integration of an autonomous transposable element is desired in a plant the presence of a negative selectable marker may make it possible to eliminate the autonomous element during the breeding process.

### 2.2. Screenable Markers

Screenable markers that may be employed include, but are not limited to, a beta-glucuronidase (GUS) or uidA gene which encodes an enzyme for which various chromogenic substrates are known; an R-locus gene, which encodes a product that regulates the production of anthocyanin pigments (red color) in plant tissues (Dellaporta 1988); a beta-lactamase gene (Sutcliffe 1978), which encodes an enzyme for which various chromogenic substrates are known (e.g., PADAC, a chromogenic cephalosporin); a xylE gene (Zukowsky 1983) which encodes a catechol dioxygenase that can convert chromogenic catechols; an α-amylase gene (Ikuta 1990); a tyrosinase gene (Katz 1983) which encodes an enzyme capable of oxidizing tyrosine to DOPA and dopaquinone which in turn condenses to form the easily detectable compound melanin; β-galactosidase gene, which encodes an enzyme for which there are chromogenic substrates; a luciferase (lux) gene (Ow 1986), which allows for bioluminescence detection; or even an aequorin gene (Prasher 1985), which may be employed in calcium-sensitive bioluminescence detection, or a green fluorescent protein gene (Niedz 1995).

Genes from the maize R gene complex are contemplated to be particularly useful as screenable markers. The R gene complex in maize encodes a protein that acts to regulate the production of anthocyanin pigments in most seed and plant tissue. A gene from the R gene complex was applied to maize transformation, because the expression of this gene in transformed cells does not harm the cells. Thus, an R gene introduced into such cells will cause the expression of a red pigment and, if stably incorporated, can be visually scored as a red sector. If a maize line is dominant for genes encoding the enzymatic intermediates in the anthocyanin biosynthetic pathway (C2, A1, A2, Bz1 and Bz2), but carries a recessive allele at the R locus, transformation of any cell from that line with R will result in red pigment formation. Exemplary lines include Wisconsin 22 which contains the rg-Stadler allele and TR112, a K55 derivative which is r-g, b, P1. Alternatively any genotype of maize can be utilized if the C1 and R alleles are introduced together.

It is further proposed that R gene regulatory regions may be employed in chimeric constructs in order to provide mechanisms for controlling the expression of chimeric genes. More diversity of phenotypic expression is known at the R locus than at any other locus (Coe 1988). It is contemplated that regulatory regions obtained from regions 5' to the structural R gene would be valuable in directing the expression of genes, e.g., insect resistance, drought resistance, herbicide tolerance or other protein coding regions. For the purposes of the present invention, it is believed that any of the various R gene family members may be successfully employed (e.g., P, S, Lc, etc.). However, the most preferred will generally be Sn (particularly Sn:bol3). Sn is a dominant member of the R gene complex and is functionally similar to the R and B loci in that Sn controls the tissue specific deposition of anthocyanin pigments in certain seedling and plant cells, therefore, its phenotype is similar to R.

A further screenable marker contemplated for use in the present invention is firefly luciferase, encoded by the lux gene. The presence of the lux gene in transformed cells may be detected using, for example, X-ray film, scintillation counting, fluorescent spectrophotometry, low-light video cameras, photon counting cameras or multiwell luminometry. It is also envisioned that this system may be developed for populational screening for bioluminescence, such as on tissue culture plates, or even for whole plant screening. Where use of a screenable marker gene such as lux or GFP is desired, benefit may be realized by creating a gene fusion between the screenable marker gene and a selectable marker gene, for example, a GFP-NPTII gene fusion. This could allow, for example, selection of transformed cells followed by screening of transgenic plants or seeds.

### 3. Exemplary DNA Molecules

The invention provides an isolated nucleic acid molecule, e.g., DNA or RNA, comprising a plant nucleotide sequence comprising an open reading frame that is preferentially expressed in a specific plant tissue, i.e., in seeds, roots, green tissue (leaf and stem), panicles or pollen, or is expressed constitutively, or a promoter thereof.

These promoters include, but are not limited to, constitutive, inducible, temporally regulated, developmentally regulated, spatially-regulated, chemically regulated, stressresponsive, tissue-specific, viral and synthetic promoters. Promoter sequences are known to be strong or weak. A strong promoter provides for a high level of gene expression, whereas a weak promoter provides for a very low level of gene expression.

An inducible promoter is a promoter that provides for the tuming on and off of gene expression in response to an exogenously added agent, or to an environmental or developmental stimulus. A bacterial promoter such as the $P_{tac}$ promoter can be induced to varying levels of gene expression depending on the level of isothiopropytgaladoside added to the transformed bacterial cells. An isolated promoter sequence that is a strong promoter for heterologous nucleic acid is advantageous because it provides for a sufficient level of gene expression to allow for easy detection and selection of transformed cells and provides for a high level of gene expression when desired.

Within a plant promoter region there are several domains that are necessary for full function of the promoter. The first of these domains lies immediately upstream of the structural gene and forms the "core promoter region" containing consensus sequences, normally 70 base pairs immediately upstream of the gene. The core promoter region contains the characteristic CAAT and TATA boxes plus surrounding sequences, and represents a transcription initiation sequence that defines the transcription start point for the strudural gene.

The presence of the core promoter region defines a sequence as being a promoter: if the region is absent, the promoter is non-fundional. Furthermore, the core promoter region is insufficient to provide full promoter activity. A series of regulatory sequences upstream of the core constitute the remainder of the promoter. The regulatory sequences determine expression level, the spatial and temporal pattern of expression and, for an important subset of promoters, expression under inductive conditions (regulation by external factors such as light, temperature, chemicals, hormones). Regulated expression of the chimeric transacting viral replication protein can be further regulated by other genetic strategies. For example, Cre-mediated gene activation as described by Odell et al. 1990. Thus, a DNA fragment containing 3' regulatory sequence bound by lox sites between the promoter and the replication protein coding sequence that blocks the expression of a chimeric replication gene from the promoter can be removed by Cre-mediated excision and result in the expression of the transacting replication gene. In this case, the chimeric Cre gene, the chimeric trans-acting replication gene, or both can be under the control of tissue- and developmental-specific or inducible promoters. An alternate genetic strategy is the use of tRNA suppressor gene. For example, the regulated expression of a tRNA suppressor gene can conditionally control expression of a trans-acting replication protein coding sequence containing an appropriate termination codon as described by Ulmasov et al. 1997. Again, either the chimeric tRNA suppressor gene, the chimeric transacting replication gene, or both can be under the control of tissue- and developmental-specific or inducible promoters.

Frequently it is desirable to have continuous or inducible expression of a DNA sequence throughout the cells of an organism in a tissue-independent manner. For example, increased resistance of a plant t6 infection by soil- and airbome-pathogens might be accomplished by genetic manipulation of the plant's genome to comprise a continuous promoter operably linked to a heterologous pathogen-resistance gene such that pathogen-resistance proteins are continuously expressed throughout the plant's tissues.

Alternatively, it might be desirable to inhibit expression of a native DNA sequence within the seeds of a plant to achieve a desired phenotype. In this case, such inhibition might be accomplished with transformation of the plant to comprise a promoter operably linked to an antisense nucleotide sequence, such that seed-preferential or seed-specific expression of the antisense sequence produces an RNA transcript that interferes with translation of the mRNA of the native DNA sequence.

To define a minimal promoter region, a DNA segment representing the promoter region is removed from the 5' region of the gene of interest and operably linked to the coding sequence of a marker (reporter) gene by recombinant DNA techniques well known to the art. The reporter gene is operably linked downstream of the promoter, so that transcripts initiating at the promoter proceed through the reporter gene. Reporter genes generally encode proteins, which are easily measured, including, but not limited to, chloramphenicol acetyl transferase (CAT), beta-glucuronidase (GUS), green fluorescent protein (GFP), beta-galactosidase (beta-GAL), and luciferase.

The construct containing the reporter gene under the control of the promoter is then introduced into an appropriate cell type by transfection techniques well known to the art. To assay for the reporter protein, cell lysates are prepared and appropriate assays, which are well known in the art, for the reporter protein are performed. For example, if CAT were the reporter gene of choice, the lysates from cells transfected with constructs containing CAT under the control of a promoter under study are mixed with isotopically labeled chloramphenicol and acetyl-coenzyme A (acetyl-CoA). The CAT enzyme transfers the acetyl group from acetyl-CoA to the 2- or 3-position of chloramphenicol. The reaction is monitored by thin-layer chromatography, which separates acetylated chloramphenicol from unreacted material. The reaction products are then visualized by autoradiography.

The level of enzyme activity corresponds to the amount of enzyme that was made, which in turn reveals the level of expression from the promoter of interest. This level of expression can be compared to other promoters to determine the relative strength of the promoter under study. In order to be sure that the level of expression is determined by the promoter, rather than by the stability of the mRNA, the level of the reporter mRNA can be measured directly, such as by Northern blot analysis.

Once activity is detected, mutational and/or deletional analyses may be employed to determine the minimal region and/or sequences required to initiate transcription. Thus, sequences can be deleted at the 5' end of the promoter region and/or at the 3' end of the promoter region, and nucleotide substitutions introduced. These constructs are then introduced to cells and their activity determined.

In one embodiment, the promoter may be a gamma zein promoter, an oleosin ole16 promoter, a globulins promoter, an actin I promoter, an actin cl promoter, a sucrose synthetase promoter, an INOPS promoter, an EXM5 promoter, a globulin2 promoter, a b-32, ADPG-pyrophosphorylase promoter, an Ltpl promoter, an Ltp2 promoter, an oleosin ole17 promoter, an oleosin ole18 promoter, an actin 2 promoter, a pollen-specific protein promoter, a pollen-specific pectate lyase promoter, an anther-specific protein promoter, an anther-specific gene RTS2 promoter, a pollen-specific gene promoter, a tapeturn-specific gene promoter, tapeturn-specific gene RAB24 promoter, a anthranilate synthase alpha subunit promoter, an alpha zein promoter, an anthranilate synthase beta subunit promoter, a dihydrodipicolinate synthase promoter, a Thil promoter, an alcohol dehydrogenase promoter, a cab binding protein promoter, an H3C4 promoter, a RUBISCO SS starch branching enzyme promoter, an ACCase promoter, an actin3 promoter, an actin7 promoter, a regulatory protein GF14-12 promoter, a ribosomal protein L9 promoter, a cellulose biosynthetic enzyme promoter, an S-adenosyl-L-homocysteine hydrolase promoter, a superoxide dismutase promoter, a C-kinase receptor promoter, a phosphoglycerate mutase promoter, a root-specific RCc3 mRNA promoter, a glucose-6 phosphate isomerase promoter, a pyrophosphate-fructose 6-phosphatelphosphotransferase promoter, an ubiquitin promoter, a beta-ketoacyl-ACP synthase promoter, a 33 kDa photosystem 11 promoter, an oxygen evolving protein promoter, a 69 kDa vacuolar ATPase subunit promoter, a metatlothioneiMike protein promoter, a glyceraidehyde-3-phosphate dehydrogenase promoter, an ABA- and ripening-inducible-like protein promoter, a phenylalanine ammonia lyase promoter, an adenosine triphosphatase S-adenosyl-L-homocysteine hydrolase promoter, an a-tubulin promoter, a cab promoter, a PEPCase promoter, an R gene promoter, a lectin promoter, a light harvesting complex promoter, a heat shock protein promoter, a chalcone synthase promoter, a zein promoter, a globulin-1 promoter, an ABA promoter, an auxin-binding protein promoter, a UDP glucose flavonoid glycosyl-transferase gene promoter, an NTI promoter, an actin promoter, an opaque 2 promoter, a b70 promoter, an oleosin promoter, a CaMV 35S promoter, a CaMV 34S promoter, a CaMV 19S promoter, a histone promoter, a turgor-inducible promoter, a pea small subunit RuBP carboxylase promoter, a Ti plasmid mannopine synthase promoter, Ti plasmid nopaline synthase promoter, a petunia chalcone isomerase promoter, a bean glycine rich protein I promoter, a CaMV 35S transcript promoter, a potato patatin promoter, or a S-E9 small subunit RuBP carboxylase promoter.

4. Transformed (Transgenic) Plants of the Invention and Methods of Preparation

Plant species may be transformed with the DNA construct of the present invention by the DNA-mediated transformation of plant cell protoplasts and subsequent regeneration of the plant from the transformed protoplasts in accordance with procedures well known in the art.
Any plant tissue capable of subsequent clonal propagation, whether by organogenesis or embryogenesis, may be transformed with a vector of the present invention. The term "organogenesis," as used herein, means a process by which shoots and roots are developed sequentially from meristematic centers; the term "embryogenesis," as used herein, means a process by which shoots and roots develop together in a concerted fashion (not sequentially), whether from somatic cells or gametes. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristems, axillary buds, and root meristems), and induced meristem tissue (e.g., cotyledon meristem and ultilane meristem).
Plants of the present invention may take a variety of forms. The plants may be chimeras of transformed cells and non-transformed cells; the plants may be clonal transformants (e.g., all cells transformed to contain the expression cassette); the plants may comprise grafts of transformed and untransformed tissues (e.g., a transformed root stock grafted to an untransformed scion in citrus species). The transformed plants may be propagated by a variety of means, such as by clonal propagation or classical breeding techniques. For example, first generation (or T1) transformed plants may be seffed to give homozygous second generation (or T2) transformed plants, and the T2 plants further propagated through classical breeding techniques. A dominant selectable marker (such as npt II) can be associated with the expression cassette to assist in breeding.
Thus, the present invention provides a transformed (transgenic) plant cell, *in planta* or *ex planta*, including a transformed plastid or other organelle, e.g., nucleus, mitochondria or chloroplast. The present invention may be used for transformation of any plant species, including, but not limited to, cells from the plant species specified above in the DEFINITION section. Preferably, transgenic plants of the present invention are crop plants and in particular cereals (for example, corn, alfalfa, sunflower, rice, Brassica, canola, soybean, barley, soybean, sugarbeet, cotton, safflower, peanut, sorghum, wheat, millet, tobacco, Linum usitatissimum (linseed and fax), Camelina sativa, Brassica juncea, etc.), and even more preferably corn, rice and soybean. Other embodiments of the invention are related to cells, cell cultures, tissues, parts (such as plants organs, leaves, roots, etc.) and propagation material (such as seeds) of such plants.
The transgenic expression cassette of the invention may not only be comprised in plants or plant cells but may advantageously also be containing in other organisms such for example bacteria. Thus, another embodiment of the invention relates to transgenic cells or non-human, transgenic organisms comprising an expression cassette of the invention. Preferred are prokaryotic and eukaryotic organisms. Both microorganism and higher organisms are comprised. Preferred microorganisms are bacteria, yeast, algae, and fungi. Preferred bacteria are those of the genus *Escherichia, Erwinia, Agrobacterium, Flavobacterium, AlcaGgenes, Pseudomonas, Bacillus or Cyanobacterim* such as - for example - *Synechocystis* and other bacteria described in Brock Biology of Microorganisms Eighth Edition (pages A-8, A-9, A10 and A11).
Especially preferred are microorganisms capable to infect plants and to transfer DNA into their genome, especially bacteria of the genus Agrobacterium, preferably Agrobacterium tumefaciens and rhizogenes. Preferred yeasts are Candida, Saccharomyces, Hansenula and Pichia. Preferred Fungi are Aspergillus, Trichoderma, Ashbya, Neurospora, Fusarium, and Beauveria. Most preferred are plant organisms as defined above.
Transformation of plants can be undertaken with a single DNA molecule or multiple DNA molecules (i.e., cotransformation), and both these techniques are suitable for use with the expression cassettes of the present invention. Numerous transformation vectors are available for plant transformation, and the expression cassettes of this invention can be used in conjunction with any such vectors. The selection of vector will depend upon the preferred transformation technique and the target species for transformation.
A variety of techniques are available and known to those skilled in the art for introduction of constructs into a plant cell host. These techniques generally include transformation with DNA employing *A. tumefaciens* or A. rhizogenes as the transforming agent, liposomes, PEG precipitation, electroporation, DNA injection, direct DNA uptake, microprojectile bombardment, particle acceleration, and the like (See, for example, EP 295959 and EP 138341) (see below). However, cells other than plant cells may be transformed with the expression cassettes of the invention. The general descriptions of plant expression vectors and reporter genes, and Agrobacterium and Agrobacterium-mediated gene transfer, can be found in Gruber et al. (1993).

Expression vectors containing genomic or synthetic fragments can be introduced into protoplasts or into intact tissues or isolated cells. Preferably expression vectors are introduced into intact tissue. General methods of culturing plant tissues are provided for example by Maki et al., (1993); and by Phillips et al. (1988). Preferably, expression vectors are introduced into maize or other plant tissues using a direct gene transfer method such as microprojectile-mediated delivery, DNA injection, electroporation and the like. More preferably expression vectors are introduced into plant tissues using the microprojectile media delivery with the biolistic device. See, for example, Tomes et al. (1995). The vectors of the invention can not only be used for expression of structural genes but may also be used in exon-trap cloning, or promoter trap procedures to detect differential gene expression in varieties of tissues (Lindsey 1993; Auch & Reth 1990).

It is particularly preferred to use the binary type vectors of Ti and Ri plasmids of Agrobacterium spp. Ti-derived vectors transform a wide variety of higher plants, including monocotyledonous and dicotyledonous plants, such as soybean, cotton, rape, tobacco, and rice (Pacciotti 1985: Byrne 1987; Sukhapinda 1987; Lorz 1985; Potrykus, 1985; Park 1985: Hiei 1994). The use of T-DNA to transform plant cells has received extensive study and is amply described (EP 120516; Hoekema, 1985; Knauf, 1983; and An 1985). For introduction into plants, the chimeric genes of the invention can be inserted into binary vectors as described in the examples.

Other transformation methods are available to those skilled in the art, such as direct uptake of foreign DNA constructs (see EP 295959), techniques of electroporation (Fromm 1986) or high velocity ballistic bombardment with metal particles coated with the nucleic acid constructs (Kline 1987, and US 4,945,050). Once transformed, the cells can be regenerated by those skilled in the art. Of particular relevance are the recently described methods to transform foreign genes into commercially important crops, such as rapeseed (De Block 1989), sunflower (Everett 1987), soybean (McCabe 1988; Hinchee 1988; Chee 1989; Christou 1989; EP 301749), rice (Hiei 1994), and corn (Gordon-Kamm 1990; Fromm 1990).

Those skilled in the art will appreciate that the choice of method might depend on the type of plant, i.e., monocotyledonous or dicotyledonous, targeted for transformation. Suitable methods of transforming plant cells include, but are not limited to, microinjection (Crossway 1986), electroporation (Riggs 1986), Agrobacterium-mediated transformation (Hinchee 1988), direct gene transfer (Paszkowski 1984), and ballistic particle acceleration using devices available from Agracetus, Inc., Madison, Wis. And BioRad, Hercules, Calif. (see, for example, US 4,945,050; and McCabe 1988). Also see, Weissinger 1988; Sanford 1987 (onion); Christou 1988 (soybean); McCabe 1988 (soybean); Datta 1990 (rice); Klein 1988 (maize); Klein 1988 (maize); Klein 1988 (maize); Fromm 1990 (maize); and Gordon-Kamm 1990 (maize); Svab 1990 (tobacco chloroplast); Koziel 1993 (maize); Shimamoto 1989 (rice); Christou 1991 (rice); European Patent Application EP 0 332 581 (orchardgrass and other Pooideae); Vasil 1993 (wheat); Weeks 1993 (wheat).

In another embodiment, a nucleotide sequence of the present invention is directly transformed into the plastid genome. Plastid transformation technology is extensively described in US 5,451,513, 5,545,817, and 5,545,818, in PCT application no. WO 95116783, and in McBride et al., 1994. The basic technique for chloroplast transformation involves introducing regions of cloned plastid DNA flanking a seledable marker together with the gene of interest into a suitable target tissue, e.g., using biolistics or protoplast transformation (e.g., calcium chloride or PEG mediated transformation). The 1 to 1.5 kb flanking regions, termed targeting sequences, facilitate orthologous recombination with the plastid genome and thus allow the replacement or modification of specific regions of the plastome. Initially, point mutations in the chloroplast 16S rRNA and rps12 genes conferring resistance to spectinomycin and/or streptomycin are utilized as selectable markers for transformation (Svab 1990; Staub 1992). This resulted in stable homoplasmic transformants at a frequency of approximately one per 100 bombardments of target leaves. The presence of cloning sites between these markers allowed creation of a plastid-targeting vector for introduction of foreign genes (Staub 1993). Substantial increases in transformation frequency are obtained by replacement of the recessive rRNA or r-protein antibiotic resistance genes with a dominant selectable marker, the bacterial aadA gene encoding the spectinomycin-detoxifying enzyme aminoglycoside-3N-adenyltransferase (Svab 1993). Other seledable markers useful for plastid transformation are known in the art and encompassed within the scope of the invention. Typically, approximately 15-20 cell division cycles following transformation are required to reach a homoplastidic state. Plastid expression, in which genes are inserted by orthologous recombination into all of the several thousand copies of the circular plastid genome present in each plant cell, takes advantage of the enormous copy number advantage over nuclear-expressed genes to permit expression levels that can readily exceed 10% of the total soluble plant protein. In a preferred embodiment, a nucleotide sequence of the present invention is inserted into a plastid-targeting vector and transformed into the plastid genome of a desired plant host. Plants homoplastic for plastid genomes containing a nucleotide sequence of the present invention are obtained, and are preferentially capable of high expression of the nucleotide sequence.

*Agrobacterium tumefaciens* cells containing a vector comprising an expression cassette of the present invention, wherein the vector comprises a Ti plasmid, are useful in methods of making transformed plants. Plant cells are infected with an *Agrobacterium tumefaciens* as described above to produce a transformed plant cell, and then a plant is regenerated from the transformed plant cell. Numerous Agrobacterium vector systems useful in carrying out the present invention are known.

Various *Agrobacterium* strains can be employed, preferably disarmed *Agrobacterium tumefaciens* or *rhizogenes* strains. In a preferred embodiment, *Agrobacterium* strains for use in the practice of the invention include octopine strains, e.g., LBA4404 or agropine strains, e.g., EHA101 or EHA105. Suitable strains of *A. tumefaciens* for DNA transfer are for example EHA101[pEHA101] (Hood 1986), EHA105[pEHA105] (Li 1992), LBA4404[pAL4404] (Hoekema 1983), C58C1[pMP90] (Koncz & Schell 1986), and C58C1[pGV2260] (Deblaere 1985). Other suitable strains are *Agrobacterium* tumefaciens C58, a nopaline strain. Other suitable strains are *A. tumefaciens* C58C1 (Van Larebeke 1974), A136 (Watson 1975) or LBA4011 (Klapwijk 1980). In another preferred embodiment the soil-borne bacterium is a disarmed variant of *Agrobacterium rhizogenes* strain K599 (NCPPB 2659). Preferably, these strains are comprising a disarmed plasmid variant of a Ti- or Ri-plasmid providing the functions required for T-DNA transfer into plant cells (e.g., the vir genes). In a preferred embodiment, the *Agrobacterium* strain used to transform the plant tissue pre-cultured with the plant phenolic compound contains a L,L-succinamopine type Ti-plasmid, preferably disarmed, such as pEHA101. In another preferred embodiment, the *Agrobacterium* strain used to transform the plant tissue pre-cultured with the plant phenolic compound contains an octopine-type Ti-plasmid, preferably disarmed, such as pAL4404. Generally, when using octopine-type Ti-plasmids or helper plasmids, it is preferred that the virF gene be deleted or inactivated (Jarschow 1991).

The method of the invention can also be used in combination with particular *Agrubacterium* strains, to further increase the transformation efficiency, such as *Agrobacterium* strains wherein the vir gene expression and/or induction thereof is altered due to the presence of mutant or chimeric virA or virG genes (e.g. Hansen 1994; Chen and Winans 1991; Scheeren-Groot, 1994). Preferred are further combinations of *Agrobacterium* tumefaciens strain LBA4404 (Hiei 1994) with super-virulent plasmids. These are preferably pTOK246-based vectors (Ishida 1996).

A binary vector or any other vector can be modified by common DNA recombination techniques, multiplied in E. coli, and introduced into *Agrobacterium* by e.g., electroporation or other transformation techniques (Mozo & Hooykaas 1991).

*Agrobacterium* is grown and used in a manner similar to that described in Ishida (1996). The vector comprising *Agrobacterium* strain may, for example, be grown for 3 days on YP medium (5 g/l yeast extract, 10 g/l peptone, 5 g/l NaCl, 15 g/l agar, pH 6.8) supplemented with the appropriate antibiotic (e.g., 50 mg/l spectinomycin). Bacteria are collected with a loop from the solid medium and resuspended. In a preferred embodiment of the invention, *Agrobacterium* cultures are started by use of aliquots frozen at - 80°C.

The transformation of the target tissue (e.g., an immature embryo) by the *Agrobacterium* may be carried out by merely contacting the target tissue with the *Agrobacterfum.* The concentration of *Agrobacterium* used for infection and co-cultivation may need to be varied. For example, a cell suspension of the *Agrobacterium* having a population density of approximately from $10^5$ - 10", preferably $10^6$ to $10^{10}$, more preferably about $10^8$ cells or cfu / ml is prepared and the target tissue is immersed in this suspension for about 3 to 10 minutes. The resulting target tissue is then cultured on a solid medium for several days together with the *Agrobacterium.*

Preferably, the bacterium is employed in concentration of $10^6$ to $10^{10}$ cfu/ml. In a preferred embodiment for the co-cultivation step about 1 to 10 μl of a suspension of the soil-borne bacterium (e.g., *Agrobacteria*) in the co-cultivation medium are directly applied to each target tissue explant and air-dried. This is saving labor and time and is reducing unintended Agrobacterium-mediated damage by excess Agrobacterium usage.

For *Agrobacterium* treatment, the baderia are resuspended in a plant compatible co-cultivation medium. Supplementation of the co-culture medium with antioxidants (e.g., silver nitrate), phenol-absorbing compounds (like polyvinylpyrrolidone, Perl 1996) or thiol compounds (e.g., dithiothreitol, L-cysteine, Olhoft 2001) which can decrease tissue necrosis due to plant defence responses (like phenolic oxidation) may further improve the efficiency of *Agrobacterium*-mediated transformation. In another preferred embodiment, the co-cultivation medium of comprises least one thiol compound, preferably selected from the group consisting of sodium thiolsulfate, dithiotrietol (DTT) and cysteine. Preferably the concentration is between about 1 mM and 10mM of L-Cysteine, 0.1 mM to 5 mM DTT, and/or 0.1 mM to 5 mM sodium thiolsulfate. Preferably, the medium employed during co-cultivation comprises from about 1 μM to about 10 μM of silver nitrate and from about 50 mg/L to about 1,000 mg/L of L-Cystein. This results in a highly reduced vulnerability of the target tissue against Agrobacterium-mediated damage (such as induced necrosis) and highly improves overall transformation efficiency.

Various vector systems can be used in combination with Agrobacteria. Preferred are binary vector systems. Common binary vectors are based on "broad host range"-plasmids like pRK252 (Bevan 1984) or pTJS75 (Watson 1985) derived from the P-type plasmid RK2. Most of these vectors are derivatives of pBIN19 (Bevan 1984). Various binary vectors are known, some of which are commercially available such as, for example, pBI101.2 or pBIN19 (Clontech Laboratories, Inc. USA). Additional vectors were improved with regard to size and handling (e.g. pPZP; Hajdukiewicz 1994). Improved vector systems are described also in WO 02/00900.

Methods using either a form of direct gene transfer or Agrobacterium-mediated transfer usually, but not necessarily, are undertaken with a selectable marker, which may provide resistance to an antibiotic (e.g., kanamycin, hygromycin or methotrexate) or a herbicide (e.g., phosphinothricin). The choice of selectable marker for plant transformation is not, however, critical to the invention.

For certain plant species, different antibiotic or herbicide selection markers may be preferred. Selection markers used routinely in transformation include the nptII gene which confers resistance to kanamycin and related antibiotics (Messing & Vierra, 1982; Bevan 1983), the bar gene which confers resistance to the herbicide phosphinothricin (White 1990, Spencer 1990), the hph gene which confers resistance to the antibiotic hygromycin (Blochlinger & Diggelmann), and the dhfr gene, which confers resistance to methotrexate (Bourouis 1983).

**5. Production and Characterization of Stably Transformed Plants**

Transgenic plant cells are then placed in an appropriate selective medium for selection of transgenic cells, which are then grown to callus. Shoots are grown from callus. Plantlets are generated from the shoot by growing in rooting medium. The various constructs normally will be joined to a marker for selection in plant cells. Conveniently, the marker may be resistance to a biocide (particularly an antibiotic, such as kanamycin, G418, bleomycin, hygromycin, chloramphenicol, herbicide, or the like). The particular marker used will allow for selection of transformed cells as compared to cells lacking the DNA, which has been introduced. Components of DNA constructs including transcription cassettes of this invention may be prepared from sequences, which are native (endogenous) or foreign (exogenous) to the host. By "foreign" it is meant that the sequence is not found in the wild-type host into which the construct is introduced. Heterologous constructs will contain at least one region, which is not native to the gene from which the transcription-initiation-region is derived.

To confirm the presence of the transgenes in transgenic cells and plants, a variety of assays may be performed. Such assays include, for example, "molecular biological" assays well known to those of skill in the art, such as Southern and Northern blotting, in situ hybridization and nucleic acid-based amplification methods such as PCR or RT-PCR or TaqMan; "biochemical" assays, such as detecting the presence of a protein product, e.g., by immunological means (ELISAs and Western blots) or by enzymatic function; plant part assays, such as seed assays; and also, by analyzing the phenotype of the whole regenerated plant, e.g., for disease or pest resistance.

DNA may be isolated from cell lines or any plant parts to determine the presence of the preselected nucleic acid segment through the use of techniques well known to those skilled in the art. Note that intact sequences will not always be present, presumably due to rearrangement or deletion of sequences in the cell.

The presence of nucleic acid elements introduced through the methods of this invention may be determined by polymerase chain reaction (PCR). Using these technique discreet fragments of nucleic acid are amplified and detected by gel electrophoresis. This type of analysis permits one to determine whether a preselected nucleic acid segment is present in a stable transformant, but does not prove integration of the introduced preseleded nucleic acid segment into the host cell genome. In addition, it is not possible using PCR techniques to determine whether transformants have exogenous genes introduced into different sites in the, genome, i.e., whether transformants are of independent origin. It is contemplated that using PCR techniques it would be possible to clone fragments of the host genomic DNA adjacent to an introduced preselected DNA segment.

Positive proof of DNA integration into the host genome and the independent identities of transformants may be determined using the technique of Southern hybridization. Using this technique specific DNA sequences that were introduced into the host genome and flanking host DNA sequences can be identified. Hence the Southern hybridization pattern of a given transformant serves as an identifying characteristic of that transformant. In addition it is possible through Southern hybridization to demonstrate the presence of introduced preselected DNA segments in high molecular weight DNA, i.e., confirm that the introduced preselected, DNA segment has been integrated into the host cell genome. The technique of Southern hybridization provides information that is obtained using PCR, e.g., the presence of a preselected DNA segment, but also demonstrates integration into the genome and characterizes each individual transformant.

It is contemplated that using the techniques of dot or slot blot hybridization which are modifications of Southern hybridization techniques one could obtain the same information that is derived from PCR, e.g., the presence of a preselected DNA segment.

Both PCR and Southern hybridization techniques can be used to demonstrate transmission of a preselected DNA segment to progeny. In most instances the characteristic Southern hybridization pattern for a given transformant will segregate in progeny as one or more Mendelian genes (Spencer 1992); Laursen 1994) indicating stable inheritance of the gene. The non-chimeric nature of the callus and the parental transformants ($R_0$) was suggested by germline transmission and the identical Southern blot hybridization patterns and intensities of the transforming DNA in callus, $R_0$ plants and $R_1$ progeny that segregated for the transformed gene.

Whereas DNA analysis techniques may be conducted using DNA isolated from any part of a plant, RNA may only be expressed in particular cells or tissue types and hence it will be necessary to prepare RNA for analysis from these tissues. PCR techniques may also be used for detection and quantitation of RNA produced from introduced preselected DNA segments. In this application of PCR it is first necessary to reverse transcribe RNA into DNA, using enzymes such as reverse transcriptase, and then through the use of conventional PCR techniques amplify the DNA. In most instances PCR techniques, while useful, will not demonstrate integrity of the RNA product. Further information about the nature of the RNA product may be obtained by Northern blotting. This technique will demonstrate the presence of an RNA species and give information about the integrity of that RNA. The presence or absence of an RNA species can also be determined using dot or slot blot Northern hybridizations. These techniques are modifications of Northern blotting and will only demonstrate the presence or absence of an RNA species.

While Southern blotting and PCR may be used to detect the preselected DNA segment in question, they do not provide information as to whether the preselected DNA segment is being expressed. Expression may be evaluated by specifically identifying the protein products of the introduced preselected DNA segments or evaluating the phenotypic changes brought about by their expression.

Assays for the production and identification of specific proteins may make use of physical-chemical, structural, functional, or other properties of the proteins. Unique physical-chemical or structural properties allow the proteins to be separated and identified by electrophoretic procedures, such as native or denaturing gel electrophoresis or isoelectric focusing, or by chromatographic techniques such as ion exchange or gel exclusion chromatography. The unique structures of individual proteins offer opportunities for use of specific antibodies to detect their presence in formats such as an ELISA assay. Combinations of approaches may be employed with even greater specificity such as Western blotting in which antibodies are used to locate individual gene products that have been separated by electrophoretic techniques. Additional techniques may be employed to absolutely confirm the identity of the product of interest such as evaluation by amino acid sequencing following purification. Although these are among the most commonly employed, other procedures may be additionally used.

Assay procedures may also be used to identify the expression of proteins by their functionality, especially the ability of enzymes to catalyze specific chemical reactions involving specific substrates and products. These reactions may be followed by providing and quantifying the loss of substrates or the generation of products of the reactions by physical or chemical procedures. Examples are as varied as the enzyme to be analyzed.

Very frequently the expression of a gene product is determined by evaluating the phenotypic results of its expression. These assays also may take many forms including but not limited to analyzing changes in the chemical composition, morphology, or physiological properties of the plant. Morphological changes may include greater stature or thicker stalks. Most often changes in response of plants or plant parts to imposed treatments are evaluated under carefully controlled conditions termed bioassays.

**6. Uses of Transgenic Plants**

Once an expression cassette of the invention has been transformed into a particular plant species, it may be propagated in that species or moved into other varieties of the same species, particularly induding commercial varieties, using traditional breeding techniques. Particularly preferred plants of the invention include the agronomically important crops listed above. The genetic properties engineered into the transgenic seeds and plants described above are passed on by sexual reproduction and can thus be maintained and propagated in progeny plants. The present invention also relates to a transgenic plant cell, tissue, organ, seed or plant part obtained from the transgenic plant. Also included within the invention are transgenic descendants of the plant as well as transgenic plant cells, tissues, organs, seeds and plant parts obtained from the descendants.

Preferably, the expression cassette in the transgenic plant is sexually transmitted. In one preferred embodiment, the coding sequence is sexually transmitted through a complete normal sexual cycle of the R0 plant to the R1 generation. Additionally preferred, the expression cassette is expressed in the cells, tissues, seeds or plant of a transgenic plant in an amount that is different than the amount in the cells, tissues, seeds or plant of a plant, which only differs in that the expression cassette is absent.

The transgenic plants produced herein are thus expected to be useful for a variety of commercial and research purposes. Transgenic plants can be created for use in traditional agriculture to possess traits beneficial to the grower (e.g., agronomic traits such as resistance to water deficit, pest resistance, herbicide resistance or increased yield), beneficial to the consumer of the grain harvested from the plant (e.g., improved nutritive content in human food or animal feed; increased vitamin, amino acid, and antioxidant content; the production of antibodies (passive immunization) and nutriceuticals), or beneficial to the food processor (e.g., improved processing traits). In such uses, the plants are generally grown for the use of their grain in human or animal foods. Additionally, the use of root-specific promoters in transgenic plants can provide beneficial traits that are localized in the consumable (by animals and humans) roots of plants such as carrots, parsnips, and beets. However, other parts of the plants, including stalks, husks, vegetative parts, and the like, may also have utility, including use as part of animal silage or for ornamental purposes. Often, chemical constituents (e.g., oils or starches) of maize and other crops are extracted for foods or industrial use and transgenic plants may be created which have enhanced or modified levels of such components.

Transgenic plants may also find use in the commercial manufacture of proteins or other molecules, where the molecule of interest is extracted or purified from plant parts, seeds, and the like. Cells or tissue from the plants may also be cultured, grown in vitro, or fermented to manufacture such molecules. The transgenic plants may also be used in commercial breeding programs, or may be crossed or bred to plants of related crop species. Improvements encoded by the expression cassette may be transferred, e.g., from maize cells to cells of other species, e.g., by protoplast fusion.

The transgenic plants may have many uses in research or breeding, including creation of new mutant plants through insertional mutagenesis, in order to identify beneficial mutants that might later be created by traditional mutation and selection. An example would be the introduction of a recombinant DNA sequence encoding a transposable element that may be used for generating genetic variation. The methods of the invention may also be used to create plants having unique "signature sequences" or other marker sequences which can be used to identify proprietary lines or varieties.

Thus, the transgenic plants and seeds according to the invention can be used in plant breeding, which aims at the development of plants with improved properties conferred by the expression cassette, such as tolerance of drought, disease, or other stresses. The various breeding steps are characterized by well-defined human intervention such as selecting the lines to be crossed, directing pollination of the parental lines, or selecting appropriate descendant plants. Depending on the desired properties different breeding measures are taken. The relevant techniques are well known in the art and include but are not limited to hybridization, inbreeding, backcross breeding, multilane breeding, variety blend, interspecific hybridization, aneuploid techniques, etc. Hybridization techniques also include the sterilization of plants to yield male or female sterile plants by mechanical, chemical or biochemical means. Cross-pollination of a male sterile plant with pollen of a different line assures that the genome of the male sterile but female fertile plant will uniformly obtain properties of both parental lines. Thus, the transgenic seeds and plants according to the invention can be used for the breeding of improved plant lines, which for example increase the effectiveness of conventional methods such as herbicide or pesticide treatment or allow dispensing with said methods due to their modified genetic properties. Alternatively new crops with improved stress tolerance can be obtained which, due to their optimized genetic "equipment", yield harvested product of better quality than products, which were not able to tolerate comparable adverse developmental conditions.

## EXAMPLES

### Materials and General Methods

Unless indicated otherwise, chemicals and reagents in the Examples were obtained from Sigma Chemical Company (St. Louis, MO), restriction endonucleases were from New England Biolabs (Beverly, MA) or Roche (Indianapolis, IN), oligonucleotides were synthesized by MWG Biotech Inc. (High Point, NC), and other modifying enzymes or kits regarding biochemicals and molecular biological assays were from Clontech (Palo Alto, CA), Pharmacia Biotech (Piscataway, NJ), Promega Corporation (Madison, WI), or Stratagene (La Jolla, CA). Materials for cell culture media were obtained from Gibco/BRL (Gaithersburg, MD) or DIFCO (Detroit, MI). The cloning steps carried out for the purposes of the present invention, such as, for example, restriction cleavages, agarose gel electrophoresis, purification of DNA fragments, transfer of nucleic acids to nitrocellulose and nylon membranes, linking DNA fragments, transformation of *E. coli* cells, growing bacteria, multiplying phages and sequence analysis of recombinant DNA, are carried out as described by Sambrook (1989). The sequencing of recombinant DNA molecules is carried out using ABI laser fluorescence DNA sequencer following the method of Sanger (Sanger 1977).

### Example 1: Generation of transgenic plants

#### 1.1 Generation of transgenic Arabidopsis thaliana plants

For generating transgenic *Arabidopsis* plants *Agrobacterium tumefaciens* (strain C58C1[pMP90]) is transformed with the various promoter::GUS vector constructs (see below). Resulting *Agrobacterium* strains are subsequently employed to obtain transgenic plants. For this purpose a isolated transformed *Agrobacterium* colony is incubated in 4 ml culture (Medium: YEB medium with 50 μg/ml Kanamycin and 25 μg/ml Rifampicin) over night at 28°C. With this culture a 400 ml culture of the same medium is inoculated and incubated over night (28 °C, 220 rpm). The bacteria a precipitated by centrifugation (GSA-Rotor, 8.000 U/min, 20 min) and the pellet is resuspended in infiltration medium (1/2 MS-Medium; 0,5 g/l MES, pH 5,8; 50 g/l sucrose). The suspension is placed in a plant box (Duchefa) and 100 ml SILVET L-77 (Osi Special-ties Inc., Cat. P030196) are added to a final concentration of 0.02%. The plant box with 8 to 12 Plants is placed into an exsiccator for 10 to 15 min. under vacuum with subsequent, spontaneous ventilation (expansion). This process is repeated 2-3 times. Thereafter all plants are transferred into pods with wet-soil and grown under long daytime conditions (16 h light; day temperature 22-24°C, night temperature 19°C; 65% rel. humidity). Seeds are harvested after 6 weeks.

### 1.2 Generation of transgenic linseed

Transgenic linseed plants can be generated for example by the method of Bell et al., 1999, In Vitro Cell. Dev. Biol.-Plant. 35(6):456-465 by means of particle bombardment. Agrobacteria-mediated transformations can be generated for example by the method of Mlynarova et al. (1994), Plant Cell Report 13: 282-285.

## EXAMPLE 2: Growth conditions for plants for tissue-specific expression analysis

To obtain 4 and 7 days old seedlings, about 400 seeds *(Arabidopsis* thaliana ecotype Columbia) are sterilized with a 80% (v/v) ethanol:water solution for 2 minutes, treated with a sodium hypochlorite solution (0.5% v/v) for 5 minutes, washed three times with distillated water and incubated at 4°C for 4 days to ensure a standardized germination. Subsequently, seeds are incubated on Petri dishes with MS medium (Sigma M5519) supplemented with 1% sucrose, 0.5 g/l MES (Sigma M8652), 0.8% Difco-BactoAgar (Difco 0140-01), adjusted to pH 5.7. The seedlings are grown under 16 h light / 8 h dark cyklus (Philips 58W/33 white light) at 22°C and harvested after 4 or 7 days, respectively.

To obtain root tissue, 100 seeds are sterilized as described above, incubated at 4°C for 4 days, and transferred into 250ml flasks with MS medium (Sigma M5519) supplemented with additional 3% sucrose and 0.5 g/l MES (Sigma M8652), adjusted to pH 5.7 for further growing. The seedlings are grown at a 16 h light / 8 h dark cycle (Philips 58W/33 white light) at 22°C and 120 rpm and harvested after 3 weeks. For all other plant organs employed, seeds are sown on standard soil (Type VM, Manna-Italia, Via S. Giacomo 42, 39050 San Giacomo/ Laives, Bolzano, Italien), incubated for 4 days at 4°C to ensure uniform germination, and subsequently grown under a 16 h light / 8 darkness regime (OSRAM Lumi-lux Daylight 36W/12) at 22°C. Young rosette leaves are harvested at the 8-leaf stage (after about 3 weeks), mature rosette leaves are harvested after 8 weeks briefly before stem formation. Apices of out-shooting stems are harvested briefly after out-shooting. Stem, stem leaves, and flower buds are harvested in development stage 12 (Bowmann J (ed.), *Arabidopsis,* Atlas of Morphology, Springer New York, 1995) prior to stamen development. Open flowers are harvested in development stage 14 immediately after stamen development. Wilting flowers are harvested in stage 15 to 16. Green and yellow shoots used for the analysis have a length of 10 to 13 mm.

## EXAMPLE 3: Demonstration of expression profile

To demonstrate and analyze the transcription regulating properties of a promoter of the useful to operably link the promoter or its fragments to a reporter gene, which can be employed to monitor its expression both quatitatively and quantitatively. Preferably bacterial β-glucuronidase is used (Jefferson 1987). β-glucuronidase activity can be monitored *in planta* with chromogenic substrates such as 5-bromo-4-Chloro-3-indolyl-β-D-glucuronic acid during corresponding activity assays (Jefferson 1987). For determination of promoter activity and tissue specificity plant tissue is dissected, embedded, stained and analyzed as described (e.g., Bäumlein 1991).

For quantitative β-glucuronidase activity analysis MUG (methylumbelliferyl glucuronide) is used as a substrate, which is converted into MU (methylumbelifferone) and glucuronic acid. Under alkaline conditions this conversion can be quantitatively monitored fluorometrically (excitation at 365 nm, measurement at 455 nm; SpectroFluorimeter Thermo Life Sciences Fluoroscan) as described (Bustos 1989).

## EXAMPLE 4: Cloning of the promoter fragments

To isolate the promoter fragments described by SEQ ID NO: 1, 2, 3, 4, 5, 6, 7, 8, 9, 10, 11, 12, 15, 16, 17, 18, 19, 20, 23, 24, 25, 26, 27, 28, 31, 32, 33, 36, 37, 38, 39, 40, 41, 42, 43, 44, 45, 46, 47, 50, 51, 52, 53, 54, 55, 56, 57, 60, 61, 62, 63, 64, 65, 66, 67, 68, 69, 70, 71, 74, 75, 76, 77, 80, 81, 84, 85, 86, 87, 148, 149, 150, 151, 152, 153, 154, 155, 158, 159, 160, 161, 162, 163, 164, 165, 168, 169, 170, 171, 172, 173, 174, 175, 178, 179, 180, 181, 182, 183, 184, and 185, genomic DNA is isolated from *Arabidopsis thaliana* (ecotype Columbia) as described (Galbiati 2000). The isolated genomic DNA is employed as matrix DNA for a polymerase chain reaction (PCR) mediated amplification using the oligonucleotide primers and protocols indicated below (Table 3).

**Table 3**: PCR oligonucleotide primers for amplification of the various transcription regulating nucleotide sequences and restriction enzymes for modifying the resulting PCR products

| SEQ ID | Promoter | Forward Primer | Reverse Primer | Restriction enzymes |
|---|---|---|---|---|
| SEQ ID NO: 1 | pSUK222L | SUK222for SEQ ID NO: 90 | SUK222Lrev SEQ ID NO: 91 | BamHI/NcoI |

Table continued

| SEQ ID | Promoter | Forward Primer | Reverse Primer | Restriction enzymes |
|---|---|---|---|---|
| SEQ ID NO: 2 | pSUK222LGB | SUK222for SEQ ID NO: 90 | SUK222Lrev SEQ ID NO: 91 | BamHI/Ncol |
| SEQ ID NO: 3 | pSUK222S | SUK222for SEQ ID NO: 90 | SUK222Srev SEQ ID NO: 92 | BamHI/Ncol |
| SEQ ID NO: 4 | pSUK222SGB | SUK222for SEQ ID NO: 90 | SUK222Srev SEQ ID NO: 92 | BamHI/Ncol |
| SEQ ID NO: 5 | pSUK224L | SUK224for SEQ ID NO: 93 | SUK224Lrev SEQ ID NO: 94 | BamHI/Ncol |
| SEQ ID NO: 6 | pSUK224LGB | SUK224for SEQ ID NO: 93 | SUK224Lrev SEQ ID NO: 94 | BamHI/Ncol |
| SEQ ID NO: 7 | pSUK224S | SUK224for SEQ ID NO: 93 | SUK224Srev SEQ ID NO: 95 | BamHI/Ncol |
| SEQ ID NO: 8 | pSUK224SGB | SUK224for SEQ ID NO: 93 | SUK224Srev SEQ ID NO: 95 | BamHI/Ncol |
| SEQ ID NO: 9 | pSUK226L | SUK226for SEQ ID NO: 96 | SUK226Lrev SEQ ID NO: 97 | BamHI/Ncol |
| SEQ ID NO: 10 | pSUK226LGB | SUK226for SEQ ID NO: 96 | SUK226Lrev SEQ ID NO: 97 | BamHI/Ncol |
| SEQ ID NO: 11 | pSUK226S | SUK226for SEQ ID NO: 96 | SUK226Srev SEQ ID NO: 98 | BamHI/Ncol |
| SEQ ID NO: 12 | pSUK226SGB | SUK226for SEQ ID NO: 96 | SUK226Srev SEQ ID NO: 98 | BamHI/Ncol |
| SEQ ID NO: 15 | pSUK322L | SUK322for SEQ ID NO: 99 | SUK322Lrev SEQ ID NO: 100 | Xhol/BamHI |
| SEQ ID NO: 16 | pSUK322LGB | SUK322for SEQ ID NO: 99 | SUK322Lrev SEQ ID NO: 100 | Xhol/BamHI |
| SEQ ID NO: 17 | pSUK322S | SUK322for SEQ ID NO: 99 | SUK322Srev SEQ ID NO: 101 | Xhol/HindIII |
| SEQ ID NO: 18 | pSUK322SGB | SUK322for SEQ ID NO: 99 | SUK322Srev SEQ ID NO: 101 | Xhol/HindIII |
| SEQ ID NO: 19 | pSUK324LGB | SUK324for SEQ ID NO: 102 | SUK324Lrev SEQ ID NO: 103 | EcoRI/EcoRI |
| SEQ ID NO: 20 | pSUK324SGB | SUK324for SEQ ID NO: 102 | SUK324Srev SEQ ID NO: 104 | EcoRI/HindIII |
| SEQ ID NO: 23 | pSUK250L | SUK250for SEQ ID NO: 105 | SUK250Lrev SEQ ID NO: 106 | BamHI/Nool - |
| SEQ ID NO: 24 | pSUK250LGB | SUK250for SEQ ID NO: 105 | SUK250Lrev SEQ ID NO: 106 | BamHI/Ncol |
| SEQ ID NO: 25 | pSUK250S | SUK250for SEQ ID NO: 105 | SUK250Srev SEQ ID NO: 107 | BamHI/Ncol |
| SEQ ID NO: 26 | pSUK250SGB | SUK250for SEQ ID NO: 105 | SUK250Srev SEQ ID NO: 107 | BamHI/Ncol |
| SEQ ID NO: 27 | pSUK252LGB | SUK252for SEQ ID NO: 108 | SUK252Lrev SEQ ID NO: 109 | BamHI/Ncol |

Table continued

| SEQ ID | Promoter | Forward Primer | Reverse Primer | Restriction enzymes |
|---|---|---|---|---|
| SEQ ID NO: 28 | pSUK252SGB | SUK252for SEQ ID NO: 108 | SUK252Srev SEQ ID NO: 110 | BamHI/NcoI |
| SEQ ID NO: 31 | pSUK19S | SUK19Sfor SEQ ID NO: 111 | SUK19Srev SEQ ID NO: 112 | HindIII/XbaI |
| SEQ ID NO: 32 | pSUK19SGB | SUK19Sfor SEQ ID NO: 111 | SUK19Srev SEQ ID NO: 112 | HindIII/XbaI |
| SEQ ID NO: 33 | pSUK19LGB | SUK19Lfor SEQ ID NO: 113 | SUK19Lrev SEQ ID NO: 114 | BamHI/NcoI |
| SEQ ID NO: 36 | pSUK28L | SUK28for SEQ ID NO: 115 | SUK28Lrev SEQ ID NO: 116 | SalI/SmaI |
| SEQ ID NO: 37 | pSUK28LGB | SUK28for SEQ ID NO: 115 | SUK28Lrev SEQ ID NO: 116 | SalI/SmaI |
| SEQ ID NO: 38 | pSUK28S | SUK28for SEQ ID NO: 115 | SUK28Srev SEQ ID NO: 117 | SalI/XhoI |
| SEQ ID NO: 39 | pSUK28SGB | SUK28for SEQ ID NO: 115 | SUK28Srev SEQ ID NO: 117 | SalI/XhoI |
| SEQ ID NO: 40 | pSUK29L | SUK29for SEQ ID NO: 118 | SUK29Lrev SEQ ID NO: 119 | SalI/SmaI |
| SEQ ID NO: 41 | pSUK29LGB | SUK29for SEQ ID NO: 118 | SUK29Lrev SEQ ID NO: 119 | SalI/SmaI |
| SEQ ID NO: 42 | pSUK29S | SUK29for SEQ ID NO: 118 | SUK29Srev SEQ ID NO: 120 | SalI/XhoI |
| SEQ ID NO: 43 | pSUK29SGB | SUK29for SEQ ID NO: 118 | SUK29Srev SEQ ID NO: 120 | SalI/XhoI |
| SEQ ID NO: 44 | pSUK30L | SUK30for SEQ ID NO: 121 | SUK30Lrev SEQ ID NO: 122 | BamHI/NcoI |
| SEQ ID NO: 45 | pSUK30LGB | SUK30for SEQ ID NO: 121 | SUK30Lrev SEQ ID NO: 122 | BamHI/NcoI |
| SEQ ID NO: 46 | pSUK30S | SUK30for SEQ ID NO: 121 | SUK30Srev SEQ ID NO: 123 | BamHI/XhoI |
| SEQ ID NO: 47 | pSUK30SGB | SUK30for SEQ ID NO: 121 | SUK30Srev SEQ ID NO: 123 | BamHI/XhoI |
| SEQ ID NO: 50 | pSUK298L | SUK298for SEQ ID NO: 124 | SUK298Lrev SEQ ID NO: 125 | BamHI/NcoI |
| SEQ ID NO: 51 | pSUK298LGB | SUK298for SEQ ID NO: 124 | SUK298Lrev SEQ ID NO: 125 | BamHI/NcoI |
| SEQ ID NO: 52 | pSUK298S | SUK298for SEQ ID NO: 124 | SUK298Srev SEQ ID NO: 126 | BamHI/NcoI |
| SEQ ID NO: 53 | pSUK298SGB | SUK298for SEQ ID NO: 124 | SUK298Srev SEQ ID NO: 126 | BamHI/NcoI |
| SEQ ID NO: 54 | pSUK300L | SUK300for SEQ ID NO: 127 | SUK300Lrev SEQ ID NO: 128 | BamHI/NcoI |
| SEQ ID NO: 55 | pSUK300LGB | SUK300for SEQ ID NO: 127 | SUK300Lrev SEQ ID NO: 128 | BamHI/NcoI |

Table continued

| SEQ ID | Promoter | Forward Primer | Reverse Primer | Restriction enzymes |
|---|---|---|---|---|
| SEQ ID NO: 56 | pSUK300S | SUK300for SEQ ID NO: 127 | SUK300Srev SEQ ID NO: 129 | BamHI/Ncol |
| SEQ ID NO: 57 | pSUK300SGB | SUK300for SEQ ID NO: 127 | SUK300Srev SEQ ID NO: 129 | BamHI/Ncol |
| SEQ ID NO: 60 | pSUK292L | SUK292for SEQ ID NO: 130 | SUK292Lrev SEQ ID NO: 131 | BamHI/Ncol |
| SEQ ID NO: 61 | pSUK292LGB | SUK292for SEQ ID NO: 130 | SUK292Lrev SEQ ID NO: 131 | BamHI/Ncol |
| SEQ ID NO:62 | pSUK292S | SUK292for SEQ ID NO: 130 | SUK292Srev SEQ ID NO: 132 | BamHI/Ncol |
| SEQ ID NO: 63 | pSUK292SGB | SUK292for SEQ ID NO: 130 | SUK292Srev SEQ ID NO: 132 | BamHI/Ncol |
| SEQ ID NO: 64 | pSUK294L | SUK294for SEQ ID NO: 133 | SUK294Lrev SEQ ID NO: 134 | BamHI/Ncol |
| SEQ ID NO: 65 | pSUK294LGB | SUK294for SEQ ID NO: 133 | SUK294Lrev SEQ ID NO: 134 | BamHI/Ncol |
| SEQ ID NO: 66 | pSUK294S | SUK294for SEQ ID NO: 133 | SUK294Srev SEQ ID NO: 135 | BamHI/Ncol |
| SEQ ID NO: 67 | pSUK294SGB | SUK294for SEQ ID NO: 133 | SUK294Srev SEQ ID NO: 135 | BamHI/Ncol |
| SEQ ID NO: 68 | pSUK296L | SUK296for SEQ ID NO: 136 | SUK296Lrev SEQ ID NO: 137 | BamHI/Ncol |
| SEQ ID NO: 69 | pSUK296LGB | SUK296for SEQ ID NO: 136 | SUK296Lrev SEQ ID NO: 137 | BamHI/Ncol |
| SEQ ID NO: 70 | pSUK296S | SUK296for SEQ ID NO: 136 | SUK296Srev SEQ ID NO: 138 | BamHI/Ncol |
| SEQ ID NO: 71 | pSUK296SGB | SUK296for SEQ ID NO: 136 | SUK296Srev SEQ ID NO: 138 | BamHI/Ncol |
| SEQ ID NO: 74 | pSUK262L | SUK262for SEQ ID NO: 139 | SUK262Lrev SEQ ID NO: 140 | BamHI/Ncol |
| SEQ ID NO: 75 | pSUK262LGB | SUK262for SEQ ID NO: 139 | SUK262Lrev SEQ ID NO: 140 | BamHI/Ncol |
| SEQ ID NO: 76 | pSUK262S | SUK262for SEQ ID NO: 139 | SUK262Srev SEQ ID NO: 141 | BamHI/Ncol |
| SEQ ID NO: 77 | pSUK262SGB | SUK262for SEQ ID NO: 139 | SUK262Srev SEQ ID NO: 141 | BamHllNcol |
| SEQ ID NO: 80 | pSUK164 | SUK164for SEQ ID NO: 142 | SUK164rev SEQ ID NO: 143 | BamHllNcol |
| SEQ ID NO: 81 | pSUK164GB | SUK164for SEQ ID NO: 142 | SUK164rev SEQ ID NO: 143 | BamHllNcol |
| SEQ ID NO: 84 | pSUK352L | SUK352for SEQ ID NO: 144 | SUK352Lrev SEQ ID NO: 145 | BamHI/Ncol |
| SEQ ID NO: 85 | pSUK352LGB | SUK352for SEQ ID NO: 144 | SUK352Lrev SEQ ID NO: 145 | BamHllNcol |

Table continued

| SEQ ID | Promoter | Forward Primer | Reverse Primer | Restriction enzymes |
|---|---|---|---|---|
| SEQ ID NO: 86 | pSUK352S | SUK352for SEQ ID NO: 144 | SUK352Srev SEQ ID NO: 146 | BamHI/Ncol |
| SEQ ID NO: 87 | pSUK352SGB | SUK352for SEQ ID NO: 144 | SUK352Srev SEQ ID NO: 146 | BamHI/Ncol |
| SEQ ID NO: 148 | pSUK40L | SUK40for SEQ ID NO: 188 | SUK40Lrev SEQ ID NO: 189 | BamHI/Ncol |
| SEQ ID NO: 149 | pSUK40LGB | SUK40for SEQ ID NO: 188 | SUK40Lrev SEQ ID NO: 189 | BamHI/Ncol |
| SEQ ID NO: 150 | pSUK40S | SUK40for SEQ ID NO: 188 | SUK40Srev SEQ ID NO: 190 | BamHI/Ncol |
| SEQ ID NO: 151 | pSUK40SGB | SUK40for SEQ ID NO: 188 | SUK40Srev SEQ ID NO: 190 | BamHI/Ncol |
| SEQ ID NO: 152 | pSUK42L | SUK42for SEQ ID NO: 191 | SUK42Lrev SEQ ID NO: 192 | BamHI/Ncol |
| SEQ ID NO: 153 | pSUK42LGB | SUK42for SEQ ID NO: 191 | SUK42Lrev SEQ ID NO: 192 | BamHI/Ncol |
| SEQ ID NO: 154 | pSUK42S | SUK42for SEQ ID NO: 191 | SUK42Srev SEQ ID NO: 193 | BamHI/Ncol |
| SEQ ID NO: 155 | pSUK42SGB | SUK42for SEQ ID NO: 191 | SUK42Srev SEQ ID NO: 193 | BamHI/Ncol |
| SEQ ID NO: 158 | pSUK48L | SUK48for SEQ ID NO: 194 | SUK48Lrev SEQ ID NO: 195 | BamHI/Ncol |
| SEQ ID NO: 159 | pSUK48LGB | SUK48for SEQ ID NO: 194 | SUK48Lrev SEQ ID NO: 195 | BamHI/Ncol |
| SEQ ID NO: 160 | pSUK48S | SUK48for SEQ ID NO: 194 | SUK48Srev SEQ ID NO: 196 | BamHI/Ncol |
| SEQ ID NO: 161 | pSUK48SGB | SUK48for SEQ ID NO: 194 | SUK48Srev SEQ ID NO: 196 | BamHI/Ncol |
| SEQ ID NO: 162 | pSUK50L | SUK50for SEQ ID NO: 197 | SUK50Lrev SEQ ID NO: 198 | BamHI/Ncol |
| SEQ ID NO: 163 | pSUK50LGB | SUK50for SEQ ID NO: 197 | SUK50Lrev SEQ ID NO: 198 | BamHI/Ncol |
| SEQ ID NO: 164 | pSUK50S | SUK50for SEQ ID NO: 197 | SUK50Srev SEQ ID NO: 199 | BamHI/Ncol |
| SEQ ID NO: 165 | pSUK50SGB | SUK50for SEQ ID NO: 197 | SUK50Srev SEQ ID NO: 199 | BamHI/Ncol |
| SEQ ID NO: 168 | pSUK96L | SUK96for SEQ ID NO: 200 | SUK96Lrev SEQ ID NO: 201 | Xhol/Ncol |
| SEQ ID NO: 169 | pSUK96LGB | SUK96for SEQ ID NO: 200 | SUK96Lrev SEQ ID NO: 201 | Xhol/Ncol |
| SEQ ID NO: 170 | pSUK96S | SUK96for SEQ ID NO: 200 | SUK96Srev SEQ ID NO: 202 | Xhol/Ncol |
| SEQ ID NO: 171 | pSUK96SSGB | SUK96for SEQ ID NO: 200 | SUK96Srev SEQ ID NO: 202 | Xhol/Ncol |

Table continued

| SEQ ID | Promoter | Forward Primer | Reverse Primer | Restriction enzymes |
|---|---|---|---|---|
| SEQ ID NO: 172 | pSUK98L | SUK98for SEQ ID NO: 203 | SUK98Lrev SEQ ID NO: 204 | Xhol/Ncol |
| SEQ ID NO: 173 | pSUK98LGB | SUK98for SEQ ID NO: 203 | SUK98rev SEQ ID NO: 204 | Xhol/Ncol |
| SEQ ID NO: 174 | pSUK98S | SUK98for SEQ ID NO: 203 | SUK98Srev SEQ ID NO: 205 | Xhol/Ncol |
| SEQ ID NO: 175 | pSUK98SGB | SUK98for SEQ ID NO: 203 | SUK98Srev SEQ ID NO: 205 | Xhol/Nool |
| SEQ ID NO: 178 | pSUK152L | SUK152for SEQ ID NO: 206 | SUK152Lrev SEQ ID NO: 207 | BamHI/Ncol |
| SEQ ID NO: 179 | pSUK152LGB | SUK152for SEQ ID NO: 206 | SUK152Lrev SEQ ID NO: 207 | BamHI/Ncol |
| SEQ ID NO: 180 | pSUK152S | SUK152for SEQ ID NO: 206 | SUK152Srev SEQ ID NO: 208 | BamHI/Ncol |
| SEQ ID NO: 181 | pSUK152SGB | SUK152for SEQ ID NO: 206 | SUK152Srev SEQ ID NO: 208 | BamHI/Ncol |
| SEQ ID NO: 182 | pSUK154L | SUK154for SEQ ID NO: 209 | SUK154Lrev SEQ ID NO: 210 | BamHI/Ncol |
| SEQ ID NO: 183 | pSUK154LGB | SUK154for SEQ ID NO: 209 | SUK154Lrev SEQ ID NO: 210 | BamHI/Ncol |
| SEQ ID NO: 184 | pSUK154S | SUK154for SEQ ID NO: 209 | SUK154Srev SEQ ID NO: 211 | BamHIINcol |
| SEQ ID NO: 185 | pSUK154SGB | SUK154for SEQ ID NO: 209 | SUK154Srev SEQ ID NO: 211 | BamHI/Ncol |

Amplification is carried out as follows:

100 ng genomic DNA
1X PCR buffer
2,5 mM $MgCl_2$,
200 $\mu$M each of dATP, dCTP, dGTP und dTTP
10 pmol of each oligonucleotide primers
2,5 Units Pfu DNA Polymerase (Stratagene)
in a final volume of 50 $\mu$l

The following temperature program is employed for the various amplifications (BIORAD Thermocycler).

1. 95°C for 5 min
2. 54°C for 1 min, followed by 72°C for 5 min and 95°C for 30 sec. Repeated 25 times.
3. 54°C for 1 min, followed by 72°C for 10min.
4. Storage at 4°C

The resulting PCR-products are digested with the restriction endonucleases specified in the Table above (Table 3) and cloned into the vector pSUN0301 (SEQ ID NO: 147) (pre-digested with the same enzymes) upstream and in operable linkage to the glucuronidase (GUS) gene. Following stable transformation of each of these constructs into *Arabidopsis* thaliana tissue specificity and expression profile was analyzed by a histochemical and quantitative GUS-assay, respectively.

**EXAMPLE 5: Expression profile of the various promoter::GUS constructs in stably transformed *A. thaliana* plants**

**5.1 pSUK222L, pSUK222LGB, pSUK222S, pSUK222SGB, pSUK224L, pSUK224LGB, pSUK224S, pSUK224SGB, pSUK226L, pSUK226LGB, pSUK226S, pSUK226SGB**

All promoter fragments conferred seed-preferred expression to the reporter gene construct tested. GUS expression was not limited to embryos but covered also seed coat and endosperm tissue. GUS expression was detected early in seed development and continued into maturing siliques. Promoter activity was also observed in various organs and tissues of adult plants at varying strength, e.g. in hydathodes of rosette and caudal leaves, vessels of stalks and rosette leaves, and also in sepals, petals and pollen. GUS expression in seedlings was detected in vasculature of rosette leaves as well as in the central cylinder of roots and also in root tips. These side activities were least apparent while expression in seeds was strongest for the longest promoter fragment analyzed.

**5.2 pSUK322L, pSUK322LGB, pSUK322S, pSUK322SGB, pSUK324LGB, pSUK324SGB**

These promoter fragments conferred seed-preferred GUS expression. GUS activity in seeds including expression in embryos became apparent early during silique development and continued well into later stages. GUS expression was also detected at strongly varying degrees in rosette leaves and lateral roots of seedlings, as well as rosette leaves, stalks, siliques and anthers (mainly pollen) of adult plants analyzed.

**5.3 pSUK250L, pSUK250LGB, pSUK250S, pSUK250SGB, pSUK262LGB, pSUK252SGB**

Seed-preferred GUS expression conferred by the promoters analyzed commenced early in seed development and continued well into mature siliques. The promoters were found to be active only partially in embryos but strongly in seed coat and endosperm. In seedlings, side activities were mainly detected in petioles and leaf tips as well as root tips while GUS expression in adult plants was highly specific for seeds.

**5.4 pSUK19S, pSUK19SGB, pSUK19LGB**

Rather strong and seed-specific GUS expression was detected late during seed development in maturing siliques. The promoters were predominantly active in embryos but side activities were also observed at cotyledons of seedlings as well as guard cells of stalks and siliques and (rarely) in midveins of rosette leaves and anthers.

**5.5 pSUK28L, pSUK28LGB, pSUK28S, pSUK28SGB, pSUK29L, pSUK29LGB, pSUK29S, pSUK29SGB, pSUK30L, pSUK30LGB, pSUK30S, pSUK30SGB**

Embryos as well as seed coat and endosperm in late developing to mature seeds revealed GUS expression driven by all promoters analyzed. While pSUK28L and pSUK30L were also characterized by promoter side activity in vascular tissue of stalks and leaves of adult plants as well as silique and flower bottoms and mainly vascular tissue of seedlings, pSUK29L was highly seed-specific in its expression pattern observed.

**5.6 pSUK298L, pSUK298LGB, pSUK298S, pSUK298SGB, pSUK300L, pSUK300LGB, pSUK300S, pSUK300SGB**

These promoter fragments drive expression in entire seeds, i.e. embryos and seed coats as well as endosperm are affected. However, promoter activity is confined to an intermediate developmental phase of the seeds, i.e. neither young nor completely mature seeds reveal GUS expression. Side activities occur in patchy styles in hypocotyl, petioles and vascular leaf tissue of seedlings as well as in stalks, siliques, rosette leaves and floral organs of full-grown plants. These side activities are less pronounced for the shorter promoter fragment pSUK298L compared with pSUK300L.

**5.7 pSUK292L, pSUK292LGB, pSUK292S, pSUK292SGB, pSUK294L, pSUK294LGB, pSUK294S, pSUK294SGB, pSUK296L, pSUK296LGB, pSUK296S, pSUK296SGB**

Reporter gene expression was driven by all promoter fragments in embryos, endosperm and seed coat. GUS activity commenced rather early in seed development and continued into mature seeds. However, multiple side activities were observed, in particular for the longer promoter fragments analyzed. The most prominent side activity was obvious in guard cells, but other organs of seedlings and adult plants transformed with reporter gene constructs harboring the longer promoter variants also clearly showed GUS expression (e.g. in styles, trichomes).

**5.8 pSUK262L, pSUK262LGB, pSUK262S, pSUK262SGB**

These weak to medium-strong promoters are active in maturing seeds while expression was not detected in very young and completely mature seeds. Reporter gene expression was observed in seed coat and endosperm but could not be proven for the embryo. Side activity is marginal compared to other promoters disclosed: Expression apart from the seeds became only apparent for sepals, midveins and hydathodes of adult plants and seedlings analyzed.

**5.9 pSUK164, pSUK164GB**

Promoter activity was detected in embryo, endosperm and seed coat of seeds starting early in development and continuing into the maturing phase. Side activities were observed in cotyledons and first rosette leaves of seedlings as well as in vascular tissue of rosette leaves of adult plants and in pollen.

**5.10 pSUK352L, pSUK352LGB, pSUK352S, pSUK352SGB**

Promoter activity commences early in seed development and ceases before full maturation. Expression is specific for embryos, i.e. no reporter gene activity was detected in seeds coat or endosperm. Weak to medium side activity was also observed in cotyledons and leaves of seedlings.

**5.11 pSUK40L, pSUK40LGB, pSUK40S, pSUK40SGB, pSUK42L, pSUK42LGB, pSUK42S, pSUK42SGB**

These promoters confer seed-preferential expression when tested in Arabidopsis thaliana. Expression is strongest in young seeds and covers the developing embryo as well as the seed coat. Side activities are observed in root tips, rarely in vascular tissue of pods and stalks and also in anthers. Longer promoter fragments confer stronger expression in seeds but are also accompanied by more pronounced side activities.

**5.12 pSUK48L, pSUK48LGB, pSUK48S, pSUK48SGB, pSUK50L, pSUK50LGB, pSUK50S, pSUK50SGB**

These promoters drive seed-preferential expression starting at rather early but maintained also in later stages of seed development of Arabidopsis thaliana. Endosperm and seed coat clearly revealed reporter gene activity while it could not been shown that these promoters are also active in embryos. Whereas the shorter promoter fragments (pSUK48 derivatives) are almost void of side activity apart from some expression observed in vascular tissue of stalks and rarely observed expression in root tips, pSUK50 derivatives are also characterized by varying degrees of reporter gene activity in various organs and tissues analyzed.

**5.13 pSUK96L, pSUK96LGB, pSUK96S, pSUK96SGB, pSUK98L, pSUK98LGB, pSUK98S, pSUK98SGB**

These promoters confer seed-preferential expression in Arabidopsis thaliana. Expression in seed coat is stronger than in any other seed tissue. Side activity of the promoters was observed mainly in vascular tissue of stalks, ovaries and siliques but also in roots (particularly root tips). Those side activity but also the expression detected in seeds was stronger with longer promoter fragments (pSUK98 derivatives) where expression of the marker gene was also visible in vascular tissue of flowers and leaves of a number of plants analyzed.

**5.14 pSUK152L, pSUK152LGB, pSUK152S, pSUK152SGB, pSUK154L, pSUK154LGB, pSUK154S, pSUK154SGB**

These promoters drive strong seed-preferential expression of the marker gene analyzed in Arabidopsis thaliana. However, expression is weaker or not detectable in very young and fully mature seeds. The promoters are active in endosperm and embryo but not in the seed coat. The strength and variation of side activity does not depend on the length of the promoter analyzed. Marker gene expression was (besides seeds) observed in roots, apical meristems and petioles of seedlings, axillary meristems and vascular tissue of stalks of adult plants as well as in various flower organs.

**EXAMPLE 6: Expression profile of the various promoter::GUS constructs in stably transformed linseed plants**

**6.1 pSUK40L**

pSUK40L is a seed-specific promoter in linseed. No GUS expression was detected in early and young embryos. Middle strong to strong GUS expression was found in middle, late and mature embryos.

### 6.2 pSUK48L, and pSUK50L

pSUK48L is a seed-specific promoter in linseed. No GUS expression was detected in early and young embryos. Middle strong to strong GUS expression was found in middle, late and mature embryos.
pSUK50L is a seed-specific promoter in linseed. No GUS expression was detected in early and young embryos. Middle strong GUS expression was found mid-phase during embryo development. Middle strong to strong GUS expression was found in late and mature embryos.

### 6.3 pSUK96L

pSUK96L is a seed-specific promoter in linseed. No GUS expression was detected in early and young embryos. Weak to medium-strong expression was found in medium, late and mature embryos.

### 6.4 pSUK152L

pSUK152L is a seed-specific promoter in linseed. No to very weak GUS expression was detected in early and young embryos. Middle strong to strong GUS expression was found in middle, late and mature embryos. Often medium strong to strong GUS expression was also found in the seed capsula and in the flower.

## EXAMPLE 7: Vector Construction for Overexpression and Gene "Knockout" Experiments

### 7.1 Overexpression

Vectors used for expression of full-length "candidate genes" of interest in plants (overexpression) are designed to overexpress the protein of interest and are of two general types, biolistic and binary, depending on the plant transformation method to be used.
For biolistic transformation (biolistic vectors), the requirements are as follows:

1. a backbone with a bacterial selectable marker (typically, an antibiotic resistance gene) and origin of replication functional in Escherichia coli *(E. coli ;* e.g., ColE1), and

2. a plant-specific portion consisting of:

   a. a gene expression cassette consisting of a promoter (eg. ZmUBlint MOD), the gene of interest (typically, a full-length cDNA) and a transcriptional terminator (*e.g.*, Agrobacterium tumefaciens nos terminator);
   b. a plant selectable marker cassette, consisting of a suitable promoter, selectable marker gene (*e.g.*, D-amino acid oxidase; dao1) and transcriptional terminator (eg. nos terminator).

Vectors designed for transformation by Agrobacterium tumefaciens (*A. tumefaciens*; binary vectors) consist of:

1. a backbone with a bacterial selectable marker functional in both E. coli and *A. tumefaciens (e.g.,* spectinomycin resistance mediated by the aadA gene) and two origins of replication, functional in each of aforementioned bacterial hosts, plus the *A. tumefaciens* virG gene;
2. a plant-specific portion as described for biolistic vectors above, except in this instance this portion is flanked by A. *tumefaciens* right and left border sequences which mediate transfer of the DNA flanked by these two sequences to the plant.

### 7.2 Gene Silencing Vectors

Vectors designed for reducing or abolishing expression of a single gene or of a family or related genes (gene silencing vectors) are also of two general types corresponding to the methodology used to downregulate gene expression: antisense or double-stranded RNA interference (dsRNAi).

(a) Anti-sense

For antisense vectors, a full-tength or partial gene fragment (typically, a portion of the cDNA) can be used in the same vectors described for full-length expression, as part of the gene expression cassette. For antisense-mediated down-regulation of gene expression, the coding region of the gene or gene fragment will be in the opposite orientation relative to the promoter; thus, mRNA will be made from the non-coding (antisense) strand in planta.

(b) dsRNAi

For dsRNAi vectors, a partial gene fragment (typically, 300 to 500 basepairs long) is used in the gene expression cassette, and is expressed in both the sense and antisense orientations, separated by a spacer region (typically, a plant intron, eg. the OsSH1 intron 1, or a selectable marker, eg. conferring kanamycin resistance). Vectors of this type are designed to form a double-stranded mRNA stem, resulting from the basepairing of the two complementary gene fragments *in planta*.

Biolistic or binary vectors designed for overexpression or knockout can vary in a number of different ways, including eg. the seledable markers used in plant and bacteria, the transcriptional terminators used in the gene expression and plant seledable marker cassettes, and the methodologies used for cloning in gene or gene fragments of interest (typically, conventional restriction enzyme-mediated or Gateway™ recombinase-based cloning).

**References**


1. Abel et al., Science, 232:738 (1986).
2. Altschul et al., Nucleic Acids Res., 25:3389 (1997).
3. Altschul et al., J. Mol. Biol., 215:403 (1990).
4. An et al., EMBO J., 4:277 (1985).
5. Auch & Reth, Nucleic Acids Research, 18:6743 (1990).
6. Ausubel et al. (1987) Current Protocols in Molecular Biology, Greene Publishing Assoc. and Wiley Interscience
7. Ballas et al., Nucleic Acids Res., 17:7891 (1989).
8. Barkai-Golan et al., Arch. Microbiol., 116:119 (1978).
9. Batzer et al., Nucleic Acid Res., 19:5081 (1991).
10. Bäumlein et al. Mol Gen Genet 225:121-128 (1991)
11. Becker et al. (1994) Plant J., 5:299-307,
12. Bernal-Lugo and Leopold, Plant Physiol., 98:1207 (1992).
13. Bevan et al., Nature, 304:184 (1983).
14. Bevan et al., Nucl. Acids Res., 11:369 (1983).
15. Bevan, Nucl. Acids Res., 12:8711 (1984).
16. Blackman et al., Plant Physiol., 100:225 (1992).
17. Blochlinger & Diggelmann, Mol Cell Biol, 4:2929 (1984).
18. Bol et al., Ann. Rev. Phytopath., 28:113 (1990).
19. Bouchez et al., EMBO J., 8:4197 (1989).
20. Bourouis et al., EMBO J., 2:1099 (1983).
21. Bowler et al., Ann. Rev. Plant Physiol., 43:83 (1992).
22. Branson and Guss, Proc. North Central Branch Entomological Society of America (1972).
23. Broakgert et al., Science, 245:110 (1989).
24. Bustos MM et al. (1989) Plant Gell 1:839-853
25. Byme et al. Plant Cell Tissue and Organ Culture, 8:3 (1987).
26. Callis et al., Genes and Develop., 1:1183 (1987).
27. Campbell and Gowri, Plant Physiol., 92:1 (1990).
28. Campbell, W. C., ed. Ivermectin and Abamectin, Springer-Verlag, New York, 1989.
29. Chee et al. Plant Physiol., 91:1212 (1989).
30. Chen and Winans (1991) J. Bacteriol. 173: 1139-1144
31. Christou et al. Proc. Natl. Acad. Sci USA, 86:7500 (1989).
32. Christou et al., Biotechnology, 9:957 (1991).
33. Christou et al., Plant Physiol., 87:671 (1988).
34. Chui et al. (1996) Curr Biol 6:325-330
35. Coe et al., In: Corn and Corn Improvement, Sprague et al. (eds.) pp. 81-258 (1988).
36. Corpet et al. Nucleic Acids Res., 16:10881 (1988).
37. Coxson et al., Biotropica, 24:121 (1992).
38. Crameri et al., Nature Biotech., 15:436 (1997).
39. Crameri et al., Nature, 391:288 (1998).
40. Crossway et al., BioTechniques, 4:320 (1986).
41. Cuozzo et al., Bio/Technology, 6:549 (1988).
42. Cutler et al., J. Plant Physiol., 135:351 (1989).
43. Czapla and Lang, J. Econ. Entomol., 83:2480 (1990).

44. Datta et al., Bio/Technology, 8:736 (1990).
45. Davies et al., Plant Physiol., 93:588 (1990).
46. Dayhoff et al., Atlas of Protein Sequence and Structure, Natl. Biomed. Res. Found., Washington, C. D. (1978).
47. De Blaere et al., Meth. Enzymol., 143:277 (1987).
48. De Block et al. Plant Physiol., 91:694 (1989).
49. De Block et al., EMBO Journal, 6:2513 (1987).
50. Deblaere et al. Nucl Acids Res 13:4777-4788 (1985)
51. Della-Cioppa et al. Bio/Technology 5:579-584 (1987)
52. Della-Cioppa et al., Plant Physiology, 84:965-968 (1987).
53. Dellaporta et al., in Chromosome Structure and Function, Plenum Press, 263-282 (1988).
54. Depicker et al., Plant Cell Reports, 7:63 (1988).
55. Dunn et al., Can. J. Plant Sci., 61:583 (1981).
56. Dure et al., Plant Mol. Biol., 12:475 (1989).
57. Ebinuma et al. Proc Natl Acad Sci USA 94:2117-2121 (2000a)
58. Ebinuma et al. Selection of Marker-free transgenic plants using the oncogenes (*ipt, rol* A, B, C) of *Agrobacterium* as selectable markers, In Molecular Biology of Woody Plants. Kluwer Academic Publishers (2000b)
59. Eichholtz et al. Somatic Cell and Molecular Genetics 13, 67-76 (1987)
60. Ellis et al., EMBO Journal, 6:3203 (1987).
61. Elroy-Stein et al., Proc. Natl. Acad. Sci. U.S.A., 86:6126 (1989).
62. English et al., Plant Cell, 8:179 (1996).
63. Erdmann et al., J. Gen. Microbiol., 138:363 (1992).
64. Erikson et al. Nat Biotechnol. 22(4):455-8 (2004)
65. Everett et al., Bio/Technology, 5:1201(1987).
66. Fedoroff NV & Smith DL Plant J 3:273- 289 (1993)
67. Fire A et al Nature 391:806-811 (1998)
68. Fitzpatrick, Gen. Engineering News, 22:7 (1993).
69. Fraley et al. Proc Natl Acad Sci USA 80:4803 (1983)
70. Fromm et al., Bio/Technology, 8:833 (1990).
71. Fromm et al., Nature (London), 319:791 (1986).
72. Galbiati et al. Funct. Integr Genozides 2000, 20 1:25-34
73. Gallie et at. Nucl Acids Res 15:8693-8711 (1987)
74. Gallie et al., Nucleic Acids Res., 15:3257 (1987).
75. Gallie et al., The Plant Cell, 1:301 (1989).
76. Gan et al., Science. 270:1986 (1995).
77. Gatehouse et al., J. Sci. Food Agric., 35:373 (1984).
78. Gelfand, eds., PCR Strategies Academic Press, New York (1995).
79. Gelvin et al., Plant Molecular Biology Manual, (1990).
80. Gleave et al. Plant Mol Biol. 40(2):223-35 (1999)
81. Gordon-Kamm et al., Plant Cell, 2:603 (1990).
82. Goring et al, PNAS, 88:1770 (1991).
83. Gruber, et al., Vectors for Plant Transformation, in: Methods in Plant Molecular Biology & Biotechnology" in Glich et al., (Eds. pp. 89-119, CRC Press, 1993).
84. Guerineau et al., Mol. Gen. Genet., 262:141 (1991).
85. Guerrero et al., Plant Mol. Biol., 15:11 (1990).
86. Gupta et al., PNAS, 90:1629 (1993).
87. Haines and Higgins (eds.), Nucleic Acid Hybridization, IRL Press, Oxford, U.K.
88. Hajdukiewicz et al. Plant Mol Biol 25:989-994 (1994)
89. Hammock et al., Nature, 344:458 (1990).
90. Hansen et al. Proc. Natl. Acad. Sci. USA 91:7603-7607 (1994)
91. Hayford et al. Plant Physiol. 86:1216 (1988)
92. Hemenway et al., EMBO Journal, 7:1273 (1988).
93. Henikoff & Henikoff, Proc. Natl. Acad. Sci. USA, 89:10915 (1989).
94. Hiei et al. Plant J 6: 271-282 (1994)
95. Higgins et al., Gene, 73:237 (1988).
96. Higo et al. (1999) Nud Acids Res 27(1): 297-300
97. Hilder et al., Nature, 330:160 (1987).
98. Hille et al. Plant Mol. Biol. 7:171 (1986)
99. Hinchee et al. Bio/Technology 6:915 (1988).

100. Hoekema *et al.* (1983) Nature 303:179-181
101. Hoekema, In: The Binary Plant Vector System. Offset-drukkerij Kanters B.V.; Alblasserdam (1985).
102. Hood et al. J Bacteriol 168:1291-1301 (1986)
103. Huang et al., CABIOS, 8:155 (1992).
104. Ikeda et al., J. Bacteriol., 169:5612 (1987).
105. Ikuta et al., Biotech., 8:241 (1990).
106. Ingelbrecht et al., Plant Cell, 1:671 (1989).
107. Innis and Gelfand, eds., PCR Methods Manual (Academic Press, New York) (1999).
108. Innis et al., eds., PCR Protocols: A Guide to Methods and Applications (Academic Press, New York (1995).
109. Innis et al., PCR Protocols: A Guide to Methods and Applications, Academic Press, Inc., San Diego, Calif. (1990).
110. Ishida Y et al. Nature Biotech 745-750 (1996)
111. Jefferson et al. EMBO J 6:3901-3907 (1987)
112. Jefferson et al. Plant Mol Biol Rep 5:387-405 (1987)
113. Jenes B et al. Techniques for Gene Transfer, in: Recombinant Plants, Vol. 1, Engineering and Utilization, edited by SD Kung and R Wu, Academic Press, pp. 128-143 (1993)
114. Jobling et al., Nature, 325:622 (1987).
115. Johnson et al., PNAS USA, 86:9871 (1989)
116. Jones et al. Mol. Gen. Genet., 210:86 (1987)
117. Joshi et al., Nucleic Acid Res., 15:9627 (1987).
118. Kaasen et al., J. Bacteriol., 174:889 (1992).
119. Karlin and Altschul, Proc. Natl. Acad Sci. USA, 87:2264 (1990).
120. Karlin and Altschul, Proc. Natl. Acad. Sci. USA, 90:5873 (1993).
121. Karsten et al., Botanica Marina, 35:11 (1992).
122. Katz et al., J. Gen. Microbiol., 129:2703 (1983).
123. Keller et al., EMBO Journal, 8:1309 (1989).
124. Keller et al., Genes Dev., 3:1639 (1989). (Eine der beiden « Keller » Referenzen ist zuviel)
125. Klapwijk et al. J. Bacteriol., 141,128-136 (1980)
126. Klein et al., Bio/Technology, 6:559 (1988).
127. Klein et al., Plant Physiol., 91:440 (1988).
128. Klein et al., Proc. Natl. Acad. Sci. USA, 85:4305 (1988).
129. Knauf, et al., Genetic Analysis of Host Range Expression by Agrobacterium In: Molecular Genetics of the Bacteria-Plant Interaction, Puhler, A. ed., Springer-Verlag, New York, 1983.
130. Koncz & Schell Mol Gen Genet 204:383-396 (1986)
131. Koprek T et al. Plant J 19(6): 719-726 (1999)
132. Koster and Leopold, Plant Physiol., 88:829 (1988).
133. Koziel et al., Biotechnology, 11:194 (1993).
134. Kunkel et al., Methods in Enzymol., 154:367 (1987).
135. Kunkel, Proc. Natl. Acad. Sci. USA, 82:488 (1985).
136. Lam E und Chua NH, J Biol Chem; 266(26):17131-17135 (1991)
137. Laufs et al., PNAS, 87:7752 (1990).
138. Lawton et al., Mol. Cell Biol., 7:335 (1987).
139. Lee and Saier, J. Bacteriol., 153 (1982).
140. Leffel et al. Biotechniques 23(5):912-8 (1997)
141. Lescot et al. Nucleic Adds Res 30(1):325-7 (2002)
142. Levings. Science, 250:942 (1990).
143. U et al. Plant Mol Biol 20:1037-1048 (1992)
144. Lindsey et al., Transgenic Research, 2:3347 (1993).
145. Liu et al., Plant J. 8, 457-463 (1995)
146. Lommel et al., Virology, 181:382 (1991).
147. Loomis et al., J. Expt. Zool., 252:9 (1989).
148. Lorz et al., Mol. Gen. Genet., 199:178 (1985).
149. Ma et al., Nature, 334 :631 (1988).
150. Macejak et al., Nature, 353:90 (1991).
151. Maki et al., Methods in Plant Molecular Biology & Biotechnology, Glich et al., 67-88 CRC Press, (1993).
152. Maniatis T, Fritsch EF, and Sambrook J Molecular Cloning: A Laboratory Manual, Cold Spring Harbor Laboratory, Cold Spring Harbor (NY), (1989)
153. Mariani et al, Nature, 347:737 (1990).
154. Matzke et al. (2000) Plant Mol Biol 43:401-415;

155. McBride et al., PNAS USA, 91:7301 (1994).
156. McCabe et al., Bio/Technology, 6:923 (1988).
157. Meinkoth and Wahl, Anal. Biochem., 138:267 (1984).
158. Messing and Vierra, Gene, 19:259 (1982).
159. Michael et al., J. Mol. Biol., 26 :585 (1990). (im Text steht: Michael et al. 1994)
160. Millar et al. Plant Mol Biol Rep 10:324-414 (1992)
161. Mogen et al., Plant Cell, 2:1261 (1990).
162. Moore et al., J. Mol. Biol., 272:336 (1997).
163. Mozo & Hooykaas Plant Mol. Biol. 16:917-918 (1991)
164. Mundy and Chua, EMBO J., 7:2279 (1988).
165. Munroe et al., Gene, 91:151 (1990).
166. Murakami et al., Mol. Gen. Genet., 205:42 (1986).
167. Murata et al., FEBS Lett., 296:187 (1992).
168. Murdock et al., Phytochemistry, 29:85 (1990).
169. Murray et al., Nucleic Acids Res., 17:477 (1989).
170. Myers and Miller, CABIOS, 4:11 (1988).
171. Naested H Plant J 18:571-576 (1999)
172. Napoli et al., Plant Cell, 2:279 (1990).
173. Needleman and Wunsch, J. Mol. Biol., 48:443-453 (1970).
174. Nehra et al. Plant J. 5:285-297 (1994)
175. Niedz et al., Plant Cell Reports, 14:403 (1995).
176. Odell et al., Mol. Gen. Genet., 113:369 (1990).
177. Odell et al., Nature, 313:810 (1985).
178. Ohtsuka et al., J. Biol. Chem., 260:2605 (1985)
179. Olhoft et al. Plant Cell Rep 20: 706-711 (2001)
180. Ow et al., Science, 234:856 (1986).
181. Pacciotti et al., Bio/Technology, 3:241 (1985).
182. Park et al., J. Plant Biol., 38:365 (1985).
183. Paszkowski et al., EMBO J., 3:2717 (1984).
184. Pearson and Lipman, Proc. Natl. Acad. Sci., 85:2444 (1988).
185. Pearson et al., Meth. Mol. Biol., 24:307 (1994).
186. Perera RJ et al. Plant Mol. Biol 23(4): 793-799 (1993)
187. Perlak et al., Proc. Natl. Acad. Sci. USA, 88:3324 (1991).
188. Phillips et al., In Corn & Corn Improvement, 3rd Edition 10 Sprague et al. (Eds. pp. 345-387)(1988).
189. Phi-Van et al., Mol. Cell. Biol., 10:2302 (1990).
190. Piatkowski et al., Plant Physiol., 94:1682 (1990).
191. Potrykus et al., Mol. Gen. Genet., 199:183 (1985).
192. Potrykus, Trends Biotech., 7:269 (1989).
193. Prasher et al., Biochem. Biophys. Res. Comm., 126:1259 (1985).
194. Proudfoot, Cell, 64:671 (1991).
195. Reed et al., J. Gen. Microbiol., 130:1 (1984).
196. Riggs et al., Proc. Natl. Acad. Sci. USA, 83:5602 (1986).
197. Rossolini et al., Mol. Cell. Probes, 8:91 (1994).
198. Ruiz, Plant Cell, 10:937 (1998).
199. Sambrook et al., Molecular Cloning: A Laboratory Manual (2d ed., Cold Spring Harbor Laboratory Press, Plainview, N.Y.) (1989).
200. Sanfacon et al., Genes Dev., 5:141 (1991).
201. Sanford et al., Particulate Science and Technology, 5:27 (1987).
202. Scheeren-Groot et al. J. Bacteriol 176: 6418-6426 (1994)
203. Schenbom and Groskreutz Mol Biotechnol 13(1): 29-44 (1999)
204. Schtaman and Hooykaas Plant J 11:1377-1385 (1997)
205. Schoffl F et al. (1989) Mol Gen Genetics 217(2-3):246-53
206. Shagan et al., Plant Physiol., 101:1397 (1993).
207. Shah et al. Science 233: 478 (1986)
208. Shapiro, Mobile Genetic Elements, Academic Press, N.Y. (1983).
209. Shimamoto et al., Nature, 338:274 (1989).
210. Silhavy TJ, Berman ML, and Enquist LW Experiments with Gene Fusions, Cold Spring Harbor Laboratory Press, Cold Spring Harbor (NY), (1984)

211. Skuzeski et al., Plant Molec. Biol. 15: 65-79 (1990).
212. Smith et al., Adv. Appl. Math., 2:482.(1981).
213. Smith et al., Mol. Gen. Genet., 224:447 (1990).
214. Spencer et al., Theor. Appl. Genet, 79:625 (1990). Spencer 1992 Referenz fehlt
215. Stalker et al., Science, 242:419 (1988).
216. Staub et al., EMBO J., 12:601 (1993).
217. Staub et al., Plant Cell, 4:39 (1992).
218. Steifel et al., The Plant Cell, 2:785 (1990).
219. Stemmer, Nature, 370:389 (1994).
220. Stemmer, Proc. Natl. Acad. Sd. USA, 91:10747 (1994).
221. Stief et al., Nature, 341:343 (1989).
222. Stougaard Plant J 3:755-761 (1993)
223. Sukhapinda et al., Plant Mol. Biol., 8:209 (1987).
224. Sundaresan et al. Gene Develop 9: 1797-1810 (1995)
225. Sutcliffe, PNAS USA, 75:3737 (1978).
226. Svab et al., Plant Mol. Biol. 14:197 (1990)
227. Svab et al., Proc. Natl. Acad. Sci. USA, 87:8526 (1990).
228. Svab et al., Proc. Natl. Acad. Sci. USA, 90:913 (1993).
229. Tarczynski et al., PNAS USA, 89:2600 (1992).
230. Thillet et al., J. Biol. Chem., 263:12500 (1988).
231. Tijssen, Laboratory Techniques in Biochemistry and Molecular Biology-Hybridization with Nucleic Acid Probes, Elsevier, N.Y. (1993).
232. Tomes et al., Plant Cell, Tissue and Organ Culture: Fundamental Methods, Springer Verlag, Berlin (1995).
233. Tomic et al., NAR, 12:1656 (1990).
234. Turner et al., Molecular Biotechnology, 3:225 (1995).
235. Twell et al., Plant Physiol., 91:1270 (1989).
236. Ugaki et al., Nucl. Adds Res., 19:371 (1991).
237. Ulmasov et al., Plant Mol. Biol., 35:417 (1997).
238. Upender et al., Biotechniques, 18:29 (1995).
239. van der Krol et al., Plant Cell, 2291 (1990).
240. Vanden Elzen et al. Plant Mol Biol. 5:299 (1985)
241. Vasil et al. Bio/Technology, 10:667-674 (1992)
242. Vasil et al. Bio/Technology, 11:1153-1158 (1993)
243. Vasil et al., Mol. Microbiol., 3:371 (1989).
244. Vasil et al., Plant Physiol., 91:1575 (1989).
245. Vemon and Bohnert, EMBO J., 11:2077 (1992).
246. Walker and Gaastra, eds., Techniques in Molecular Biology, MacMillan Publishing Company, New York (1983).
247. Wan & Lemaux (1994) Plant Physiol., 104:3748
248. Wang et al., Mol. Cell. Biol., 12:3399 (1992).
249. Waterman, M. S. introduction to Computational Biology: Maps, sequences and genomes. Chapman & Hall. London (1995).
250. Watrud et al., in Engineered Organisms and the Environment (1985).
251. Watson et al. J. Bacteriol 123, 255-264 (1975)
252. Watson et al., Corn: Chemistry and Technology (1987).
253. Weeks et al. Plant Physiol 102:1077-1084 (1993)
254. Weissinger et al., Annual Rev. Genet., 22:421 (1988).
255. White et al, Nucl Acids Res, 18, 1062 (1990).
256. Wingender E et al. Nucleic Acids Res 29(1):281-3 (2001)
257. Wolter et al., EMBO Journal, 11:4685 (1992).
258. Wyn-Jones and Storey, Physiology and Biochemistry of Drought Resistance in Plants, Paleg et al. (eds.), pp. 171-204 (1981).
259. Yamaguchi-Shinozaki et al., Plant Cell Physiol., 33:217 (1992).
260. Zhang et al., Proc. Natl. Acad. Sci. USA, 94:4504 (1997).
261. Zukowsky et al., PNAS USA, 80:1101 (1983).

All publications, patents and patent applications are incorporated herein by reference. While in the foregoing specification this invention has been described in relation to certain preferred embodiments thereof, and many details have been set forth for purposes of illustration, it will be apparent to those skilled in the art that the invention is susceptible to additional embodiments and that certain of the details described herein may be varied considerably without departing from the basic principles of the invention.

## Claims

1. An expression cassette for seed-specific or seed-preferential transcription in plants comprising

i) at least one transcription regulating nucleotide sequence of a plant gene, said plant gene selected from the group of genes described by the GenBank *Arabidopsis thaliana* genome loci At4g12910, At1g66250, At4g00820, At2g36640, At2g34200, At3g11180, At4g00360, At2g48030, At2g38590, At1g23000, At3g15510, At3g50870, At4g00220, and At4g26320, or a functional equivalent thereof, and functionally linked thereto
ii) at least one nucleic acid sequence which is heterologous in relation to said transcription regulating nucleotide sequence.

2. The expression cassette of Claim 1, wherein the transcription regulating nucleotide sequence is selected from the group of sequences consisting of

i) the sequences described by SEQ ID NOs: 1, 2, 3, 4, 5, 6, 7, 8, 9, 10, 11, 12, 15, 16, 17, 18, 19, 20, 23, 24, 25, 26, 27, 28, 31, 32, 33, 36, 37, 38, 39, 40, 41, 42, 43, 44, 45, 46, 47, 50,51, 51, 52, 53, 54, 55, 56, 57, 60, 61, 62, 63, 64, 65, 66, 67, 68, 69, 70, 71, 74, 75, 76, 77, 80, 81, 84, 85, 86, 87, 148, 149. 150, 151, 152, 153, 154, 155, 158, 159, 160, 161, 162, 163, 164, 165, 168, 169, 170,171, 172,173,174,175,178,179,180,181, 182,183,184, and 185,
ii) a fragment of at least 50 consecutive bases of a sequence under i) which has substantially the same promoter activity as the corresponding transcription regulating nucleotide sequence described by SEQ ID NO: 1, 2, 3, 4, 5, 6, 7, 8, 9, 10, 11, 12, 15, 16, 17, 18, 19, 20, 23, 24, 25, 26, 27, 28, 31, 32, 33, 36, 37, 38, 39, 40, 41, 42, 43, 44, 45, 46, 47, 50, 51, 51, 52, 53, 54, 55, 56, 57, 60, 61, 62, 63, 64, 65, 66, 67, 68, 69, 70, 71, 74, 75, 76, 77, 80, 81, 84, 85, 86, 87, 148, 149, 150, 151, 152, 153, 154, 155, 158, 159, 160, 161, 162, 163, 164, 165, 168, 169, 170, 171, 172, 173, 174, 175, 178, 179, 180, 181, 182, 183, 184, or 185;
iii) a nucleotide sequence having substantial similarity with a sequence identity of at least 40% to a transcription regulating nucleotide sequence described by SEQ ID NO: 1, 2, 3, 4, 5, 6, 7, 8, 9, 10, 11, 12, 15, 16, 17, 18, 19, 20, 23, 24, 25, 26, 27, 28, 31, 32, 33, 36, 37, 38, 39, 40, 41, 42, 43, 44, 45, 46, 47, 50,51, 51, 52, 53, 54, 55, 56, 57, 60, 61, 62, 63, 64, 65, 66, 67, 68, 69, 70, 71, 74, 75, 76, 77, 80, 81, 84, 85, 86, 87, 148, 149, 150, 151, 152, 153, 154, 155, 158, 159, 160, 161, 162, 163, 164, 165, 168, 169, 170, 171, 172, 173, 174, 175, 178, 179, 180, 181, 182, 183, 184, or 185 ;
iv) a nucleotide sequence capable of hybridizing under conditions equivalent to hybridization in 7% sodium dodecyl sulfate (SDS), 0.5 M $NaPO_4$, 1 mM EDTA at 50°C with washing in 2 X SSC, 0. 1% SDS at 50°C to a transcription regulating nucleotide sequence described by SEQ ID NO: 1, 2, 3, 4, 5, 6, 7, 8, 9, 10, 11, 12, 15, 16, 17, 18, 19, 20, 23, 24, 25, 26, 27, 28, 31, 32, 33, 36, 37, 38, 39, 40, 41, 42, 43, 44, 45, 46, 47, 50,51, 51, 52, 53, 54, 55, 56, 57, 60, 61, 62, 63, 64, 65, 66, 67, 68, 69, 70, 71, 74, 75, 76, 77, 80, 81, 84, 85, 86, 87, 148, 149, 150, 151, 152, 153, 154, 155, 158, 159, 160, 161, 162, 163, 164, 165, 168, 169, 170, 171, 172, 173, 174, 175, 178, 179, 180, 181, 182, 183, 184, or 185 , or the complement thereof;
v) a nucleotide sequence capable of hybridizing under conditions equivalent to hybridization in 7% sodium dodecyl sulfate (SDS), 0.5 M $NaPO_4$, 1 mM EDTA at 50°C with washing in 2 X SSC, 0. 1% SDS at 50°C to a nucleic acid comprising 50 to 200 or more consecutive nucleotides of a transcription regulating nucleotide sequence described by SEQ ID NO: 1, 2, 3, 4, 5, 6, 7, 8, 9, 10, 11, 12, 15, 16, 17, 18, 19, 20, 23, 24, 25, 26, 27, 28, 31, 32, 33, 36, 37, 38, 39, 40, 41, 42, 43, 44, 45, 46, 47, 50,51, 51, 52, 53, 54, 55, 56, 57, 60, 61, 62, 63, 64, 65, 66, 67, 68, 69, 70, 71, 74, 75, 76, 77, 80, 81, 84, 85, 86, 87, 148, 149, 150, 151, 152, 153, 154, 155, 158, 159, 160, 161, 162, 163, 164, 165, 168, 169, 170, 171, 172, 173, 174, 175, 178, 179, 180, 181, 182, 183, 184, or 185 , or the complement thereof;
vi) a nucleotide sequence which is the complement or reverse complement of any of the previously mentioned nucleotide sequences under i) to v).

3. The expression cassette of Claim 1 or 2, wherein the functional equivalent of the transcription regulating nucleotide sequence is obtained or obtainable from plant genomic DNA from a gene encoding a polypeptide which has at least 70% amino acid sequence identity to a polypeptide selected from the group described by SEQ ID NO: 14, 22, 30, 35, 49, 59, 73, 79, 83, 89, 157, 167, 177, and 187, respectively.

4. The expression cassette of any of Claim 1 to 3, wherein expression of the nucleic acid sequence results in expression of a protein, or expression of a antisense RNA, sense or double-stranded RNA.

5. The expression cassette of any of Claim 1 to 4, wherein expression of the nucleic acid sequence confers to the plant an agronomically valuable trait.

**6.** A vector comprising an expression cassette of any of Claim 1 to 5.

**7.** A transgenic host cell or non-human organism comprising an expression cassette of any of Claim 1 to 5, or a vector of Claim 6.

**8.** A transgenic plant comprising the expression cassette of any of Claim 1 to 5, a vector of Claim 6, or a cell of claim 7.

**9.** A method for identifying and/or isolating a sequence with seed-specific or seed-preferential transcription regulating activity **characterized** that said identification and/or isolation utilizes a nucleic acid sequence encoding a amino acid sequence as described by SEQ ID NO: 14, 22, 30, 35, 49, 59, 73, 79, 83, 89, 157, 167, 177, or 187 or a part of at least 15 bases thereof.

**10.** The method of Claim 9, wherein the nucleic acid sequences is described by SEQ ID NO: 13, 21, 29, 34, 48, 58, 72, 78, 82, 88, 156, 166, 176, or 186, or a part of at least 15 bases thereof.

**11.** The method of Claim 9 or 10, wherein said identification and/or isolation is realized by a method selected from polymerase chain reaction, hybridization, and database screening.

**12.** A method for providing a transgenic expression cassette for seed-specific or seed-preferential transcription in plants comprising the steps of:

I. isolating of a seed-specific or seed-preferential transcription regulating nucleotide sequence utilizing at least one nucleic acid sequence or a part thereof, wherein said sequence is encoding a polypeptide described by SEQ ID NO: 14, 22, 30, 35, 49, 59, 73, 79, 83, 89, 157, 167, 177, or 187, or a part of at least 15 bases thereof, and
II. functionally linking said seed-specific or seed-preferential transcription regulating nucleotide sequence to another nucleotide sequence of interest, which is heterolog in relation to said seed-specific or seed-preferential transcription regulating nucleotide sequence.

SEQUENCE LISTING

<110> BASF Plant Science GmbH
<120> Expression cassettes for seed-preferential expression in plants
<130> PF56024 AT (PF56118-2 PF56118 PF56024 / AE20040854 AE20041012)
<160> 211
<170> PatentIn version 3.3

<210> 1
<211> 980
<212> DNA
<213> Arabidopsis thaliana

<220>
<221> promoter
<222> (1)..(980)
<223> transcription regulating sequence from gene At4g12910

<400> 1
```
ttttatctgt ataataaaac cggtagtttc ttgtacccaa ccaaattctt acttcaaaat     60
tttgaaaaca taatacaacg aactttattt atttattacc tttttcaaat tcttcccttc    120
caaaactttc tgattttatt attctttaca tatactctta taatgttata acaattccag    180
agaaaacaat atgaaaagaa gaaaaactaa ctaaacaaaa tagtagtgtg gacctggatc    240
tattcttcta ttaaaatttg ctttccacta gattttagat atataggttt aaaagctaac    300
catagctttt aacctcataa atttcaaaat gctttccaaa atacggatta tactattccc    360
aataattgaa taatcagttt attttctaca taatttaaca atcggaaagc taattcgtag    420
aagttatcgt tcaaaataaa accaaaactt tttaatcagt atgtaggaat cttttccaaa    480
caaaacgcct aaaacttaac aaaaagaatt cacaaacacc agccacgtgt caccttttgt    540
taagccagtc gtcactttaa gtaaccgctt gtcactttaa gaagcggtcc caagtcaact    600
cgcgccactc aaatcaatca tgcttatgta aataacaact aaacgcagac gcaatcacca    660
ttttctttat agaaatgctc aaaatcgtgc cttttaagga aactgttgct ttgcagaatc    720
acaaaatcta tataccaacc ataagtttcc aaattttgtt ctaaaaatca aaaaaattcg    780
tatcatcttt ttgtcttttt ttaccagaat ttcatcctta tttcgtgcta aatcattttc    840
caaacattca taatactttt tgttggttta tggccaacta acaaaatatc ctctagattt    900
tcttctttgt gttagtctat ataaagcaat accacagtga tcctcttttt taattcattc    960
tgtgtttatg tcaataatca                                                980
```

<210> 2
<211> 994
<212> DNA
<213> Arabidopsis thaliana

<220>
<221> promoter
<222> (1)..(994)

<223> transcription regulating sequence from gene At4g12910

<400> 2
```
aacttgttat ctttttatct gtataataaa accggtagtt tcttgtacca aaccaaattc     60
ttagttcaaa attttgaaaa cataatacaa cgaactttat ttatttatta ccttttttcaa   120
attcttccct tccaaaactt tctgatttta ttattcttta catatactct tataatgtta    180
taacaattcc agagaaaaca atatgaaaag aagaaaaact aactaaacaa aatagtagtg    240
tggacctgga tctattcttc tattaaaatt tgctttccac tagattttag atatataggt    300
ttaaaagcta accatagctt ttaacctcat aaatttcaaa atgctttcca aaatacggat    360
tatactattc ccaataattg aataatcagt ttattttcta cgtaatttaa caatgggaaa    420
gctaattcgt agaagttatc gttcaaaata aaaccaaaac tttttaatca ttatgtagga    480
atcttttcca aacaaaacgc ctaaaactta acaaaaagaa ttcacaaaaa ccagccacgt    540
gtcacctttt gttaagccag tcgtcacttt aagtaaccgc ttgtcacttt aagaagcggt    600
cccaagtcaa ctcgcgccac tcaaatcaat catgcttatg taaataacaa ctaaacgcag    660
acgcaatcac cattttcttt atagaaatgc tcaaaatcgt gccttttaag gaaactgttg    720
ctttgcagaa tcacaaaatc tatataccaa ccataagttt ccaaattttg ttctaaaaat    780
caaaaaaatt catatcatct ttttgtcttt ttttaccaga atttcatcct tatttcgtgc    840
taaatcattt tccaaacatt cataatactt tttgttggtt tatggccaac taacaaaata    900
tcctctagat tttcttcttt gtgttagtct atataaagca ataccacagt gatcctcttt    960
tttaattcat tctgtgttta tgtcaataat caca                                994
```

<210> 3
<211> 686
<212> DNA
<213> Arabidopsis thaliana

<220>
<221> promoter
<222> (1)..(686)
<223> transcription regulating sequence from gene At4g12910

<400> 3
```
ttttatctgt ataataaaac cggtagtttc ttgtacccaa ccaaattctt acttcaaaat     60
tttgaaaaca taatacaacg aactttattt atttattacc tttttcaaat tcttcccttc    120
caaaactttc tgattttatt attctttaca tatactctta taatgttata acaattccag    180
agaaaacaat atgaaaagaa gaaaaactaa ctaaacaaaa tagtagtgtg gacctggatc    240
tattcttcta ttaaaatttg ctttccacta gattttagat atataggttt aaaagctaac    300
catagctttt aacctcataa atttcaaaat gctttccaaa atacggatta tactattccc    360
aataattgaa taatcagttt attttctaca taatttaaca atcggaaagc taattcgtag    420
aagttatcgt tcaaaataaa accaaaactt tttaatcagt atgtaggaat cttttccaaa    480
caaaacgcct aaaacttaac aaaaagaatt cacaaacacc agccacgtgt caccttttgt    540
taagccagtc gtcactttaa gtaaccgctt gtcactttaa gaagcggtcc caagtcaact    600
cgcgccactc aaatcaatca tgcttatgta aataacaact aaacgcagac gcaatcacca    660
ttttctttat agaaatgctc aaaatc                                          686
```

<210> 4
<211> 698
<212> DNA
<213> Arabidopsis thaliana

<220>
<221> promoter
<222> (1)..(698)
<223> transcription regulating sequence from gene At4g12910

<400> 4
aacttgttat ctttttatct gtataataaa accggtagtt tcttgtacca aaccaaattc 60
ttagttcaaa attttgaaaa cataatacaa cgaactttat ttatttatta ccttttttcaa 120
attcttccct tccaaaactt tctgatttta ttattcttta catatactct tataatgtta 180
taacaattcc agagaaaaca atatgaaaag aagaaaaact aactaaacaa aatagtagtg 240
tggacctgga tctattcttc tattaaaatt tgctttccac tagattttag atatataggt 300
ttaaaagcta accatagctt ttaacctcat aaatttcaaa atgctttcca aaatacggat 360
tatactattc ccaataattg aataatcagt ttattttcta cgtaatttaa caatgggaaa 420
gctaattcgt agaagttatc gttcaaaata aaaccaaaac tttttaatca ttatgtagga 480
atcttttcca aacaaaacgc ctaaaactta acaaaaagaa ttcacaaaaa ccagccacgt 540
gtcacctttt gttaagccag tcgtcacttt aagtaaccgc ttgtcacttt aagaagcggt 600
cccaagtcaa ctcgcgccac tcaaatcaat catgcttatg taaataacaa ctaaacgcag 660
acgcaatcac cattttcttt atagaaatgc tcaaaatc 698

<210> 5
<211> 2051
<212> DNA
<213> Arabidopsis thaliana

<220>
<221> promoter
<222> (1)..(2051)
<223> transcription regulating sequence from gene At4g12910

<400> 5
ctcccttttt atgtctagaa ttttcaagta tatctaacat ctcaaatgtg cattacatgc 60
aaaaacttgc atgtctttgt tactaactta ccataagaca cataaatgaa aagaaagaag 120
gtcatatgat aaatcatatc cacaaaacaa ataataagaa aatattcact gtaaaattag 180
taacttaata agaggttagg tagcaaacat ataaactaca acacaagtaa ttgttttagc 240
aattatcaag taatgaccat taataaataa aataaactaa tgaaagaaga agagtagaat 300
gaaagtaata cttgatacat tagcatgatt ttgtattttt tagtattttt aacaaatgaa 360
ttaagagtaa gattaatgga tgacttaatt cacaatagga gaattttggt ttttcttcaa 420
ttaatgtagc taatcttttt atttaaataa tataaaagga caaatgtcat tttttcatgt 480
gataatggta tatctatatg gagaaaattg accaactttc atatatatga ttatataagt 540
aataatatgt aaagaagaaa agaaacgaaa gagtgaaaga gtgagcaaga gaaacttgta 600

taattaactt gtatatatgc gttaaaaaaa tgagaaccga ctcttttact aaatcattta 660
ttaagcagag atttagctga cattatactt ctccatctat atgatagaaa agtataattt 720
tattcaatcc agatgattgg attttcaaaa atatattttg aaagttttcg caaaaagtaa 780
gtcgaaaacg ttatcaacca attattttgc tagtaggacc aaattaaaat gattcacatt 840
ccgaaatcct attccacttt tcgatttact attgcataat ttcttctaaa ctatgtagtt 900
tatacccttc gatttaccat ttttactcca ctaaaattta gtaaccgcat ttaaaagttt 960
agaagtaatt atttttagta ctttgaagta agaaatcaca taaattgata atggcgcagc 1020
tatacacttt attagcactt ctttttacc tttcaaaaaa acttgttatc tttttatctg 1080
tataataaaa ccggtagttt cttgtaccca accaaattct tacttcaaaa ttttgaaaac 1140
ataatacaac gaactttatt tatttattac ctttttcaaa ttcttccctt ccaaaacttt 1200
ctgattttat tattctttac atatactctt ataatgttat aacaattcca gagaaaacaa 1260
tatgaaaaga agaaaaacta actaaacaaa atagtagtgt ggacctggat ctattcttct 1320
attaaaattt gctttccact agatttaga tatataggtt taaaagctaa ccatagcttt 1380
taacctcata aatttcaaaa tgctttccaa aatacggatt atactattcc caataattga 1440
ataatcagtt tattttctac ataatttaac aatcggaaag ctaattcgta gaagttatcg 1500
ttcaaaataa aaccaaaact ttttaatcag tatgtaggaa tcttttccaa acaaaacgcc 1560
taaaacttaa caaaaagaat tcacaaacac cagccacgtg tcaccttttg ttaagccagt 1620
cgtcacttta agtaaccgct tgtcacttta agaagcggtc ccaagtcaac tcgcgccact 1680
caaatcaatc atgcttatgt aaataacaac taaacgcaga cgcaatcacc attttcttta 1740
tagaaatgct caaaatcgtg ccttttaagg aaactgttgc tttgcagaat cacaaaatct 1800
atataccaac cataagtttc caaattttgt tctaaaaatc aaaaaaattc gtatcatctt 1860
tttgtctttt tttaccagaa tttcatcctt atttcgtgct aaatcatttt ccaaacattc 1920
ataatacttt ttgttggttt atggccaact aacaaaatat cctctagatt ttcttctttg 1980
tgttagtcta tataaagcaa taccacagtg atcctctttt ttaattcatt ctgtgtttat 2040
gtcaataatc a 2051

<210> 6
<211> 2066
<212> DNA
<213> Arabidopsis thaliana

<220>
<221> promoter
<222> (1)..(2066)
<223> transcription regulating sequence from gene At4g12910

<400> 6
tactttagat ctctcccttt ttatgtctag aattttcaag tatatctaac atctcaaatg 60
tgcattacgt gcaaaaactt gcatgtcttt gttactaact taccataaga cacataaatg 120
aaaagagaaa aggtcatatg ataaatcata tccacaaaac aaataataag aaaatattca 180
ctgtaaaatt agtaacttaa taagaggtta ggtagcaaac atctaaacta caacacaagt 240
aattgtttta gcaattatca agtaatgatc attaataaat aaaaaaaact aatgaaagaa 300
gaagagtaga atgaaagtaa tacttgatac attagcatga ttttgtattt ttttagtatt 360
tttaacaaat gaattaagag taagattaat ggatgactta attcacaata ggagaatttt 420
ggtttttctt caattaatgt agctaatctt tttatttaaa taatataaaa cgacaaatgt 480

```
cattttctca tgtgataatg gtacacttat atggagaaaa tttaccaact ttcatatata    540
tgattatata agtaataata tgtaaagaag aaaagaaacg aaagagtgaa agagtgagca    600
agagaaactt gtataattaa cttgtatata tgcgttaaaa aaatgagaac cgactctttt    660
actaaatcat ttattaagca gagatttagc tgacattata cttctccatc tatatgatag    720
aaaagtataa ttttattcaa tccagatgat tggattttca aaaatatatt ttgaaagttt    780
tcgcaaaaag taagtcgaaa acgttatcaa ccaattattt tgctagtagg accaaattaa    840
aatgattcac attccgaaat cctattccac ttttcgattt actattgcat aatttcttct    900
aaactatgta gtttataccc ttcgatttac catttttact ccactaaaat ttagtaaccg    960
catttaaaag tttagaagta attattttta gtactttgaa gtaagaaatc acataaattg   1020
ataatggcgc agctatacac tttattagca cttcttttttt acctttcaaa aaaacttgtt  1080
atctttttat ctgtataata aaaccggtag tttcttgtac caaaccaaat tcttagttca   1140
aaattttgaa aacataatac aacgaacttt atttatttat taccttttttc aaattcttcc  1200
cttccaaaac tttctgattt tattattctt tacatatact cttataatgt tataacaatt   1260
ccagagaaaa caatatgaaa agaagaaaaa ctaactaaac aaaatagtag tgtggacctg   1320
gatctattct tctattaaaa tttgctttcc actagatttt agatatatag gtttaaaagc   1380
taaccatagc ttttaacctc ataaatttca aaatgctttc caaaatacgg attatactat   1440
tcccaataat tgaataatca gtttattttc tacgtaattt aacaatggga aagctaattc   1500
gtagaagtta tcgttcaaaa taaaaccaaa actttttaat cattatgtag gaatcttttc   1560
caaacaaaac gcctaaaact taacaaaaag aattcacaaa aaccagccac gtgtcacctt   1620
ttgttaagcc agtcgtcact ttaagtaacc gcttgtcact ttaagaagcg gtcccaagtc   1680
aactcgcgcc actcaaatca atcatgctta tgtaaataac aactaaacgc agacgcaatc   1740
accattttct ttatagaaat gctcaaaatc gtgcctttta aggaaactgt tgctttgcag   1800
aatcacaaaa tctatatacc aaccataagt ttccaaattt tgttctaaaa atcaaaaaaa   1860
ttcatatcat ctttttgtct ttttttacca gaatttcatc cttatttcgt gctaaatcat   1920
tttccaaaca ttcataatac tttttgttgg tttatggcca actaacaaaa tatcctctag   1980
attttcttct ttgtgttagt ctatataaag caataccaca gtgatcctct tttttaattc   2040
attctgtgtt tatgtcaata atcaca                                         2066
```

```
<210>  7
<211>  1757
<212>  DNA
<213>  Arabidopsis thaliana

<220>
<221>  promoter
<222>  (1)..(1757)
<223>  transcription regulating sequence from gene At4g12910

<400>  7
ctccctttttt atgtctagaa ttttcaagta tatctaacat ctcaaatgtg cattacatgc    60
aaaaacttgc atgtctttgt tactaactta ccataagaca cataaatgaa aagaaagaag   120
gtcatatgat aaatcatatc cacaaaacaa ataataagaa aatattcact gtaaaattag   180
taacttaata agaggttagg tagcaaacat ataaactaca acacaagtaa ttgttttagc   240
aattatcaag taatgaccat taataaataa aataaactaa tgaaagaaga agagtagaat   300
gaaagtaata cttgatacat tagcatgatt ttgtattttt tagtattttt aacaaatgaa   360
```

ttaagagtaa gattaatgga tgacttaatt cacaatagga gaattttggt ttttcttcaa 420
ttaatgtagc taatcttttt atttaaataa tataaaagga caaatgtcat tttttcatgt 480
gataatggta tatctatatg gagaaaattg accaactttc atatatatga ttatataagt 540
aataatatgt aaagaagaaa agaaacgaaa gagtgaaaga gtgagcaaga gaaacttgta 600
taattaactt gtatatatgc gttaaaaaaa tgagaaccga ctcttttact aaatcattta 660
ttaagcagag atttagctga cattatactt ctccatctat atgatagaaa agtataattt 720
tattcaatcc agatgattgg attttcaaaa atatattttg aaagttttcg caaaaagtaa 780
gtcgaaaacg ttatcaacca attattttgc tagtaggacc aaattaaaat gattcacatt 840
ccgaaatcct attccacttt tcgatttact attgcataat ttcttctaaa ctatgtagtt 900
tatacccttc gatttaccat ttttactcca ctaaaattta gtaaccgcat ttaaaagttt 960
agaagtaatt atttttagta ctttgaagta agaaatcaca taaattgata atggcgcagc 1020
tatacacttt attagcactt ctttttaccc tttcaaaaaa acttgttatc tttttatctg 1080
tataataaaa ccggtagttt cttgtaccca accaaattct tacttcaaaa ttttgaaaac 1140
ataatacaac gaactttatt tatttattac ctttttcaaa ttcttccctt ccaaaacttt 1200
ctgattttat tattctttac atatactctt ataatgttat aacaattcca gagaaaacaa 1260
tatgaaaaga agaaaaacta actaaacaaa atagtagtgt ggacctggat ctattcttct 1320
attaaaattt gctttccact agattttaga tatataggtt taaaagctaa ccatagcttt 1380
taacctcata aatttcaaaa tgctttccaa aatacggatt atactattcc caataattga 1440
ataatcagtt tattttctac ataatttaac aatcggaaag ctaattcgta gaagttatcg 1500
ttcaaaataa aaccaaaact ttttaatcag tatgtaggaa tcttttccaa acaaaacgcc 1560
taaaacttaa caaaaagaat tcacaaacac cagccacgtg tcaccttttg ttaagccagt 1620
cgtcacttta agtaaccgct tgtcacttta agaagcggtc ccaagtcaac tcgcgccact 1680
caaatcaatc atgcttatgt aaataacaac taaacgcaga cgcaatcacc atttctttta 1740
tagaaatgct caaaatc 1757

<210> 8
<211> 1770
<212> DNA
<213> Arabidopsis thaliana

<220>
<221> promoter
<222> (1)..(1770)
<223> transcription regulating sequence from gene At4g12910

<400> 8
tactttagat ctctcccttt ttatgtctag aattttcaag tatatctaac atctcaaatg 60
tgcattacgt gcaaaaactt gcatgtcttt gttactaact taccataaga cacataaatg 120
aaaagagaaa aggtcatatg ataaatcata tccacaaaac aaataataag aaaatattca 180
ctgtaaaatt agtaacttaa taagaggtta ggtagcaaac atctaaacta caacacaagt 240
aattgtttta gcaattatca agtaatgatc attaataaat aaaaaaaact aatgaaagaa 300
gaagagtaga atgaaagtaa tacttgatac attagcatga ttttgtattt ttttagtatt 360
tttaacaaat gaattaagag taagattaat ggatgactta attcacaata ggagaatttt 420
ggtttttctt caattaatgt agctaatctt tttatttaaa taatataaaa cgacaaatgt 480
cattttctca tgtgataatg gtacacttat atggagaaaa tttaccaact ttcatatata 540

tgattatata agtaataata tgtaaagaag aaaagaaacg aaagagtgaa agagtgagca 600
agagaaactt gtataattaa cttgtatata tgcgttaaaa aaatgagaac cgactctttt 660
actaaatcat ttattaagca gagatttagc tgacattata cttctccatc tatatgatag 720
aaaagtataa ttttattcaa tccagatgat tggattttca aaaatatatt ttgaaagttt 780
tcgcaaaaag taagtcgaaa acgttatcaa ccaattattt tgctagtagg accaaattaa 840
aatgattcac attccgaaat cctattccac ttttcgattt actattgcat aatttcttct 900
aaactatgta gtttataccc ttcgatttac catttttact ccactaaaat ttagtaaccg 960
catttaaaag tttagaagta attattttta gtactttgaa gtaagaaatc acataaattg 1020
ataatggcgc agctatacac tttattagca cttctttttt acctttcaaa aaaacttgtt 1080
atctttttat ctgtataata aaaccggtag tttcttgtac caaaccaaat tcttagttca 1140
aaattttgaa aacataatac aacgaacttt atttatttat taccttttc aaattcttcc 1200
cttccaaaac tttctgattt tattattctt tacatatact cttataatgt tataacaatt 1260
ccagagaaaa caatatgaaa agaagaaaaa ctaactaaac aaaatagtag tgtggacctg 1320
gatctattct tctattaaaa tttgctttcc actagatttt agatatatag gtttaaaagc 1380
taaccatagc ttttaacctc ataaatttca aaatgctttc caaaatacgg attatactat 1440
tcccaataat tgaataatca gtttattttc tacgtaattt aacaatggga aagctaattc 1500
gtagaagtta tcgttcaaaa taaaaccaaa actttttaat cattatgtag gaatcttttc 1560
caaacaaaac gcctaaaact taacaaaaag aattcacaaa aaccagccac gtgtcacctt 1620
ttgttaagcc agtcgtcact ttaagtaacc gcttgtcact ttaagaagcg gtcccaagtc 1680
aactcgcgcc actcaaatca atcatgctta tgtaaataac aactaaacgc agacgcaatc 1740
accattttct ttatagaaat gctcaaaatc 1770

<210> 9
<211> 3954
<212> DNA
<213> Arabidopsis thaliana

<220>
<221> promoter
<222> (1)..(3954)
<223> transcription regulating sequence from gene At4g12910

<400> 9
actagtcatc tatttcttga gaatatccct aagcttgacg tcttaatcat ctgaatttat 60
acacatacaa tttgttgctt gcttgtttgc tttctcttta tttattttta cttattgatt 120
tagtttagtc tcgatactat aaccttctgt gtgaacaaaa aagttttgtg gaaatcgatc 180
cttaagtatt acaactggcc tcttatttga gagagtagtc taggttaatt tgatcctata 240
ttagttatcg tccatatgtg taccacacca tcacacaaga aaatgacatc attaccaagg 300
attaaattgc gagaggttct agattctctc atccgacaaa ggcaaaaaga gaacttcgag 360
ccaaactccc cagaatggag tactttccaa ccctagccct aatatgcccg ttctagtaaa 420
atccgaaccg ttgctatact cgttgcatcc aaaatacaaa catgcaacat tttgaactca 480
ctatcaagtg tatataatgt aagcatttct tgcacaagag ttcctttcat gttgttctcg 540
tctacataat gttgtcggta ggttattttc ttatcaaatg atcaatgtga gatacatatt 600
tgattttgga ttaggatttc cacacatcgc attgtagttt gttattgaag ctaagagcat 660
ttgaataaac cttatggtca taatagtagg agcaagggaa gcattgagtg cagtgaggtt 720

```
gctccccata ttcatgtgat gagtttatcg cttttgagga gatttaaaat gaatttcagc    780
cataaatgct atatctcggt gggagtggat cgagtgacga gagaggtgga aaacaacttt    840
gttgtcgtaa aaactttcca ctgtgcatat atggatatat agttaaacaa cttttttttt    900
tattcggcca ttatggaata atgactattg ataaaaaaat aaaaataata agtagagtag    960
aatgaaagta gtgtttgata cattattatg attttttttt cattaaatgt ttttagaaaa   1020
tcatttaaga gtaagattaa tagagaattt aacataacaa aagacttttg atttttcctc   1080
aattagtttt gtttatcatt ttatttaatt aatgtagcat gacacatgcc attttcttgt   1140
gtgatgatat gttacttata tggagagagt tgaccaattt ttatatatat gattaattaa   1200
aagacggagg aaagaaaaac aagaaaagtc tatgtgattt tcatttatat tttttttttt   1260
actgattaat taaggatgtg atttgtagta aaatgtgatg attttaaagt agtgaacgaa   1320
agatttgtgg aagtaagagg taagttggtt taatggttaa agagtgaggg aagaaagaaa   1380
aaatgagtta cttagtagaa attttgttgg tagtgtatgt tgggatataa tttggtttga   1440
tgtggggata tgaaagtatg taataaaaat atatgtaaat catctaatat catatgaata   1500
agggtatttt gaaaattgct aacaaaacaa atatttatgg ttattattgt tatttatgtt   1560
catttttgtg ttattgtgaa aataataaac tcaaatctat ctcatatagc tgcaaatttt   1620
atgattagat tcatctttga ttcgaaatat caattatacg ttttatgtac aaatctaaat   1680
ttttatacat ctaaaatcct aaatttttac tatcatgaat acatagaaca acattataag   1740
atatattgat acaatagtat ataattatgt tacttatatg gtttgaaact tgcataattt   1800
gattctgaac aaacattttt ttgttccacg tactcttttc tcatctacta ctctaaccat   1860
atcctgatat gctattaatt taagtcaata ctttttttaga tctctcccta tttatgtcta   1920
gaattttcaa gtatatctaa catctcaaat gtgcattaca tgcaaaaact tgcatgtctt   1980
tgttactaac ttaccataag acacataaat gaaaagaaag aaggtcatat gataaatcat   2040
atccacaaaa caaataataa gaaaatattc actgtaaaat tagtaactta ataagaggtt   2100
aggtagcaaa catataaact acaacacaag taattgtttt agcaattatc aagtaatgac   2160
cattaataaa taaaataaac taatgaaaga agaagagtag aatgaaagta atacttgata   2220
cattagcatg attttgtatt ttttagtatt tttaacaaat gaattaagag taagattaat   2280
ggatgactta attcacaata ggagaatttt ggtttttctt caattaatgt agctaatctt   2340
tttatttaaa taatataaaa ggacaaatgt catttttttca tgtgataatg gtatatctat   2400
atggagaaaa ttgaccaact ttcatatata tgattatata agtaataata tgtaaagaag   2460
aaaagaaacg aaagagtgaa agagtgagca agagaaactt gtataattaa cttgtatata   2520
tgcgttaaaa aaatgagaac cgactctttt actaaatcat ttattaagca gagatttagc   2580
tgacattata cttctccatc tatatgatag aaaagtataa ttttattcaa tccagatgat   2640
tggattttca aaaatatatt ttgaaagttt tcgcaaaaag taagtcgaaa acgttatcaa   2700
ccaattattt tgctagtagg accaaattaa aatgattcac attccgaaat cctattccac   2760
ttttcgattt actattgcat aatttcttct aaactatgta gtttataccc ttcgatttac   2820
catttttact ccactaaaat ttagtaaccg catttaaaag tttagaagta attattttta   2880
gtactttgaa gtaagaaatc acataaattg ataatggcgc agctatacac tttattagca   2940
cttctttttt acctttcaaa aaaacttgtt atctttttat ctgtataata aaaccggtag   3000
tttcttgtac ccaaccaaat tcttacttca aaattttgaa aacataatac aacgaacttt   3060
atttatttat taccttttc aaattcttcc cttccaaaac tttctgattt tattattctt   3120
tacatatact cttataatgt tataacaatt ccagagaaaa caatatgaaa agaagaaaaa   3180
ctaactaaac aaaatagtag tgtggacctg gatctattct tctattaaaa tttgctttcc   3240
actagatttt agatatatag gtttaaaagc taaccatagc ttttaacctc ataaatttca   3300
aaatgctttc caaaatacgg attatactat tcccaataat tgaataatca gtttattttc   3360
tacataattt aacaatcgga aagctaattc gtagaagtta tcgttcaaaa taaaaccaaa   3420
```

acttttaat cagtatgtag gaatcttttc caaacaaaac gcctaaaact taacaaaaag 3480
aattcacaaa caccagccac gtgtcacctt ttgttaagcc agtcgtcact ttaagtaacc 3540
gcttgtcact ttaagaagcg gtcccaagtc aactcgcgcc actcaaatca atcatgctta 3600
tgtaaataac aactaaacgc agacgcaatc accattttct ttatagaaat gctcaaaatc 3660
gtgcctttta aggaaactgt tgctttgcag aatcacaaaa tctatatacc aaccataagt 3720
ttccaaattt tgttctaaaa atcaaaaaaa ttcgtatcat ctttttgtct ttttttacca 3780
gaatttcatc cttatttcgt gctaaatcat tttccaaaca ttcataatac tttttgttgg 3840
tttatggcca actaacaaaa tatcctctag attttcttct ttgtgttagt ctatataaag 3900
caataccata gtgatcctct tttttaattc attctgtgtt tatgtcaaga atca 3954

<210> 10
<211> 3962
<212> DNA
<213> Arabidopsis thaliana

<220>
<221> promoter
<222> (1)..(3962)
<223> transcription regulating sequence from gene At4g12910

<400> 10
ataattagat ctactagtca tctatttctt gagaatatcc ctaagcttga cgtcttaatc 60
atctgaattt atacacatac aatttgttgc ttgcttattt gctttctctt tatttatttt 120
tacttattga tttagtttag tctcgatact ataaccttct gtgtgaacaa aaaagtttcg 180
tggaaatcga tccttaagta ttacaactgg cctcttattt gagagagtag tctaggttaa 240
tttgatccta tatcagttat cgtccatatg tgtaccacac catcacacaa gaaaatgaca 300
tcattaccaa ggattaaatt gtgagaggtt ctagattctc tcatccgacc aaggcaaaaa 360
gagaacttcg agccaaactc cccagaatgg agtactttcc aaccctagcc ctaatatgcc 420
cgttctagta aaatccgaac cgttgctata ctcgttgcat ccaaaataca aacatgcaac 480
attttgaact cactatcaag tgtatataat gtaagcattt cttgcacaag agttcctttc 540
atgttgttct cgtctacata atgttgtcgg taggttattt tcttatcaaa tgatcaatgt 600
gagatacata tttgattttg gattaggatt tccacacatc gcattgtagt ttgttattga 660
agctaagagc atttgaataa accttatggt cataatagta ggagcaaggg aagcattgag 720
tgcagtgaag ttgctcccca tattcatgtg atgagtttat cgcttttgag gagatttaaa 780
atgaatttca gccataaatg ctatatctcg gtgggagtgg agtgacgaga gaggaggaaa 840
acaactttgt tgtcgtaaaa actttccatt gtgcatatat ggatatatag ttaaacaact 900
ttttttttta ttcggccatt atggaataat gactattgat aaaaaaataa aaataataag 960
tagagtagaa tgaaagtagt gcttgataca ttattatgat ttttttttca ttaaatgttt 1020
ttagaaaatc atttaagagt aagattaata gagaatttaa cataacaaaa gacttttgat 1080
ttttcctcaa ttagttttgt taatcatttt atttaattaa tgtagcatga cacatgccat 1140
tttcttgtgt gatgatatgt tacttatatg gagagagttg accaattttt atatatatga 1200
ttaattaaaa gacggaggaa agaaaaacaa gaaaagtcaa tgtgattttc atttatatta 1260
aattttttga ctgattaatt aaggatgtga tttgtagtaa aatgtgatga ttttaaagta 1320
gtgaacgaaa gatttgtgga agtaagaggt aagttgggtt aatggttaaa gagtgaggga 1380
agaaagaaaa aatgagttac ttagtagaaa ttttgttggt agtgtatgtt gggatataat 1440

ttggtttgat gtggggatat gaaagtatgt aataaaaata tatgtaaatc atctaatatc 1500
atatgaataa gggtattttg aaaattgcta acaaaacaaa tatttatggt tattattgtt 1560
atttatgttc atttttgtgt tattgtgaaa ataataaact caaatctatc tcatatagct 1620
gcaaatttta tgattagatt catctttgat tcgaaatatc aattatacgt tttatgtaca 1680
aatctaaatt tttatacatc taaaatccta aatttttact atcatgaata catagaataa 1740
cattataaga tatattgata caatagtata taattatgtt acttatatgg tttgaaactt 1800
gcataatttg ttctgaacaa acattttttt gttccacgta ctcttttctc atctactact 1860
ctaaccatat cctgatatgc tattaattta agtcaatact ttagatctct cccttttat 1920
gtctagaatt ttcaagtata tctaacatct caaatgtgca ttacgtgcaa aaacttgcat 1980
gtctttgtta ctaacttacc ataagacaca taaatgaaaa gagaaaaggt catatgataa 2040
atcatatcca caaaacaaat aataagaaaa tattcactgt aaaattagta acttaataag 2100
aggttaggta gcaaacatct aaactacaac acaagtaatt gttttagcaa ttatcaagta 2160
atgatcatta ataaataaaa aaaactaatg aaagaagaag agtagaatga aagtaatact 2220
tgatacatta gcatgatttt gtattttttt agtattttta acaaatgaat taagagtaag 2280
attaatggat gacttaattc acaataggag aattttggtt tttcttcaat taatgtagct 2340
aatcttttta tttaaataat ataaaacgac aaatgtcatt ttctcatgtg ataatggtac 2400
acttatatgg agaaaattta ccaactttca tatatatgat tatataagta ataatatgta 2460
aagaagaaaa gaaacgaaag agtgaaagag tgagcaagag aaacttgtat aattaacttg 2520
tatatatgcg ttaaaaaaat gagaaccgac tcttttacta aatcatttat taagcagaga 2580
tttagctgac attatacttc tccatctata tgatagaaaa gtataatttt attcaatcca 2640
gatgattgga ttttcaaaaa tatattttga aagttttcgc aaaaagtaag tcgaaaacgt 2700
tatcaaccaa ttattttgct agtaggacca aattaaaatg attcacattc cgaaatccta 2760
ttccactttt cgatttacta ttgcataatt tcttctaaac tatgtagttt atacccttcg 2820
atttaccatt tttactccac taaaatttag taaccgcatt taaaagttta gaagtaatta 2880
tttttagtac tttgaagtaa gaaatcacat aaattgataa tggcgcagct atacacttta 2940
ttagcacttc ttttttacct ttcaaaaaaa cttgttatct ttttatctgt ataataaaac 3000
cggtagtttc ttgtaccaaa ccaaattctt agttcaaaat tttgaaaaca taatacaacg 3060
aactttattt atttattacc tttttcaaat tcttcccttc caaaactttc tgattttatt 3120
attctttaca tatactctta taatgttata acaattccag agaaaacaat atgaaaagaa 3180
gaaaaactaa ctaaacaaaa tagtagtgtg gacctggatc tattcttcta ttaaaatttg 3240
ctttccacta gattttagat atataggttt aaaagctaac catagctttt aacctcataa 3300
atttcaaaat gctttccaaa atacggatta tactattccc aataattgaa taatcagttt 3360
attttctacg taatttaaca atgggaaagc taattcgtag aagttatcgt tcaaaataaa 3420
accaaaactt tttaatcatt atgtaggaat cttttccaaa caaaacgcct aaaacttaac 3480
aaaaagaatt cacaaaaacc agccacgtgt caccttttgt taagccagtc gtcactttaa 3540
gtaaccgctt gtcactttaa gaagcggtcc caagtcaact cgcgccactc aaatcaatca 3600
tgcttatgta aataacaact aaacgcagac gcaatcacca ttttctttat agaaatgctc 3660
aaaatcgtgc cttttaagga aactgttgct ttgcagaatc acaaaatcta tataccaacc 3720
ataagtttcc aaattttgtt ctaaaaatca aaaaaattca tatcatcttt ttgtcttttt 3780
ttaccagaat ttcatcctta tttcgtgcta aatcattttc caaacattca taatactttt 3840
tgttggttta tggccaacta acaaaatatc ctctagattt tcttctttgt gttagtctat 3900
ataaagcaat accacagtga tcctcttttt taattcattc tgtgtttatg tcaataatca 3960
ca 3962

<210> 11

<211> 3660
<212> DNA
<213> Arabidopsis thaliana

<220>
<221> promoter
<222> (1)..(3660)
<223> transcription regulating sequence from gene At4g12910

<400> 11
```
actagtcatc tatttcttga gaatatccct aagcttgacg tcttaatcat ctgaatttat      60
acacatacaa tttgttgctt gcttgtttgc tttctcttta tttattttta cttattgatt     120
tagtttagtc tcgatactat aaccttctgt gtgaacaaaa aagttttgtg gaaatcgatc     180
cttaagtatt acaactggcc tcttatttga gagagtagtc taggttaatt tgatcctata     240
ttagttatcg tccatatgtg taccacacca tcacacaaga aaatgacatc attaccaagg     300
attaaattgc gagaggttct agattctctc atccgacaaa ggcaaaaaga gaacttcgag     360
ccaaactccc cagaatggag tactttccaa ccctagccct aatatgcccg ttctagtaaa     420
atccgaaccg ttgctatact cgttgcatcc aaaatacaaa catgcaacat tttgaactca     480
ctatcaagtg tatataatgt aagcatttct tgcacaagag ttcctttcat gttgttctcg     540
tctacataat gttgtcggta ggttattttc ttatcaaatg atcaatgtga gatacatatt     600
tgattttgga ttaggatttc cacacatcgc attgtagttt gttattgaag ctaagagcat     660
ttgaataaac cttatggtca taatagtagg agcaagggaa gcattgagtg cagtgaggtt     720
gctccccata ttcatgtgat gagtttatcg cttttgagga gatttaaaat gaatttcagc     780
cataaatgct atatctcggt gggagtggat cgagtgacga gagaggtgga aaacaacttt     840
gttgtcgtaa aaactttcca ctgtgcatat atggatatat agttaaacaa cttttttttt     900
tattcggcca ttatggaata atgactattg ataaaaaaat aaaaataata agtagagtag     960
aatgaaagta gtgtttgata cattattatg attttttttt cattaaatgt ttttagaaaa    1020
tcatttaaga gtaagattaa tagagaattt aacataacaa aagacttttg atttttcctc    1080
aattagtttt gtttatcatt ttatttaatt aatgtagcat gacacatgcc attttcttgt    1140
gtgatgatat gttacttata tggagagagt tgaccaattt ttatatatat gattaattaa    1200
aagacggagg aaagaaaaac aagaaaagtc tatgtgattt tcatttatat tttttttttt    1260
actgattaat taaggatgtg atttgtagta aaatgtgatg attttaaagt agtgaacgaa    1320
agatttgtgg aagtaagagg taagttggtt taatggttaa agagtgaggg aagaaagaaa    1380
aaatgagtta cttagtagaa attttgttgg tagtgtatgt tgggatataa tttggtttga    1440
tgtggggata tgaaagtatg taataaaaat atatgtaaat catctaatat catatgaata    1500
agggtatttt gaaaattgct aacaaaacaa atatttatgg ttattattgt tatttatgtt    1560
catttttgtg ttattgtgaa aataataaac tcaaatctat ctcatatagc tgcaaatttt    1620
atgattagat tcatctttga ttcgaaatat caattatacg ttttatgtac aaatctaaat    1680
ttttatacat ctaaaatcct aaatttttac tatcatgaat acatagaaca acattataag    1740
atatattgat acaatagtat ataattatgt tacttatatg gtttgaaact tgcataattt    1800
gattctgaac aaacattttt ttgttccacg tactcttttc tcatctacta ctctaaccat    1860
atcctgatat gctattaatt taagtcaata ctttttttaga tctctcccta tttatgtcta    1920
gaattttcaa gtatatctaa catctcaaat gtgcattaca tgcaaaaact tgcatgtctt    1980
tgttactaac ttaccataag acacataaat gaaaagaaag aaggtcatat gataaatcat    2040
atccacaaaa caaataataa gaaaatattc actgtaaaat tagtaactta ataagaggtt    2100
```

```
aggtagcaaa catataaact acaacacaag taattgtttt agcaattatc aagtaatgac   2160
cattaataaa taaaataaac taatgaaaga agaagagtag aatgaaagta atacttgata   2220
cattagcatg attttgtatt ttttagtatt tttaacaaat gaattaagag taagattaat   2280
ggatgactta attcacaata ggagaatttt ggtttttctt caattaatgt agctaatctt   2340
tttatttaaa taatataaaa ggacaaatgt catttttca tgtgataatg gtatatctat   2400
atggagaaaa ttgaccaact ttcatatata tgattatata agtaataata tgtaaagaag   2460
aaaagaaacg aaagagtgaa agagtgagca agagaaactt gtataattaa cttgtatata   2520
tgcgttaaaa aaatgagaac cgactctttt actaaatcat ttattaagca gagatttagc   2580
tgacattata cttctccatc tatatgatag aaaagtataa ttttattcaa tccagatgat   2640
tggattttca aaaatatatt ttgaaagttt tcgcaaaaag taagtcgaaa acgttatcaa   2700
ccaattattt tgctagtagg accaaattaa aatgattcac attccgaaat cctattccac   2760
ttttcgattt actattgcat aatttcttct aaactatgta gtttataccc ttcgatttac   2820
catttttact ccactaaaat ttagtaaccg catttaaaag tttagaagta attattttta   2880
gtactttgaa gtaagaaatc acataaattg ataatggcgc agctatacac tttattagca   2940
cttctttttt acctttcaaa aaaacttgtt atctttttat ctgtataata aaaccggtag   3000
tttcttgtac ccaaccaaat tcttacttca aaattttgaa aacataatac aacgaacttt   3060
atttatttat tacctttttc aaattcttcc cttccaaaac tttctgattt tattattctt   3120
tacatatact cttataatgt tataacaatt ccagagaaaa caatatgaaa agaagaaaaa   3180
ctaactaaac aaaatagtag tgtggacctg gatctattct tctattaaaa tttgctttcc   3240
actagatttt agatatatag gtttaaaagc taaccatagc ttttaacctc ataaatttca   3300
aaatgctttc caaaatacgg attatactat tcccaataat tgaataatca gtttattttc   3360
tacataattt aacaatcgga aagctaattc gtagaagtta tcgttcaaaa taaaaccaaa   3420
actttttaat cagtatgtag gaatcttttc caaacaaaac gcctaaaact taacaaaaag   3480
aattcacaaa caccagccac gtgtcacctt ttgttaagcc agtcgtcact ttaagtaacc   3540
gcttgtcact ttaagaagcg gtcccaagtc aactcgcgcc actcaaatca atcatgctta   3600
tgtaaataac aactaaacgc agacgcaatc accattttct ttatagaaat gctcaaaatc   3660
```

<210> 12
<211> 3666
<212> DNA
<213> Arabidopsis thaliana

<220>
<221> promoter
<222> (1)..(3666)
<223> transcription regulating sequence from gene At4g12910

<400> 12

```
ataattagat ctactagtca tctatttctt gagaatatcc ctaagcttga cgtcttaatc     60
atctgaattt atacacatac aatttgttgc ttgcttattt gctttctctt tatttatttt    120
tacttattga tttagtttag tctcgatact ataaccttct gtgtgaacaa aaaagtttcg    180
tggaaatcga tccttaagta ttacaactgg cctcttattt gagagagtag tctaggttaa    240
tttgatccta tatcagttat cgtccatatg tgtaccacac catcacacaa gaaaatgaca    300
tcattaccaa ggattaaatt gtgagaggtt ctagattctc tcatccgacc aaggcaaaaa    360
gagaacttcg agccaaactc cccagaatgg agtactttcc aaccctagcc ctaatatgcc    420
```

cgttctagta aaatccgaac cgttgctata ctcgttgcat ccaaaataca aacatgcaac 480
attttgaact cactatcaag tgtatataat gtaagcattt cttgcacaag agttcctttc 540
atgttgttct cgtctacata atgttgtcgg taggttattt tcttatcaaa tgatcaatgt 600
gagatacata tttgattttg gattaggatt tccacacatc gcattgtagt ttgttattga 660
agctaagagc atttgaataa accttatggt cataatagta ggagcaaggg aagcattgag 720
tgcagtgaag ttgctcccca tattcatgtg atgagtttat cgcttttgag gagatttaaa 780
atgaatttca gccataaatg ctatatctcg gtgggagtgg agtgacgaga gaggaggaaa 840
acaactttgt tgtcgtaaaa actttccatt gtgcatatat ggatatatag ttaaacaact 900
ttttttttta ttcggccatt atggaataat gactattgat aaaaaaataa aaataataag 960
tagagtagaa tgaaagtagt gcttgataca ttattatgat tttttttca ttaaatgttt 1020
ttagaaaatc atttaagagt aagattaata gagaatttaa cataacaaaa gacttttgat 1080
ttttcctcaa ttagttttgt taatcatttt atttaattaa tgtagcatga cacatgccat 1140
tttcttgtgt gatgatatgt tacttatatg gagagagttg accaattttt atatatatga 1200
ttaattaaaa gacggaggaa agaaaaacaa gaaaagtcaa tgtgattttc atttatatta 1260
aatttttga ctgattaatt aaggatgtga tttgtagtaa aatgtgatga ttttaaagta 1320
gtgaacgaaa gatttgtgga agtaagaggt aagttgggtt aatggttaaa gagtgaggga 1380
agaaagaaaa aatgagttac ttagtagaaa ttttgttggt agtgtatgtt gggatataat 1440
ttggtttgat gtggggatat gaaagtatgt aataaaaata tatgtaaatc atctaatatc 1500
atatgaataa gggtattttg aaaattgcta acaaaacaaa tatttatggt tattattgtt 1560
atttatgttc atttttgtgt tattgtgaaa ataataaact caaatctatc tcatatagct 1620
gcaaatttta tgattagatt catctttgat tcgaaatatc aattatacgt tttatgtaca 1680
aatctaaatt tttatacatc taaaatccta aatttttact atcatgaata catagaataa 1740
cattataaga tatattgata caatagtata taattatgtt acttatatgg tttgaaactt 1800
gcataatttg ttctgaacaa acattttttt gttccacgta ctcttttctc atctactact 1860
ctaaccatat cctgatatgc tattaattta agtcaatact ttagatctct cccttttat 1920
gtctagaatt ttcaagtata tctaacatct caaatgtgca ttacgtgcaa aaacttgcat 1980
gtctttgtta ctaacttacc ataagacaca taaatgaaaa gagaaaaggt catatgataa 2040
atcatatcca caaaacaaat aataagaaaa tattcactgt aaaattagta acttaataag 2100
aggttaggta gcaaacatct aaactacaac acaagtaatt gttttagcaa ttatcaagta 2160
atgatcatta ataaataaaa aaaactaatg aaagaagaag agtagaatga aagtaatact 2220
tgatacatta gcatgatttt gtattttttt agtattttta acaaatgaat taagagtaag 2280
attaatggat gacttaattc acaataggag aattttggtt tttcttcaat taatgtagct 2340
aatcttttta tttaaataat ataaaacgac aaatgtcatt ttctcatgtg ataatggtac 2400
acttatatgg agaaaattta ccaactttca tatatatgat tatataagta ataatatgta 2460
aagaagaaaa gaaacgaaag agtgaaagag tgagcaagag aaacttgtat aattaacttg 2520
tatatatgcg ttaaaaaaat gagaaccgac tcttttacta aatcatttat taagcagaga 2580
tttagctgac attatacttc tccatctata tgatagaaaa gtataatttt attcaatcca 2640
gatgattgga ttttcaaaaa tatattttga aagttttcgc aaaaagtaag tcgaaaacgt 2700
tatcaaccaa ttattttgct agtaggacca aattaaaatg attcacattc cgaaatccta 2760
ttccactttt cgatttacta ttgcataatt tcttctaaac tatgtagttt ataccccttcg 2820
atttaccatt tttactccac taaaatttag taaccgcatt taaaagttta gaagtaatta 2880
tttttagtac tttgaagtaa gaaatcacat aaattgataa tggcgcagct atacacttta 2940
ttagcacttc ttttttacct ttcaaaaaaa cttgttatct ttttatctgt ataataaaac 3000
cggtagtttc ttgtaccaaa ccaaattctt agttcaaaat tttgaaaaca taatacaacg 3060
aactttattt atttattacc tttttcaaat tcttcccttc caaaactttc tgattttatt 3120

```
attctttaca tatactctta taatgttata acaattccag agaaaacaat atgaaaagaa   3180
gaaaaactaa ctaaacaaaa tagtagtgtg gacctggatc tattcttcta ttaaaatttg   3240
ctttccacta gattttagat atataggttt aaaagctaac catagctttt aacctcataa   3300
atttcaaaat gctttccaaa atacggatta tactattccc aataattgaa taatcagttt   3360
attttctacg taatttaaca atgggaaagc taattcgtag aagttatcgt tcaaaataaa   3420
accaaaactt tttaatcatt atgtaggaat cttttccaaa caaaacgcct aaaacttaac   3480
aaaaagaatt cacaaaaacc agccacgtgt caccttttgt taagccagtc gtcactttaa   3540
gtaaccgctt gtcactttaa gaagcggtcc caagtcaact cgcgccactc aaatcaatca   3600
tgcttatgta aataacaact aaacgcagac gcaatcacca ttttctttat agaaatgctc   3660
aaaatc                                                              3666

<210>  13
<211>  1914
<212>  DNA
<213>  Arabidopsis thaliana

<220>
<221>  CDS
<222>  (282)..(1736)
<223>  encoding serine carboxypeptidase I precursor-like protein

<400>  13
gtgcctttta aggaaactgt tgctttgcag aatcacaaaa tctatatacc aaccataagt     60
ttccaaattt tgttctaaaa atcaaaaaaa ttcatatcat ctttttgtct tttttacca    120
gaatttcatc cttatttcgt gctaaatcat tttccaaaca ttcataatac tttttgttgg    180
tttatggcca actaacaaaa tatcctctag attttcttct ttgtgttagt ctatataaag    240
caataccaca gtgatcctct tttttaattc attctgtgtt t atg tca ata atc aca    296
                                              Met Ser Ile Ile Thr
                                              1               5
atg gtt tgg tta atg aaa gtt ttt gtc ttt gtg aca tta ctc tct tta      344
Met Val Trp Leu Met Lys Val Phe Val Phe Val Thr Leu Leu Ser Leu
                10                  15                  20
gtg ttt gta ata aca gaa tca gct cct gaa tct gct ctt atc acc aaa      392
Val Phe Val Ile Thr Glu Ser Ala Pro Glu Ser Ala Leu Ile Thr Lys
            25                  30                  35
ctt cct ggt ttc gaa ggc act ttt cct tcg aaa cat tac tcc ggg tat      440
Leu Pro Gly Phe Glu Gly Thr Phe Pro Ser Lys His Tyr Ser Gly Tyr
        40                  45                  50
gtg aca att gat aag gaa cat gga aag aat cta tgg tat tac ttt att      488
Val Thr Ile Asp Lys Glu His Gly Lys Asn Leu Trp Tyr Tyr Phe Ile
    55                  60                  65
gaa tcg gag aag aat cca tcg aaa gat ccg gtg gtg ctt tgg ctc aat      536
Glu Ser Glu Lys Asn Pro Ser Lys Asp Pro Val Val Leu Trp Leu Asn
70                  75                  80                  85
ggt ggt cca ggt tgt tca agc atg gat gga ttt gtg tat gag cat ggt      584
```

```
Gly Gly Pro Gly Cys Ser Ser Met Asp Gly Phe Val Tyr Glu His Gly
                90                  95                  100
cca ttc aat ttc gaa cta cca aag aaa aac aat agt ctc cca ctc ttg      632
Pro Phe Asn Phe Glu Leu Pro Lys Lys Asn Asn Ser Leu Pro Leu Leu
            105                 110                 115
cat ctt aat cct tat agt tgg tcc aag gtc tct aac att ata tac ctt      680
His Leu Asn Pro Tyr Ser Trp Ser Lys Val Ser Asn Ile Ile Tyr Leu
        120                 125                 130
gat tct cct gtt ggt gtg gga ttc tct tac tca aac aat aag tct gat      728
Asp Ser Pro Val Gly Val Gly Phe Ser Tyr Ser Asn Asn Lys Ser Asp
    135                 140                 145
tac ata acc ggt gat att aaa acc gcg gtt gac tct cac gcg ttc ctt      776
Tyr Ile Thr Gly Asp Ile Lys Thr Ala Val Asp Ser His Ala Phe Leu
150                 155                 160                 165
ctc aag tgg ttc caa atg ttt cct gag ttt caa tca aat cct ttt ttc      824
Leu Lys Trp Phe Gln Met Phe Pro Glu Phe Gln Ser Asn Pro Phe Phe
                170                 175                 180
atc tct gga gaa tct tac gcc gga gtt tat gtc cca act ctt gct tca      872
Ile Ser Gly Glu Ser Tyr Ala Gly Val Tyr Val Pro Thr Leu Ala Ser
            185                 190                 195
gaa gtt gtc ata ggg aac aaa aat gga gta aag ccg gct ctt aac ttc      920
Glu Val Val Ile Gly Asn Lys Asn Gly Val Lys Pro Ala Leu Asn Phe
        200                 205                 210
aag ggg tat ttg gtt gga aat gga gtt gcg gat ccg aag ttt gat gga      968
Lys Gly Tyr Leu Val Gly Asn Gly Val Ala Asp Pro Lys Phe Asp Gly
    215                 220                 225
aat gct ttt gtc ccg ttt gca cat ggg atg gga cta atc tct gat gaa     1016
Asn Ala Phe Val Pro Phe Ala His Gly Met Gly Leu Ile Ser Asp Glu
230                 235                 240                 245
ctc ttt gag aac gtt aca aaa gct tgc aag gga aat ttc tat gaa ata     1064
Leu Phe Glu Asn Val Thr Lys Ala Cys Lys Gly Asn Phe Tyr Glu Ile
                250                 255                 260
gag gga ctc gag tgc gag gaa cag tat acg aaa gtt aac gat gat act     1112
Glu Gly Leu Glu Cys Glu Glu Gln Tyr Thr Lys Val Asn Asp Asp Thr
            265                 270                 275
aac cag ttg aat ata tac aac att ctt gag cca tgt tac cac gga aca     1160
Asn Gln Leu Asn Ile Tyr Asn Ile Leu Glu Pro Cys Tyr His Gly Thr
        280                 285                 290
tcg cta tct gca ttc gac att aga tcg ctt cct tca agt ctc ctt cag     1208
Ser Leu Ser Ala Phe Asp Ile Arg Ser Leu Pro Ser Ser Leu Leu Gln
    295                 300                 305
cta ggc aaa act gaa aaa cgg tta cct ata aga aaa aga atg ttt ggt     1256
Leu Gly Lys Thr Glu Lys Arg Leu Pro Ile Arg Lys Arg Met Phe Gly
310                 315                 320                 325
cga gcg tgg cca gtt cgt gcg cct gtt cat cca ggc att gtc cct agc     1304
```

```
Arg Ala Trp Pro Val Arg Ala Pro Val His Pro Gly Ile Val Pro Ser
                330                 335                 340
tgg tct caa ctt ctt gcc gac gtc act gtt cct tgc att gat gat aga      1352
Trp Ser Gln Leu Leu Ala Asp Val Thr Val Pro Cys Ile Asp Asp Arg
            345                 350                 355
gtt gca aca gcg tgg ttg aac gat cca gag att agg aaa gct att cat      1400
Val Ala Thr Ala Trp Leu Asn Asp Pro Glu Ile Arg Lys Ala Ile His
        360                 365                 370
act aaa gag gag agc gag att gga agg tgg gaa ctt tgc agt ggg aaa      1448
Thr Lys Glu Glu Ser Glu Ile Gly Arg Trp Glu Leu Cys Ser Gly Lys
    375                 380                 385
ctc tca ttc tat cac gac gca gga agc atg atc gat ttc cat aga aac      1496
Leu Ser Phe Tyr His Asp Ala Gly Ser Met Ile Asp Phe His Arg Asn
390                 395                 400                 405
ctt aca cta agt gga tat aga gct ctc att tac agc ggg gat cac gat      1544
Leu Thr Leu Ser Gly Tyr Arg Ala Leu Ile Tyr Ser Gly Asp His Asp
                410                 415                 420
atg tgt gtt ccg ttt act ggc tcg gaa gct tgg aca aaa tct ctt gga      1592
Met Cys Val Pro Phe Thr Gly Ser Glu Ala Trp Thr Lys Ser Leu Gly
            425                 430                 435
tac aaa gtt att gat gaa tgg agg gca tgg ata tca aat gac caa gtc      1640
Tyr Lys Val Ile Asp Glu Trp Arg Ala Trp Ile Ser Asn Asp Gln Val
        440                 445                 450
gcg ggg tat acg caa gga tat gca aac aat ctc aca ttt tta acc atc      1688
Ala Gly Tyr Thr Gln Gly Tyr Ala Asn Asn Leu Thr Phe Leu Thr Ile
    455                 460                 465
aag ggt gca gga cat acg gtt cct gag aca aac cgc ggg agg ctt tag      1736
Lys Gly Ala Gly His Thr Val Pro Glu Thr Asn Arg Gly Arg Leu
470                 475                 480
acttctatag ccgatttcta gaaggaagca agatttaaga gagctgtctc atcagtttca    1796
tattctgtgc tgtatttaaa agtgatcatc ttatagcaat atagaatctt gattgatttg    1856
aataagaaag atatattctt caactcatta ttcaacatag taaaaatcat ttacctct      1914

<210>  14
<211>  484
<212>  PRT
<213>  Arabidopsis thaliana

<400>  14
Met Ser Ile Ile Thr Met Val Trp Leu Met Lys Val Phe Val Phe Val
1               5                   10                  15

Thr Leu Leu Ser Leu Val Phe Val Ile Thr Glu Ser Ala Pro Glu Ser
            20                  25                  30
```

```
Ala Leu Ile Thr Lys Leu Pro Gly Phe Glu Gly Thr Phe Pro Ser Lys
        35                  40                  45

His Tyr Ser Gly Tyr Val Thr Ile Asp Lys Glu His Gly Lys Asn Leu
    50                  55                  60

Trp Tyr Tyr Phe Ile Glu Ser Glu Lys Asn Pro Ser Lys Asp Pro Val
65                  70                  75                  80

Val Leu Trp Leu Asn Gly Gly Pro Gly Cys Ser Ser Met Asp Gly Phe
                85                  90                  95

Val Tyr Glu His Gly Pro Phe Asn Phe Glu Leu Pro Lys Lys Asn Asn
            100                 105                 110

Ser Leu Pro Leu Leu His Leu Asn Pro Tyr Ser Trp Ser Lys Val Ser
        115                 120                 125

Asn Ile Ile Tyr Leu Asp Ser Pro Val Gly Val Gly Phe Ser Tyr Ser
    130                 135                 140

Asn Asn Lys Ser Asp Tyr Ile Thr Gly Asp Ile Lys Thr Ala Val Asp
145                 150                 155                 160

Ser His Ala Phe Leu Leu Lys Trp Phe Gln Met Phe Pro Glu Phe Gln
                165                 170                 175

Ser Asn Pro Phe Phe Ile Ser Gly Glu Ser Tyr Ala Gly Val Tyr Val
            180                 185                 190

Pro Thr Leu Ala Ser Glu Val Val Ile Gly Asn Lys Asn Gly Val Lys
        195                 200                 205

Pro Ala Leu Asn Phe Lys Gly Tyr Leu Val Gly Asn Gly Val Ala Asp
    210                 215                 220

Pro Lys Phe Asp Gly Asn Ala Phe Val Pro Phe Ala His Gly Met Gly
225                 230                 235                 240

Leu Ile Ser Asp Glu Leu Phe Glu Asn Val Thr Lys Ala Cys Lys Gly
                245                 250                 255

Asn Phe Tyr Glu Ile Glu Gly Leu Glu Cys Glu Glu Gln Tyr Thr Lys
            260                 265                 270
```

Val Asn Asp Asp Thr Asn Gln Leu Asn Ile Tyr Asn Ile Leu Glu Pro
275 280 285

Cys Tyr His Gly Thr Ser Leu Ser Ala Phe Asp Ile Arg Ser Leu Pro
290 295 300

Ser Ser Leu Leu Gln Leu Gly Lys Thr Glu Lys Arg Leu Pro Ile Arg
305 310 315 320

Lys Arg Met Phe Gly Arg Ala Trp Pro Val Arg Ala Pro Val His Pro
325 330 335

Gly Ile Val Pro Ser Trp Ser Gln Leu Leu Ala Asp Val Thr Val Pro
340 345 350

Cys Ile Asp Asp Arg Val Ala Thr Ala Trp Leu Asn Asp Pro Glu Ile
355 360 365

Arg Lys Ala Ile His Thr Lys Glu Glu Ser Glu Ile Gly Arg Trp Glu
370 375 380

Leu Cys Ser Gly Lys Leu Ser Phe Tyr His Asp Ala Gly Ser Met Ile
385 390 395 400

Asp Phe His Arg Asn Leu Thr Leu Ser Gly Tyr Arg Ala Leu Ile Tyr
405 410 415

Ser Gly Asp His Asp Met Cys Val Pro Phe Thr Gly Ser Glu Ala Trp
420 425 430

Thr Lys Ser Leu Gly Tyr Lys Val Ile Asp Glu Trp Arg Ala Trp Ile
435 440 445

Ser Asn Asp Gln Val Ala Gly Tyr Thr Gln Gly Tyr Ala Asn Asn Leu
450 455 460

Thr Phe Leu Thr Ile Lys Gly Ala Gly His Thr Val Pro Glu Thr Asn
465 470 475 480

Arg Gly Arg Leu

<210> 15
<211> 2254
<212> DNA

<213> Arabidopsis thaliana

<220>
<221> promoter
<222> (1)..(2254)
<223> transcription regulating sequence from gene At1g66250

<400> 15

```
tacctcgagc tcgtcatgga ctcatggcag tggtaatttg gtcaccaaaa tctggctgcg     60
agactccaaa gacgattaca tgtgtcggca tcccctaaag ttatgtcatg tatgctcagt    120
gtttcctcgc attactctcc aatttttgcc ttgcaagata cttttgtgca tgggcatggc    180
ttgtcacttg tgtcgcgaac cttgtcttca catatttcct cgatatattt caatctcttt    240
ttctagtata ttctttaatc cttccagata taatctgaaa cttcattgaa ttaacaagtt    300
cataaccaag atcgtctatc atttaaaggc cttaattaaa aaagaaaagt caataacaaa    360
gaacactttg ttaatatcct cacattaaat taattaataa tcatttataa atatatcatt    420
aaccaacaga cccgttattc atttttaaaa cttgattaat tctctcgcaa ccacccacaa    480
aagtgataaa agcataagta taaaaatgtc aagagatttg acaaaatttt gtaggccgag    540
ttggattgac ttaggttcgt cccaaacggc taaatcactt gtccatgctt accaagatgc    600
caaaatacac gacatattcc acaacaaaca aattccgcta tctgataatt cccgaaaata    660
tgttccaaaa atatatagaa atgatggaaa caaaataata ttttaaaatc tttaatattc    720
attaactaat gatttatgaa aaattattaa aaaatgattt gaatatttta taaatctatt    780
aagacgaaaa caagccatgt ttattctctc ttataagcta tgtatagtat ttgtaagtta    840
ctattagtat gggtaacaat tataagaaaa cgaatcaatt tattaataga taaattaaga    900
taaaatcaca taaaagtaag aaaaaggacc aaatgtaata aagagtaaga gaaatacaga    960
gaaacaaaca aaagagtcgc aacggctatt tcgtagttac caacttattt tgctgtccaa   1020
tgcacaactt gttcttacac caaaagttgt gctctttaaa cttgcattta ttctacccaa   1080
aaaaaaaaaa attgcacaat atatatacaa atatatacca tagtttatca ctaacaatca   1140
gtagtactat gtttcacact aatatactca aaattaatca tcaaatcata ttaatattag   1200
tgattaacaa acaacaaaga agctaattaa gacttttctt tcccacagta caactctctg   1260
actgtgtaac tcactctgtc aactacaaga ttacaaaaga ccgttagatt tcatctttga   1320
ccatttgaaa tcttaagatc aaatctcagc cgtccattcc accgttacca atctctctct   1380
ttcgatataa tcaacaaatt ttctaacttt ttctcggtgt cctttctcaa tcagaagctt   1440
taccctattt tctctatacc tcccacgcaa tttctctcat tcctttcttt gtgggttttt   1500
gtttattctt cacaaagtta acatttttag actcaaaact cattcttttg cccttcctct   1560
gtttctcctc tcttggggtt tatcttagat tctccaaaaa agtctctctc tttccatggc   1620
ttctcttctc catcttcttc tcctttctct ttctctttta gttcttgctt cagcttctcc   1680
ttctcctcca gctgacgaag gtgagattct tgtatctgat tcaatcttgt actttgagtt   1740
tcagattcaa tctttactcc caaatttgaa agttttgatt tttgcatgtg ttaatgttct   1800
tctagaaaac caaatgttcc aatttttat gtagtaataa ttccactgtc agtgattaat   1860
gaaaatagct ttaatataaa gtaattaaag acaagtcctt ttgtatgtat gtgttggatt   1920
tgattcattg agtatgtttg agagtgtggt aggttatgat aataaatgtc agtatgtgtg   1980
tttggcaggt gggtttgttt ttaaatgctc actaagaaac aagaaatggt gaattttcat   2040
gtccccaaat atttattaca agacaaataa ttcaatgtct aatgttttga ccagagacat   2100
agttcttttt agttaagtct atgaatccaa atctttgagt attgtacact cttttggact   2160
agttctagat atgttgatac taatggttta gttgaaatca atatattttt tgtaggttca   2220
```

tatattggag taaacatagg gactgatctt tctg 2254

<210> 16
<211> 2259
<212> DNA
<213> Arabidopsis thaliana

<220>
<221> promoter
<222> (1)..(2259)
<223> transcription regulating sequence from gene At1g66250

<400> 16
taaaagctcg agctcgtcat ggactcatgg cagtggtaat ttggtcacca aaatctggct 60
gcgagactcc aaagacgatt acatgtgtcg gcatcccta aagttatgtc atgtatgctc 120
agtgtttcct cgcattactc tccaattttt gccttgcaag atacttttgt gcatgggcat 180
ggcttgtcac ttgtgtcgcg aaccttgtct tcacatattt cctcgatata tttcaatctc 240
tttttctagt atattcttta atccttccag atataatctg aaacttcatt gaattaacaa 300
gttcataacc aagatcgtct atcatttaaa ggccttaatt aaaaaagaaa agtcaataac 360
aaagaacact ttgttaatat cctcacatta aattaattaa taatcatcta taaatatatc 420
attaaccaac agacccgtta ttcattttta aaacttgatt aattctctcg caaccaccca 480
caaaagtgat aaaagcataa gtataaaaat gtcaagagat ttgacaaaat tttgtaggcc 540
gagttagatt gacttaggtt cgtcccaaac ggctaaatca cttgtccatg cttaccaaga 600
tgccaaaata cacgacatat tccacaacaa acaaattccg ctatctgata attcccgaaa 660
atatgttcca aaaatatata gaaatgatgg aaacaaaata atattttaaa atctttaata 720
ttcattaact aatgatttat gaaaaattat taaaaaatga tttgaatatt ttataaatct 780
attaagacga aaacaagcca tgtttattct ctcttataag ctatgtatag tatttgtaag 840
ttactattag tatgggtaac aattataaga aaacgaatca atttattaat agataaatta 900
agataaaatc acataaaagt aagaaaaagg accaaatgta ataaagagta agagaaatac 960
agagaaacaa acaaaagagt cgcaacggct atttcgtagt taccaactta ttttgctgtc 1020
caatgcacaa cttgttctta caccaaaagt tgtgctcttt aaacttgcat ttattctacc 1080
caaaaaaaaa aaaaattgct caatatatat acaaatatat accatagttt atcactaaca 1140
atcagtagta ttatgtttca cactaatata ctcaaaatta atcatcaaat catattaata 1200
ttagtgatta acaaacaaca aagaagctaa ttaagacttt tctttcccac agtacaactc 1260
tctgactgtg taactcactc tgtcaactac aagattacaa aagaccgtta gatttcatct 1320
ttgaccattt gaaatcttaa gatcaaatct cagccgtcca ttccaccgtt accaatctct 1380
ctctttcgat ataatcaaca aattttctaa ctttttctcg gtgtcctttc tcaatcagaa 1440
gctttaccct attttctcta tacctcccac gcaatttctc tcattccttt ctttgtgggt 1500
ttttgtttat tcttcacaaa gttaacattt ttagactcaa aactcattct tttgcccttc 1560
ctctgtttct cctctcttgg ggtttatctt agattctcca aaaaagtctc tctctttcca 1620
tggcttctct tctccatctt cttctccttt ctctttctct tttagttctt gcttcagctt 1680
ctccttctcc tccagctgat gaaggtgaga ttcttgtatc tgattcaatc ttgtactttg 1740
agtttcagat tcaatcttta ctcccaaatt tgaaagtttt gatttttgca tgtgttaatg 1800
ttcttctaga aaaccaaatg ttccattttt tatgtagtaa taattccact gtcagtgatt 1860
aatgaaaata gctttaatat aaagtaatta aagacaagtc cttttgtatg tatgtgttgg 1920

atttgattca ttgagtatgt ttgagagtgt ggtaggttat gataataaat gtcagtatgt 1980
gtgtttggca ggtgggtttg tttttaaatg ctcactaaga aacaagaaat ggtgaatttt 2040
catgtcccca aatatttatt acaagacaaa taattcaatg tctaatgttt tgaccagaga 2100
catagttctt tttagttaag tctatgaatc caaatctttg agtattgtac actcttttgg 2160
actagttcta gatatgttga tactaatggt ttagttgaaa tcaatatatt ttttgtaggt 2220
tcatatattg gagtaaacat agggactgat ctttctgat 2259

<210> 17
<211> 1446
<212> DNA
<213> Arabidopsis thaliana

<220>
<221> promoter
<222> (1)..(1446)
<223> transcription regulating sequence from gene At1g66250

<400> 17
tacctcgagc tcgtcatgga ctcatggcag tggtaatttg gtcaccaaaa tctggctgcg 60
agactccaaa gacgattaca tgtgtcggca tcccctaaag ttatgtcatg tatgctcagt 120
gtttcctcgc attactctcc aatttttgcc ttgcaagata cttttgtgca tgggcatggc 180
ttgtcacttg tgtcgcgaac cttgtcttca catatttcct cgatatattt caatctcttt 240
ttctagtata ttctttaatc cttccagata taatctgaaa cttcattgaa ttaacaagtt 300
cataaccaag atcgtctatc atttaaaggc cttaattaaa aaagaaaagt caataacaaa 360
gaacactttg ttaatatcct cacattaaat taattaataa tcatttataa atatatcatt 420
aaccaacaga cccgttattc atttttaaaa cttgattaat tctctcgcaa ccacccacaa 480
aagtgataaa agcataagta taaaaatgtc aagagatttg acaaaatttt gtaggccgag 540
ttggattgac ttaggttcgt cccaaacggc taaatcactt gtccatgctt accaagatgc 600
caaaatacac gacatattcc acaacaaaca aattccgcta tctgataatt cccgaaaata 660
tgttccaaaa atatatagaa atgatggaaa caaaataata ttttaaaatc tttaatattc 720
attaactaat gatttatgaa aaattattaa aaaatgattt gaatatttta taaatctatt 780
aagacgaaaa caagccatgt ttattctctc ttataagcta tgtatagtat ttgtaagtta 840
ctattagtat gggtaacaat tataagaaaa cgaatcaatt tattaataga taaattaaga 900
taaaatcaca taaaagtaag aaaaaggacc aaatgtaata aagagtaaga gaaatacaga 960
gaaacaaaca aaagagtcgc aacggctatt tcgtagttac caacttattt tgctgtccaa 1020
tgcacaactt gttcttacac caaaagttgt gctctttaaa cttgcattta ttctacccaa 1080
aaaaaaaaaa attgcacaat atatatacaa atatatacca tagtttatca ctaacaatca 1140
gtagtactat gtttcacact aatatactca aaattaatca tcaaatcata ttaatattag 1200
tgattaacaa acaacaaaga agctaattaa gacttttctt tcccacagta caactctctg 1260
actgtgtaac tcactctgtc aactacaaga ttacaaaaga ccgttagatt tcatctttga 1320
ccatttgaaa tcttaagatc aaatctcagc cgtccattcc accgttacca atctctctct 1380
ttcgatataa tcaacaaatt ttctaacttt ttctcggtgt cctttctcaa tcagaagctt 1440
taccct 1446

<210> 18

<211> 1450
<212> DNA
<213> Arabidopsis thaliana

<220>
<221> promoter
<222> (1)..(1450)
<223> transcription regulating sequence from gene At1g66250

<400> 18
```
taaaagctcg agctcgtcat ggactcatgg cagtggtaat ttggtcacca aaatctggct      60
gcgagactcc aaagacgatt acatgtgtcg gcatccccta aagttatgtc atgtatgctc     120
agtgtttcct cgcattactc tccaattttt gccttgcaag atacttttgt gcatgggcat     180
ggcttgtcac ttgtgtcgcg aaccttgtct tcacatattt cctcgatata tttcaatctc     240
tttttctagt atattcttta atccttccag atataatctg aaacttcatt gaattaacaa     300
gttcataacc aagatcgtct atcatttaaa ggccttaatt aaaaaagaaa agtcaataac     360
aaagaacact ttgttaatat cctcacatta aattaattaa taatcatcta taaatatatc     420
attaaccaac agacccgtta ttcattttta aaacttgatt aattctctcg caaccaccca     480
caaaagtgat aaaagcataa gtataaaaat gtcaagagat ttgacaaaat tttgtaggcc     540
gagttagatt gacttaggtt cgtcccaaac ggctaaatca cttgtccatg cttaccaaga     600
tgccaaaata cacgacatat tccacaacaa acaaattccg ctatctgata attcccgaaa     660
atatgttcca aaaatatata gaaatgatgg aaacaaaata atattttaaa atctttaata     720
ttcattaact aatgatttat gaaaaattat taaaaaatga tttgaatatt ttataaatct     780
attaagacga aaacaagcca tgtttattct ctcttataag ctatgtatag tatttgtaag     840
ttactattag tatgggtaac aattataaga aaacgaatca atttattaat agataaatta     900
agataaaatc acataaaagt aagaaaaagg accaaatgta ataaagagta agagaaatac     960
agagaaacaa acaaaagagt cgcaacggct atttcgtagt taccaactta ttttgctgtc    1020
caatgcacaa cttgttctta caccaaaagt tgtgctcttt aaacttgcat ttattctacc    1080
caaaaaaaaa aaaaattgct caatatatat acaaatatat accatagttt atcactaaca    1140
atcagtagta ttatgtttca cactaatata ctcaaaatta atcatcaaat catattaata    1200
ttagtgatta acaaacaaca aagaagctaa ttaagacttt tctttcccac agtacaactc    1260
tctgactgtg taactcactc tgtcaactac aagattacaa aagaccgtta gatttcatct    1320
ttgaccattt gaaatcttaa gatcaaatct cagccgtcca ttccaccgtt accaatctct    1380
ctctttcgat ataatcaaca aatttctaa cttttctcg gtgtcctttc tcaatcagaa    1440
gctttaccct                                                          1450
```

<210> 19
<211> 3892
<212> DNA
<213> Arabidopsis thaliana

<220>
<221> promoter
<222> (1)..(3892)
<223> transcription regulating sequence from gene At1g66250

<400> 19

caataagaat tcggttagtt ttctaatata catgatgtag acatgtagtt tggtttatat 60
atgttaaata tatgtataaa ttgttaccga attaatagtt tgtaaccaaa taattacatt 120
gctagattta tttaattcat taaaaaaaca ccttaataat gttgcggcag gatatcatta 180
tgcataggta ggtattccga caaaaaatga ccttttaagt tctaaaatta gaataaacca 240
ctggaactca aaaatgtagt tcgttaatcc aaatatacat attaaaagta ggattgccaa 300
gaaaatcttc aaattctggt aaaattggca cgtaggatag agttttgttg ttgtcgtaaa 360
ttcatgttaa atcttggttt gatggttgta aagtgtgttg tttagaagtt aaaaccgcgt 420
tttataatta acaacatttt aaatgacagc aaataaacaa aacgaattac ttcttttggg 480
tgttcattat cttttataaa taacttttc ttcattttc ttaaattttt cattagccta 540
taagtagttc tcgggaacaa agttttctag tacttaaata tataaatctt acatatctta 600
tgttacttgt tgatatcaaa agaaaagttg aaaatgaaga agattacaaa aaagctatca 660
atgaaatacg tgaagctcca taagcaaaaa aagctccgcc aagacgggtc catgaagtca 720
gaggaagacg gtggagagcc tttccgggaa tctcattcgc cggattatgc ggaaagtaat 780
gaaacaaagg agactcctaa ggttacaaag attatggagt ccatgcatcg taagctgatg 840
ctaaaagaca aggcaaataa aaagaaaatt cacgtggacg atcaatccca aatgtcagaa 900
cgttcggtta ggaatggtgg tcgtgatcgc aaggaccagc ttgatgaagg ctcggttaga 960
aacatgaatc gcgatggtac ggaccagctt gtaggctcag ttaaaaacag tcatatggat 1020
catcttgaag atttgattag gggtcgtgat cgtatggaag gttccaatag gaatggtgct 1080
cgtaatcgtg tggaccagct tgaaggtttt agcaaaaacg gtgagaaaca tcagcatgaa 1140
gaaaatactg ttaaaacaag cgatcaaata gatcaatcag aaaaatcaac taaaagtcca 1200
tctgattttg cttcaaaaaa agattatctt gattggattg aatatgtgga aggatcgaat 1260
catcattgtt tcgatcgatc cgaagattcc gaaaaatctt atataagaga ggatatcgat 1320
catgacgcga taagcgttgg gatatcagaa ggatccatag aggggaacaa cgatgagagt 1380
ctattactca agagttctaa gtatagaaaa aacgagaaat ttgaagattc aaaaggttat 1440
aagaaaggaa aaggtagcaa ggctaaagat tcactgaaat gtcaattagt tgactaaatg 1500
ttgaatgtgc attttagtcc taacaaaata aatgtttctt tcataatgta taatatgatc 1560
attgatcatt tagttagtat gaacaactac tctgtatgat cattactttc aatgaaaatt 1620
gtggttttct tattaaaagc tcgagctcgt catggactca tggcagtggt aatttggtca 1680
ccaaaatctg gctgcgagac tccaaagacg attacatgtg tcggcatccc ctaaagttat 1740
gtcatgtatg ctcagtgttt cctcgcatta ctctccaatt tttgccttgc aagatacttt 1800
tgtgcatggg catggcttgt cacttgtgtc gcgaaccttg tcttcacata tttcctcgat 1860
atatttcaat ctctttttct agtatattct ttaatccttc cagatataat ctgaaacttc 1920
attgaattaa caagttcata accaagatcg tctatcattt aaaggcctta attaaaaaag 1980
aaaagtcaat aacaaagaac actttgttaa tatcctcaca ttaaattaat taataatcat 2040
ctataaatat atcattaacc aacagacccg ttattcattt ttaaaacttg attaattctc 2100
tcgcaaccac ccacaaaagt gataaaagca taagtataaa aatgtcaaga gatttgacaa 2160
aattttgtag gccgagttag attgacttag gttcgtccca aacggctaaa tcacttgtcc 2220
atgcttacca agatgccaaa atacacgaca tattccacaa caaacaaatt ccgctatctg 2280
ataattcccg aaaatatgtt ccaaaaatat atagaaatga tggaaacaaa ataatatttt 2340
aaaatcttta atattcatta actaatgatt tatgaaaaat tattaaaaaa tgatttgaat 2400
attttataaa tctattaaga cgaaaacaag ccatgtttat tctctcttat aagctatgta 2460
tagtatttgt aagttactat tagtatgggt aacaattata agaaaacgaa tcaatttatt 2520
aatagataaa ttaagataaa atcacataaa agtaagaaaa aggaccaaat gtaataaaga 2580

```
gtaagagaaa tacagagaaa caaacaaaag agtcgcaacg gctatttcgt agttaccaac   2640
ttattttgct gtccaatgca caacttgttc ttacaccaaa agttgtgctc tttaaacttg   2700
catttattct acccaaaaaa aaaaaaaatt gctcaatata tatacaaata tataccatag   2760
tttatcacta acaatcagta gtattatgtt tcacactaat atactcaaaa ttaatcatca   2820
aatcatatta atattagtga ttaacaaaca acaaagaagc taattaagac ttttctttcc   2880
cacagtacaa ctctctgact gtgtaactca ctctgtcaac tacaagatta caaaagaccg   2940
ttagatttca tctttgacca tttgaaatct taagatcaaa tctcagccgt ccattccacc   3000
gttaccaatc tctctctttc gatataatca acaaattttc taacttttc tcggtgtcct   3060
ttctcaatca gaagctttac cctattttct ctatacctcc cacgcaattt ctctcattcc   3120
tttctttgtg ggtttttgtt tattcttcac aaagttaaca tttttagact caaaactcat   3180
tcttttgccc ttcctctgtt tctcctctct tggggtttat cttagattct ccaaaaaagt   3240
ctctctcttt ccatggcttc tcttctccat cttcttctcc tttctctttc tcttttagtt   3300
cttgcttcag cttctccttc tcctccagct gatgaaggtg agattcttgt atctgattca   3360
atcttgtact ttgagtttca gattcaatct ttactcccaa atttgaaagt tttgattttt   3420
gcatgtgtta atgttcttct agaaaaccaa atgttccatt ttttatgtag taataattcc   3480
actgtcagtg attaatgaaa atagctttaa tataaagtaa ttaaagacaa gtccttttgt   3540
atgtatgtgt tggatttgat tcattgagta tgtttgagag tgtggtaggt tatgataata   3600
aatgtcagta tgtgtgtttg gcaggtgggt ttgtttttaa atgctcacta agaaacaaga   3660
aatggtgaat tttcatgtcc ccaaatattt attacaagac aaataattca atgtctaatg   3720
ttttgaccag agacatagtt ctttttagtt aagtctatga atccaaatct ttgagtattg   3780
tacactcttt tggactagtt ctagatatgt tgatactaat ggtttagttg aaatcaatat   3840
atttttgta ggttcatata ttggagtaaa catagggact gatctttctg at             3892
```

<210> 20
<211> 3083
<212> DNA
<213> Arabidopsis thaliana

<220>
<221> promoter
<222> (1)..(3083)
<223> transcription regulating sequence from gene At1g66250

<400> 20

```
caataagaat tcggttagtt ttctaatata catgatgtag acatgtagtt tggtttatat     60
atgttaaata tatgtataaa ttgttaccga attaatagtt tgtaaccaaa taattacatt    120
gctagattta tttaattcat taaaaaaaca ccttaataat gttgcggcag gatatcatta    180
tgcataggta ggtattccga caaaaaatga cctttaagt tctaaaatta gaataaacca     240
ctggaactca aaaatgtagt tcgttaatcc aaatatacat attaaaagta ggattgccaa    300
gaaaatcttc aaattctggt aaaattggca cgtaggatag agttttgttg ttgtcgtaaa    360
ttcatgttaa atcttggttt gatggttgta aagtgtgttg tttagaagtt aaaaccgcgt    420
tttataatta acaacatttt aaatgacagc aaataaacaa aacgaattac ttcttttggg    480
tgttcattat cttttataaa taactttttc ttcatttttc ttaaattttt cattagccta    540
taagtagttc tcgggaacaa agttttctag tacttaaata tataaatctt acatatctta    600
tgttacttgt tgatatcaaa agaaaagttg aaaatgaaga agattacaaa aaagctatca    660
```

```
atgaaatacg tgaagctcca taagcaaaaa aagctccgcc aagacgggtc catgaagtca     720
gaggaagacg gtggagagcc tttccgggaa tctcattcgc cggattatgc ggaaagtaat     780
gaaacaaagg agactcctaa ggttacaaag attatggagt ccatgcatcg taagctgatg     840
ctaaaagaca aggcaaataa aaagaaaatt cacgtggacg atcaatccca aatgtcagaa     900
cgttcggtta ggaatggtgg tcgtgatcgc aaggaccagc ttgatgaagg ctcggttaga     960
aacatgaatc gcgatggtac ggaccagctt gtaggctcag ttaaaaacag tcatatggat    1020
catcttgaag atttgattag ggtcgtgat cgtatggaag gttccaatag gaatggtgct    1080
cgtaatcgtg tggaccagct tgaaggtttt agcaaaaacg gtgagaaaca tcagcatgaa    1140
gaaaatactg ttaaaacaag cgatcaaata gatcaatcag aaaaatcaac taaaagtcca    1200
tctgattttg cttcaaaaaa agattatctt gattggattg aatatgtgga aggatcgaat    1260
catcattgtt tcgatcgatc cgaagattcc gaaaaatctt atataagaga ggatatcgat    1320
catgacgcga taagcgttgg gatatcagaa ggatccatag aggggaacaa cgatgagagt    1380
ctattactca agagttctaa gtatagaaaa aacgagaaat ttgaagattc aaaaggttat    1440
aagaaaggaa aaggtagcaa ggctaaagat tcactgaaat gtcaattagt tgactaaatg    1500
ttgaatgtgc atttagtcc taacaaaata aatgtttctt tcataatgta taatatgatc    1560
attgatcatt tagttagtat gaacaactac tctgtatgat cattactttc aatgaaaatt    1620
gtggttttct tattaaaagc tcgagctcgt catggactca tggcagtggt aatttggtca    1680
ccaaaatctg gctgcgagac tccaaagacg attacatgtg tcggcatccc ctaaagttat    1740
gtcatgtatg ctcagtgttt cctcgcatta ctctccaatt tttgccttgc aagatacttt    1800
tgtgcatggg catggcttgt cacttgtgtc gcgaaccttg tcttcacata tttcctcgat    1860
atatttcaat ctctttttct agtatattct ttaatccttc cagatataat ctgaaacttc    1920
attgaattaa caagttcata accaagatcg tctatcattt aaaggcctta attaaaaaag    1980
aaaagtcaat aacaaagaac actttgttaa tatcctcaca ttaaattaat taataatcat    2040
ctataaatat atcattaacc aacagacccg ttattcattt ttaaaacttg attaattctc    2100
tcgcaaccac ccacaaaagt gataaaagca taagtataaa aatgtcaaga gatttgacaa    2160
aattttgtag gccgagttag attgacttag gttcgtccca aacggctaaa tcacttgtcc    2220
atgcttacca agatgccaaa atacacgaca tattccacaa caaacaaatt ccgctatctg    2280
ataattcccg aaaatatgtt ccaaaaatat atagaaatga tggaaacaaa ataatatttt    2340
aaaatcttta atattcatta actaatgatt tatgaaaaat tattaaaaaa tgatttgaat    2400
attttataaa tctattaaga cgaaaacaag ccatgtttat tctctcttat aagctatgta    2460
tagtatttgt aagttactat tagtatgggt aacaattata agaaaacgaa tcaatttatt    2520
aatagataaa ttaagataaa atcacataaa agtaagaaaa aggaccaaat gtaataaaga    2580
gtaagagaaa tacagagaaa caaacaaaag agtcgcaacg gctatttcgt agttaccaac    2640
ttattttgct gtccaatgca caacttgttc ttacaccaaa agttgtgctc tttaaacttg    2700
catttattct acccaaaaaa aaaaaaaatt gctcaatata tatacaaata tataccatag    2760
tttatcacta acaatcagta gtattatgtt tcacactaat atactcaaaa ttaatcatca    2820
aatcatatta atattagtga ttaacaaaca acaaagaagc taattaagac ttttctttcc    2880
cacagtacaa ctctctgact gtgtaactca ctctgtcaac tacaagatta caaaagaccg    2940
ttagatttca tctttgacca tttgaaatct taagatcaaa tctcagccgt ccattccacc    3000
gttaccaatc tctctctttc gatataatca acaaattttc taactttttc tcggtgtcct    3060
ttctcaatca gaagctttac cct                                            3083
```

<210> 21
<211> 1786
<212> DNA

```
<213>  Arabidopsis thaliana

<220>
<221>  CDS
<222>  (170)..(1687)
<223>  encoding glycosyl hydrolase family 17 protein

<400>  21
atttctcta tacctcccac gcaatttctc tcattccttt ctttgtgggt ttttgtttat      60
tcttcacaaa gttaacattt ttagactcaa aactcattct tttgcccttc ctctgtttct     120
cctctcttgg ggtttatctt agattctcca aaaaagtctc tctctttcc atg gct tct     178
                                                       Met Ala Ser
                                                       1
ctt ctc cat ctt ctt ctc ctt tct ctt tct ctt tta gtt ctt gct tca     226
Leu Leu His Leu Leu Leu Leu Ser Leu Ser Leu Leu Val Leu Ala Ser
    5                   10                  15
gct tct cct tct cct cca gct gat gaa ggt tca tat att gga gta aac     274
Ala Ser Pro Ser Pro Pro Ala Asp Glu Gly Ser Tyr Ile Gly Val Asn
20                  25                  30                  35
ata ggg act gat ctt tct gat atg cca cat cca aca caa gtt gtt gct     322
Ile Gly Thr Asp Leu Ser Asp Met Pro His Pro Thr Gln Val Val Ala
                40                  45                  50
cta cta aaa gct caa gaa atc cga cat att cga tta tac aac gct gat     370
Leu Leu Lys Ala Gln Glu Ile Arg His Ile Arg Leu Tyr Asn Ala Asp
            55                  60                  65
ccc ggg ttg ttg att gct cta gca aac act ggt atc aaa gtt ata atc     418
Pro Gly Leu Leu Ile Ala Leu Ala Asn Thr Gly Ile Lys Val Ile Ile
        70                  75                  80
tcc att cct aat gac cag ctt ctt ggt ata ggt caa tcc aat tca acc     466
Ser Ile Pro Asn Asp Gln Leu Leu Gly Ile Gly Gln Ser Asn Ser Thr
    85                  90                  95
gca gcg aat tgg gtt aaa cgc aat gtc att gca cat tac cct gca acg     514
Ala Ala Asn Trp Val Lys Arg Asn Val Ile Ala His Tyr Pro Ala Thr
100                 105                 110                 115
atg ata acc gcg gtt tct gtt ggc tct gag gtg cta acc agt ctc tct     562
Met Ile Thr Ala Val Ser Val Gly Ser Glu Val Leu Thr Ser Leu Ser
                120                 125                 130
aat gca gca cct gtt cta gtc agt gct ata aag aac gtt cat gct gct     610
Asn Ala Ala Pro Val Leu Val Ser Ala Ile Lys Asn Val His Ala Ala
            135                 140                 145
tta ctc tcg gcg aat ctt gac aag ttg ata aaa gtt tct act cct tta     658
Leu Leu Ser Ala Asn Leu Asp Lys Leu Ile Lys Val Ser Thr Pro Leu
        150                 155                 160
tca act tcc ttg att ctt gat cct ttt cct cca tcg caa gcg ttc ttt     706
Ser Thr Ser Leu Ile Leu Asp Pro Phe Pro Pro Ser Gln Ala Phe Phe
```

```
    165                 170                 175
aac cgt tct ttg aat gcg gtt ata gtt ccg tta cta agc ttc ttg cag      754
Asn Arg Ser Leu Asn Ala Val Ile Val Pro Leu Leu Ser Phe Leu Gln
180                 185                 190                 195
tca aca aac tca tac ctg atg gtg aat gtg tat cca tac att gac tat      802
Ser Thr Asn Ser Tyr Leu Met Val Asn Val Tyr Pro Tyr Ile Asp Tyr
                200                 205                 210
atg caa tca aac ggt gtg att ccg tta gac tac gcg ttg ttc aaa ccg      850
Met Gln Ser Asn Gly Val Ile Pro Leu Asp Tyr Ala Leu Phe Lys Pro
            215                 220                 225
ata cct cca aac aaa gaa gct gtt gat gca aac act ctt gtt cgt tac      898
Ile Pro Pro Asn Lys Glu Ala Val Asp Ala Asn Thr Leu Val Arg Tyr
        230                 235                 240
tca aac gct ttt gat gca atg gtt gat gca acg tac ttc gct atg gcg      946
Ser Asn Ala Phe Asp Ala Met Val Asp Ala Thr Tyr Phe Ala Met Ala
    245                 250                 255
ttc ctc aac ttc aca aac att ccg gtt tta gta acc gaa tca ggc tgg      994
Phe Leu Asn Phe Thr Asn Ile Pro Val Leu Val Thr Glu Ser Gly Trp
260                 265                 270                 275
cct tcg aaa gga gaa acc aat gag cct gat gct aca ctt gac aac gcc     1042
Pro Ser Lys Gly Glu Thr Asn Glu Pro Asp Ala Thr Leu Asp Asn Ala
                280                 285                 290
aac act tac aac agt aat ctc att aga cat gtt ctt aac aag acc gga     1090
Asn Thr Tyr Asn Ser Asn Leu Ile Arg His Val Leu Asn Lys Thr Gly
            295                 300                 305
act ccg aaa cgc ccg ggg atc gct gtg agt act tat att tac gag ctt     1138
Thr Pro Lys Arg Pro Gly Ile Ala Val Ser Thr Tyr Ile Tyr Glu Leu
        310                 315                 320
tac aat gaa gat aca aaa gca ggt tta tca gag aag aat tgg ggg ttg     1186
Tyr Asn Glu Asp Thr Lys Ala Gly Leu Ser Glu Lys Asn Trp Gly Leu
    325                 330                 335
ttt aat gca aat ggt gaa ccg gtt tac gta ctt cgg ttg act aac tcg     1234
Phe Asn Ala Asn Gly Glu Pro Val Tyr Val Leu Arg Leu Thr Asn Ser
340                 345                 350                 355
ggc tcg gtt cta gct aac gat aca acg aac cag act tat tgt act gca     1282
Gly Ser Val Leu Ala Asn Asp Thr Thr Asn Gln Thr Tyr Cys Thr Ala
                360                 365                 370
aga gaa ggt gct gat aca aag atg ctt caa gct gct ctt gat tgg gct     1330
Arg Glu Gly Ala Asp Thr Lys Met Leu Gln Ala Ala Leu Asp Trp Ala
            375                 380                 385
tgt ggt cct gga aag att gat tgt tcg cct att aag caa gga gag act     1378
Cys Gly Pro Gly Lys Ile Asp Cys Ser Pro Ile Lys Gln Gly Glu Thr
        390                 395                 400
tgt tat gaa ccg gat aat gtc gtc gcg cat gct aac tac gcg ttt gat     1426
Cys Tyr Glu Pro Asp Asn Val Val Ala His Ala Asn Tyr Ala Phe Asp
```

```
    405                 410                 415
act tat tat cat cag act ggg aat aat cct gat gct tgc aac ttc aat      1474
Thr Tyr Tyr His Gln Thr Gly Asn Asn Pro Asp Ala Cys Asn Phe Asn
420                 425                 430                 435
ggt gtt gct agt atc act acc aca gat cca agt cat ggt aca tgt gtg      1522
Gly Val Ala Ser Ile Thr Thr Thr Asp Pro Ser His Gly Thr Cys Val
                440                 445                 450
ttt gct ggg agc cgc ggt aat ggt aga aac ggg acg tct gtg aac ata      1570
Phe Ala Gly Ser Arg Gly Asn Gly Arg Asn Gly Thr Ser Val Asn Ile
            455                 460                 465
aca gcg cca tcg gca aac tca aca act tct tct gga ata cgg agt gat      1618
Thr Ala Pro Ser Ala Asn Ser Thr Thr Ser Ser Gly Ile Arg Ser Asp
        470                 475                 480
ttg tat tac agt cgg ggt ata tgg agt atc ttg aca gta atg att ttg      1666
Leu Tyr Tyr Ser Arg Gly Ile Trp Ser Ile Leu Thr Val Met Ile Leu
    485                 490                 495
aac gtt gct aat atc ttg tga gaaatctttg ggacataaca atgttagttt         1717
Asn Val Ala Asn Ile Leu
500                 505
ctgttccgag aagttttttt aactttctta ggtgggattg gagttagaat tgaggccgat   1777
tgtgttgga                                                            1786
```

<210> 22
<211> 505
<212> PRT
<213> Arabidopsis thaliana

<400> 22

```
Met Ala Ser Leu Leu His Leu Leu Leu Leu Ser Leu Ser Leu Leu Val
1               5                   10                  15

Leu Ala Ser Ala Ser Pro Ser Pro Pro Ala Asp Glu Gly Ser Tyr Ile
            20                  25                  30

Gly Val Asn Ile Gly Thr Asp Leu Ser Asp Met Pro His Pro Thr Gln
        35                  40                  45

Val Val Ala Leu Leu Lys Ala Gln Glu Ile Arg His Ile Arg Leu Tyr
    50                  55                  60

Asn Ala Asp Pro Gly Leu Leu Ile Ala Leu Ala Asn Thr Gly Ile Lys
65                  70                  75                  80

Val Ile Ile Ser Ile Pro Asn Asp Gln Leu Leu Gly Ile Gly Gln Ser
                85                  90                  95
```

```
Asn Ser Thr Ala Ala Asn Trp Val Lys Arg Asn Val Ile Ala His Tyr
            100                 105                 110

Pro Ala Thr Met Ile Thr Ala Val Ser Val Gly Ser Glu Val Leu Thr
        115                 120                 125

Ser Leu Ser Asn Ala Ala Pro Val Leu Val Ser Ala Ile Lys Asn Val
    130                 135                 140

His Ala Ala Leu Leu Ser Ala Asn Leu Asp Lys Leu Ile Lys Val Ser
145                 150                 155                 160

Thr Pro Leu Ser Thr Ser Leu Ile Leu Asp Pro Phe Pro Pro Ser Gln
                165                 170                 175

Ala Phe Phe Asn Arg Ser Leu Asn Ala Val Ile Val Pro Leu Leu Ser
            180                 185                 190

Phe Leu Gln Ser Thr Asn Ser Tyr Leu Met Val Asn Val Tyr Pro Tyr
      · 195                 200                 205

Ile Asp Tyr Met Gln Ser Asn Gly Val Ile Pro Leu Asp Tyr Ala Leu
    210                 215                 220

Phe Lys Pro Ile Pro Pro Asn Lys Glu Ala Val Asp Ala Asn Thr Leu
225                 230                 235                 240

Val Arg Tyr Ser Asn Ala Phe Asp Ala Met Val Asp Ala Thr Tyr Phe
                245                 250                 255

Ala Met Ala Phe Leu Asn Phe Thr Asn Ile Pro Val Leu Val Thr Glu
            260                 265                 270

Ser Gly Trp Pro Ser Lys Gly Glu Thr Asn Glu Pro Asp Ala Thr Leu
        275                 280                 285

Asp Asn Ala Asn Thr Tyr Asn Ser Asn Leu Ile Arg His Val Leu Asn
    290                 295                 300

Lys Thr Gly Thr Pro Lys Arg Pro Gly Ile Ala Val Ser Thr Tyr Ile
305                 310                 315                 320

Tyr Glu Leu Tyr Asn Glu Asp Thr Lys Ala Gly Leu Ser Glu Lys Asn
                325                 330                 335
```

```
Trp Gly Leu Phe Asn Ala Asn Gly Glu Pro Val Tyr Val Leu Arg Leu
            340                 345                 350

Thr Asn Ser Gly Ser Val Leu Ala Asn Asp Thr Thr Asn Gln Thr Tyr
        355                 360                 365

Cys Thr Ala Arg Glu Gly Ala Asp Thr Lys Met Leu Gln Ala Ala Leu
    370                 375                 380

Asp Trp Ala Cys Gly Pro Gly Lys Ile Asp Cys Ser Pro Ile Lys Gln
385                 390                 395                 400

Gly Glu Thr Cys Tyr Glu Pro Asp Asn Val Val Ala His Ala Asn Tyr
                405                 410                 415

Ala Phe Asp Thr Tyr Tyr His Gln Thr Gly Asn Asn Pro Asp Ala Cys
            420                 425                 430

Asn Phe Asn Gly Val Ala Ser Ile Thr Thr Thr Asp Pro Ser His Gly
        435                 440                 445

Thr Cys Val Phe Ala Gly Ser Arg Gly Asn Gly Arg Asn Gly Thr Ser
    450                 455                 460

Val Asn Ile Thr Ala Pro Ser Ala Asn Ser Thr Thr Ser Ser Gly Ile
465                 470                 475                 480

Arg Ser Asp Leu Tyr Tyr Ser Arg Gly Ile Trp Ser Ile Leu Thr Val
                485                 490                 495

Met Ile Leu Asn Val Ala Asn Ile Leu
            500                 505
```

<210> 23
<211> 1026
<212> DNA
<213> Arabidopsis thaliana

<220>
<221> promoter
<222> (1)..(1026)
<223> transcription regulating sequence from gene At4g00820

<400> 23

```
tatagaatta aattgtattt gtattgaaaa tttggattgt atttgaattg tatacaatcg     60
aattaaattg tattctatta tgataaaaaa aaatatttaa attgtattgt aaaatcgaat    120
ttgtaagatt gaaatttatt ttaaagaaat actaaattaa atactgaaaa aaaatatgag    180
ttactattta aaattcaaat atcatcaata agttggagtg tatagagtta aaagacagtt    240
tattatgata aaagaaatag ttaaaatgtt tagtaaaatt gaatgtatat gattgaaatt    300
ttatttaatt caaaaattac taaattaaac attagaaaag tgtggatcac tatttactat    360
tcaaatttta aatattatca atacatattt gaataaattt atatcttaca cccgcctatt    420
taggcgggcc ttatctagta tatatataag tagaataata ttcatgcata tcttaagaaa    480
acccaaaaat tagaatgtat gaaatagaat tgcataatcg aaccaaggga aagaagaatt    540
gttttgcata atgaaataga attagtttat attgtatttg tttaaaatta actctcaaaa    600
caacaaaact ctatctttca atttaacagc aacaaatctt ctagaattga atgtacaaca    660
aatgcaagta ggtacatgat ataataataa taataatgtg atattactag tttagtgaaa    720
tgagttaaac aaaaataaca aaataaatga agtggagaaa gtaaatgaag aggaagagtc    780
atcgctgtca tggtcgcagt taaagaggtg gtcagagtca gaagtctccc ttaagcaacc    840
ttccaaattt attgacattt ctgtttccca agatccaccc accactctcc tctctctctt    900
cctcatttct gcgcaagaat ctaaaccaaa tctctcatcc tctcttctat gtcccaaaac    960
aggaacataa ctactactac taccatgtct tgactctgtt gagaatctta aatcctacct   1020
aacctc                                                              1026
```

<210> 24
<211> 1030
<212> DNA
<213> Arabidopsis thaliana

<220>
<221> promoter
<222> (1)..(1030)
<223> transcription regulating sequence from gene At4g00820

<400> 24

```
taagatggag tgtatagaat taaattgtat ttgtattgaa aatttggatt gtatttgaat     60
tgtatacaat cgaattaaat tgtattctat tatgataaaa aataatattt aaattgtatt    120
gtaaaatcga agttgtaaga ttaaaattta ttttaaagaa atactaaatt aaatactgaa    180
aaaaatatga gttactattt aaaattcaaa tatcatcaat aagttggagt gtatagagtt    240
aaaaggcagt ttattatgat aaaagaaata gttaaaatgt atagtaaaat tgaatgtata    300
tgattgaaat tttatttaat tcaaaaatta ctaaatttaa cattagaaaa gtgtggatca    360
ctatttacca ttcaaatttt aaatattatc aatacatatt tgaataaatt tatatcttag    420
gcgggcctta tctagtatat atatatatat ataagtagaa taatattcat gcatatctta    480
agaaaaccca aaaattagaa tgtatgaaat ataattgcat aatcgaacca agggaaagaa    540
gaattgtttt gcataatgaa atagaattag tttatattgt atttgtttaa aattaactct    600
caaaacaaca aaactctatc tttcaattta acagcaacaa atcttctaga attgaatgta    660
caacaaatgc aagtaggtac atgatataat aataataatg tgatataact agtttagtga    720
aatgagttaa acaaaaataa caaaataaat gaagtggaga aagtaaatga agaggaagag    780
tcatcgctgt catggtcgca gttaaagagg tggtcagagt cagaagtctc ccttaagcaa    840
ccttccaaat ttattgacat ttctgtttcc caagatccac ccaccactct cctctctctc    900
```

ttcctcattt ctgcgcaaga atctaaacca aatctctcat cctctcttct atgtcccaaa 960
acaggaacat aactactact actaccatgt cttgactctg ttgagaatct taaatcctac 1020
ctaacctcca 1030

<210> 25
<211> 844
<212> DNA
<213> Arabidopsis thaliana

<220>
<221> promoter
<222> (1)..(844)
<223> transcription regulating sequence from gene At4g00820

<400> 25
tatagaatta aattgtattt gtattgaaaa tttggattgt atttgaattg tatacaatcg 60
aattaaattg tattctatta tgataaaaaa aaatatttaa attgtattgt aaaatcgaat 120
ttgtaagatt gaaatttatt ttaaagaaat actaaattaa atactgaaaa aaaatatgag 180
ttactattta aaattcaaat atcatcaata agttggagtg tatagagtta aaagacagtt 240
tattatgata aaagaaatag ttaaaatgtt tagtaaaatt gaatgtatat gattgaaatt 300
ttatttaatt caaaaattac taaattaaac attagaaaag tgtggatcac tatttactat 360
tcaaatttta aatattatca atacatattt gaataaattt atatcttaca cccgcctatt 420
taggcgggcc ttatctagta tatatataag tagaataata ttcatgcata tcttaagaaa 480
acccaaaaat tagaatgtat gaaatagaat tgcataatcg aaccaaggga aagaagaatt 540
gttttgcata atgaaataga attagtttat attgtatttg tttaaaatta actctcaaaa 600
caacaaaact ctatctttca atttaacagc aacaaatctt ctagaattga atgtacaaca 660
aatgcaagta ggtacatgat ataataataa taataatgtg atattactag tttagtgaaa 720
tgagttaaac aaaaataaca aaataaatga agtggagaaa gtaaatgaag aggaagagtc 780
atcgctgtca tggtcgcagt taaagaggtg gtcagagtca gaagtctccc ttaagcaacc 840
ttcc 844

<210> 26
<211> 846
<212> DNA
<213> Arabidopsis thaliana

<220>
<221> promoter
<222> (1)..(846)
<223> transcription regulating sequence from gene At4g00820

<400> 26
taagatggag tgtatagaat taaattgtat ttgtattgaa aatttggatt gtatttgaat 60
tgtatacaat cgaattaaat tgtattctat tatgataaaa aataatattt aaattgtatt 120
gtaaaatcga agttgtaaga ttaaaattta ttttaaagaa atactaaatt aaatactgaa 180

```
aaaaatatga gttactattt aaaattcaaa tatcatcaat aagttggagt gtatagagtt    240
aaaaggcagt ttattatgat aaaagaaata gttaaaatgt atagtaaaat tgaatgtata    300
tgattgaaat tttatttaat tcaaaaatta ctaaatttaa cattagaaaa gtgtggatca    360
ctatttacca ttcaaatttt aaatattatc aatacatatt tgaataaatt tatatcttag    420
gcgggcctta tctagtatat atatatatat ataagtagaa taatattcat gcatatctta    480
agaaaaccca aaaattagaa tgtatgaaat ataattgcat aatcgaacca agggaaagaa    540
gaattgtttt gcataatgaa atagaattag tttatattgt atttgtttaa aattaactct    600
caaaacaaca aaactctatc tttcaattta acagcaacaa atcttctaga attgaatgta    660
caacaaatgc aagtaggtac atgatataat aataataatg tgatataact agtttagtga    720
aatgagttaa acaaaaataa caaaataaat gaagtggaga aagtaaatga agaggaagag    780
tcatcgctgt catggtcgca gttaaagagg tggtcagagt cagaagtctc ccttaagcaa    840
ccttcc                                                               846
```

<210> 27
<211> 2352
<212> DNA
<213> Arabidopsis thaliana

<220>
<221> promoter
<222> (1)..(2352)
<223> transcription regulating sequence from gene At4g00820

<400> 27

```
ctgtcgacat tgtctctgca aaaccaagta atcacaaaaa ccataaacaa cagattcaaa     60
tctcaacaac aacaagaaat caaacgagaa atttcctaaa tagccactaa atttactaat    120
atatgcttat cgtacgagca caataaatcc gaagattggt gagaaatctg taacgaataa    180
gcctttaggt gttggaaatg tttgaggatc gagaaaaata cctcagacta cgattatgaa    240
gcttcggctc aaatttcttt ggtggcgatg ataaataaac ctaatctcca gaaatattgg    300
gctgggaata atccttgaaa ggcccattgg aaacttttca cttatataca attgggcctt    360
tgataaacaa aaaaaaagta gggtttgatg taaggaagcc cagatgagag tgttgttgac    420
cagatgtttg actgttaaat gtaaaaaggc cacgtgtatg gttgtaggga agaaatggac    480
taccgtggtc catcatgcac ctgatgatgt ctctgtctga ctggctccat gcacgtgata    540
acacgagact tcaccagttc gttttaaaaa atttcttctt tttttagttt gttatttatt    600
ggccaataag caaagcttga aagctgtcat tttcgactta aatcattggt ctggtttaat    660
atgcttaata aatatatgat aatgatatcc agctttttcc taaggtaaaa gatgaataat    720
tggtttacgc attcgatggt ttaaacttta ttaatttttc aaaacatgaa tcacaatcgt    780
cattcgtcag tattgaaaac ataataacct tacgctcaca agtttatatt tgaaggtata    840
ttcaataatc atgcgtacgt ataatagggt tcgtccgtat gcatagatca tagctaggtg    900
cctaggtctg tctacattgt ttgttcgtct atatatttat gcttatatat aaaaaaaata    960
tatatatata tatatatata aaaacatgta ttgtatacac tttgtacatt tatatgatac   1020
ttattaaaat catatttaaa aaagtaaact agtaatgata gtttaatgtt attgtcgatc   1080
taataaatat atatatatat atatatgaaa catataagca tatatcttat tatataaagt   1140
acagttttga aagttactaa ctcacgagat catgacacgt gtcaggtcta tcattgagat   1200
gttgacatat gtcaaaaaaa attctttaaa aaataaataa acatatttac atatactaat   1260
```

```
ttatatttct atatctaaaa atttaactta taattttatt aattaaaaag gaaaactaac   1320
aataagatgg agtgtataga attaaattgt atttgtattg aaaatttgga ttgtatttga   1380
attgtataca atcgaattaa attgtattct attatgataa aaaataatat ttaaattgta   1440
ttgtaaaatc gaagttgtaa gattaaaatt tattttaaag aaatactaaa ttaaatactg   1500
aaaaaaatat gagttactat ttaaaattca aatatcatca ataagttgga gtgtatagag   1560
ttaaaaggca gtttattatg ataaaagaaa tagttaaaat gtatagtaaa attgaatgta   1620
tatgattgaa attttattta attcaaaaat tactaaattt aacattagaa aagtgtggat   1680
cactatttac cattcaaatt ttaaatatta tcaatacata tttgaataaa tttatatctt   1740
aggcgggcct tatctagtat atatatatat atataagtag aataatattc atgcatatct   1800
taagaaaacc caaaaattag aatgtatgaa atataattgc ataatcgaac caagggaaag   1860
aagaattgtt ttgcataatg aaatagaatt agtttatatt gtatttgttt aaaattaact   1920
ctcaaaacaa caaaactcta tctttcaatt taacagcaac aaatcttcta gaattgaatg   1980
tacaacaaat gcaagtaggt acatgatata ataataataa tgtgatataa ctagtttagt   2040
gaaatgagtt aaacaaaaat aacaaaataa atgaagtgga gaaagtaaat gaagaggaag   2100
agtcatcgct gtcatggtcg cagttaaaga ggtggtcaga gtcagaagtc tcccttaagc   2160
aaccttccaa atttattgac atttctgttt cccaagatcc acccaccact ctcctctctc   2220
tcttcctcat ttctgcgcaa gaatctaaac caaatctctc atcctctctt ctatgtccca   2280
aaacaggaac ataactacta ctactaccat gtcttgactc tgttgagaat cttaaatcct   2340
acctaacctc ca                                                       2352
```

```
<210>  28
<211>  2168
<212>  DNA
<213>  Arabidopsis thaliana

<220>
<221>  promoter
<222>  (1)..(2168)
<223>  transcription regulating sequence from gene At4g00820

<400>  28
ctgtcgacat tgtctctgca aaaccaagta atcacaaaaa ccataaacaa cagattcaaa     60
tctcaacaac aacaagaaat caaacgagaa atttcctaaa tagccactaa atttactaat    120
atatgcttat cgtacgagca caataaatcc gaagattggt gagaaatctg taacgaataa    180
gcctttaggt gttggaaatg tttgaggatc gagaaaaata cctcagacta cgattatgaa    240
gcttcggctc aaatttcttt ggtggcgatg ataaataaac ctaatctcca gaaatattgg    300
gctgggaata atccttgaaa ggcccattgg aaacttttca cttatataca attgggcctt    360
tgataaacaa aaaaaaagta gggtttgatg taaggaagcc cagatgagag tgttgttgac    420
cagatgtttg actgttaaat gtaaaaaggc cacgtgtatg gttgtaggga agaaatggac    480
taccgtggtc catcatgcac ctgatgatgt ctctgtctga ctggctccat gcacgtgata    540
acacgagact tcaccagttc gttttaaaaa atttcttctt tttttagttt gttatttatt    600
ggccaataag caaagcttga aagctgtcat tttcgactta aatcattggt ctggtttaat    660
atgcttaata aatatatgat aatgatatcc agctttttcc taaggtaaaa gatgaataat    720
tggtttacgc attcgatggt ttaaacttta ttaatttttc aaaacatgaa tcacaatcgt    780
cattcgtcag tattgaaaac ataataacct tacgctcaca agtttatatt tgaaggtata    840
```

```
ttcaataatc atgcgtacgt ataatagggt tcgtccgtat gcatagatca tagctaggtg    900
cctaggtctg tctacattgt ttgttcgtct atatatttat gcttatatat aaaaaaaata    960
tatatatata tatatatata aaaacatgta ttgtatacac tttgtacatt tatatgatac   1020
ttattaaaat catatttaaa aaagtaaact agtaatgata gtttaatgtt attgtcgatc   1080
taataaatat atatatatat atatatgaaa catataagca tatatcttat tatataaagt   1140
acagttttga aagttactaa ctcacgagat catgacacgt gtcaggtcta tcattgagat   1200
gttgacatat gtcaaaaaaa attctttaaa aaataaataa acatatttac atatactaat   1260
ttatatttct atatctaaaa atttaactta taattttatt aattaaaaag gaaaactaac   1320
aataagatgg agtgtataga attaaattgt atttgtattg aaaatttgga ttgtatttga   1380
attgtataca atcgaattaa attgtattct attatgataa aaaataatat ttaaattgta   1440
ttgtaaaatc gaagttgtaa gattaaaatt tattttaaag aaatactaaa ttaaatactg   1500
aaaaaaatat gagttactat ttaaaattca aatatcatca ataagttgga gtgtatagag   1560
ttaaaaggca gtttattatg ataaaagaaa tagttaaaat gtatagtaaa attgaatgta   1620
tatgattgaa attttattta attcaaaaat tactaaattt aacattagaa aagtgtggat   1680
cactatttac cattcaaatt ttaaatatta tcaatacata tttgaataaa tttatatctt   1740
aggcgggcct tatctagtat atatatatat atataagtag aataatattc atgcatatct   1800
taagaaaacc caaaaattag aatgtatgaa atataattgc ataatcgaac caagggaaag   1860
aagaattgtt ttgcataatg aaatagaatt agtttatatt gtatttgttt aaaattaact   1920
ctcaaaacaa caaaactcta tctttcaatt taacagcaac aaatcttcta gaattgaatg   1980
tacaacaaat gcaagtaggt acatgatata ataataataa tgtgatataa ctagtttagt   2040
gaaatgagtt aaacaaaaat aacaaaataa atgaagtgga gaaagtaaat gaagaggaag   2100
agtcatcgct gtcatggtcg cagttaaaga ggtggtcaga gtcagaagtc tcccttaagc   2160
aaccttcc                                                            2168
```

<210> 29
<211> 2032
<212> DNA
<213> Arabidopsis thaliana

<220>
<221> CDS
<222> (185)..(1789)
<223> encoding calmodulin-binding protein-related protein

<400> 29

```
aaatttattg acatttctgt ttcccaagat ccacccacca ctctcctctc tctcttcctc     60
atttctgcgc aagaatctaa accaaatctc tcatcctctc ttctatgtcc caaaacagga    120
acataactac tactactacc atgtcttgac tctgttgaga atcttaaatc ctacctaacc    180
tcca atg ggt aag aag agc ggt tct tct tct tct tgg ctc act gcc gtg     229
     Met Gly Lys Lys Ser Gly Ser Ser Ser Ser Trp Leu Thr Ala Val
     1               5                   10                  15
aaa cga gct ttc cga tct ccc acc aag aaa gaa cac aac aac aat gct      277
Lys Arg Ala Phe Arg Ser Pro Thr Lys Lys Glu His Asn Asn Asn Ala
                20                  25                  30
cat ggt aat gaa gtc gac gaa gat gaa gac aag aag aaa gag aag aga      325
```

```
His Gly Asn Glu Val Asp Glu Asp Glu Asp Lys Lys Lys Glu Lys Arg
            35                  40                  45
cgt tgg tta ttt aga aaa tct acg aat cat gac tct ccg gtg aag acc      373
Arg Trp Leu Phe Arg Lys Ser Thr Asn His Asp Ser Pro Val Lys Thr
        50                  55                  60
tcc ggc gtc gga aaa gac gct ccg gcg cag aaa tcc aca gaa aca acg      421
Ser Gly Val Gly Lys Asp Ala Pro Ala Gln Lys Ser Thr Glu Thr Thr
    65                  70                  75
acg atc atc aac cca acc gtt tta tcc tct gtt aca gaa cag agg tac      469
Thr Ile Ile Asn Pro Thr Val Leu Ser Ser Val Thr Glu Gln Arg Tyr
80                  85                  90                  95
gac gca tct aca ccg ccg gcc acc gtc tcc gcc gca tcg gaa act cat      517
Asp Ala Ser Thr Pro Pro Ala Thr Val Ser Ala Ala Ser Glu Thr His
                100                 105                 110
cct cct tcg aca acg aag gag tta cca aat ctt aca aga cgt act tat      565
Pro Pro Ser Thr Thr Lys Glu Leu Pro Asn Leu Thr Arg Arg Thr Tyr
            115                 120                 125
acc gca aga gaa gat tac gca gct gtt gta atc caa act ggt ttc aga      613
Thr Ala Arg Glu Asp Tyr Ala Ala Val Val Ile Gln Thr Gly Phe Arg
        130                 135                 140
ggc tat ttg gca aga aga gca tta aga gca ttg aaa ggg cta gtg aag      661
Gly Tyr Leu Ala Arg Arg Ala Leu Arg Ala Leu Lys Gly Leu Val Lys
    145                 150                 155
tta caa gca cta gtg aga ggt cac aat gtg agg aag caa gca aag atg      709
Leu Gln Ala Leu Val Arg Gly His Asn Val Arg Lys Gln Ala Lys Met
160                 165                 170                 175
act tta agg tgt atg caa gct cta gtt cga gtc caa tct cgt gtg ctt      757
Thr Leu Arg Cys Met Gln Ala Leu Val Arg Val Gln Ser Arg Val Leu
                180                 185                 190
gac caa cgg aaa cgc ttg tct cac gat ggt agt cgc aaa tct gcc ttc      805
Asp Gln Arg Lys Arg Leu Ser His Asp Gly Ser Arg Lys Ser Ala Phe
            195                 200                 205
agc gac act caa agt gtg ctc gaa tct cgt tat ctt caa gaa ata tca      853
Ser Asp Thr Gln Ser Val Leu Glu Ser Arg Tyr Leu Gln Glu Ile Ser
        210                 215                 220
gac aga aga tcc atg tca aga gaa gga agt agc att gcg gaa gat tgg      901
Asp Arg Arg Ser Met Ser Arg Glu Gly Ser Ser Ile Ala Glu Asp Trp
    225                 230                 235
gat gat cga cca cac acg att gag gaa gtg aaa gca atg ttg caa caa      949
Asp Asp Arg Pro His Thr Ile Glu Glu Val Lys Ala Met Leu Gln Gln
240                 245                 250                 255
aga cga gac aat gcg ttg aga cgt gag agt aac aat agt ata tca caa      997
Arg Arg Asp Asn Ala Leu Arg Arg Glu Ser Asn Asn Ser Ile Ser Gln
                260                 265                 270
gct ttc tct cac cag gtt cgg aga aca aga ggt agt tat tct aca gga     1045
```

```
Ala Phe Ser His Gln Val Arg Arg Thr Arg Gly Ser Tyr Ser Thr Gly
            275                 280                 285
gac gag tat gaa gaa gag aga ccg aaa tgg tta gac aga tgg atg gct      1093
Asp Glu Tyr Glu Glu Glu Arg Pro Lys Trp Leu Asp Arg Trp Met Ala
        290                 295                 300
tct aaa ccg tgg gat aaa cga gct tca acg gat caa aga gta cca ccg      1141
Ser Lys Pro Trp Asp Lys Arg Ala Ser Thr Asp Gln Arg Val Pro Pro
    305                 310                 315
gtt tac aaa acc gtg gag atc gat act tct caa ccg tat tta acc cgc      1189
Val Tyr Lys Thr Val Glu Ile Asp Thr Ser Gln Pro Tyr Leu Thr Arg
320                 325                 330                 335
ggt aac tcg aga acc ggt gca agt cca agc cgt agc caa agg cct agt      1237
Gly Asn Ser Arg Thr Gly Ala Ser Pro Ser Arg Ser Gln Arg Pro Ser
                340                 345                 350
tca cca tca agg acc agc cac cat tac caa caa cac aat ttc tca tca      1285
Ser Pro Ser Arg Thr Ser His His Tyr Gln Gln His Asn Phe Ser Ser
            355                 360                 365
gct aca cca tct ccg gct aaa tct aga ccg ata caa att cga tcc gct      1333
Ala Thr Pro Ser Pro Ala Lys Ser Arg Pro Ile Gln Ile Arg Ser Ala
        370                 375                 380
agt ccg cgg atc caa aga gat gat cgg tca gcg tac aac tac aca tca      1381
Ser Pro Arg Ile Gln Arg Asp Asp Arg Ser Ala Tyr Asn Tyr Thr Ser
    385                 390                 395
aac aca cct agc ttg aga tct aac tat agt ttc aca gca aga agt ggt      1429
Asn Thr Pro Ser Leu Arg Ser Asn Tyr Ser Phe Thr Ala Arg Ser Gly
400                 405                 410                 415
tat agt gtt tgt acc act act act act gct aca aat gct gca ttg cca      1477
Tyr Ser Val Cys Thr Thr Thr Thr Thr Ala Thr Asn Ala Ala Leu Pro
                420                 425                 430
aac tac atg gcg att acg gaa tct gct aag gct aga atc cgg tct cag      1525
Asn Tyr Met Ala Ile Thr Glu Ser Ala Lys Ala Arg Ile Arg Ser Gln
            435                 440                 445
agt gca cca agg caa cgg cct tca aca ccc gag aaa gaa cgt atc agt      1573
Ser Ala Pro Arg Gln Arg Pro Ser Thr Pro Glu Lys Glu Arg Ile Ser
        450                 455                 460
tca gct aga aaa cgg ctt tcg ttt cca gtt cca ccg ctg ccg cag caa      1621
Ser Ala Arg Lys Arg Leu Ser Phe Pro Val Pro Pro Leu Pro Gln Gln
    465                 470                 475
atg gat ggt cag agt tta agg agt cca agt ttc aag agt ata ggt ggc      1669
Met Asp Gly Gln Ser Leu Arg Ser Pro Ser Phe Lys Ser Ile Gly Gly
480                 485                 490                 495
tca caa ttg ggt gca ttg gaa caa caa tca aat tac tct tct tgt tgt      1717
Ser Gln Leu Gly Ala Leu Glu Gln Gln Ser Asn Tyr Ser Ser Cys Cys
                500                 505                 510
act gag tct ctt ggt ggt ggt gga gag ata tca cct gct tct act agc      1765
```

Thr Glu Ser Leu Gly Gly Gly Gly Glu Ile Ser Pro Ala Ser Thr Ser
515 520 525

gat tat agg cga tgg tta aga tga ttccataatc caaccaaatc aaccggtcat 1819
Asp Tyr Arg Arg Trp Leu Arg
530

gattttttc ctccctttt tttttttgtt atatataaat aaaaacaatt tgacaaaaag 1879
aaccataact ttttcagttt ttattgatga tctttgagat tggtgtgtaa ttttgtttgt 1939
gtttatgttt gtgtgtgtga atttgtagct tataattgga agtgtttact aactaatctc 1999
tatataaata tgtatgctca tatttttatc tat 2032

<210> 30
<211> 534
<212> PRT
<213> Arabidopsis thaliana

<400> 30
Met Gly Lys Lys Ser Gly Ser Ser Ser Ser Trp Leu Thr Ala Val Lys
1 5 10 15

Arg Ala Phe Arg Ser Pro Thr Lys Lys Glu His Asn Asn Asn Ala His
20 25 30

Gly Asn Glu Val Asp Glu Asp Glu Asp Lys Lys Lys Glu Lys Arg Arg
35 40 45

Trp Leu Phe Arg Lys Ser Thr Asn His Asp Ser Pro Val Lys Thr Ser
50 55 60

Gly Val Gly Lys Asp Ala Pro Ala Gln Lys Ser Thr Glu Thr Thr Thr
65 70 75 80

Ile Ile Asn Pro Thr Val Leu Ser Ser Val Thr Glu Gln Arg Tyr Asp
85 90 95

Ala Ser Thr Pro Pro Ala Thr Val Ser Ala Ala Ser Glu Thr His Pro
100 105 110

Pro Ser Thr Thr Lys Glu Leu Pro Asn Leu Thr Arg Arg Thr Tyr Thr
115 120 125

Ala Arg Glu Asp Tyr Ala Ala Val Val Ile Gln Thr Gly Phe Arg Gly
130 135 140

Tyr Leu Ala Arg Arg Ala Leu Arg Ala Leu Lys Gly Leu Val Lys Leu
145 150 155 160

```
Gln Ala Leu Val Arg Gly His Asn Val Arg Lys Gln Ala Lys Met Thr
                165                 170                 175

Leu Arg Cys Met Gln Ala Leu Val Arg Val Gln Ser Arg Val Leu Asp
            180                 185                 190

Gln Arg Lys Arg Leu Ser His Asp Gly Ser Arg Lys Ser Ala Phe Ser
        195                 200                 205

Asp Thr Gln Ser Val Leu Glu Ser Arg Tyr Leu Gln Glu Ile Ser Asp
    210                 215                 220

Arg Arg Ser Met Ser Arg Glu Gly Ser Ser Ile Ala Glu Asp Trp Asp
225                 230                 235                 240

Asp Arg Pro His Thr Ile Glu Glu Val Lys Ala Met Leu Gln Gln Arg
                245                 250                 255

Arg Asp Asn Ala Leu Arg Arg Glu Ser Asn Asn Ser Ile Ser Gln Ala
            260                 265                 270

Phe Ser His Gln Val Arg Arg Thr Arg Gly Ser Tyr Ser Thr Gly Asp
        275                 280                 285

Glu Tyr Glu Glu Glu Arg Pro Lys Trp Leu Asp Arg Trp Met Ala Ser
    290                 295                 300

Lys Pro Trp Asp Lys Arg Ala Ser Thr Asp Gln Arg Val Pro Pro Val
305                 310                 315                 320

Tyr Lys Thr Val Glu Ile Asp Thr Ser Gln Pro Tyr Leu Thr Arg Gly
                325                 330                 335

Asn Ser Arg Thr Gly Ala Ser Pro Ser Arg Ser Gln Arg Pro Ser Ser
            340                 345                 350

Pro Ser Arg Thr Ser His His Tyr Gln Gln His Asn Phe Ser Ser Ala
        355                 360                 365

Thr Pro Ser Pro Ala Lys Ser Arg Pro Ile Gln Ile Arg Ser Ala Ser
    370                 375                 380

Pro Arg Ile Gln Arg Asp Asp Arg Ser Ala Tyr Asn Tyr Thr Ser Asn
385                 390                 395                 400
```

Thr Pro Ser Leu Arg Ser Asn Tyr Ser Phe Thr Ala Arg Ser Gly Tyr
405 410 415

Ser Val Cys Thr Thr Thr Thr Thr Ala Thr Asn Ala Ala Leu Pro Asn
420 425 430

Tyr Met Ala Ile Thr Glu Ser Ala Lys Ala Arg Ile Arg Ser Gln Ser
435 440 445

Ala Pro Arg Gln Arg Pro Ser Thr Pro Glu Lys Glu Arg Ile Ser Ser
450 455 460

Ala Arg Lys Arg Leu Ser Phe Pro Val Pro Pro Leu Pro Gln Gln Met
465 470 475 480

Asp Gly Gln Ser Leu Arg Ser Pro Ser Phe Lys Ser Ile Gly Gly Ser
485 490 495

Gln Leu Gly Ala Leu Glu Gln Gln Ser Asn Tyr Ser Ser Cys Cys Thr
500 505 510

Glu Ser Leu Gly Gly Gly Gly Glu Ile Ser Pro Ala Ser Thr Ser Asp
515 520 525

Tyr Arg Arg Trp Leu Arg
530

<210> 31
<211> 1201
<212> DNA
<213> Arabidopsis thaliana

<220>
<221> promoter
<222> (1)..(1201)
<223> transcription regulating sequence from gene At2g36640

<400> 31
tctttttgtt gtggaggata catgtcttgt aagattattc ttttccctta actttttgtt 60
gagtttttac aataagataa ttaattgaga tcggttgctt tttgaccatt gcttttgctt 120
gcttactcgt ttgggcatcg gattctccac ttacacgaca tacgtacgtg tgtactcata 180
ctcacacgta cctacacatt atgaaagcca tgatgctagc tagaagttcc atatattgtt 240
atatggttga tcaaatcgaa actcaaacta agtgtttctg atttgatctg atcttaacta 300
tatacttgag atcttaaggt actcacgtta gtgtatatca tatttacgac aaatcatgta 360

gaaaacgtga ccgatttgga tgattcggca tcaaatgcgc gtcaacaaag ttgtcggaga 420
aataataaaa aaaggacgta cctaaataag tataaaagta atctatgtga gaacatgatt 480
aacgtgattt atttattttt gataaaaaaa aaagttaagg tgactaatag agatgtttaa 540
caaaaaagag agttgtctat tcggttaaga caatagactc atgtgctaac ctgaattagc 600
tggcaaatta gagaaaattt ggaaataaat ccaaactatt gcaatgaact tttttgtata 660
ccctcccaag aaccgtcaaa aactcaaaat tatattgata accgacttga tcaataaacg 720
gctctatcca atgattaact tgcgtcctta tttttctaaa atgcttgttt ccaagttttt 780
gatgttgagg caataacttc caataaactg tgtggcaata actaagtaat ctttcatatc 840
tctaaattgc ttgtttccaa gttatgtgcc tcagtaagtt tgaaatggcg aatgtgatct 900
tcacctaaca ttttcctcga ccaacacaaa ttatgatgtc aaaagatttg atacgaccga 960
ccatcgatgc ttgcttgctt atgtcttagc tcttaagtca tgccatgaac actcaagaat 1020
catgatccat gcatgaaaag tccacgtgtc ataccacgtc cgtagaagat aaagctaagt 1080
aaattgtacg tggcagctgt tgcgtccctc tatctaccac gtagcgtctc aacagaaaca 1140
gagcattact ataagaagta acaaaagcag ctcaactgat agcacaagtt aaagaattct 1200
a 1201

<210> 32
<211> 1216
<212> DNA
<213> Arabidopsis thaliana

<220>
<221> promoter
<222> (1)..(1216)
<223> transcription regulating sequence from gene At2g36640

<400> 32
acagtcctct ttttgttgtg gaggatacat gtcttgtaag attattcttt tcccttaact 60
ttttattgag tttttacaat aagataatta attgagatcg gttgcttttt gaccattgct 120
tttgcttgct tactcgtttg ggcatcggat tctccactta cacgacatac gtacgtgtgt 180
actcatactc acacgtacct acacattatg aaagccatga tgctagctag aagttccata 240
tattgttata tggttgatca aatcgaaact caaactaagt gtttctgatt tgatctgatc 300
ttaactatat acttgagatc ttaaggtact cacgttagtg tatatcatat ttacgacaaa 360
tcatgtagaa aacgtgaccg atttggatga ttcggcatca aatgcgcgtc aacaaagttg 420
tcggagaaat aataaaaaaa ggacgtacct aattaagtat aaaagtaatc tatgtgagaa 480
catgattaac gtgatttatt tatttttgat aaaaaaaaaa agttaaggtg actaatagag 540
atgtttaaca aaaaagagag ttgtctattc ggttaagaca atagactcat gtgctaacct 600
gaattagctg gcaaattaga gaaaatttgg aaataaatcc aaactattgc aatgaacttt 660
tttgtatacc ctcccaagaa ccgtcaaaaa ctcaaaatta tattgataac cgacttgatc 720
aataaacggc tctatccaat gattaacttg cgtccttatt tttctaaaat gcttgtttcc 780
aagtttttga tgttgaggca ataacttcca ataaactgtg tggcaataac taagtaatct 840
ttcatatctc taaattgctt gtttccaagt tatgtgcctc agtaagtttg aaatggcgaa 900
tgtgatcttc acctaacatt ttcctcgacc aacacaaatt atgatatcaa aagatttgat 960
acgaccgacc atcgatgctt gcttgcttat gtcttagctc ttaagtcatg ccatgaacac 1020
tcaagaatca tgatccatgc atgaaaagtc cacgtgtcat accacgtccg tagaagataa 1080

```
agctaagtaa attgtacgtg gcagctgttg cgtccctcta tctaccacgt agcgtctcaa   1140
cagaaacaga gcattactat aagaagtaac aaaagcagct caactgatag cacaagttaa   1200
agaattctaa aatcgt                                                   1216

<210>  33
<211>  1441
<212>  DNA
<213>  Arabidopsis thaliana

<220>
<221>  promoter
<222>  (1)..(1441)
<223>  transcription regulating sequence from gene At2g36640

<400>  33
taatctctta tgttgctttc aactccaaaa atctttcttt ctgactaaaa aagtttcctt     60
tcggttatgt gaccgaaaca gagactcgag aattgattga tcaatcttgt gagggtttga    120
tcaaatttaa atggtttata cgaatcattg gtctcatgaa aaaaatagtt ttatacagtc    180
ctctttttgt tgtggaggat acatgtcttg taagattatt cttttccctt aactttttat    240
tgagttttta caataagata attaattgag atcggttgct ttttgaccat tgcttttgct    300
tgcttactcg tttgggcatc ggattctcca cttacacgac atacgtacgt gtgtactcat    360
actcacacgt acctacacat tatgaaagcc atgatgctag ctagaagttc catatattgt    420
tatatggttg atcaaatcga aactcaaact aagtgtttct gatttgatct gatcttaact    480
atatacttga gatcttaagg tactcacgtt agtgtatatc atatttacga caaatcatgt    540
agaaaacgtg accgatttgg atgattcggc atcaaatgcg cgtcaacaaa gttgtcggag    600
aaataataaa aaaaggacgt acctaattaa gtataaaagt aatctatgtg agaacatgat    660
taacgtgatt tatttatttt tgataaaaaa aaaaagttaa ggtgactaat agagatgttt    720
aacaaaaaag agagttgtct attcggttaa gacaatagac tcatgtgcta acctgaatta    780
gctggcaaat tagagaaaat ttggaaataa atccaaacta ttgcaatgaa ctttttgta    840
taccctccca agaaccgtca aaaactcaaa attatattga taaccgactt gatcaataaa    900
cggctctatc caatgattaa cttgcgtcct tatttttcta aaatgcttgt ttccaagttt    960
ttgatgttga ggcaataact tccaataaac tgtgtggcaa taactaagta atctttcata   1020
tctctaaatt gcttgtttcc aagttatgtg cctcagtaag tttgaaatgg cgaatgtgat   1080
cttcacctaa cattttcctc gaccaacaca aattatgata tcaaaagatt tgatacgacc   1140
gaccatcgat gcttgcttgc ttatgtctta gctcttaagt catgccatga acactcaaga   1200
atcatgatcc atgcatgaaa agtccacgtg tcataccacg tccgtagaag ataaagctaa   1260
gtaaattgta cgtggcagct gttgcgtccc tctatctacc acgtagcgtc tcaacagaaa   1320
cagagcatta ctataagaag taacaaaagc agctcaactg atagcacaag ttaaagaatt   1380
ctaaaatcgt aaagttctaa agcggttttt catcaatttt caagcaatcg agaaaaaagc   1440
a                                                                   1441

<210>  34
<211>  1572
<212>  DNA
<213>  Arabidopsis thaliana
```

<220>
<221> CDS
<222> (61)..(1407)
<223> encoding late embryogenesis abundant protein (ECP63) / LEA
protein

<400> 34

```
taaaatcgta aagttctaaa gcggtttttc atcaattttc aagcaatcga gaaaaaagca      60
atg gcg tca gac aaa caa aag gcg gag aga gcc gag gtt gcg gcg agg       108
Met Ala Ser Asp Lys Gln Lys Ala Glu Arg Ala Glu Val Ala Ala Arg
1               5                   10                  15
cta gcg gct gag gac ttg cat gac att aac aaa tcc ggt ggt gct gat       156
Leu Ala Ala Glu Asp Leu His Asp Ile Asn Lys Ser Gly Gly Ala Asp
            20                  25                  30
gtc aca atg tat aag gtg acg gag aga aca act gaa cat cca ccg gag       204
Val Thr Met Tyr Lys Val Thr Glu Arg Thr Thr Glu His Pro Pro Glu
        35                  40                  45
caa gat agg ccc ggt gtg ata gga tca gtg ttc agg gct gtc caa gga       252
Gln Asp Arg Pro Gly Val Ile Gly Ser Val Phe Arg Ala Val Gln Gly
    50                  55                  60
acg tat gag cat gcg aga gac gct gta gtt gga aaa acc cac gaa gcg       300
Thr Tyr Glu His Ala Arg Asp Ala Val Val Gly Lys Thr His Glu Ala
65                  70                  75                  80
gct gag tct acc aaa gaa gga gct cag ata gct tca gag aaa gcg gtt       348
Ala Glu Ser Thr Lys Glu Gly Ala Gln Ile Ala Ser Glu Lys Ala Val
                85                  90                  95
gga gca aag gac gca acc gtc gag aaa gct aag gaa acc gct gat tat       396
Gly Ala Lys Asp Ala Thr Val Glu Lys Ala Lys Glu Thr Ala Asp Tyr
            100                 105                 110
act gcg gag aag gtg ggt gag tat aaa gac tat acg gtt gat aaa gct       444
Thr Ala Glu Lys Val Gly Glu Tyr Lys Asp Tyr Thr Val Asp Lys Ala
        115                 120                 125
aaa gag gct aag gac aca act gca gag aag gcg aag gag act gct aat       492
Lys Glu Ala Lys Asp Thr Thr Ala Glu Lys Ala Lys Glu Thr Ala Asn
    130                 135                 140
tat act gcg gat aag gcg gtg gaa gca aag gat aag acg gcg gag aag       540
Tyr Thr Ala Asp Lys Ala Val Glu Ala Lys Asp Lys Thr Ala Glu Lys
145                 150                 155                 160
att ggt gag tac aaa gac tat gcg gtg gat aag gca gta gaa gct aaa       588
Ile Gly Glu Tyr Lys Asp Tyr Ala Val Asp Lys Ala Val Glu Ala Lys
                165                 170                 175
gat aag aca gcg gag aag gcg aag gag act gcg aat tat acg gcg gat       636
Asp Lys Thr Ala Glu Lys Ala Lys Glu Thr Ala Asn Tyr Thr Ala Asp
            180                 185                 190
```

```
aag gct aaa gag gct aag gac aag acg gct gag aag gtt ggt gag tat      684
Lys Ala Lys Glu Ala Lys Asp Lys Thr Ala Glu Lys Val Gly Glu Tyr
        195                 200                 205
aag gat tac acg gtg gac aag gcc gtg gaa gct agg gat tac aca gcg      732
Lys Asp Tyr Thr Val Asp Lys Ala Val Glu Ala Arg Asp Tyr Thr Ala
    210                 215                 220
gag aag gct att gaa gca aag gat aag aca gct gag aag act gga gag      780
Glu Lys Ala Ile Glu Ala Lys Asp Lys Thr Ala Glu Lys Thr Gly Glu
225                 230                 235                 240
tat aag gac tat acg gtg gag aag gcg acg gag ggg aaa gat gtt acg      828
Tyr Lys Asp Tyr Thr Val Glu Lys Ala Thr Glu Gly Lys Asp Val Thr
                245                 250                 255
gtg agt aag cta gga gag ctg aag gat agt gcc gtt gag aca gcg aag      876
Val Ser Lys Leu Gly Glu Leu Lys Asp Ser Ala Val Glu Thr Ala Lys
            260                 265                 270
aga gct atg ggt ttc ttg tcg ggg aag aca gag gag gcc aaa gga aaa      924
Arg Ala Met Gly Phe Leu Ser Gly Lys Thr Glu Glu Ala Lys Gly Lys
        275                 280                 285
gct gtg gag acc aaa gat act gcc aag gaa aac atg gag aaa gct gga      972
Ala Val Glu Thr Lys Asp Thr Ala Lys Glu Asn Met Glu Lys Ala Gly
    290                 295                 300
gaa gta aca aga caa aag atg gag gaa atg aga ttg gaa ggt aaa gag     1020
Glu Val Thr Arg Gln Lys Met Glu Glu Met Arg Leu Glu Gly Lys Glu
305                 310                 315                 320
ctc aaa gaa gaa gct gga gca aaa gcc caa gag gca tct caa aag act     1068
Leu Lys Glu Glu Ala Gly Ala Lys Ala Gln Glu Ala Ser Gln Lys Thr
                325                 330                 335
agg gag agt act gag tcg gga gct caa aaa gcc gaa gag acc aaa gat     1116
Arg Glu Ser Thr Glu Ser Gly Ala Gln Lys Ala Glu Glu Thr Lys Asp
            340                 345                 350
tct gct gcc gtg agg gga aat gaa gcg aaa ggg act att ttt ggt gca     1164
Ser Ala Ala Val Arg Gly Asn Glu Ala Lys Gly Thr Ile Phe Gly Ala
        355                 360                 365
tta ggg aat gta acg gaa gca ata aag agc aaa ctg aca atg cca tca     1212
Leu Gly Asn Val Thr Glu Ala Ile Lys Ser Lys Leu Thr Met Pro Ser
    370                 375                 380
gac att gtg gag gaa aca cgc gcg gca cgt gag cat gga ggg acg ggt     1260
Asp Ile Val Glu Glu Thr Arg Ala Ala Arg Glu His Gly Gly Thr Gly
385                 390                 395                 400
agg act gtg gtt gaa gtc aag gtc gag gat tca aag ccg ggt aag gtg     1308
Arg Thr Val Val Glu Val Lys Val Glu Asp Ser Lys Pro Gly Lys Val
                405                 410                 415
gcg act tca ctg aag gcg tcg gat caa atg acc ggt caa aca ttc aac     1356
Ala Thr Ser Leu Lys Ala Ser Asp Gln Met Thr Gly Gln Thr Phe Asn
            420                 425                 430
```

```
gac gtt gga cgg atg gat gat gat gct cgg aaa gat aag gga aag ctg     1404
Asp Val Gly Arg Met Asp Asp Asp Ala Arg Lys Asp Lys Gly Lys Leu
        435                 440                 445
tga gaatactaga aaaatgacaa tgtttttgg gccttttgtt ctggatgaat           1457
atctctgttg tttttgggc cttttgtttt ggatacgtct gttatagcag ataacgtttt    1517
ctgatagttt ctatgtttgt attcttgttt tggaatagga aagctttctt cagtt        1572

<210>  35
<211>  448
<212>  PRT
<213>  Arabidopsis thaliana

<400>  35
Met Ala Ser Asp Lys Gln Lys Ala Glu Arg Ala Glu Val Ala Ala Arg
1               5                   10                  15

Leu Ala Ala Glu Asp Leu His Asp Ile Asn Lys Ser Gly Gly Ala Asp
            20                  25                  30

Val Thr Met Tyr Lys Val Thr Glu Arg Thr Thr Glu His Pro Pro Glu
        35                  40                  45

Gln Asp Arg Pro Gly Val Ile Gly Ser Val Phe Arg Ala Val Gln Gly
    50                  55                  60

Thr Tyr Glu His Ala Arg Asp Ala Val Val Gly Lys Thr His Glu Ala
65                  70                  75                  80

Ala Glu Ser Thr Lys Glu Gly Ala Gln Ile Ala Ser Glu Lys Ala Val
                85                  90                  95

Gly Ala Lys Asp Ala Thr Val Glu Lys Ala Lys Glu Thr Ala Asp Tyr
            100                 105                 110

Thr Ala Glu Lys Val Gly Glu Tyr Lys Asp Tyr Thr Val Asp Lys Ala
        115                 120                 125

Lys Glu Ala Lys Asp Thr Thr Ala Glu Lys Ala Lys Glu Thr Ala Asn
    130                 135                 140

Tyr Thr Ala Asp Lys Ala Val Glu Ala Lys Asp Lys Thr Ala Glu Lys
145                 150                 155                 160

Ile Gly Glu Tyr Lys Asp Tyr Ala Val Asp Lys Ala Val Glu Ala Lys
                165                 170                 175
```

```
Asp Lys Thr Ala Glu Lys Ala Lys Glu Thr Ala Asn Tyr Thr Ala Asp
            180                 185                 190

Lys Ala Lys Glu Ala Lys Asp Lys Thr Ala Glu Lys Val Gly Glu Tyr
        195                 200                 205

Lys Asp Tyr Thr Val Asp Lys Ala Val Glu Ala Arg Asp Tyr Thr Ala
    210                 215                 220

Glu Lys Ala Ile Glu Ala Lys Asp Lys Thr Ala Glu Lys Thr Gly Glu
225                 230                 235                 240

Tyr Lys Asp Tyr Thr Val Glu Lys Ala Thr Glu Gly Lys Asp Val Thr
                245                 250                 255

Val Ser Lys Leu Gly Glu Leu Lys Asp Ser Ala Val Glu Thr Ala Lys
            260                 265                 270

Arg Ala Met Gly Phe Leu Ser Gly Lys Thr Glu Glu Ala Lys Gly Lys
        275                 280                 285

Ala Val Glu Thr Lys Asp Thr Ala Lys Glu Asn Met Glu Lys Ala Gly
    290                 295                 300

Glu Val Thr Arg Gln Lys Met Glu Glu Met Arg Leu Glu Gly Lys Glu
305                 310                 315                 320

Leu Lys Glu Glu Ala Gly Ala Lys Ala Gln Glu Ala Ser Gln Lys Thr
                325                 330                 335

Arg Glu Ser Thr Glu Ser Gly Ala Gln Lys Ala Glu Glu Thr Lys Asp
            340                 345                 350

Ser Ala Ala Val Arg Gly Asn Glu Ala Lys Gly Thr Ile Phe Gly Ala
        355                 360                 365

Leu Gly Asn Val Thr Glu Ala Ile Lys Ser Lys Leu Thr Met Pro Ser
    370                 375                 380

Asp Ile Val Glu Glu Thr Arg Ala Ala Arg Glu His Gly Gly Thr Gly
385                 390                 395                 400

Arg Thr Val Val Glu Val Lys Val Glu Asp Ser Lys Pro Gly Lys Val
                405                 410                 415
```

```
Ala Thr Ser Leu Lys Ala Ser Asp Gln Met Thr Gly Gln Thr Phe Asn
            420                 425                 430

Asp Val Gly Arg Met Asp Asp Asp Ala Arg Lys Asp Lys Gly Lys Leu
        435                 440                 445

<210>  36
<211>  1587
<212>  DNA
<213>  Arabidopsis thaliana

<220>
<221>  promoter
<222>  (1)..(1587)
<223>  transcription regulating sequence from gene At2g34200

<400>  36
tttatctaat ggataaccac cacccattag gatcaccata aaggtcatat tttggagcat      60
tccaatatca taacacttta ctcttaaggg tctaatttga gttggtggca acatgtcata     120
tagacttcag agctgcttcc aaaatgggtt tccaatctac accatttatt acagattctt     180
ttttgctttt cgtatttgga tagtttacga gtttagtttt tacttttttt agattcagga     240
agtctttaat gggtttcacc ggtttagtag gttcaccttt atcggccaac aagaattaca     300
tttctctcta tttatgacga ctgccaccaa taattcaaaa caagtttaca tattaagttt     360
tcttaaacta ctcctatata ttaagtctaa gattatatta atcttattta tataatttaa     420
ttcaactaaa tttttagttt gtttatttac atgatatacc atggtcctgt tattttagct     480
aaagtatata ttaaatttat tactttttta tttcaaagat tttttctatt gtttttttatt     540
ttgttttctt ttcatttaag cttatattat tattattata cacacacatt taaggtttat     600
atttatattt cttgcaatgc aaacatgtgt caatattagt tggtttaaga tacaaaaata     660
ttaaggacta aatggttgag ataatactac tttgatatat atatatataa ctatattcta     720
taaacttaat ttatttgaga gggtaaggat tagatgacca agaaattggt aaaaattgga     780
aaaataatta tagacattat tattttacaa gatattctca aatggctaga gatttgtgaa     840
gttctgtttt tttttttcctc aaagttcaag tgtaatattt ttcgatgaca atgattattc     900
caaatttatt tttttgttaa tcgtaaacag tgaattttgc ataattggtt tgaattagag     960
ttgataatag ttgttctcat catcaccaaa cattcttctg cttaccaaaa gtaagggact    1020
ttcattagct aaaacaaaaa tcatacacaa atgggttttg gttgtactgt ccaaagtgga    1080
acacacagtt ttatagaaag acaaaactga aacggcaagt gagagttaaa gtaaaaggaa    1140
attgcaatcg agagccagct gagattttta gatattttgc aggtaactct taacgccgag    1200
catacatacg tttctcaaag acataaagct gattgtgttt ctccatttgc tgaaagatct    1260
gagccataaa gctgaaatat aaaccttttt tgtgttttgt tgttccccag attctctgac    1320
gctgcttaaa ccttaaagtt cgtagctttt tcttgctagt gggtcttttg aggtggatct    1380
ggattctggg taaggttttg ttttttatat ctttaccagt aaattgaagt tcttatggtt    1440
tcaagaaatt ttacagggtt ttcaattatt ggaatgttgt ttctgttggc tctttatctg    1500
aatacaatct tactaagagg ttacggatct tggactttgt aggaacttga gcaaaagcca    1560
tcctcttttg ttgtatatat gaattaa                                        1587
```

<210> 37
<211> 1602
<212> DNA
<213> Arabidopsis thaliana

<220>
<221> promoter
<222> (1)..(1602)
<223> transcription regulating sequence from gene At2g34200

<400> 37
```
tttttcttt atctaatgga taaccaccac ccattaggat caccataaag atcatatttt     60
ggagcattcc aatatcataa cactttactc ttaagggtct aatttgagtt ggtggcaaca    120
tgtcatatag acttcagagc tgcttccaaa atgggtttcc aatctacacc atttattaca    180
gattcatttt tgcttttcgt atttggttag tttaggagtt tagtttttac ttttttaga     240
ttcaggaagt ctttaatggg tttcaccggt ttagtaggtt cacctttatc ggccaacaag    300
aattacattt ctctctattt atgacgactg ccaccaataa ttcaaaacaa gtttacatat    360
taagttttct taaactactc ctatatatta agtctaagat tatattaatc ttatttatat    420
aatttaattc aactaaattt ttagtttgtt tatttacatg atataccatg gtcctgttat    480
tttagctaaa gtatatatta aatttattac ttttttattt caaagatttt ttctattgtt    540
ttttattttg ttttcttttc atttaagctt atattattat tattatacac acacatttaa    600
ggtttatatt tatatttctt gcaatgcaaa catgtgtcaa tattagttga tttaagatac    660
aaaaatatta aggactaaat ggttgagata atactacttt gatatatata tatatatata    720
tataactata ttctataaac ttaatttatt tgagagggta aggattagat gaccaagaaa    780
ttggtaaaaa ttggaaaaat aattatagac attattattt tacaagatat tctcaaatgg    840
ctagagattt gtgaagttct gtttttttt tcctcaaagt tcaagtgtaa tatttttcga    900
tgacaatgat tattccaaat ttattttttt gttaatcgta aacagtgaat tttgcataat    960
tggtttgaat tagagttgat aatagttgtt ctcatcatca ccaaacattc ttctgcttac   1020
caaaagtaag ggactttcat tagcaaaaac aaaaatcata cacaaatggg ttttggttgt   1080
actgtccaaa atggaacaca cagttttata gaaagacaaa actgaaacgg caagtgagag   1140
ttaaagtaaa aggaaattgc aatcgagagc cagctgagat ttttagatat tttgcaggta   1200
actcttaacg ccgagcatac atacgtttct caaagacata aagctgattg tgtttctcca   1260
tttgctgaaa gatctgagcc ataaagctga aatagaaacc ttttttgtgt tttgttgttc   1320
cccagattct ctgacgctgc ttaaacctta aagttcgtag ctttttcttg ctagtgggtc   1380
ttttgaggtg gatctggatt ctgggtaagg ttttgttttt tatatcttta ccagtaaatt   1440
gaagttctta tggtttcaag aaattttaca gggttttcaa ttattggaat gttgtttctg   1500
ttggctcttt atctgaatac aatcttacta agaggttacg gatcttggac tttgtaggaa   1560
cttgagcaaa agccatcctc ttttgttgta tatatgaatt aa                      1602
```

<210> 38
<211> 1130
<212> DNA
<213> Arabidopsis thaliana

<220>
<221> promoter
<222> (1)..(1130)
<223> transcription regulating sequence from gene At2g34200

<400> 38
```
tttatctaat ggataaccac cacccattag gatcaccata aaggtcatat tttggagcat     60
tccaatatca taacacttta ctcttaaggg tctaatttga gttggtggca acatgtcata    120
tagacttcag agctgcttcc aaaatgggtt tccaatctac accatttatt acagattctt    180
ttttgctttt cgtatttgga tagtttacga gtttagtttt tacttttttt agattcagga    240
agtctttaat gggtttcacc ggtttagtag gttcaccttt atcggccaac aagaattaca    300
tttctctcta tttatgacga ctgccaccaa taattcaaaa caagtttaca tattaagttt    360
tcttaaacta ctcctatata ttaagtctaa gattatatta atcttattta tataatttaa    420
ttcaactaaa tttttagttt gtttatttac atgatatacc atggtcctgt tattttagct    480
aaagtatata ttaaatttat tacttttta tttcaaagat tttttctatt gtttttttatt    540
ttgtttttctt ttcatttaag cttatattat tattattata cacacacatt taaggtttat    600
atttatattt cttgcaatgc aaacatgtgt caatattagt tggtttaaga tacaaaaata    660
ttaaggacta aatggttgag ataatactac tttgatatat atatatataa ctatattcta    720
taaacttaat ttatttgaga gggtaaggat tagatgacca agaaattggt aaaaattgga    780
aaaataatta tagacattat tattttacaa gatattctca aatggctaga gatttgtgaa    840
gttctgtttt ttttttcctc aaagttcaag tgtaatattt ttcgatgaca atgattattc    900
caaatttatt tttttgttaa tcgtaaacag tgaattttgc ataattggtt tgaattagag    960
ttgataatag ttgttctcat catcaccaaa cattcttctg cttaccaaaa gtaagggact   1020
ttcattagct aaaacaaaaa tcatacacaa atgggttttg gttgtactgt ccaaagtgga   1080
acacacagtt ttatagaaag acaaaactga aacggcaagt gagagttaaa              1130
```

<210> 39
<211> 1145
<212> DNA
<213> Arabidopsis thaliana

<220>
<221> promoter
<222> (1)..(1145)
<223> transcription regulating sequence from gene At2g34200

<400> 39
```
tttttcttt atctaatgga taaccaccac ccattaggat caccataaag atcatatttt     60
ggagcattcc aatatcataa cactttactc ttaagggtct aatttgagtt ggtggcaaca    120
tgtcatatag acttcagagc tgcttccaaa atgggtttcc aatctacacc atttattaca    180
gattcatttt tgcttttcgt atttggttag tttaggagtt tagtttttac ttttttttaga    240
ttcaggaagt ctttaatggg tttcaccggt ttagtaggtt cacctttatc ggccaacaag    300
aattacattt ctctctattt atgacgactg ccaccaataa ttcaaaacaa gtttacatat    360
taagttttct taaactactc ctatatatta agtctaagat tatattaatc ttatttatat    420
aatttaattc aactaaattt ttagtttgtt tatttacatg atataccatg gtcctgttat    480
```

```
tttagctaaa gtatatatta aatttattac ttttttattt caaagatttt ttctattgtt     540
ttttattttg ttttcttttc atttaagctt atattattat tattatacac acacatttaa     600
ggtttatatt tatatttctt gcaatgcaaa catgtgtcaa tattagttga tttaagatac     660
aaaaatatta aggactaaat ggttgagata atactacttt gatatatata tatatatata     720
tataactata ttctataaac ttaatttatt tgagagggta aggattagat gaccaagaaa     780
ttggtaaaaa ttggaaaaat aattatagac attattattt tacaagatat tctcaaatgg     840
ctagagattt gtgaagttct gttttttttt tcctcaaagt tcaagtgtaa tatttttcga     900
tgacaatgat tattccaaat ttattttttt gttaatcgta aacagtgaat tttgcataat     960
tggtttgaat tagagttgat aatagttgtt ctcatcatca ccaaacattc ttctgcttac    1020
caaaagtaag ggactttcat tagcaaaaac aaaaatcata cacaaatggg ttttggttgt    1080
actgtccaaa atggaacaca cagttttata gaaagacaaa actgaaacgg caagtgagag    1140
ttaaa                                                                1145
```

<210> 40
<211> 1169
<212> DNA
<213> Arabidopsis thaliana

<220>
<221> promoter
<222> (1)..(1169)
<223> transcription regulating sequence from gene At2g34200

<400> 40

```
tttatctaat ggataaccac cacccattag gatcaccata aagatcatat tttggagcat      60
tccaatatca taacacttta ctcttaaggg tctaatttga gttggtggca acatgtcata     120
tagacttcag agctgcttcc aaaatgggtt tccaatctac accatttatt acagattctt     180
ttttgctttt cgtatttgga tagtttacga gtttagtttt tacttttttt agattcagga     240
agtctttaat gggtttcacc ggtttagtag gttcaccttt atcggccaac aagaattaca     300
tttctctcta tttatgacga ctgccaccaa taattcaaaa caagtttaca tattaagttt     360
tcttaaacta ctcctatata ttaagtctaa gattatatta atcttattta tataatttaa     420
ttcaactaaa tttttagttt gtttatttac atgatatacc atggtcctgt tattttagct     480
aaagtatata ttaaatttat tactttttta tttcaaagat tttttctatt gtttttttatt    540
ttgttttctt ttcatttaag cttatattat tattattata cacacacatt taaggtttat     600
atttatattt cttgcaatgc aaacatgtgt caatattagt tggtttaaga tacaaaaata     660
ttaaggacta aatggttgag ataatactac tttgatatat atatatataa ctatattcta     720
taaacttaat ttatttgaga gggtaaggat tagatgacca agaaattggt aaaaattgga     780
aaaataatta tagacattat tattttacaa gatattctca aatggctaga gatttgtgaa     840
gttctgtttt tttttttcctc aaagttcaag tgtaatattt ttcgatgaca atgattattc    900
caaatttatt tttttgttaa tcgtaaacag tgaattttgc ataattggtt tgaattagag     960
ttgataatag ttgttctcat catcaccaaa cattcttctg cttaccaaaa gtaagggact    1020
ttcattagct aaaacaaaaa tcatacacaa atgggttttg gttgtactgt ccaaaatgga    1080
acacacagtt ttatagaaag acaaaactga aacggcaagt gagagttaaa gtaaaaggaa    1140
attgcaatcg agagccagct gagatttttt                                     1169
```

<210> 41
<211> 1191
<212> DNA
<213> Arabidopsis thaliana

<220>
<221> promoter
<222> (1)..(1191)
<223> transcription regulating sequence from gene At2g34200

<400> 41
```
tttttcttt atctaatgga taaccaccac ccattaggat caccataaag atcatatttt      60
ggagcattcc aatatcataa cactttactc ttaagggtct aatttgagtt ggtggcaaca    120
tgtcatatag acttcagagc tgcttccaaa atgggtttcc aatctacacc atttattaca    180
gattcatttt tgcttttcgt atttggttag tttaggagtt tagtttttac tttttttaga    240
ttcaggaagt ctttaatggg tttcaccggt ttagtaggtt cacctttatc ggccaacaag    300
aattacattt ctctctattt atgacgactg ccaccaataa ttcaaaacaa gtttacatat    360
taagttttct taaactactc ctatatatta agtctaagat tatattaatc ttatttatat    420
aatttaattc aactaaattt ttagtttgtt tatttacatg atataccatg gtcctgttat    480
tttagctaaa gtatatatta aatttattac tttttatatt caaagatttt ttctattgtt    540
ttttattttg ttttcttttc atttaagctt atattattat tattatacac acacatttaa    600
ggtttatatt tatatttctt gcaatgcaaa catgtgtcaa tattagttga tttaagatac    660
aaaaatatta aggactaaat ggttgagata atactacttt gatatatata tatatatata    720
tataactata ttctataaac ttaatttatt tgagagggta aggattagat gaccaagaaa    780
ttggtaaaaa ttggaaaaat aattatagac attattattt tacaagatat tctcaaatgg    840
ctagagattt gtgaagttct gttttttttt tcctcaaagt tcaagtgtaa tatttttcga    900
tgacaatgat tattccaaat ttattttttt gttaatcgta aacagtgaat tttgcataat    960
tggtttgaat tagagttgat aatagttgtt ctcatcatca ccaaacattc ttctgcttac   1020
caaaagtaag ggactttcat tagcaaaaac aaaaatcata cacaaatggg ttttggttgt   1080
actgtccaaa atggaacaca cagttttata gaaagacaaa actgaaacgg caagtgagag   1140
ttaaagtaaa aggaaattgc aatcgagagc cagctgagat ttttagatat t            1191
```

<210> 42
<211> 1130
<212> DNA
<213> Arabidopsis thaliana

<220>
<221> promoter
<222> (1)..(1130)
<223> transcription regulating sequence from gene At2g34200

<400> 42
```
tttatctaat ggataaccac cacccattag gatcaccata aagatcatat tttggagcat     60
tccaatatca taacacttta ctcttaaggg tctaatttga gttggtggca acatgtcata    120
```

```
tagacttcag agctgcttcc aaaatgggtt tccaatctac accatttatt acagattctt    180
ttttgctttt cgtatttgga tagtttacga gtttagtttt tacttttttt agattcagga    240
agtctttaat gggtttcacc ggtttagtag gttcaccttt atcggccaac aagaattaca    300
tttctctcta tttatgacga ctgccaccaa taattcaaaa caagtttaca tattaagttt    360
tcttaaacta ctcctatata ttaagtctaa gattatatta atcttattta tataatttaa    420
ttcaactaaa tttttagttt gtttatttac atgatatacc atggtcctgt tattttagct    480
aaagtatata ttaaatttat tacttttttta tttcaaagat tttttctatt gtttttttatt    540
ttgttttctt ttcatttaag cttatattat tattattata cacacacatt taaggtttat    600
atttatattt cttgcaatgc aaacatgtgt caatattagt tggtttaaga tacaaaaata    660
ttaaggacta aatggttgag ataatactac tttgatatat atatatataa ctatattcta    720
taaacttaat ttatttgaga gggtaaggat tagatgacca agaaattggt aaaaattgga    780
aaaataatta tagacattat tattttacaa gatattctca aatggctaga gatttgtgaa    840
gttctgtttt ttttttcctc aaagttcaag tgtaatattt ttcgatgaca atgattattc    900
caaatttatt tttttgttaa tcgtaaacag tgaattttgc ataattggtt tgaattagag    960
ttgataatag ttgttctcat catcaccaaa cattcttctg cttaccaaaa gtaagggact   1020
ttcattagct aaaacaaaaa tcatacacaa atgggttttg gttgtactgt ccaaaatgga   1080
acacacagtt ttatagaaag acaaaactga aacggcaagt gagagttaaa              1130
```

<210> 43
<211> 1145
<212> DNA
<213> Arabidopsis thaliana

<220>
<221> promoter
<222> (1)..(1145)
<223> transcription regulating sequence from gene At2g34200

<400> 43

```
tttttcttt atctaatgga taaccaccac ccattaggat caccataaag atcatatttt      60
ggagcattcc aatatcataa cactttactc ttaagggtct aatttgagtt ggtggcaaca    120
tgtcatatag acttcagagc tgcttccaaa atgggtttcc aatctacacc atttattaca    180
gattcatttt tgcttttcgt atttggttag tttaggagtt tagtttttac ttttttttaga    240
ttcaggaagt ctttaatggg tttcaccggt ttagtaggtt caccttttatc ggccaacaag    300
aattacattt ctctctattt atgacgactg ccaccaataa ttcaaaacaa gtttacatat    360
taagttttct taaactactc ctatatatta agtctaagat tatattaatc ttatttatat    420
aatttaattc aactaaattt ttagtttgtt tatttacatg atataccatg gtcctgttat    480
tttagctaaa gtatatatta aatttattac tttttttattt caaagatttt ttctattgtt    540
ttttattttg ttttcttttc atttaagctt atattattat tattatacac acacatttaa    600
ggtttatatt tatatttctt gcaatgcaaa catgtgtcaa tattagttga tttaagatac    660
aaaaatatta aggactaaat ggttgagata atactacttt gatatatata tatatatata    720
tataactata ttctataaac ttaatttatt tgagagggta aggattagat gaccaagaaa    780
ttggtaaaaa ttggaaaaat aattatagac attattattt tacaagatat tctcaaatgg    840
ctagagattt gtgaagttct gttttttttt tcctcaaagt tcaagtgtaa tatttttcga    900
tgacaatgat tattccaaat ttatttttttt gttaatcgta aacagtgaat tttgcataat    960
```

tggtttgaat tagagttgat aatagttgtt ctcatcatca ccaaacattc ttctgcttac 1020
caaaagtaag ggactttcat tagcaaaaac aaaaatcata cacaaatggg ttttggttgt 1080
actgtccaaa atggaacaca cagttttata gaaagacaaa actgaaacgg caagtgagag 1140
ttaaa 1145

<210> 44
<211> 1120
<212> DNA
<213> Arabidopsis thaliana

<220>
<221> promoter
<222> (1)..(1120)
<223> transcription regulating sequence from gene At2g34200

<400> 44
tgttatttta gctaaagtat atattaaatt tattactttt ttatttcaaa gattttttct 60
attgtttttt attttgtttt cttttcattt aagcttatat tattattatt atacacacac 120
atttaaggtt tatatttata tttcttgcaa tgcaaacatg tgtcaatatt agttggttta 180
agatacaaaa atattaagga ctaaatggtt gagataatac tactttgata tatatatata 240
taactatatt ctataaactt aatttatttg agagggtaag gattagatga ccaagaaatt 300
ggtaaaaatt ggaaaaataa ttatagacat tattatttta caagatattc tcaaatggct 360
agagatttgt gaagttctgt tttttttttc ctcaaagttc aagtgtaata tttttcgatg 420
acaatgatta ttccaaattt atttttttgt taatcgtaaa cagtgaattt tgcataattg 480
gtttgaatta gagttgataa tagttgttct catcatcacc aaacattctt ctgcttacca 540
aaagtaaggg actttcatta gctaaaacaa aaatcataca caaatgggtt ttggttgtac 600
tgtccaaaat ggaacacaca gttttataga aagacaaaac tgaaacggca agtgagagtt 660
aaagtaaaag gaaattgcaa tcgagagcca gctgagattt ttagatattt tgcaggtaac 720
tcttaacgcc gagcatacat acgtttctca aagacataaa gctgattgtg tttctccatt 780
tgctgaaaga tctgagccat aaagctgaaa tataaacctt ttttgtgttt tgttgttccc 840
cagattctct gacgctgctt aaaccttaaa gttcgtagct ttttcttgct agtgggtctt 900
ttgaggtgga tctggattct gggtaaggtt ttgtttttta tatctttacc agtaaattga 960
agttcttatg gtttcaagaa attttacagg gttttcaatt attggaatgt tgtttctgtt 1020
ggctctttat ctgaatacaa tcttactaag aggttacgga tcttggactt tgtaggaact 1080
tgagcaaaag ccatcctctt ttgttgtata tatgaattaa 1120

<210> 45
<211> 1135
<212> DNA
<213> Arabidopsis thaliana

<220>
<221> promoter
<222> (1)..(1135)
<223> transcription regulating sequence from gene At2g34200

<400> 45

```
atggtcctgt tattttagct aaagtatata ttaaatttat tactttttta tttcaaagat      60
tttttctatt gtttttttatt ttgttttctt ttcatttaag cttatattat tattattata    120
cacacacatt taaggtttat atttatattt cttgcaatgc aaacatgtgt caatattagt    180
tgatttaaga tacaaaaata ttaaggacta aatggttgag ataatactac tttgatatat    240
atatatatat atatataact atattctata aacttaattt atttgagagg gtaaggatta    300
gatgaccaag aaattggtaa aaattggaaa aataattata gacattatta ttttacaaga    360
tattctcaaa tggctagaga tttgtgaagt tctgtttttt ttttcctcaa agttcaagtg    420
taatattttt cgatgacaat gattattcca aatttatttt tttgttaatc gtaaacagtg    480
aattttgcat aattggtttg aattagagtt gataatagtt gttctcatca tcaccaaaca    540
ttcttctgct taccaaaagt aagggacttt cattagcaaa aacaaaaatc atacacaaat    600
gggttttggt tgtactgtcc aaaatggaac acacagtttt atagaaagac aaaactgaaa    660
cggcaagtga gagttaaagt aaaaggaaat tgcaatcgag agccagctga gatttttaga    720
tattttgcag gtaactctta acgccgagca tacatacgtt tctcaaagac ataaagctga    780
ttgtgtttct ccatttgctg aaagatctga gccataaagc tgaaatagaa accttttttg    840
tgttttgttg ttccccagat tctctgacgc tgcttaaacc ttaaagttcg tagctttttc    900
ttgctagtgg gtcttttgag gtggatctgg attctgggta aggttttgtt ttttatatct    960
ttaccagtaa attgaagttc ttatggtttc aagaaatttt acagggtttt caattattgg   1020
aatgttgttt ctgttggctc tttatctgaa tacaatctta ctaagaggtt acggatcttg   1080
gactttgtag gaacttgagc aaaagccatc ctcttttgtt gtatatatga attaa        1135
```

<210> 46
<211> 663
<212> DNA
<213> Arabidopsis thaliana

<220>
<221> promoter
<222> (1)..(663)
<223> transcription regulating sequence from gene At2g34200

<400> 46

```
tgttatttta gctaaagtat atattaaatt tattactttt ttatttcaaa gatttttctt     60
attgtttttt attttgtttt cttttcattt aagcttatat tattattatt atacacacac    120
atttaaggtt tatatttata tttcttgcaa tgcaaacatg tgtcaatatt agttggttta    180
agatacaaaa atattaagga ctaaatggtt gagataatac tactttgata tatatatata    240
taactatatt ctataaactt aatttatttg agagggtaag gattagatga ccaagaaatt    300
ggtaaaaatt ggaaaaataa ttatagacat tattatttta caagatattc tcaaatggct    360
agagatttgt gaagttctgt tttttttttc ctcaaagttc aagtgtaata tttttcgatg    420
acaatgatta ttccaaattt atttttttgt taatcgtaaa cagtgaattt tgcataattg    480
gtttgaatta gagttgataa tagttgttct catcatcacc aaacattctt ctgcttacca    540
aaagtaaggg actttcatta gctaaaacaa aaatcataca caaatgggtt ttggttgtac    600
tgtccaaaat ggaacacaca gttttataga aagacaaaac tgaaacggca agtgagagtt    660
aaa                                                                  663
```

<210> 47
<211> 678
<212> DNA
<213> Arabidopsis thaliana

<220>
<221> promoter
<222> (1)..(678)
<223> transcription regulating sequence from gene At2g34200

<400> 47
atggtcctgt tattttagct aaagtatata ttaaatttat tacttttta tttcaaagat 60
tttttctatt gtttttatt ttgttttctt ttcatttaag cttatattat tattattata 120
cacacacatt taaggtttat atttatattt cttgcaatgc aaacatgtgt caatattagt 180
tgatttaaga tacaaaaata ttaaggacta aatggttgag ataatactac tttgatatat 240
atatatatat atatataact atattctata aacttaattt atttgagagg gtaaggatta 300
gatgaccaag aaattggtaa aaattggaaa aataattata gacattatta ttttacaaga 360
tattctcaaa tggctagaga tttgtgaagt tctgtttttt ttttcctcaa agttcaagtg 420
taatattttt cgatgacaat gattattcca aatttatttt tttgttaatc gtaaacagtg 480
aattttgcat aattggtttg aattagagtt gataatagtt gttctcatca tcaccaaaca 540
ttcttctgct taccaaaagt aagggacttt cattagcaaa aacaaaaatc atacacaaat 600
gggttttggt tgtactgtcc aaaatggaac acacagtttt atagaaagac aaaactgaaa 660
cggcaagtga gagttaaa 678

<210> 48
<211> 968
<212> DNA
<213> Arabidopsis thaliana

<220>
<221> CDS
<222> (177)..(845)
<223> encoding zinc finger (C3HC4-type RING finger) family protein)

<400> 48
gtaaaaggaa attgcaatcg agagccagct gagattttta gatattttgc agattctctg 60
acgctgctta aaccttaaag ttcgtagctt tttcttgcta gtgggtcttt tgaggtggat 120
ctggattctg ggaacttgag caaaagccat cctcttttgt tgtatatatg aattaa atg 179
Met
1

gac cct aaa agt tgt gag aat tcg agt gat gtt aag ggt cag acc agt 227
Asp Pro Lys Ser Cys Glu Asn Ser Ser Asp Val Lys Gly Gln Thr Ser
5 10 15

gac tct gta tca aaa aag gtg ttg att gag gaa gag gaa gat gta aag 275

```
Asp Ser Val Ser Lys Lys Val Leu Ile Glu Glu Glu Glu Asp Val Lys
        20                  25                  30
aaa cca caa cag gga aaa gaa aat gat tca aga atg gcc aaa gat gtt      323
Lys Pro Gln Gln Gly Lys Glu Asn Asp Ser Arg Met Ala Lys Asp Val
    35                  40                  45
gtt agt tgc agt agt aac att tca gct cat gtt gtt cat gag gaa gtt      371
Val Ser Cys Ser Ser Asn Ile Ser Ala His Val Val His Glu Glu Val
50                  55                  60                  65
gcg gat aat gtt act gcg gta agc tgc aac gag gca gag agt gat ata      419
Ala Asp Asn Val Thr Ala Val Ser Cys Asn Glu Ala Glu Ser Asp Ile
                70                  75                  80
tcg aaa gca aag gcg aag gag ttc cac act att gat ttg agt ggt gta      467
Ser Lys Ala Lys Ala Lys Glu Phe His Thr Ile Asp Leu Ser Gly Val
            85                  90                  95
gga gaa aga atc tgt agg att tgt cat ttt ggt tct gat caa tca cct      515
Gly Glu Arg Ile Cys Arg Ile Cys His Phe Gly Ser Asp Gln Ser Pro
        100                 105                 110
gag gct tct ggt gat gat aag tca gta agt ccg gag ttg att gag att      563
Glu Ala Ser Gly Asp Asp Lys Ser Val Ser Pro Glu Leu Ile Glu Ile
    115                 120                 125
ggt tgc aaa tgt aaa aac gag ctt ggc ctt gca cat ttc cat tgc gct      611
Gly Cys Lys Cys Lys Asn Glu Leu Gly Leu Ala His Phe His Cys Ala
130                 135                 140                 145
gaa gcc tgg ttc aag tta aga gga aac agt gtg tgt gaa atc tgc ggt      659
Glu Ala Trp Phe Lys Leu Arg Gly Asn Ser Val Cys Glu Ile Cys Gly
                150                 155                 160
tgc aca gcg aag aat gtt aca gta agg ttg atg gag gat tgg agc ggc      707
Cys Thr Ala Lys Asn Val Thr Val Arg Leu Met Glu Asp Trp Ser Gly
            165                 170                 175
gaa aga gac aat aca ttg gat ggg aga aga aga cga gga aga gga caa      755
Glu Arg Asp Asn Thr Leu Asp Gly Arg Arg Arg Arg Gly Arg Gly Gln
        180                 185                 190
tct tgc tgc atc ttt atg gtt ttt ctg ctc act atc ctt ttg ctt cat      803
Ser Cys Cys Ile Phe Met Val Phe Leu Leu Thr Ile Leu Leu Leu His
    195                 200                 205
tgg ttt ttc aaa aag att agt ggt tac tac caa aac act tga              845
Trp Phe Phe Lys Lys Ile Ser Gly Tyr Tyr Gln Asn Thr
210                 215                 220
atatgtatat ctgttggtaa ctttatcatt tgattctgaa tctagattat atggaaaaga    905
gatgatagaa tcaaaattct tagatcaaat tctgattcat agtccaacag atggacaatt    965
tag                                                                  968
```

<210> 49
<211> 222
<212> PRT

<213> Arabidopsis thaliana

<400> 49

```
Met Asp Pro Lys Ser Cys Glu Asn Ser Ser Asp Val Lys Gly Gln Thr
1               5                   10                  15

Ser Asp Ser Val Ser Lys Lys Val Leu Ile Glu Glu Glu Glu Asp Val
            20                  25                  30

Lys Lys Pro Gln Gln Gly Lys Glu Asn Asp Ser Arg Met Ala Lys Asp
        35                  40                  45

Val Val Ser Cys Ser Ser Asn Ile Ser Ala His Val Val His Glu Glu
    50                  55                  60

Val Ala Asp Asn Val Thr Ala Val Ser Cys Asn Glu Ala Glu Ser Asp
65                  70                  75                  80

Ile Ser Lys Ala Lys Ala Lys Glu Phe His Thr Ile Asp Leu Ser Gly
                85                  90                  95

Val Gly Glu Arg Ile Cys Arg Ile Cys His Phe Gly Ser Asp Gln Ser
            100                 105                 110

Pro Glu Ala Ser Gly Asp Asp Lys Ser Val Ser Pro Glu Leu Ile Glu
        115                 120                 125

Ile Gly Cys Lys Cys Lys Asn Glu Leu Gly Leu Ala His Phe His Cys
    130                 135                 140

Ala Glu Ala Trp Phe Lys Leu Arg Gly Asn Ser Val Cys Glu Ile Cys
145                 150                 155                 160

Gly Cys Thr Ala Lys Asn Val Thr Val Arg Leu Met Glu Asp Trp Ser
                165                 170                 175

Gly Glu Arg Asp Asn Thr Leu Asp Gly Arg Arg Arg Arg Gly Arg Gly
            180                 185                 190

Gln Ser Cys Cys Ile Phe Met Val Phe Leu Leu Thr Ile Leu Leu Leu
        195                 200                 205

His Trp Phe Phe Lys Lys Ile Ser Gly Tyr Tyr Gln Asn Thr
    210                 215                 220
```

<210> 50
<211> 1069
<212> DNA
<213> Arabidopsis thaliana

<220>
<221> promoter
<222> (1)..(1069)
<223> transcription regulating sequence from gene At3g11180

<400> 50
ggtgagtgct acgttttata ttagtacagt actatgtcta gaaatatgtc tacttttgta 60
atattttact caaaagagag acgattgaag aaaatagata attacaagtt acagccattg 120
ttactctctt cttacaagaa ctatgcatac agtttatttc accatctatt gaactatata 180
atttcatatt attgccttta tagaattgta caattccaaa gagcatcctc ttccttgcgt 240
aggttatcat tttgaactat taagagcact ttgataactg aagtaagtta gaaatcactt 300
tacatgtagt aaagtatcaa taaaattagt gcaattctga gataaggaag ttaaagttaa 360
atattctttt aaaaatgttg aagtcgtcag attcttttag tatatatctc aaggcattaa 420
atatttcaag gttgagtacg tcgttaagct acccaaacat tctcaaacag aagaaaatca 480
taccgatctt aatttatatt attagggtta aaacatctac gaactttcac agctaataac 540
aaaaaaatga actccacgtg atagctaaaa ttatttgcaa ggaagctaca aaacttttca 600
ttggaaaatg cttcgtactt ttcacatttg gaattattat caaatacatc cacgtcattt 660
tcaatctttt ttaccacgta tcaaaaaaaa catttttta tcacgttttc gtgaactaac 720
aagcaacaaa ctttaatcgt atggactcta ttcgacttcc tcatcggtta caattacgga 780
ttcctacata cgttacgatt ttcttcagtt acttattaca aaggaagata attaatattt 840
tcgattcacg agaatagtct gaaacatgtc ggtccgagtt tgattttatt tgtgtatgat 900
agttaatatt cacaattcat ttatttatgt taatccaaat cttcttccct ttctctctct 960
ctttctctcc ctctaaccat ataaaccaac tatatataac agcacttcac aacttctcat 1020
gaacaagtag aaaaaaagag caagactaat aagccatata taagagaca 1069

<210> 51
<211> 1088
<212> DNA
<213> Arabidopsis thaliana

<220>
<221> promoter
<222> (1)..(1088)
<223> transcription regulating sequence from gene At3g11180

<400> 51
agagatggaa ctggtgagtg ctacgtttta tattagtaca gtactatgtc tagaaatatg 60
tctacttttg taatatttta ctcaaaagag agacgattga agaaaataga taattacaag 120
ttacagccat tgttactctc ttcttacaag aactatgcat acagtttatt tcaccatcta 180
ttgaactata taatttcata ttattgcctt tatagaattg tacaattcca aagagcatcc 240

tcttccttgc gtaggttatc attttgaact attaagagca ctttgataac tgaagtaagt 300
tagaaatcac tttacatgta gtaaagtatc aataaaatta gtgcaattct gagataagga 360
agttaaagtt aaatattatt ttaaaatgtt gaagtcgtca gattctttta gtatatatct 420
caaggcatta aatatttcaa ggttgagtac gtcgttaagc tacccaaaca ttctcaaaca 480
gaagaaaatc ataccgatct taatttatat tgttagggtt aaaacatcta cgaactttca 540
cagctaataa caaaaaaatg aactccacgt gatagctaaa attatttgta aggaagctac 600
aaaacttttc attgcaaaat gcttcgtagt tttcacattt ggaattatta tcaaatacat 660
ccacgtcatt ttcaatcttt tttaccacgt atcagaaaaa acattttttta tcacgttttc 720
ttgaactaac aagcaacaaa ctttaatcgt atggactcta ttcgacttcc tcatcggtta 780
caaacttaca attacggatt cctacatacg ttacgatttt cttcagttac ttattacaaa 840
ggaagataat taatattttc gattcacgag aatagtctga aacatgtcgg tccgagtttg 900
attttatttg tgtatgatag ttaatattca caattcattt atttatgtta atccaaatct 960
tgttcccttt ctctctctct ttctctccct ctaaccatat aaaccaacta tatataacag 1020
cacttcacaa cttctcatga acaagtagaa aaaaaagagc aagactaata agccatataa 1080
gagacaac 1088

<210> 52
<211> 1034
<212> DNA
<213> Arabidopsis thaliana

<220>
<221> promoter
<222> (1)..(1034)
<223> transcription regulating sequence from gene At3g11180

<400> 52
ggtgagtgct acgttttata ttagtacagt actatgtcta gaaatatgtc tacttttgta 60
atattttact caaaagagag acgattgaag aaaatagata attacaagtt acagccattg 120
ttactctctt cttacaagaa ctatgcatac agtttatttc accatctatt gaactatata 180
atttcatatt attgccttta tagaattgta caattccaaa gagcatcctc ttccttgcgt 240
aggttatcat tttgaactat taagagcact ttgataactg aagtaagtta gaaatcactt 300
tacatgtagt aaagtatcaa taaaattagt gcaattctga gataaggaag ttaaagttaa 360
atattctttt aaaaatgttg aagtcgtcag attcttttag tatatatctc aaggcattaa 420
atatttcaag gttgagtacg tcgttaagct acccaaacat tctcaaacag aagaaaatca 480
taccgatctt aatttatatt attagggtta aaacatctac gaactttcac agctaataac 540
aaaaaaatga actccacgtg atagctaaaa ttatttgcaa ggaagctaca aaacttttca 600
ttggaaaatg cttcgtactt ttcacatttg gaattattat caaatacatc cacgtcattt 660
tcaatctttt ttaccacgta tcaaaaaaaa catttttttta tcacgttttc gtgaactaac 720
aagcaacaaa ctttaatcgt atggactcta ttcgacttcc tcatcggtta caattacgga 780
ttcctacata cgttacgatt ttcttcagtt acttattaca aaggaagata attaatattt 840
tcgattcacg agaatagtct gaaacatgtc ggtccgagtt tgattttatt tgtgtatgat 900
agttaatatt cacaattcat ttatttatgt taatccaaat cttcttccct ttctctctct 960
ctttctctcc ctctaaccat ataaaccaac tatatataac agcacttcac aacttctcat 1020
gaacaagtag aaaa 1034

<210> 53
<211> 1053
<212> DNA
<213> Arabidopsis thaliana

<220>
<221> promoter
<222> (1)..(1053)
<223> transcription regulating sequence from gene At3g11180

<400> 53
agagatggaa ctggtgagtg ctacgtttta tattagtaca gtactatgtc tagaaatatg 60
tctacttttg taatatttta ctcaaaagag agacgattga agaaaataga taattacaag 120
ttacagccat tgttactctc ttcttacaag aactatgcat acagtttatt tcaccatcta 180
ttgaactata taatttcata ttattgcctt tatagaattg tacaattcca aagagcatcc 240
tcttccttgc gtaggttatc attttgaact attaagagca ctttgataac tgaagtaagt 300
tagaaatcac tttacatgta gtaaagtatc aataaaatta gtgcaattct gagataagga 360
agttaaagtt aaatattatt ttaaaatgtt gaagtcgtca gattctttta gtatatatct 420
caaggcatta aatatttcaa ggttgagtac gtcgttaagc tacccaaaca ttctcaaaca 480
gaagaaaatc ataccgatct taatttatat tgttagggtt aaaacatcta cgaactttca 540
cagctaataa caaaaaaatg aactccacgt gatagctaaa attatttgta aggaagctac 600
aaaacttttc attgcaaaat gcttcgtagt tttcacattt ggaattatta tcaaatacat 660
ccacgtcatt ttcaatcttt tttaccacgt atcagaaaaa acatttttta tcacgttttc 720
ttgaactaac aagcaacaaa ctttaatcgt atggactcta ttcgacttcc tcatcggtta 780
caaacttaca attacggatt cctacatacg ttacgatttt cttcagttac ttattacaaa 840
ggaagataat taatattttc gattcacgag aatagtctga aacatgtcgg tccgagtttg 900
attttatttg tgtatgatag ttaatattca caattcattt atttatgtta atccaaatct 960
tgttcccttt ctctctctct ttctctccct ctaaccatat aaaccaacta tatataacag 1020
cacttcacaa cttctcatga acaagtagaa aaa 1053

<210> 54
<211> 2012
<212> DNA
<213> Arabidopsis thaliana

<220>
<221> promoter
<222> (1)..(2012)
<223> transcription regulating sequence from gene At3g11180

<400> 54
ctgaatacga tcgatcagat ggaaacaaca aatttgtttg cgatactgaa gctatatata 60
ccatacattg cattatctta gatacattta aattctagca aaaagtataa aagaagatca 120
ttacgtgagc gacgaaggag atgttgtata tatactacaa attaagcaga tccaaatctc 180

```
tatgtttccg atactgaagc tttatactaa taaaatatac attgcaatat atattatttt     240
agattcatcc ttctgaaggt ttaataccaa taattttagg gtataattaa gaaaatagtt     300
ttctactttt ttcacattta aaatcaatca tagttattct tcctaacctc actaaatttg     360
tttgattaat taatttaaaa atgggattta aataagtatt tgttacacaa tatgtagttg     420
gatagtatcc acatgtgcct tccaaattca gtgaaccatg tttctattat tgggaagtcg     480
tcggccttct atttagatgg tcaacggacc atttactaat acaagttttg cccataatat     540
atttaaatcg actctccttc ggaattttta catttaaatt gtttaaaagc tctacttgaa     600
taatccaaca tacaataatc ttgaactaat tttttttta ataatgtagt tattagaatt     660
ataaacgaaa agtgtttgat tggtatgttg aatataagcg tacgcttaat ttgctattgc     720
atgacacata aataatcatc ttttaacact cttgaatttt taaaaattat aattgcgatt     780
ctcaaattta gtcataaatt cagttaacta tcatcgctaa gtggcataaa taatgtagtt     840
agatagtcac cgtccgttgc taatacagaa taaaagtcac cgtccaatta cacacacgta     900
tgtaaggaaa ttatacataa cttaatttta tagagatgga actggtgagt gctacgtttt     960
atattagtac agtactatgt ctagaaatat gtctactttt gtaatatttt actcaaaaga    1020
gagacgattg aagaaaatag ataattacaa gttacagcca ttgttactct cttcttacaa    1080
gaactatgca tacagtttat ttcaccatct attgaactat ataatttcat attattgcct    1140
ttatagaatt gtacaattcc aaagagcatc ctcttccttg cgtaggttat cattttgaac    1200
tattaagagc actttgataa ctgaagtaag ttagaaatca ctttacatgt agtaaagtat    1260
caataaaatt agtgcaattc tgagataagg aagttaaagt taaatattct tttaaaaatg    1320
ttgaagtcgt cagattcttt tagtatatat ctcaaggcat taaatatttc aaggttgagt    1380
acgtcgttaa gctacccaaa cattctcaaa cagaagaaaa tcataccgat cttaatttat    1440
attattaggg ttaaaacatc tacgaacttt cacagctaat aacaaaaaaa tgaactccac    1500
gtgatagcta aaattatttg caaggaagct acaaaacttt tcattggaaa atgcttcgta    1560
cttttcacat ttggaattat tatcaaatac atccacgtca ttttcaatct tttttaccac    1620
gtatcaaaaa aaacattttt ttatcacgtt ttcgtgaact aacaagcaac aaactttaat    1680
cgtatggact ctattcgact tcctcatcgg ttacaattac ggattcctac atacgttacg    1740
attttcttca gttacttatt acaaaggaag ataattaata ttttcgattc acgagaatag    1800
tctgaaacat gtcggtccga gtttgatttt atttgtgtat gatagttaat attcacaatt    1860
catttattta tgttaatcca aatcttcttc cctttctctc tctctttctc tccctctaac    1920
catataaacc aactatatat aacagcactt cacaacttct catgaacaag tagaaaaaaa    1980
gagcaagact aataagccat atataagaga ca                                  2012

<210>  55
<211>  2033
<212>  DNA
<213>  Arabidopsis thaliana

<220>
<221>  promoter
<222>  (1)..(2033)
<223>  transcription regulating sequence from gene At3g11180

<400>  55
gagttagtat ctctgaatac gatcgatcag atggaaacaa caaatttgtt tgcgatactg      60
aagctatata taccatacat tgcattatct tagatacatt taaattctag caaaaagtat     120
```

```
aaaagaagat cattacgtga gcgacgaagg agatgttgta tatatactac aaattaagca    180
gatccaaatc tctatgtttc cgatactgaa gctttatact aataaaatat acattgcaat    240
atatattatt ttagattcat ccttctgaag gtttatatac caataatttt agggtataat    300
taagaaaata gttttctact tttttcacat ttaaaatcaa tcatagttat tcttcctaac    360
ctcactaaat ttgtttgatt aattaactta aaaatgggat ttaaataagt atttgttaca    420
caatatgtag ttggatagta tccacatgtg ccttccaaat tcagtgaacc atgtttctat    480
tattgggaag tcgtcggcct tctatttaga tggtcaacgg accatttact aatacaagtt    540
ttgcccataa tatatttaaa tcgactctcc ttcggaattt ttacatttaa attgtttaaa    600
agctctactt gaataatcca acatacaata atcttgaact aattttttttt tttaataatg    660
tagttattag aattataaac gaaaagtgtt tgattggtat gttgaatata agcgtacgcc    720
taatttgcta ttgcatgaca cataaataat catcttttaa cactcttgaa tttttaaaaa    780
ttataattgc gattctcaaa tttagtcata aattcagtta actatcatcg ctaagtggca    840
taaataatgt agttagatag tcaccgtccg ttgctaatac agaataaaag tcaccgtcca    900
attacacata cgtatgtaag gaaattatac ataacttaat tttatagaga tggaactggt    960
gagtgctacg ttttatatta gtacagtact atgtctagaa atatgtctac ttttgtaata   1020
ttttactcaa aagagagacg attgaagaaa atagataatt acaagttaca gccattgtta   1080
ctctcttctt acaagaacta tgcatacagt ttatttcacc atctattgaa ctatataatt   1140
tcatattatt gcctttatag aattgtacaa ttccaaagag catcctcttc cttgcgtagg   1200
ttatcatttt gaactattaa gagcactttg ataactgaag taagttagaa atcactttac   1260
atgtagtaaa gtatcaataa aattagtgca attctgagat aaggaagtta aagttaaata   1320
ttattttaaa atgttgaagt cgtcagattc ttttagtata tatctcaagg cattaaatat   1380
ttcaaggttg agtacgtcgt taagctaccc aaacattctc aaacagaaga aaatcatacc   1440
gatcttaatt tatattgtta gggttaaaac atctacgaac tttcacagct aataacaaaa   1500
aaatgaactc cacgtgatag ctaaaattat ttgtaaggaa gctacaaaac ttttcattgc   1560
aaaatgcttc gtagttttca catttggaat tattatcaaa tacatccacg tcattttcaa   1620
tctttttttac cacgtatcag aaaaaacatt ttttatcacg ttttcttgaa ctaacaagca   1680
acaaacttta atcgtatgga ctctattcga cttcctcatc ggttacaaac ttacaattac   1740
ggattcctac atacgttacg attttcttca gttacttatt acaaaggaag ataattaata   1800
ttttcgattc acgagaatag tctgaaacat gtcggtccga gtttgatttt atttgtgtat   1860
gatagttaat attcacaatt catttattta tgttaatcca aatcttgttc cctttctctc   1920
tctctttctc tccctctaac catataaacc aactatatat aacagcactt cacaacttct   1980
catgaacaag tagaaaaaaa agagcaagac taataagcca tataagagac aac          2033
```

<210> 56
<211> 1977
<212> DNA
<213> Arabidopsis thaliana

<220>
<221> promoter
<222> (1)..(1977)
<223> *transcription regulating sequence from gene At3g11180*

<400> 56

```
ctgaatacga tcgatcagat ggaaacaaca aatttgtttg cgatactgaa gctatatata     60
```

ccatacattg cattatctta gatacattta aattctagca aaaagtataa aagaagatca 120
ttacgtgagc gacgaaggag atgttgtata tatactacaa attaagcaga tccaaatctc 180
tatgtttccg atactgaagc tttatactaa taaaatatac attgcaatat atattatttt 240
agattcatcc ttctgaaggt ttaataccaa taattttagg gtataattaa gaaaatagtt 300
ttctactttt ttcacattta aaatcaatca tagttattct tcctaacctc actaaatttg 360
tttgattaat taatttaaaa atgggattta aataagtatt tgttacacaa tatgtagttg 420
gatagtatcc acatgtgcct tccaaattca gtgaaccatg tttctattat tgggaagtcg 480
tcggccttct atttagatgg tcaacggacc atttactaat acaagttttg cccataatat 540
atttaaatcg actctccttc ggaattttta catttaaatt gtttaaaagc tctacttgaa 600
taatccaaca tacaataatc ttgaactaat ttttttttta ataatgtagt tattagaatt 660
ataaacgaaa agtgtttgat tggtatgttg aatataagcg tacgcttaat ttgctattgc 720
atgacacata aataatcatc ttttaacact cttgaatttt taaaaattat aattgcgatt 780
ctcaaattta gtcataaatt cagttaacta tcatcgctaa gtggcataaa taatgtagtt 840
agatagtcac cgtccgttgc taatacagaa taaaagtcac cgtccaatta cacacacgta 900
tgtaaggaaa ttatacataa cttaatttta tagagatgga actggtgagt gctacgtttt 960
atattagtac agtactatgt ctagaaatat gtctactttt gtaatatttt actcaaaaga 1020
gagacgattg aagaaaatag ataattacaa gttacagcca ttgttactct cttcttacaa 1080
gaactatgca tacagtttat ttcaccatct attgaactat ataatttcat attattgcct 1140
ttatagaatt gtacaattcc aaagagcatc ctcttccttg cgtaggttat cattttgaac 1200
tattaagagc actttgataa ctgaagtaag ttagaaatca ctttacatgt agtaaagtat 1260
caataaaatt agtgcaattc tgagataagg aagttaaagt taaatattct tttaaaaatg 1320
ttgaagtcgt cagattcttt tagtatatat ctcaaggcat taaatatttc aaggttgagt 1380
acgtcgttaa gctacccaaa cattctcaaa cagaagaaaa tcataccgat cttaatttat 1440
attattaggg ttaaaacatc tacgaacttt cacagctaat aacaaaaaaa tgaactccac 1500
gtgatagcta aaattatttg caaggaagct acaaaacttt tcattggaaa atgcttcgta 1560
cttttcacat ttggaattat tatcaaatac atccacgtca ttttcaatct tttttaccac 1620
gtatcaaaaa aaacattttt ttatcacgtt ttcgtgaact aacaagcaac aaactttaat 1680
cgtatggact ctattcgact tcctcatcgg ttacaattac ggattcctac atacgttacg 1740
atttcttca gttacttatt acaaaggaag ataattaata ttttcgattc acgagaatag 1800
tctgaaacat gtcggtccga gtttgatttt atttgtgtat gatagttaat attcacaatt 1860
catttattta tgttaatcca aatcttcttc cctttctctc tctctttctc tccctctaac 1920
catataaacc aactatatat aacagcactt cacaacttct catgaacaag tagaaaa 1977

<210> 57
<211> 1998
<212> DNA
<213> Arabidopsis thaliana

<220>
<221> promoter
<222> (1)..(1998)
<223> transcription regulating sequence from gene At3g11180

<400> 57
gagttagtat ctctgaatac gatcgatcag atggaaacaa caaatttgtt tgcgatactg 60

```
aagctatata taccatacat tgcattatct tagatacatt taaattctag caaaaagtat     120
aaaagaagat cattacgtga gcgacgaagg agatgttgta tatatactac aaattaagca     180
gatccaaatc tctatgtttc cgatactgaa gctttatact aataaaatat acattgcaat     240
atatattatt ttagattcat ccttctgaag gtttatatac caataatttt agggtataat     300
taagaaaata gttttctact tttttcacat ttaaaatcaa tcatagttat tcttcctaac     360
ctcactaaat ttgtttgatt aattaactta aaaatgggat ttaaataagt atttgttaca     420
caatatgtag ttggatagta tccacatgtg ccttccaaat tcagtgaacc atgtttctat     480
tattgggaag tcgtcggcct tctatttaga tggtcaacgg accatttact aatacaagtt     540
ttgcccataa tatatttaaa tcgactctcc ttcggaattt ttacatttaa attgtttaaa     600
agctctactt gaataatcca acatacaata atcttgaact aatttttttt tttaataatg     660
tagttattag aattataaac gaaaagtgtt tgattggtat gttgaatata agcgtacgcc     720
taatttgcta ttgcatgaca cataaataat catcttttaa cactcttgaa tttttaaaaa     780
ttataattgc gattctcaaa tttagtcata aattcagtta actatcatcg ctaagtggca     840
taaataatgt agttagatag tcaccgtccg ttgctaatac agaataaaag tcaccgtcca     900
attacacata cgtatgtaag gaaattatac ataacttaat tttatagaga tggaactggt     960
gagtgctacg ttttatatta gtacagtact atgtctagaa atatgtctac ttttgtaata    1020
ttttactcaa aagagagacg attgaagaaa atagataatt acaagttaca gccattgtta    1080
ctctcttctt acaagaacta tgcatacagt ttatttcacc atctattgaa ctatataatt    1140
tcatattatt gcctttatag aattgtacaa ttccaaagag catcctcttc cttgcgtagg    1200
ttatcatttt gaactattaa gagcactttg ataactgaag taagttagaa atcactttac    1260
atgtagtaaa gtatcaataa aattagtgca attctgagat aaggaagtta aagttaaata    1320
ttattttaaa atgttgaagt cgtcagattc ttttagtata tatctcaagg cattaaatat    1380
ttcaaggttg agtacgtcgt taagctaccc aaacattctc aaacagaaga aaatcatacc    1440
gatcttaatt tatattgtta gggttaaaac atctacgaac tttcacagct aataacaaaa    1500
aaatgaactc cacgtgatag ctaaaattat ttgtaaggaa gctacaaaac ttttcattgc    1560
aaaatgcttc gtagttttca catttggaat tattatcaaa tacatccacg tcattttcaa    1620
tctttttttac cacgtatcag aaaaaacatt ttttatcacg ttttcttgaa ctaacaagca    1680
acaaacttta atcgtatgga ctctattcga cttcctcatc ggttacaaac ttacaattac    1740
ggattcctac atacgttacg attttcttca gttacttatt acaaaggaag ataattaata    1800
ttttcgattc acgagaatag tctgaaacat gtcggtccga gtttgatttt atttgtgtat    1860
gatagttaat attcacaatt catttattta tgttaatcca aatcttgttc cctttctctc    1920
tctctttctc tccctctaac catataaacc aactatatat aacagcactt cacaacttct    1980
catgaacaag tagaaaaa                                                   1998
```

<210> 58
<211> 1577
<212> DNA
<213> Arabidopsis thaliana

<220>
<221> CDS
<222> (36)..(1238)
<223> encoding oxidoreductase, 2OG-Fe(II) oxygenase family protein

<400> 58

```
aaagagcaag actaataagc catataagag acaac atg aac aac cta gac gag        53
                                       Met Asn Asn Leu Asp Glu
                                       1               5

atc aag atc gag agc aag acc tgc ctc aac gat caa gaa caa gaa gtc      101
Ile Lys Ile Glu Ser Lys Thr Cys Leu Asn Asp Gln Glu Gln Glu Val
            10                  15                  20

aaa ata gac aac atg cac atg agc gac caa gac aag aac aag atc gaa      149
Lys Ile Asp Asn Met His Met Ser Asp Gln Asp Lys Asn Lys Ile Glu
        25                  30                  35

atc aag aac aag agt ggc ctc ggc gaa aag tgg cca gaa ccc atc gtc      197
Ile Lys Asn Lys Ser Gly Leu Gly Glu Lys Trp Pro Glu Pro Ile Val
    40                  45                  50

cga gtc caa tct cta gcc gag agc aac ctc act agt ctc cct gac cgt      245
Arg Val Gln Ser Leu Ala Glu Ser Asn Leu Thr Ser Leu Pro Asp Arg
55                  60                  65                  70

tac atc aag ccg ccg tct caa cgc cct caa acc acc atc atc gac cac      293
Tyr Ile Lys Pro Pro Ser Gln Arg Pro Gln Thr Thr Ile Ile Asp His
                75                  80                  85

caa ccg gaa gta gct gac ata aat ata ccg atc ata gac cta gac agt      341
Gln Pro Glu Val Ala Asp Ile Asn Ile Pro Ile Ile Asp Leu Asp Ser
            90                  95                  100

cta ttc tct ggc aat gaa gac gac aag aag agg ata tcc gag gca tgc      389
Leu Phe Ser Gly Asn Glu Asp Asp Lys Lys Arg Ile Ser Glu Ala Cys
        105                 110                 115

cgt gaa tgg gga ttc ttc cag gta atc aac cat ggc gtg aag ccg gag      437
Arg Glu Trp Gly Phe Phe Gln Val Ile Asn His Gly Val Lys Pro Glu
    120                 125                 130

ctg atg gac gca gct aga gaa act tgg aag agc ttc ttt aat ttg cct      485
Leu Met Asp Ala Ala Arg Glu Thr Trp Lys Ser Phe Phe Asn Leu Pro
135                 140                 145                 150

gtt gaa gcc aaa gaa gtt tac tca aac tcc cca aga acc tat gaa gga      533
Val Glu Ala Lys Glu Val Tyr Ser Asn Ser Pro Arg Thr Tyr Glu Gly
                155                 160                 165

tat gga agc aga ttg ggt gtg gaa aaa gga gcc att ctt gat tgg aat      581
Tyr Gly Ser Arg Leu Gly Val Glu Lys Gly Ala Ile Leu Asp Trp Asn
            170                 175                 180

gat tat tac tat ctc cat ttt ctt cct ctt gcc ttg aag gat ttc aac      629
Asp Tyr Tyr Tyr Leu His Phe Leu Pro Leu Ala Leu Lys Asp Phe Asn
        185                 190                 195

aaa tgg cct tct tta cct tcc aac att aga gaa atg aat gat gag tac      677
Lys Trp Pro Ser Leu Pro Ser Asn Ile Arg Glu Met Asn Asp Glu Tyr
    200                 205                 210

ggt aag gaa cta gtg aag cta ggt ggg aga cta atg acg atc tta tcg      725
Gly Lys Glu Leu Val Lys Leu Gly Gly Arg Leu Met Thr Ile Leu Ser
215                 220                 225                 230
```

```
tca aat ttg ggg cta aga gca gaa caa ctt caa gaa gca ttt ggt gga      773
Ser Asn Leu Gly Leu Arg Ala Glu Gln Leu Gln Glu Ala Phe Gly Gly
                235                 240                 245
gaa gac gtt ggt gca tgt ttg agg gtt aat tat tac cca aag tgc cct      821
Glu Asp Val Gly Ala Cys Leu Arg Val Asn Tyr Tyr Pro Lys Cys Pro
            250                 255                 260
caa ccg gag ctt gcc ctc ggc ctc tcc cct cat tct gat ccc ggc ggc      869
Gln Pro Glu Leu Ala Leu Gly Leu Ser Pro His Ser Asp Pro Gly Gly
        265                 270                 275
atg acc atc ctc ttg ccg gac gat caa gtc gtc ggc ctt cag gtc cgt      917
Met Thr Ile Leu Leu Pro Asp Asp Gln Val Val Gly Leu Gln Val Arg
    280                 285                 290
cac ggt gac acg tgg atc act gtc aat cct ctc cgc cac gct ttt atc      965
His Gly Asp Thr Trp Ile Thr Val Asn Pro Leu Arg His Ala Phe Ile
295                 300                 305                 310
gtc aat atc ggc gat caa att cag ata cta agc aat tcg aaa tac aag      1013
Val Asn Ile Gly Asp Gln Ile Gln Ile Leu Ser Asn Ser Lys Tyr Lys
                315                 320                 325
agc gtg gaa cat cga gtg ata gtg aat tcg gaa aaa gaa agg gtt tca      1061
Ser Val Glu His Arg Val Ile Val Asn Ser Glu Lys Glu Arg Val Ser
            330                 335                 340
cta gca ttc ttc tat aac cct aag agt gac att ccg atc caa cca atg      1109
Leu Ala Phe Phe Tyr Asn Pro Lys Ser Asp Ile Pro Ile Gln Pro Met
        345                 350                 355
caa caa ctt gtc acc tct acg atg cct ccc ttg tat cct ccc atg acc      1157
Gln Gln Leu Val Thr Ser Thr Met Pro Pro Leu Tyr Pro Pro Met Thr
    360                 365                 370
ttt gat caa tat aga ctc ttc att aga acg caa ggt cca cgt gga aaa      1205
Phe Asp Gln Tyr Arg Leu Phe Ile Arg Thr Gln Gly Pro Arg Gly Lys
375                 380                 385                 390
tcc cac gtt gag tct cat ata tct cct cgt taa ttgatatcta cttcataaaa    1258
Ser His Val Glu Ser His Ile Ser Pro Arg
                395                 400
tgtctgataa atagaataat tgataccgaa gctatggaag cttaaagaac tacctagaga    1318
ttccaaaaga ttggtgaaga atgaagatat atatacatat atattgagat caaagtgaat    1378
cgactaaaga ccgagaacta tcaaacattt gtttaagtta aattactttg tagtcacccct   1438
tgtagtgaat tatatatgtt tataattagt tcttttaggt tctattgtat ttctattatg    1498
caaaatcaat atccgtgatt tatgttgaag catgtctatc aataattatg cctcctctat    1558
ctctatgatt agtaacatt                                                 1577
```

<210> 59
<211> 400
<212> PRT
<213> Arabidopsis thaliana

<400> 59

```
Met Asn Asn Leu Asp Glu Ile Lys Ile Glu Ser Lys Thr Cys Leu Asn
1               5                   10                  15

Asp Gln Glu Gln Glu Val Lys Ile Asp Asn Met His Met Ser Asp Gln
            20                  25                  30

Asp Lys Asn Lys Ile Glu Ile Lys Asn Lys Ser Gly Leu Gly Glu Lys
        35                  40                  45

Trp Pro Glu Pro Ile Val Arg Val Gln Ser Leu Ala Glu Ser Asn Leu
    50                  55                  60

Thr Ser Leu Pro Asp Arg Tyr Ile Lys Pro Pro Ser Gln Arg Pro Gln
65                  70                  75                  80

Thr Thr Ile Ile Asp His Gln Pro Glu Val Ala Asp Ile Asn Ile Pro
                85                  90                  95

Ile Ile Asp Leu Asp Ser Leu Phe Ser Gly Asn Glu Asp Asp Lys Lys
            100                 105                 110

Arg Ile Ser Glu Ala Cys Arg Glu Trp Gly Phe Phe Gln Val Ile Asn
        115                 120                 125

His Gly Val Lys Pro Glu Leu Met Asp Ala Ala Arg Glu Thr Trp Lys
    130                 135                 140

Ser Phe Phe Asn Leu Pro Val Glu Ala Lys Glu Val Tyr Ser Asn Ser
145                 150                 155                 160

Pro Arg Thr Tyr Glu Gly Tyr Gly Ser Arg Leu Gly Val Glu Lys Gly
                165                 170                 175

Ala Ile Leu Asp Trp Asn Asp Tyr Tyr Tyr Leu His Phe Leu Pro Leu
            180                 185                 190

Ala Leu Lys Asp Phe Asn Lys Trp Pro Ser Leu Pro Ser Asn Ile Arg
        195                 200                 205

Glu Met Asn Asp Glu Tyr Gly Lys Glu Leu Val Lys Leu Gly Gly Arg
    210                 215                 220

Leu Met Thr Ile Leu Ser Ser Asn Leu Gly Leu Arg Ala Glu Gln Leu
225                 230                 235                 240
```

Gln Glu Ala Phe Gly Gly Glu Asp Val Gly Ala Cys Leu Arg Val Asn
245 250 255

Tyr Tyr Pro Lys Cys Pro Gln Pro Glu Leu Ala Leu Gly Leu Ser Pro
260 265 270

His Ser Asp Pro Gly Gly Met Thr Ile Leu Leu Pro Asp Asp Gln Val
275 280 285

Val Gly Leu Gln Val Arg His Gly Asp Thr Trp Ile Thr Val Asn Pro
290 295 300

Leu Arg His Ala Phe Ile Val Asn Ile Gly Asp Gln Ile Gln Ile Leu
305 310 315 320

Ser Asn Ser Lys Tyr Lys Ser Val Glu His Arg Val Ile Val Asn Ser
325 330 335

Glu Lys Glu Arg Val Ser Leu Ala Phe Phe Tyr Asn Pro Lys Ser Asp
340 345 350

Ile Pro Ile Gln Pro Met Gln Gln Leu Val Thr Ser Thr Met Pro Pro
355 360 365

Leu Tyr Pro Pro Met Thr Phe Asp Gln Tyr Arg Leu Phe Ile Arg Thr
370 375 380

Gln Gly Pro Arg Gly Lys Ser His Val Glu Ser His Ile Ser Pro Arg
385 390 395 400

<210> 60
<211> 949
<212> DNA
<213> Arabidopsis thaliana

<220>
<221> promoter
<222> (1)..(949)
<223> transcription regulating sequence from gene At4g00360

<400> 60
atcaattcac tctttaacac ttctttatac cattgaagaa attagatgaa agagtcacga 60
gttgcttacc aaatccctca caagaattga gaactgataa accaaattga gaagattaaa 120
tatcacgtct ccttttgatc tctattataa ttaatcgaaa ataaaaataa gagttttcaa 180

caaaacgtga tcattggttt acgatcactt gcaaagtcag acctaaaacg tagcattagt 240
acactaacct taaatattaa ttatatcatg caaaccctaa tgtcattacc taactataca 300
tgtgtaatgt gttcaacaga tcttcttaac ccacattaga tcaatattaa acaataaaaa 360
agattcttat atattctact acttacttct tcttattccc atccatattt ttctgtgcct 420
taaggttctc aactaatctc atttaattta gctagcacac agagaaacac acacgtatat 480
aaataatatg ataacacaca aaaagactca tatatataaa taattagagt cattaaatgt 540
ggattcatca ttaaatgaaa caactcttct tctctgtaca atttctcttc acaccttcac 600
caaattcttt gacttcaaaa atcttataaa atttatatat ctccaaaacc ataaaaccaa 660
aacgagtttt cacaaataaa ttacttagtt gaaatttcaa atctcattca attagggtac 720
actctctcaa caatccacat taatgagggt tgctgcttct gatggctagc agtaacagtt 780
ttatcgcctc cacttcttaa tgccatcttt ttcctcttcc ctctccttct ctatatatat 840
tttctgactc tgcaaaacct taattcatcc atctctcaaa caccattttt ggaaacacca 900
tttcacattc cttaaacttt tccattttag tatcatttca tattcattg 949

<210> 61
<211> 962
<212> DNA
<213> Arabidopsis thaliana

<220>
<221> promoter
<222> (1)..(962)
<223> transcription regulating sequence from gene At4g00360

<400> 61
cttgtgggat taatcaattc actctttaac acttctttat accattgaag aaattagatg 60
aaagagtcac gagttgctta ccaaatccct cacaagaatt gagaactgat aaaccaaatt 120
gagaagatta aatatcacgt ctccttttga tctctattat aattaatcga aaataaaaat 180
aagagttttc aacaaaacgt gatcattggt ttacgatcac ttgcaaagtc aaacctaaaa 240
cgtagcatta gtacactaac cttaaatact aattatatca tgcaaaccct aatgtcatta 300
cctaactata catgtgtaat gtgttcaaca gatcttctta acccacatta gatcaatatt 360
aaacaataaa aagattctta tatattctac tacttacttc ttcttattcc catccatatt 420
tttctgtgcc tttaggttct caactaatct catttaattt agctagcaca cagagaaaca 480
cacacgtata taaataatat gataacacac aaaaagactc atatatataa ataattagag 540
tcattaaatg tggattcatc attaaatgaa acaactcttc ttctctgtac aatttctctt 600
cacaccttca ccaaattctt tgacttcaaa aatcttataa aatttatata tctccaaaac 660
cataaaacca aaacgagttt tcacaaataa attacttagt tgaaatttca aatctcattc 720
aattagggta cactctctca acaatccaca ttaatgaggg ttgctgcttc tgatggctag 780
cagtaacagt tttatcgcct ccacttctta atgccatctt tttcctcttc cctctccttc 840
tctatatata ttttctgact ctgcaaaacc ttaattcatc catctctcaa acaccatttt 900
tggaaacacc atttcatatt ccttaaactt ttccatttta gtatcatttc atattcattg 960
at 962

<210> 62
<211> 768

<212> DNA
<213> Arabidopsis thaliana

<220>
<221> promoter
<222> (1)..(768)
<223> transcription regulating sequence from gene At4g00360

<400> 62
```
atcaattcac tctttaacac ttctttatac cattgaagaa attagatgaa agagtcacga     60
gttgcttacc aaatccctca caagaattga gaactgataa accaaattga gaagattaaa    120
tatcacgtct ccttttgatc tctattataa ttaatcgaaa ataaaaataa gagttttcaa    180
caaaacgtga tcattggttt acgatcactt gcaaagtcag acctaaaacg tagcattagt    240
acactaacct taaatattaa ttatatcatg caaaccctaa tgtcattacc taactataca    300
tgtgtaatgt gttcaacaga tcttcttaac ccacattaga tcaatattaa acaataaaaa    360
agattcttat atattctact acttacttct tcttattccc atccatattt ttctgtgcct    420
taaggttctc aactaatctc atttaattta gctagcacac agagaaacac acacgtatat    480
aaataatatg ataacacaca aaaagactca tatatataaa taattagagt cattaaatgt    540
ggattcatca ttaaatgaaa caactcttct tctctgtaca atttctcttc acaccttcac    600
caaattcttt gacttcaaaa atcttataaa atttatatat ctccaaaacc ataaaaccaa    660
aacgagtttt cacaaataaa ttacttagtt gaaatttcaa atctcattca attagggtac    720
actctctcaa caatccacat taatgagggt tgctgcttct gatggcta                  768
```

<210> 63
<211> 779
<212> DNA
<213> Arabidopsis thaliana

<220>
<221> promoter
<222> (1)..(779)
<223> transcription regulating sequence from gene At4g00360

<400> 63
```
cttgtgggat taatcaattc actctttaac acttctttat accattgaag aaattagatg     60
aaagagtcac gagttgctta ccaaatccct cacaagaatt gagaactgat aaaccaaatt    120
gagaagatta aatatcacgt ctccttttga tctctattat aattaatcga aaataaaaat    180
aagagttttc aacaaaacgt gatcattggt ttacgatcac ttgcaaagtc aaacctaaaa    240
cgtagcatta gtacactaac cttaaatact aattatatca tgcaaaccct aatgtcatta    300
cctaactata catgtgtaat gtgttcaaca gatcttctta acccacatta gatcaatatt    360
aaacaataaa aagattctta tatattctac tacttacttc ttcttattcc catccatatt    420
tttctgtgcc tttaggttct caactaatct catttaattt agctagcaca cagagaaaca    480
cacacgtata taaataatat gataacacac aaaaagactc atatatataa ataattagag    540
tcattaaatg tggattcatc attaaatgaa acaactcttc ttctctgtac aatttctctt    600
cacaccttca ccaaattctt tgacttcaaa aatcttataa aatttatata tctccaaaac    660
```

```
cataaaacca aaacgagttt tcacaaataa attacttagt tgaaatttca aatctcattc    720
aattagggta cactctctca acaatccaca ttaatgaggg ttgctgcttc tgatggcta     779
```

<210> 64
<211> 2040
<212> DNA
<213> Arabidopsis thaliana

<220>
<221> promoter
<222> (1)..(2040)
<223> transcription regulating sequence from gene At4g00360

<400> 64

```
aaaaagagag tcaacattgc gttttaaatg cattttgagg tttggcaaac tatctacaag     60
ctatctgaca tctacagact acgtacagtt gtgttccatt tgttacaatt acaacaatca    120
gcttgtttgt ttttggcaaa ttaagagtca tgagtgtgta tacgaaatta ctttttggtg    180
gtctgaattt atgaatgcag ttacactaac ccttcttcaa aaataaaaat gaatgcggtt    240
atactaaact atcaaatata tctactagca aatatgaaac tacattcagt aacagtgatc    300
tctcttttttg gatacaagat gaaacattgt tctcttctca tgctctaaat agaaaatact    360
ctacgaaatg aaaatgtatg tcatcttata agaactatac atttttactc cgatggttaa    420
ttctttgaag gattctcgac agtttagaag tctgcaaaaa atataatctc ataagaaaaa    480
aattctaagt ctcttgatac aatgcatata gaaactgaca aataatcgaa gaaattgtac    540
ttgagcccat tacgactatt gaaaattctg attttggatg aattcagcgg aaactacaaa    600
tttaagagta ctttatgtgt ataatatgaa gcctatatat atagaagtac taatgtaata    660
aaataagaac cggtgaaaag ggggacaaga tcacaaggtt ttcgcattca gtgcctttac    720
agagttatat atttgatgat gttattgctt acttgcctaa tagtactata ctactatata    780
tatctaaggt aacacatgta tatatatgtc acatagacat tactagtata tattatgtac    840
ttctatcata tatttatgat attgcagttg cagcgtacac aagtcagctc cttttgactt    900
ttacatctca tgaatgcatt gccatgacat ctaatcttac tcgagatttg tgcatgcaca    960
ttattcactt ttgtcttttg caatttttgt tattgtaaaa aaaggaaaaa caaatgtaaa   1020
agagagagag accagaaagg tctaactaaa cctaaagagt caatgaaatg tgtttctttc   1080
ttgtgggatt aatcaattca ctctttaaca cttctttata ccattgaaga aattagatga   1140
aagagtcacg agttgcttac caaatccctc acaagaattg agaactgata aaccaaattg   1200
agaagattaa atatcacgtc tccttttgat ctctattata attaatcgaa aataaaaata   1260
agagttttca acaaaacgtg atcattggtt tacgatcact tgcaaagtca gacctaaaac   1320
gtagcattag tacactaacc ttaaatatta attatatcat gcaaacccta gtgtcattac   1380
ctaactatac atgtgtaatg tgttcaacag atcttcttaa cccacattag atcaatatta   1440
aacaataaaa aagattctta tatattctac tacttacttc ttcttattcc catccatatt   1500
tttctgtgcc ttaaggttct caactaatct catttaattt agctagcaca cagagaaaca   1560
cacacgtata taaataatat gataacacac aaaaagactc atatatataa ataattagag   1620
tcattaaatg tggattcatc attaaatgaa acaactcttc ttctctgtac aatttctctt   1680
cacaccttca ccaaattctt tgacttcaaa aatcttataa aatttatata tctccaaaac   1740
cataaaacca aaacgagttt tcacaaataa attacttagt tgaaatttca aatctcattc   1800
aattagggta cactctctca acaatccaca ttaatgaggg ttgctgcttc tgatggctag   1860
```

cagtaacagt tttatcgcct ccacttctta atgccatctt tttcctcttc cctctccttc 1920
tctatatata ttttctgact ctgcaaaacc ttaattcatc catctctcaa acaccatttt 1980
tggaaacacc atttcacatt ccttaaactt ttccatttta gtatcatttc atattcattg 2040

<210> 65
<211> 2053
<212> DNA
<213> Arabidopsis thaliana

<220>
<221> promoter
<222> (1)..(2053)
<223> transcription regulating sequence from gene At4g00360

<400> 65
taaaaaggat ttaaaaagag agtcaacatt gcgttttaaa tgcattttga ggtttggcaa 60
accatctaca agctatctga catctacaga ctacgtacag ttgtgttcca tttgttacaa 120
ttacaacaat cagcttgttt gtttttggca aattaagagt catgagtgtg tatacgaaat 180
tactttttgg tggtctgaat ttatgaatgc agttacacta acccttcttc aaaaataaaa 240
atgaatgcgg ttatactaaa ctatcaaata tatctactag caaatatgaa actacattca 300
gtaacagtga tctctctttt tggatacaag atgaaacatt gttctcttct catgctctaa 360
atagaaaata ctctacgaaa tgaaaatgta tgtcatctta taagaactat acatttttac 420
tccgatggtt aattctttga aggattctcg acagtttaga agtctgcaaa aaatataatc 480
tcataagaaa aaaattctaa gtctcttgat acaatgcata tagaaactga caaataatcg 540
aagaaattgt acttgagccc attacgacta ttgaaaattc tgattttgga tgaattcagc 600
ggaaactaca aatttaagag tactttatgt gtataatatg aagcctatat atatagaatt 660
actaatgtaa taaaataaga accggtgaaa aggggga caa gatcacaagg ttttcgcatt 720
cagtgccttt acagagttat atatttgatg atgttattgc ttacttgcct aatagtacta 780
tactactata tatatctaag gtaacacatg tatatatatg tcacatagac attactagta 840
tatattatgt acttctatca tatatttatg atattgcagt tgcagcgtac acaagtcagc 900
tccttttgac ttttacatct catgaatgca ttgccatgac atctaatctt actcgagatt 960
tgtgcatgca cattattcac ttttgtcttt tgcaattttt gttattgtaa aaaaaggaaa 1020
aacaaatgta aaagagagag agaccagaaa ggtctaacta aacctaaaga gtcaatgaaa 1080
tgtgtttctt tcttgtggga ttaatcaatt cactctttaa cacttcttta taccattgaa 1140
gaaattagat gaaagagtca cgagttgctt accaaatccc tcacaagaat tgagaactga 1200
taaaccaaat tgagaagatt aaatatcacg tctccttttg atctctatta taattaatcg 1260
aaaataaaaa taagagtttt caacaaaacg tgatcattgg tttacgatca cttgcaaagt 1320
caaacctaaa acgtagcatt agtacactaa ccttaaatac taattatatc atgcaaaccc 1380
taatgtcatt acctaactat acatgtgtaa tgtgttcaac agatcttctt aacccacatt 1440
agatcaatat taaacaataa aaagattctt atatattcta ctacttactt cttcttattc 1500
ccatccatat ttttctgtgc ctttaggttc tcaactaatc tcatttaatt tagctagcac 1560
acagagaaac acacacgtat ataaataata tgataacaca caaaaagact catatatata 1620
aataattaga gtcattaaat gtggattcat cattaaatga aacaactctt cttctctgta 1680
caatttctct tcacaccttc accaaattct ttgacttcaa aaatcttata aaatttatat 1740
atctccaaaa ccataaaacc aaaacgagtt ttcacaaata aattacttag ttgaaatttc 1800

```
aaatctcatt caattagggt acactctctc aacaatccac attaatgagg gttgctgctt    1860
ctgatggcta gcagtaacag ttttatcgcc tccacttctt aatgccatct ttttcctctt    1920
ccctctcctt ctctatatat attttctgac tctgcaaaac cttaattcat ccatctctca    1980
aacaccattt ttggaaacac catttcatat tccttaaact tttccatttt agtatcattt    2040
catattcatt gat                                                       2053

<210>  66
<211>  1859
<212>  DNA
<213>  Arabidopsis thaliana

<220>
<221>  promoter
<222>  (1)..(1859)
<223>  transcription regulating sequence from gene At4g00360

<400>  66
aaaaagagag tcaacattgc gttttaaatg cattttgagg tttggcaaac tatctacaag      60
ctatctgaca tctacagact acgtacagtt gtgttccatt tgttacaatt acaacaatca     120
gcttgtttgt ttttggcaaa ttaagagtca tgagtgtgta tacgaaatta ctttttggtg     180
gtctgaattt atgaatgcag ttacactaac ccttcttcaa aaataaaaat gaatgcggtt     240
atactaaact atcaaatata tctactagca aatatgaaac tacattcagt aacagtgatc     300
tctctttttg gatacaagat gaaacattgt tctcttctca tgctctaaat agaaaatact     360
ctacgaaatg aaaatgtatg tcatcttata agaactatac atttttactc cgatggttaa     420
ttctttgaag gattctcgac agtttagaag tctgcaaaaa atataatctc ataagaaaaa     480
aattctaagt ctcttgatac aatgcatata gaaactgaca aataatcgaa gaaattgtac     540
ttgagcccat tacgactatt gaaaattctg attttggatg aattcagcgg aaactacaaa     600
tttaagagta ctttatgtgt ataatatgaa gcctatatat atagaagtac taatgtaata     660
aaataagaac cggtgaaaag ggggacaaga tcacaaggtt ttcgcattca gtgcctttac     720
agagttatat atttgatgat gttattgctt acttgcctaa tagtactata ctactatata     780
tatctaaggt aacacatgta tatatatgtc acatagacat tactagtata tattatgtac     840
ttctatcata tatttatgat attgcagttg cagcgtacac aagtcagctc cttttgactt     900
ttacatctca tgaatgcatt gccatgacat ctaatcttac tcgagatttg tgcatgcaca     960
ttattcactt ttgtcttttg caatttttgt tattgtaaaa aaaggaaaaa caaatgtaaa    1020
agagagagag accagaaagg tctaactaaa cctaaagagt caatgaaatg tgtttctttc    1080
ttgtgggatt aatcaattca ctctttaaca cttctttata ccattgaaga aattagatga    1140
aagagtcacg agttgcttac caaatccctc acaagaattg agaactgata aaccaaattg    1200
agaagattaa atatcacgtc tccttttgat ctctattata attaatcgaa aataaaaata    1260
agagttttca acaaaacgtg atcattggtt tacgatcact tgcaaagtca gacctaaaac    1320
gtagcattag tacactaacc ttaaatatta attatatcat gcaaacccta gtgtcattac    1380
ctaactatac atgtgtaatg tgttcaacag atcttcttaa cccacattag atcaatatta    1440
aacaataaaa aagattctta tatattctac tacttacttc ttcttattcc catccatatt    1500
tttctgtgcc ttaaggttct caactaatct catttaattt agctagcaca cagagaaaca    1560
cacacgtata taaataatat gataacacac aaaaagactc atatatataa ataattagag    1620
tcattaaatg tggattcatc attaaatgaa acaactcttc ttctctgtac aatttctctt    1680
```

cacaccttca ccaaattctt tgacttcaaa aatcttataa aatttatata tctccaaaac 1740
cataaaacca aaacgagttt tcacaaataa attacttagt tgaaatttca aatctcattc 1800
aattagggta cactctctca acaatccaca ttaatgaggg ttgctgcttc tgatggcta 1859

<210> 67
<211> 1870
<212> DNA
<213> Arabidopsis thaliana

<220>
<221> promoter
<222> (1)..(1870)
<223> transcription regulating sequence from gene At4g00360

<400> 67
taaaaaggat ttaaaaagag agtcaacatt gcgttttaaa tgcattttga ggtttggcaa 60
accatctaca agctatctga catctacaga ctacgtacag ttgtgttcca tttgttacaa 120
ttacaacaat cagcttgttt gtttttggca aattaagagt catgagtgtg tatacgaaat 180
tactttttgg tggtctgaat ttatgaatgc agttacacta acccttcttc aaaaataaaa 240
atgaatgcgg ttatactaaa ctatcaaata tatctactag caaatatgaa actacattca 300
gtaacagtga tctctctttt tggatacaag atgaaacatt gttctcttct catgctctaa 360
atagaaaata ctctacgaaa tgaaaatgta tgtcatctta taagaactat acatttttac 420
tccgatggtt aattctttga aggattctcg acagtttaga agtctgcaaa aaatataatc 480
tcataagaaa aaaattctaa gtctcttgat acaatgcata tagaaactga caaataatcg 540
aagaaattgt acttgagccc attacgacta ttgaaaattc tgatttttgga tgaattcagc 600
ggaaactaca aatttaagag tactttatgt gtataatatg aagcctatat atatagaatt 660
actaatgtaa taaaataaga accggtgaaa agggggacaa gatcacaagg ttttcgcatt 720
cagtgccttt acagagttat atatttgatg atgttattgc ttacttgcct aatagtacta 780
tactactata tatatctaag gtaacacatg tatatatatg tcacatagac attactagta 840
tatattatgt acttctatca tatatttatg atattgcagt tgcagcgtac acaagtcagc 900
tccttttgac ttttacatct catgaatgca ttgccatgac atctaatctt actcgagatt 960
tgtgcatgca cattattcac ttttgtcttt tgcaattttt gttattgtaa aaaaaggaaa 1020
aacaaatgta aaagagagag agaccagaaa ggtctaacta aacctaaaga gtcaatgaaa 1080
tgtgtttctt tcttgtggga ttaatcaatt cactctttaa cacttcttta taccattgaa 1140
gaaattagat gaaagagtca cgagttgctt accaaatccc tcacaagaat tgagaactga 1200
taaaccaaat tgagaagatt aaatatcacg tctccttttg atctctatta taattaatcg 1260
aaaataaaaa taagagtttt caacaaaacg tgatcattgg tttacgatca cttgcaaagt 1320
caaacctaaa acgtagcatt agtacactaa ccttaaatac taattatatc atgcaaaccc 1380
taatgtcatt acctaactat acatgtgtaa tgtgttcaac agatcttctt aacccacatt 1440
agatcaatat taaacaataa aaagattctt atatattcta ctacttactt cttcttattc 1500
ccatccatat ttttctgtgc ctttaggttc tcaactaatc tcatttaatt tagctagcac 1560
acagagaaac acacacgtat ataaataata tgataacaca caaaaagact catatatata 1620
aataattaga gtcattaaat gtggattcat cattaaatga aacaactctt cttctctgta 1680
caatttctct tcacaccttc accaaattct ttgacttcaa aaatcttata aaatttatat 1740
atctccaaaa ccataaaacc aaaacgagtt ttcacaaata aattacttag ttgaaatttc 1800

aaatctcatt caattagggt acactctctc aacaatccac attaatgagg gttgctgctt 1860
ctgatggcta 1870

<210> 68
<211> 2996
<212> DNA
<213> Arabidopsis thaliana

<220>
<221> promoter
<222> (1)..(2996)
<223> transcription regulating sequence from gene At4g00360

<400> 68
agaaaaagaa tcaaattcct ccatgtcgtt ctgtatcgcc tccatgtacg taaagaaagt 60
tggtcactct tacggaaaga caccattttt accaagtctg accaatttat aaggaaagct 120
atttttcact tgccttgtag tctattaaat actagggatg gttcagcatc cgcaaacagt 180
aaatgagaaa ccgcatgact tgcatttacc acatttatac taattattaa cttatctatt 240
tatgctttct gaatattagc aatcagcact tttgtgcata gtacatataa atataaggag 300
agaggatctt cttgtcgtag gccgcattgg ggagtaatta agcctcttga ttgaccgtta 360
agtagcgcca tatgttagac tgatgtgata caccacatca tccattgtat ttatgtctcc 420
caacaatcca tttttcataa taaactctcg ataaaatttg attcaatctg atcaaaagcc 480
tttcatgtta gtttttataa ccataaactt tcttttgtat gacgaatttg ttcagagacc 540
ataaaacatc tcttgcgtta taagaatatt atctaaaatc aatggtcttt cgataaagac 600
tgattgcgtt tctaaaacaa aatttatttt tctatttttt tatttattta attttccctc 660
ttatttttag ttgtttttat aatatactaa gcgaggtagt ataaatctag aatcctgaac 720
cagcagtcaa atatcactat ttaaagctaa ctatttaggt ttaataatac cttaggatat 780
tattaccatt tacgtaaaaa agtttgaacc tggaaaacat atatgtctac gtagagtacc 840
gaatattcta aaccatttaa aatattttat aatgatcaac catgattgaa atcaagtaga 900
aaaacttgaa caatgatctt ctagttcatt ttagttgttt cacttaaaaa ggatttaaaa 960
agagagtcaa cattgcgttt taaatgcatt ttgaggtttg gcaaaccatc tacaagctat 1020
ctgacatcta cagactacgt acagttgtgt tccatttgtt acaattacaa caatcagctt 1080
gtttgttttt ggcaaattaa gagtcatgag tgtgtatacg aaattacttt ttggtggtct 1140
gaatttatga atgcagttac actaaccctt cttcaaaaat aaaaatgaat gcggttatac 1200
taaactatca aatatatcta ctagcaaata tgaaactaca ttcagtaaca gtgatctctc 1260
tttttggata caagatgaaa cattgttctc ttctcatgct ctaaatagaa aatactctac 1320
gaaatgaaaa tgtatgtcat cttataagaa ctatacattt ttactccgat ggttaattct 1380
ttgaaggatt ctcgacagtt tagaagtctg caaaaaatat aatctcataa gaaaaaaatt 1440
ctaagtctct tgatacaatg catatagaaa ctgacaaata atcgaagaaa ttgtacttga 1500
gcccattacg actattgaaa attctgattt tggatgaatt cagcggaaac tacaaattta 1560
agagtacttt atgtgtataa tatgaagcct atatatatag aagtactaat gtaataaaat 1620
aagaaccggt gaaaaggggg acaagatcac aaggttttcg cattcagtgc ctatacagag 1680
ttatatattt gatgatgtta ttgcttactt gcctgatagt actatactac tatatatatc 1740
taaggtaaca catgtatata tatgtcacat agacattact agtatatatt atgtacttct 1800
atcatatatt tatgatattg cagttgcagc gtacacaagt cagctccctt tgacttttac 1860

atctcatgaa tgcattgcca tgacatctaa tcttactcga gatttgtgca tgcgcattat 1920
tcacttttgt cttttgcaat ttttgttatt gtaaaagaag gaaaaacaaa tgtaaaagag 1980
agagagacca gaaaggtcta actaaaccta aagagtcaat gaaatgtgtt tctttcttgt 2040
gggattaatc aattcactct ttaacacttc tttataccat tgaagaaatt agatgaaaga 2100
gtcacgagtt gcttaccaaa tccctcacaa gaattgagaa ctgataaacc aaattgagaa 2160
gattaaatat cacgtctcct tttgatctct attataatta atcgaaaata aaaataagag 2220
ttttcaacaa aacgtgatca ttggtttacg atcacttgca aagtcagacc taaaacgtag 2280
cattagtaca ctaaccttaa atattaatta tatcatgcaa accctaatgt cattacctaa 2340
ctatacatgt gtaatgtgtt caacagatct tcttaaccca cattagatca atattaaaca 2400
ataaaaaaga ttcttatata ttctactact tacttcttct tattcccatc catatttttc 2460
tgtgccttaa ggttctcaac taatctcatt taatttagct agcacacaga gaaacacaca 2520
cgtatataaa taatatgata acacacaaaa agactcatat atataaataa ttagagtcat 2580
taaatgtgga ttcatcatta aatgaaacaa ctcttcttct ctgtacaatt tctcttcaca 2640
ccttcaccaa attctttgac ttcaaaaatc ttataaaatt tatatatctc caaaaccata 2700
aaaccaaaac gagttttcac aaataaatta cttagttgaa atttcaaatc tcattcaatt 2760
agggtacact ctctcaacaa tccacattaa tgagggttgc tgcttctgat ggctagcagt 2820
aacagtttta tcgcctccac ttcttaatgc catctttttc ctcttccctc tccttctcta 2880
tatatatttt ctgactctgc aaaaccttaa ttcatccatc tctcaaacac catttttgga 2940
aacaccattt cacattcctt aaacttttcc attttagtat catttcatat tcattg 2996

<210> 69
<211> 3008
<212> DNA
<213> Arabidopsis thaliana

<220>
<221> promoter
<222> (1)..(3008)
<223> transcription regulating sequence from gene At4g00360

<400> 69
aactaaggat aaagaaaaag aatcaaattc ctccatatcg ttctgtatcg cctccatgta 60
cgtaaagaaa gttggtcact cttacggaaa gacaccattt ttaccaggtc tgagcaattt 120
ataaggaaag ctatttttca cttgccttgt actctattaa atactaggga tgattcagca 180
tccgcaaaca gtaaatgaga aaccgcatga cttgcattta ccacctttat actaattatt 240
aacttatcta tttatgcttt ctgaatatta gcaatcagca cttttgtgca tagtacatat 300
aaatatgagg agagaggatc ttcttgtcgt aggccgcatt ggggagtaat taagcctctt 360
gattgaccgt taagtagcgc catatgttag actgatgtga tacaccacat catccattgt 420
atttatgtct cccaacaatc cattttttcat aataaactct cgataaaatt tgattcaatc 480
tgatcataag cctttcatgt tagtttttat aaccataaac tttcttttgt atgacgaatt 540
tgttcagaga ccataaaaca tctcttgcgt tataagaata ttatctaaaa tcaatggtct 600
ttcgataaag actaattgtg tttctaaaac aaaatttatt tttctatttt ctatttattt 660
aatttccctt ctcattttta gttgttttta taatatacta agcgaggtag tataaatcta 720
gaatcctgaa ccagcagtca aatatcacta tttaaagcta actatttagg tttaataata 780
ccttaggata ttattaccat ttacgtaaaa aagtttgaac ctggaaaaca tatatatcta 840

cgtagagtac cgaatattct aaaccattta aaatatttta taatgatcaa ccatgattga 900
aatcaagtag aaaaacttga acaatgatct tctagttcat tttagttgtt tcacttaaaa 960
aggatttaaa aagagagtca acattgcgtt ttaaatgcat tttgaggttt ggcaaaccat 1020
ctacaagcta tctgacatct acagactacg tacagttgtg ttccatttgt tacaattaca 1080
acaatcagct tgtttgtttt tggcaaatta agagtcatga gtgtgtatac gaaattactt 1140
tttggtggtc tgaatttatg aatgcagtta cactaaccct tcttcaaaaa taaaaatgaa 1200
tgcggttata ctaaactatc aaatatatct actagcaaat atgaaactac attcagtaac 1260
agtgatctct ctttttggat acaagatgaa acattgttct cttctcatgc tctaaataga 1320
aaatactcta cgaaatgaaa atgtatgtca tcttataaga actatacatt tttactccga 1380
tggttaattc tttgaaggat tctcgacagt ttagaagtct gcaaaaaata taatctcata 1440
agaaaaaaat tctaagtctc ttgatacaat gcatatagaa actgacaaat aatcgaagaa 1500
attgtacttg agcccattac gactattgaa aattctgatt ttggatgaat tcagcggaaa 1560
ctacaaattt aagagtactt tatgtgtata atatgaagcc tatatatata gaattactaa 1620
tgtaataaaa taagaaccgg tgaaaagggg gacaagatca caaggttttc gcattcagtg 1680
cctttacaga gttatatatt tgatgatgtt attgcttact tgcctaatag tactatacta 1740
ctatatatat ctaaggtaac acatgtatat atatgtcaca tagacattac tagtatatat 1800
tatgtacttc tatcatatat ttatgatatt gcagttgcag cgtacacaag tcagctcctt 1860
ttgactttta catctcatga atgcattgcc atgacatcta atcttactcg agatttgtgc 1920
atgcacatta ttcacttttg tcttttgcaa tttttgttat tgtaaaaaaa ggaaaaacaa 1980
atgtaaaaga gagagagacc agaaaggtct aactaaacct aaagagtcaa tgaaatgtgt 2040
ttctttcttg tgggattaat caattcactc tttaacactt ctttatacca ttgaagaaat 2100
tagatgaaag agtcacgagt tgcttaccaa atccctcaca agaattgaga actgataaac 2160
caaattgaga agattaaata tcacgtctcc ttttgatctc tattataatt aatcgaaaat 2220
aaaaataaga gttttcaaca aaacgtgatc attggtttac gatcacttgc aaagtcaaac 2280
ctaaaacgta gcattagtac actaacctta aatactaatt atatcatgca aaccctaatg 2340
tcattaccta actatacatg tgtaatgtgt tcaacagatc ttcttaaccc acattagatc 2400
aatattaaac aataaaaaga ttcttatata ttctactact tacttcttct tattcccatc 2460
catatttttc tgtgccttta ggttctcaac taatctcatt taatttagct agcacacaga 2520
gaaacacaca cgtatataaa taatatgata acacacaaaa agactcatat atataaataa 2580
ttagagtcat taaatgtgga ttcatcatta aatgaaacaa ctcttcttct ctgtacaatt 2640
tctcttcaca ccttcaccaa attctttgac ttcaaaaatc ttataaaatt tatatatctc 2700
caaaaccata aaaccaaaac gagttttcac aaataaatta cttagttgaa atttcaaatc 2760
tcattcaatt agggtacact ctctcaacaa tccacattaa tgagggttgc tgcttctgat 2820
ggctagcagt aacagtttta tcgcctccac ttcttaatgc catctttttc ctcttccctc 2880
tccttctcta tatatatttt ctgactctgc aaaaccttaa ttcatccatc tctcaaacac 2940
catttttgga aacaccattt catattcctt aaactttcc attttagtat catttcatat 3000
tcattgat 3008

<210> 70
<211> 2815
<212> DNA
<213> Arabidopsis thaliana

<220>
<221> promoter

<222> (1)..(2815)

<223> transcription regulating sequence from gene At4g00360

<400> 70

```
agaaaaagaa tcaaattcct ccatgtcgtt ctgtatcgcc tccatgtacg taaagaaagt     60
tggtcactct tacggaaaga caccattttt accaagtctg accaatttat aaggaaagct    120
atttttcact tgccttgtag tctattaaat actagggatg gttcagcatc cgcaaacagt    180
aaatgagaaa ccgcatgact tgcatttacc acatttatac taattattaa cttatctatt    240
tatgctttct gaatattagc aatcagcact tttgtgcata gtacatataa atataaggag    300
agaggatctt cttgtcgtag gccgcattgg ggagtaatta agcctcttga ttgaccgtta    360
agtagcgcca tatgttagac tgatgtgata caccacatca tccattgtat ttatgtctcc    420
caacaatcca tttttcataa taaactctcg ataaaatttg attcaatctg atcaaaagcc    480
tttcatgtta gtttttataa ccataaactt tcttttgtat gacgaatttg ttcagagacc    540
ataaaacatc tcttgcgtta taagaatatt atctaaaatc aatggtcttt cgataaagac    600
tgattgcgtt tctaaaacaa aatttatttt tctatttttt tatttattta attttccctc    660
ttatttttag ttgtttttat aatatactaa gcgaggtagt ataaatctag aatcctgaac    720
cagcagtcaa atatcactat ttaaagctaa ctatttaggt ttaataatac cttaggatat    780
tattaccatt tacgtaaaaa agtttgaacc tggaaaacat atatgtctac gtagagtacc    840
gaatattcta aaccatttaa aatattttat aatgatcaac catgattgaa atcaagtaga    900
aaaacttgaa caatgatctt ctagttcatt ttagttgttt cacttaaaaa ggatttaaaa    960
agagagtcaa cattgcgttt taaatgcatt ttgaggtttg gcaaaccatc tacaagctat   1020
ctgacatcta cagactacgt acagttgtgt tccatttgtt acaattacaa caatcagctt   1080
gtttgttttt ggcaaattaa gagtcatgag tgtgtatacg aaattacttt ttggtggtct   1140
gaatttatga atgcagttac actaaccctt cttcaaaaat aaaaatgaat gcggttatac   1200
taaactatca aatatatcta ctagcaaata tgaaactaca ttcagtaaca gtgatctctc   1260
tttttggata caagatgaaa cattgttctc ttctcatgct ctaaatagaa aatactctac   1320
gaaatgaaaa tgtatgtcat cttataagaa ctatacattt ttactccgat ggttaattct   1380
ttgaaggatt ctcgacagtt tagaagtctg caaaaaatat aatctcataa gaaaaaaatt   1440
ctaagtctct tgatacaatg catatagaaa ctgacaaata atcgaagaaa ttgtacttga   1500
gcccattacg actattgaaa attctgattt tggatgaatt cagcggaaac tacaaattta   1560
agagtacttt atgtgtataa tatgaagcct atatatatag aagtactaat gtaataaaat   1620
aagaaccggt gaaaaggggg acaagatcac aaggttttcg cattcagtgc ctatacagag   1680
ttatatattt gatgatgtta ttgcttactt gcctgatagt actatactac tatatatatc   1740
taaggtaaca catgtatata tatgtcacat agacattact agtatatatt atgtacttct   1800
atcatatatt tatgatattg cagttgcagc gtacacaagt cagctccctt tgacttttac   1860
atctcatgaa tgcattgcca tgacatctaa tcttactcga gatttgtgca tgcgcattat   1920
tcacttttgt cttttgcaat ttttgttatt gtaaaagaag gaaaaacaaa tgtaaaagag   1980
agagagacca gaaaggtcta actaaaccta aagagtcaat gaaatgtgtt tctttcttgt   2040
gggattaatc aattcactct ttaacacttc tttataccat tgaagaaatt agatgaaaga   2100
gtcacgagtt gcttaccaaa tccctcacaa gaattgagaa ctgataaacc aaattgagaa   2160
gattaaatat cacgtctcct tttgatctct attataatta atcgaaaata aaaataagag   2220
ttttcaacaa aacgtgatca ttggtttacg atcacttgca aagtcagacc taaaacgtag   2280
cattagtaca ctaaccttaa atattaatta tatcatgcaa accctaatgt cattacctaa   2340
ctatacatgt gtaatgtgtt caacagatct tcttaaccca cattagatca atattaaaca   2400
ataaaaaaga ttcttatata ttctactact tacttcttct tattcccatc catatttttc   2460
```

tgtgccttaa ggttctcaac taatctcatt taatttagct agcacacaga gaaacacaca 2520
cgtatataaa taatatgata acacacaaaa agactcatat atataaataa ttagagtcat 2580
taaatgtgga ttcatcatta aatgaaacaa ctcttcttct ctgtacaatt tctcttcaca 2640
ccttcaccaa attctttgac ttcaaaaatc ttataaaatt tatatatctc caaaaccata 2700
aaaccaaaac gagttttcac aaataaatta cttagttgaa atttcaaatc tcattcaatt 2760
agggtacact ctctcaacaa tccacattaa tgagggttgc tgcttctgat ggcta 2815

<210> 71
<211> 2825
<212> DNA
<213> Arabidopsis thaliana

<220>
<221> promoter
<222> (1)..(2825)
<223> transcription regulating sequence from gene At4g00360

<400> 71
aactaaggat aaagaaaaag aatcaaattc ctccatatcg ttctgtatcg cctccatgta 60
cgtaaagaaa gttggtcact cttacggaaa gacaccattt ttaccaggtc tgagcaattt 120
ataaggaaag ctatttttca cttgccttgt actctattaa atactaggga tgattcagca 180
tccgcaaaca gtaaatgaga aaccgcatga cttgcattta ccacctttat actaattatt 240
aacttatcta tttatgcttt ctgaatatta gcaatcagca cttttgtgca tagtacatat 300
aaatatgagg agagaggatc ttcttgtcgt aggccgcatt ggggagtaat taagcctctt 360
gattgaccgt taagtagcgc catatgttag actgatgtga tacaccacat catccattgt 420
atttatgtct cccaacaatc catttttcat aataaactct cgataaaatt tgattcaatc 480
tgatcataag cctttcatgt tagtttttat aaccataaac tttcttttgt atgacgaatt 540
tgttcagaga ccataaaaca tctcttgcgt tataagaata ttatctaaaa tcaatggtct 600
ttcgataaag actaattgtg tttctaaaac aaaatttatt tttctatttt ctatttattt 660
aattttcctt ctcattttta gttgttttta taatatacta agcgaggtag tataaatcta 720
gaatcctgaa ccagcagtca aatatcacta tttaaagcta actatttagg tttaataata 780
ccttaggata ttattaccat ttacgtaaaa aagtttgaac ctggaaaaca tatatatcta 840
cgtagagtac cgaatattct aaaccattta aaatatttta taatgatcaa ccatgattga 900
aatcaagtag aaaaacttga acaatgatct tctagttcat tttagttgtt tcacttaaaa 960
aggatttaaa aagagagtca acattgcgtt ttaaatgcat tttgaggttt ggcaaaccat 1020
ctacaagcta tctgacatct acagactacg tacagttgtg ttccatttgt tacaattaca 1080
acaatcagct tgtttgtttt tggcaaatta agagtcatga gtgtgtatac gaaattactt 1140
tttggtggtc tgaatttatg aatgcagtta cactaaccct tcttcaaaaa taaaaatgaa 1200
tgcggttata ctaaactatc aaatatatct actagcaaat atgaaactac attcagtaac 1260
agtgatctct ctttttggat acaagatgaa acattgttct cttctcatgc tctaaataga 1320
aaatactcta cgaaatgaaa atgtatgtca tcttataaga actatacatt tttactccga 1380
tggttaattc tttgaaggat tctcgacagt ttagaagtct gcaaaaaata taatctcata 1440
agaaaaaaat tctaagtctc ttgatacaat gcatatagaa actgacaaat aatcgaagaa 1500
attgtacttg agcccattac gactattgaa aattctgatt ttggatgaat tcagcggaaa 1560
ctacaaattt aagagtactt tatgtgtata atatgaagcc tatatatata gaattactaa 1620

```
tgtaataaaa taagaaccgg tgaaaagggg gacaagatca caaggttttc gcattcagtg   1680
cctttacaga gttatatatt tgatgatgtt attgcttact tgcctaatag tactatacta   1740
ctatatatat ctaaggtaac acatgtatat atatgtcaca tagacattac tagtatatat   1800
tatgtacttc tatcatatat ttatgatatt gcagttgcag cgtacacaag tcagctcctt   1860
ttgactttta catctcatga atgcattgcc atgacatcta atcttactcg agatttgtgc   1920
atgcacatta ttcacttttg tcttttgcaa tttttgttat tgtaaaaaaa ggaaaaacaa   1980
atgtaaaaga gagagagacc agaaaggtct aactaaacct aaagagtcaa tgaaatgtgt   2040
ttctttcttg tgggattaat caattcactc tttaacactt ctttatacca ttgaagaaat   2100
tagatgaaag agtcacgagt tgcttaccaa atccctcaca agaattgaga actgataaac   2160
caaattgaga agattaaata tcacgtctcc ttttgatctc tattataatt aatcgaaaat   2220
aaaaataaga gttttcaaca aaacgtgatc attggtttac gatcacttgc aaagtcaaac   2280
ctaaaacgta gcattagtac actaacctta aatactaatt atatcatgca aaccctaatg   2340
tcattaccta actatacatg tgtaatgtgt tcaacagatc ttcttaaccc acattagatc   2400
aatattaaac aataaaaaga ttcttatata ttctactact tacttcttct tattcccatc   2460
catatttttc tgtgccttta ggttctcaac taatctcatt taatttagct agcacacaga   2520
gaaacacaca cgtatataaa taatatgata acacacaaaa agactcatat atataaataa   2580
ttagagtcat taaatgtgga ttcatcatta aatgaaacaa ctcttcttct ctgtacaatt   2640
tctcttcaca ccttcaccaa attctttgac ttcaaaaatc ttataaaatt tatatatctc   2700
caaaaccata aaaccaaaac gagttttcac aaataaatta cttagttgaa atttcaaatc   2760
tcattcaatt agggtacact ctctcaacaa tccacattaa tgagggttgc tgcttctgat   2820
ggcta                                                               2825
```

<210> 72
<211> 2069
<212> DNA
<213> Arabidopsis thaliana

<220>
<221> CDS
<222> (184)..(1845)
<223> encoding putative cytochrome P450

<400> 72

```
gcagtaacag ttttatcgcc tccacttctt aatgccatct ttttcctctt ccctctcctt     60
ctctatatat attttctgac tctgcaaaac cttaattcat ccatctctca aacaccattt    120
ttggaaacac catttcatat tccttaaact tttccatttt agtatcattt catattcatt    180
gat atg gat gtc tcc aac acg atg ctc ctt gta gct gtt gtt gca gct      228
    Met Asp Val Ser Asn Thr Met Leu Leu Val Ala Val Val Ala Ala
    1               5                   10                  15
tac tgg cta tgg ttc cag cgg atc tca agg tgg cta aag ggt cca cgt      276
Tyr Trp Leu Trp Phe Gln Arg Ile Ser Arg Trp Leu Lys Gly Pro Arg
                20                  25                  30
gtt tgg cca gtt ttg ggc agt ctt ccg ggt ctg atc gag cag cgt gac      324
Val Trp Pro Val Leu Gly Ser Leu Pro Gly Leu Ile Glu Gln Arg Asp
            35                  40                  45
```

```
cgt atg cac gac tgg atc act gag aac ctc cgt gcg tgt ggc ggc act      372
Arg Met His Asp Trp Ile Thr Glu Asn Leu Arg Ala Cys Gly Gly Thr
        50                  55                  60
tat cag aca tgt atc tgc gcc gta cct ttc ttg gca aaa aag caa ggt      420
Tyr Gln Thr Cys Ile Cys Ala Val Pro Phe Leu Ala Lys Lys Gln Gly
    65                  70                  75
ctc gtg acc gtc acg tgc gat ccc aag aac atc gaa cac atg ctc aag      468
Leu Val Thr Val Thr Cys Asp Pro Lys Asn Ile Glu His Met Leu Lys
80                  85                  90                  95
acc agg ttc gac aac tac cct aaa ggt cct acg tgg caa gcc gtg ttc      516
Thr Arg Phe Asp Asn Tyr Pro Lys Gly Pro Thr Trp Gln Ala Val Phe
                100                 105                 110
cat gac ttc ctc ggc caa ggt atc ttc aac tcc gac ggt gac act tgg      564
His Asp Phe Leu Gly Gln Gly Ile Phe Asn Ser Asp Gly Asp Thr Trp
            115                 120                 125
ctc ttc cag cgt aaa acc gcc gct ctt gaa ttc acc acc agg acg ttg      612
Leu Phe Gln Arg Lys Thr Ala Ala Leu Glu Phe Thr Thr Arg Thr Leu
        130                 135                 140
agg caa gcg atg ggt cgg tgg gtg aac cgg gga atc aag ctc cgg ttt      660
Arg Gln Ala Met Gly Arg Trp Val Asn Arg Gly Ile Lys Leu Arg Phe
    145                 150                 155
tgt cca att ctc gaa acg gct cag aac aat tac gag ccg gtt gat ctc      708
Cys Pro Ile Leu Glu Thr Ala Gln Asn Asn Tyr Glu Pro Val Asp Leu
160                 165                 170                 175
caa gac tta ata cta cgg ctc aca ttc gac aac att tgc ggt tta gca      756
Gln Asp Leu Ile Leu Arg Leu Thr Phe Asp Asn Ile Cys Gly Leu Ala
                180                 185                 190
ttc ggt aaa gac act cga acc tgc gca ccg gga ctt ccc gag aac ggt      804
Phe Gly Lys Asp Thr Arg Thr Cys Ala Pro Gly Leu Pro Glu Asn Gly
            195                 200                 205
ttc gcc tcg gct ttc gac cga gcc acc gaa gct tct ctc cag cgg ttt      852
Phe Ala Ser Ala Phe Asp Arg Ala Thr Glu Ala Ser Leu Gln Arg Phe
        210                 215                 220
att cta cca gag ttt cta tgg agg ctg aag aaa tgg ctt gga ctc ggc      900
Ile Leu Pro Glu Phe Leu Trp Arg Leu Lys Lys Trp Leu Gly Leu Gly
    225                 230                 235
tta gaa gtc agc ttg agc cgg agc cta gga gag atc gat ggg tat tta      948
Leu Glu Val Ser Leu Ser Arg Ser Leu Gly Glu Ile Asp Gly Tyr Leu
240                 245                 250                 255
gat gct gtc att aat aca cgt aag caa gaa ttg ctg agt cag cga gag      996
Asp Ala Val Ile Asn Thr Arg Lys Gln Glu Leu Leu Ser Gln Arg Glu
                260                 265                 270
agt ggg gtc cag cgt cac gac gat ctc ctc tct cgt ttc atg aag aag     1044
Ser Gly Val Gln Arg His Asp Asp Leu Leu Ser Arg Phe Met Lys Lys
            275                 280                 285
```

```
aaa gac cag tcg tac agc gag acg ttc tta cga cac gtg gcg ctt aac      1092
Lys Asp Gln Ser Tyr Ser Glu Thr Phe Leu Arg His Val Ala Leu Asn
        290                 295                 300
ttc atc cta gct gga cgt gac acg tca tca gta gcg ttg agc tgg ttt      1140
Phe Ile Leu Ala Gly Arg Asp Thr Ser Ser Val Ala Leu Ser Trp Phe
    305                 310                 315
ttc tgg ctc atc acg acg cat ccg acg gtt gag gat aag atc gtc cgc      1188
Phe Trp Leu Ile Thr Thr His Pro Thr Val Glu Asp Lys Ile Val Arg
320                 325                 330                 335
gag ata tgc tcc gtt ctg att gag aca cgt gga acc gat gta tcg tcg      1236
Glu Ile Cys Ser Val Leu Ile Glu Thr Arg Gly Thr Asp Val Ser Ser
                340                 345                 350
tgg acg gcg gag ccg ttg gaa ttc gat gag gtc gac cgg ttg gtt tac      1284
Trp Thr Ala Glu Pro Leu Glu Phe Asp Glu Val Asp Arg Leu Val Tyr
            355                 360                 365
ctg aag gcc gcg ctc tct gag acg ttg agg ctt tac ccg tcg gtg ccg      1332
Leu Lys Ala Ala Leu Ser Glu Thr Leu Arg Leu Tyr Pro Ser Val Pro
        370                 375                 380
gaa gat tca aag cac gtc gtg aac gac gat atc tta ccg gac gga act      1380
Glu Asp Ser Lys His Val Val Asn Asp Asp Ile Leu Pro Asp Gly Thr
    385                 390                 395
ttc gta ccg gcg gga tcg tcg gtg act tat tcg atc tac gcg gcg ggg      1428
Phe Val Pro Ala Gly Ser Ser Val Thr Tyr Ser Ile Tyr Ala Ala Gly
400                 405                 410                 415
agg atg aag agc acg tgg gga gag gat tgc ttg gaa ttc aaa ccg gag      1476
Arg Met Lys Ser Thr Trp Gly Glu Asp Cys Leu Glu Phe Lys Pro Glu
                420                 425                 430
agg tgg atc tcg ccg gac gat gga aaa ttc gtg aat cac gat cag tac      1524
Arg Trp Ile Ser Pro Asp Asp Gly Lys Phe Val Asn His Asp Gln Tyr
            435                 440                 445
cga ttc gtg gcg ttt aac gcc gga cct agg atc tgt cta gga aaa gat      1572
Arg Phe Val Ala Phe Asn Ala Gly Pro Arg Ile Cys Leu Gly Lys Asp
        450                 455                 460
cta gcg tat ctg cag atg aag acg atc gcg gcg gcg gtt tta ctc agg      1620
Leu Ala Tyr Leu Gln Met Lys Thr Ile Ala Ala Ala Val Leu Leu Arg
    465                 470                 475
cat aga cta acg gtg gcg ccg gga cac aag gtg gag cag aag atg tcg      1668
His Arg Leu Thr Val Ala Pro Gly His Lys Val Glu Gln Lys Met Ser
480                 485                 490                 495
ttg act ttg ttc atg aag aac gga ctt ttg gtc aac gtg cac aag agg      1716
Leu Thr Leu Phe Met Lys Asn Gly Leu Leu Val Asn Val His Lys Arg
                500                 505                 510
gat ttg gaa gtg atg atg aag agt ctc gta ccc aaa gag aga aac gac      1764
Asp Leu Glu Val Met Met Lys Ser Leu Val Pro Lys Glu Arg Asn Asp
            515                 520                 525
```

```
gtc gtt gtt ctt aac gga aaa tgc aac ggc ggt atc ggt gaa ggc gtc      1812
Val Val Val Leu Asn Gly Lys Cys Asn Gly Gly Ile Gly Glu Gly Val
        530                 535                 540
gcc gtt aat gcg gct gtg gcg gtt gcc gtg taa tgcagcttct cgggttgata    1865
Ala Val Asn Ala Ala Val Ala Val Ala Val
    545                 550
acatttttat agtaattaat acatacattt acctttgggt tttgtgattg ttgtgtaaaa    1925
cagtaaaaaa aatgatttcg ttgtgctatg taaactttgt aagcaaatac tcttcttcct    1985
aaagtaaaaa agtttgattg tttttgtttt ggtttctttt tctccatccc catcaaattg    2045
taagcttcaa aagagtcaag ttcc                                           2069

<210>  73
<211>  553
<212>  PRT
<213>  Arabidopsis thaliana

<400>  73
Met Asp Val Ser Asn Thr Met Leu Leu Val Ala Val Val Ala Ala Tyr
1               5                   10                  15

Trp Leu Trp Phe Gln Arg Ile Ser Arg Trp Leu Lys Gly Pro Arg Val
            20                  25                  30

Trp Pro Val Leu Gly Ser Leu Pro Gly Leu Ile Glu Gln Arg Asp Arg
        35                  40                  45

Met His Asp Trp Ile Thr Glu Asn Leu Arg Ala Cys Gly Gly Thr Tyr
    50                  55                  60

Gln Thr Cys Ile Cys Ala Val Pro Phe Leu Ala Lys Lys Gln Gly Leu
65                  70                  75                  80

Val Thr Val Thr Cys Asp Pro Lys Asn Ile Glu His Met Leu Lys Thr
                85                  90                  95

Arg Phe Asp Asn Tyr Pro Lys Gly Pro Thr Trp Gln Ala Val Phe His
            100                 105                 110

Asp Phe Leu Gly Gln Gly Ile Phe Asn Ser Asp Gly Asp Thr Trp Leu
        115                 120                 125

Phe Gln Arg Lys Thr Ala Ala Leu Glu Phe Thr Thr Arg Thr Leu Arg
    130                 135                 140

Gln Ala Met Gly Arg Trp Val Asn Arg Gly Ile Lys Leu Arg Phe Cys
```

145 150 155 160

Pro Ile Leu Glu Thr Ala Gln Asn Asn Tyr Glu Pro Val Asp Leu Gln
165 170 175

Asp Leu Ile Leu Arg Leu Thr Phe Asp Asn Ile Cys Gly Leu Ala Phe
180 185 190

Gly Lys Asp Thr Arg Thr Cys Ala Pro Gly Leu Pro Glu Asn Gly Phe
195 200 205

Ala Ser Ala Phe Asp Arg Ala Thr Glu Ala Ser Leu Gln Arg Phe Ile
210 215 220

Leu Pro Glu Phe Leu Trp Arg Leu Lys Lys Trp Leu Gly Leu Gly Leu
225 230 235 240

Glu Val Ser Leu Ser Arg Ser Leu Gly Glu Ile Asp Gly Tyr Leu Asp
245 250 255

Ala Val Ile Asn Thr Arg Lys Gln Glu Leu Leu Ser Gln Arg Glu Ser
260 265 270

Gly Val Gln Arg His Asp Asp Leu Leu Ser Arg Phe Met Lys Lys Lys
275 280 285

Asp Gln Ser Tyr Ser Glu Thr Phe Leu Arg His Val Ala Leu Asn Phe
290 295 300

Ile Leu Ala Gly Arg Asp Thr Ser Ser Val Ala Leu Ser Trp Phe Phe
305 310 315 320

Trp Leu Ile Thr Thr His Pro Thr Val Glu Asp Lys Ile Val Arg Glu
325 330 335

Ile Cys Ser Val Leu Ile Glu Thr Arg Gly Thr Asp Val Ser Ser Trp
340 345 350

Thr Ala Glu Pro Leu Glu Phe Asp Glu Val Asp Arg Leu Val Tyr Leu
355 360 365

Lys Ala Ala Leu Ser Glu Thr Leu Arg Leu Tyr Pro Ser Val Pro Glu
370 375 380

Asp Ser Lys His Val Val Asn Asp Asp Ile Leu Pro Asp Gly Thr Phe

385 390 395 400

Val Pro Ala Gly Ser Ser Val Thr Tyr Ser Ile Tyr Ala Ala Gly Arg
405 410 415

Met Lys Ser Thr Trp Gly Glu Asp Cys Leu Glu Phe Lys Pro Glu Arg
420 425 430

Trp Ile Ser Pro Asp Asp Gly Lys Phe Val Asn His Asp Gln Tyr Arg
435 440 445

Phe Val Ala Phe Asn Ala Gly Pro Arg Ile Cys Leu Gly Lys Asp Leu
450 455 460

Ala Tyr Leu Gln Met Lys Thr Ile Ala Ala Ala Val Leu Leu Arg His
465 470 475 480

Arg Leu Thr Val Ala Pro Gly His Lys Val Glu Gln Lys Met Ser Leu
485 490 495

Thr Leu Phe Met Lys Asn Gly Leu Leu Val Asn Val His Lys Arg Asp
500 505 510

Leu Glu Val Met Met Lys Ser Leu Val Pro Lys Glu Arg Asn Asp Val
515 520 525

Val Val Leu Asn Gly Lys Cys Asn Gly Gly Ile Gly Glu Gly Val Ala
530 535 540

Val Asn Ala Ala Val Ala Val Ala Val
545 550

<210> 74
<211> 1564
<212> DNA
<213> Arabidopsis thaliana

<220>
<221> promoter
<222> (1)..(1564)
<223> transcription regulating sequence from gene At2g48030

<400> 74
aaaccagtct ctcacactaa agaaacactt tgtcatattt gtacatactg agcggaatat 60
tctgagggat ttgttttgtt ttcaatcagc ttgtagttga ttattgtagt tgcgacataa 120

```
tgaaaagagg agaaatgaga gatgggttca aagaagcatt ccacattcag aagtccaatc     180
tcctaaaaga ctaaaccagc caaaatgaaa agaggagaca gagccacaca tccgagacga     240
tgggccgaaa cacacatagt ataattgaat agtgggccgt ggcccaatat taaataatca     300
tcccttgatt attgattatc tatgacctca taaacgataa taattactaa ttagtagtct     360
acgaatgaga aatataataa aatagtcgtt ttctagtgtg tttgttaagt tgttatagat     420
caatatacaa gtatcattca ttggaagaca acaacatgta tatatctgag tgggaatgtg     480
gcagaaaaag gatttggata aaataagtac acgaagaaaa aagacagagt atgtaatgcc     540
gttttggtag gatggaaaca gcatgcagtt gttttggcaa ctgtattgta gtattagtat     600
tattagaaaa tccagctaag agattattag actttaccca tatctaaata agtcatcctt     660
ccttaccttt ttcatataca aaacaattaa cacttgacct aacttgcaac gctctctctc     720
gattccatcc ttttttgttc ttttcttttt gacacttttt ttttctttct ttcttttctt     780
aaatagatta ttattcaatc tttcctcctc catataataa taaaagtgat tgtgactgat     840
atcaaattcc ttatttaaat accacaatca cacagataca acatattact aattttaaat     900
tacagagcag ttggtccaca tatgcttgaa attgatagtc taattagttt taatgaggag     960
gtccatgtca aataggcctg acccaccagc tatatatata ccatgcatta aacctatata    1020
taaaaataaa atctctataa ctttttttgg gagttgttaa tttccttttc gttaattata    1080
gaatatgctc cctttctctt ctctgacgat ggttcataaa cctattcctt cgtagttgtt    1140
taacgtccat aataattgta tacttttagt tcgagattta cctaaacaaa acaaaaacag    1200
aataccatac tgtatttcat gatttctgta ttggaaaata aaagactgaa accaaatgat    1260
tggtgggctt ttctaaaata atagtaataa aatacaatta agacacacaa aaaagaaaag    1320
gatttggtac atttattaac atcgatgatg agttgataat gccacgtaag ccaaatacca    1380
ttttcccaag aagctcatat gaccttcttt tttgtgcagc aaactccaca ttccacaact    1440
ctctctctct ctcatttctt cgtataaaag gaaatctata tcatctctct ctctcattgt    1500
caaaactcaa aagtatatac ttttactagt tttagactta ataaatgcga tcgccctttt    1560
ttta                                                                  1564
```

<210> 75
<211> 1578
<212> DNA
<213> Arabidopsis thaliana

<220>
<221> promoter
<222> (1)..(1578)
<223> transcription regulating sequence from gene At2g48030

<400> 75

```
tgcttaggtc caaaaccagt ctctcacact aaagaaacac tttgtcatat ttgtacatac      60
tgagcggaat attctgaggg atttgttttg ttttcaatca gcttgtagtt gattattgta     120
gttgcgacat aatgaaaaga ggagaaatga gagatgggtt caaagaagca ttccacattc     180
agaagtccaa tctcctaaaa gactaaacca gccaaaatga aaagaggaga cagagccaca     240
catccgagac gatgggccga aacacacata gtataattga atgtgggccg tggcccaata     300
ttaaataatc atcccttgat tattgattat ctatgacctc ataaacgata ataattacta     360
attagtactc tacgaatgag aaatataata aaatagtcgt tttctagtgt gtttgttaag     420
ttgttataga tcaatataca agtatcattc attggaagac aacaacatgt atatatctga     480
```

```
gtgggaatgt ggcagaaaaa ggatttggat aaaataagta cacgaagaaa aaagacagag    540
tatgtaatgc cgttttggta ggatggaaac agcatgcagt tgttttggca actgtattgt    600
agtattagta ttattagaaa atccagctaa gagattatat tagactttac ccatatctaa    660
ataagtcatc cttccttacc tttttcatat acaaaacaat taacacttga cctaacttgc    720
aacgctctct ctcgattcca tccttttttg ttcttttctt tttgacactt tttttttct    780
ttctttcttt tcttaaatag attattattc aatctttcct cctccatata ataataaaag    840
tgattgtgac tgatatcaaa ttccttattt aaataccaca atcacacaga tacaacatat    900
tactaatttt aaattacaga gcagttggtc cacatatgct tgaaattgat agtctaatta    960
gttttaatga ggaggtccat gtcaaatagg cctgacccac tagctatata tataccatgc   1020
attaaaccta tatataaaaa taaaatctct ataacttttt ttgggagttg ttaatttcct   1080
tttcgttaat tatagaatat gctccctttc tcttctctga cgatggttca taaacctatt   1140
ccttcgtagt tgtttaacgt ccataataat tgtatacttt tagttcgaga tttacctaaa   1200
caaaacaaaa acagaatacc atactgtatt tcatgatttc tgtattggaa aataaaagac   1260
tgaaaccaaa tgattggtgg gcttttctaa aataatagta ataaaataca attaagacac   1320
acaaaaaaga aaaggatttg gtacatttat taacatcgat gatgagttga taatgccacg   1380
taagccaaat accattttcc caagaagctc atatgacctt ctttttgtg cagcaaactc   1440
cacattccac aactctctct ctctcatttc ttcgtataaa aggaaatcta tatcatctct   1500
ctctctcatt gtcaaaactc aaaagtatat acttttacta gttttagact taataaatgc   1560
gatcgccctt tttttagt                                                  1578
```

<210> 76
<211> 1404
<212> DNA
<213> Arabidopsis thaliana

<220>
<221> promoter
<222> (1)..(1404)
<223> transcription regulating sequence from gene At2g48030

<400> 76

```
aaaccagtct ctcacactaa agaaacactt tgtcatattt gtacatactg agcggaatat     60
tctgagggat ttgttttgtt ttcaatcagc ttgtagttga ttattgtagt tgcgacataa    120
tgaaaagagg agaaatgaga gatgggttca aagaagcatt ccacattcag aagtccaatc    180
tcctaaaaga ctaaaccagc caaaatgaaa agaggagaca gagccacaca tccgagacga    240
tgggccgaaa cacacatagt ataattgaat agtgggccgt ggcccaatat taaataatca    300
tcccttgatt attgattatc tatgacctca taaacgataa taattactaa ttagtagtct    360
acgaatgaga aatataataa aatagtcgtt ttctagtgtg tttgttaagt tgttatagat    420
caatatacaa gtatcattca ttggaagaca acaacatgta tatatctgag tgggaatgtg    480
gcagaaaaag gatttggata aaataagtac acgaagaaaa aagacagagt atgtaatgcc    540
gttttggtag gatggaaaca gcatgcagtt gttttggcaa ctgtattgta gtattagtat    600
tattagaaaa tccagctaag agattattag actttaccca tatctaaata agtcatcctt    660
ccttaccttt ttcatataca aaacaattaa cacttgacct aacttgcaac gctctctctc    720
gattccatcc tttttgttc ttttcttttt gacacttttt ttttctttct ttcttttctt    780
aaatagatta ttattcaatc tttcctcctc catataataa taaaagtgat tgtgactgat    840
```

```
atcaaattcc ttatttaaat accacaatca cacagataca acatattact aattttaaat     900
tacagagcag ttggtccaca tatgcttgaa attgatagtc taattagttt taatgaggag     960
gtccatgtca aataggcctg acccaccagc tatatatata ccatgcatta aacctatata    1020
taaaaataaa atctctataa cttttttgg gagttgttaa tttccttttc gttaattata    1080
gaatatgctc cctttctctt ctctgacgat ggttcataaa cctattcctt cgtagttgtt    1140
taacgtccat aataattgta tacttttagt tcgagattta cctaaacaaa acaaaaacag    1200
aataccatac tgtatttcat gatttctgta ttggaaaata aaagactgaa accaaatgat    1260
tggtgggctt ttctaaaata atagtaataa aatacaatta agacacacaa aaaagaaaag    1320
gatttggtac atttattaac atcgatgatg agttgataat gccacgtaag ccaaatacca    1380
ttttcccaag aagctcatat gacc                                           1404
```

<210> 77
<211> 1418
<212> DNA
<213> Arabidopsis thaliana

<220>
<221> promoter
<222> (1)..(1418)
<223> transcription regulating sequence from gene At2g48030

<400> 77

```
tgcttaggtc caaaaccagt ctctcacact aaagaaacac tttgtcatat ttgtacatac      60
tgagcggaat attctgaggg atttgttttg ttttcaatca gcttgtagtt gattattgta     120
gttgcgacat aatgaaaaga ggagaaatga gagatgggtt caaagaagca ttccacattc     180
agaagtccaa tctcctaaaa gactaaacca gccaaaatga aaagaggaga cagagccaca     240
catccgagac gatgggccga aacacacata gtataattga atgtgggccg tggcccaata     300
ttaaataatc atcccttgat tattgattat ctatgacctc ataaacgata ataattacta     360
attagtactc tacgaatgag aaatataata aaatagtcgt tttctagtgt gtttgttaag     420
ttgttataga tcaatataca agtatcattc attggaagac aacaacatgt atatatctga     480
gtgggaatgt ggcagaaaaa ggatttggat aaaataagta cacgaagaaa aaagacagag     540
tatgtaatgc cgttttggta ggatggaaac agcatgcagt tgttttggca actgtattgt     600
agtattagta ttattagaaa atccagctaa gagattatat tagactttac ccatatctaa     660
ataagtcatc cttccttacc tttttcatat acaaaacaat taacacttga cctaacttgc     720
aacgctctct ctcgattcca tcctttttg ttcttttctt tttgacactt ttttttttct     780
ttctttcttt tcttaaatag attattattc aatctttcct cctccatata ataataaaag     840
tgattgtgac tgatatcaaa ttccttattt aaataccaca atcacacaga tacaacatat     900
tactaatttt aaattacaga gcagttggtc cacatatgct tgaaattgat agtctaatta     960
gttttaatga ggaggtccat gtcaaatagg cctgacccac tagctatata tataccatgc    1020
attaaaccta tatataaaaa taaaatctct ataacttttt ttgggagttg ttaatttcct    1080
tttcgttaat tatagaatat gctccctttc tcttctctga cgatggttca taaacctatt    1140
ccttcgtagt tgtttaacgt ccataataat tgtatacttt tagttcgaga tttacctaaa    1200
caaaacaaaa acagaatacc atactgtatt tcatgatttc tgtattggaa aataaaagac    1260
tgaaaccaaa tgattggtgg gcttttctaa aataatagta ataaaataca attaagacac    1320
acaaaaaaga aaaggatttg gtacatttat taacatcgat gatgagttga taatgccacg    1380
```

```
taagccaaat accattttcc caagaagctc atatgacc                          1418

<210>  78
<211>  1784
<212>  DNA
<213>  Arabidopsis thaliana

<220>
<221>  CDS
<222>  (161)..(1477)
<223>  encoding endonuclease/exonuclease/phosphatase family protein

<400>  78
ttcttttttg tgcagcaaac tccacattcc acaactctct ctctctcatt tcttcgtata    60
aaaggaaatc tatatcatct ctctctctca ttgtcaaaac tcaaaagtat atacttttac   120
tagttttaga cttaataaat gcgatcgccc tttttttagt atg ttg aac ctc att     175
                                            Met Leu Asn Leu Ile
                                            1               5
gct ttc ctc cgc cgt cgt ctc cgc cgt ccc cgg aaa gcc aga atc tcc     223
Ala Phe Leu Arg Arg Arg Leu Arg Arg Pro Arg Lys Ala Arg Ile Ser
                10                  15                  20
gtc aac cac cac cac ctc tca gtt gat tct tct cct gag act cat cat     271
Val Asn His His His Leu Ser Val Asp Ser Ser Pro Glu Thr His His
            25                  30                  35
cat caa aat ggc ttt tca tcg gca gcc gcc atc cac cca aac ccg gac     319
His Gln Asn Gly Phe Ser Ser Ala Ala Ala Ile His Pro Asn Pro Asp
        40                  45                  50
aaa acc atc aca gtg gcc acc ttt aac gct gct atg ttc tcc atg gct     367
Lys Thr Ile Thr Val Ala Thr Phe Asn Ala Ala Met Phe Ser Met Ala
    55                  60                  65
ccc gcc gtc cct agc aac aag ggc ttg cca ttc cgg tcc aag tct acg     415
Pro Ala Val Pro Ser Asn Lys Gly Leu Pro Phe Arg Ser Lys Ser Thr
70                  75                  80                  85
gtt gac cgc ccc aag agc att ctc aag ccc atg aac gcc gcc gca tct     463
Val Asp Arg Pro Lys Ser Ile Leu Lys Pro Met Asn Ala Ala Ala Ser
                90                  95                  100
cct act cac gac tct aga aag caa cag agg ttt gcc aaa tcc agg cct     511
Pro Thr His Asp Ser Arg Lys Gln Gln Arg Phe Ala Lys Ser Arg Pro
            105                 110                 115
agg aga gtc tcc atc aat ctc ccc gac aac gag atc agc cgc cag ctc     559
Arg Arg Val Ser Ile Asn Leu Pro Asp Asn Glu Ile Ser Arg Gln Leu
        120                 125                 130
agc ttc cgg gag gat cca cag cac tct ccc ctc agg ccc ggc gag atc     607
Ser Phe Arg Glu Asp Pro Gln His Ser Pro Leu Arg Pro Gly Glu Ile
    135                 140                 145
```

```
ggt ctc cgg agc acc aga acg gct ctg gaa gtg ctg agt gag cta gac      655
Gly Leu Arg Ser Thr Arg Thr Ala Leu Glu Val Leu Ser Glu Leu Asp
150                 155                 160                 165
gca gac gtg ctg gct ctt caa gac gtc aag gcg gac gag gct gac caa      703
Ala Asp Val Leu Ala Leu Gln Asp Val Lys Ala Asp Glu Ala Asp Gln
                170                 175                 180
atg aga ccg ctc tcc gat ctc gct gcg gcg ctg ggg atg aat tac gtc      751
Met Arg Pro Leu Ser Asp Leu Ala Ala Ala Leu Gly Met Asn Tyr Val
            185                 190                 195
ttc gcc gag agc tgg gcg ccg gag tac ggc aac gcc att ctc tct aag      799
Phe Ala Glu Ser Trp Ala Pro Glu Tyr Gly Asn Ala Ile Leu Ser Lys
        200                 205                 210
tgg ccc atc aaa agc tcc aat gtt cta aga atc ttc gac cac act gat      847
Trp Pro Ile Lys Ser Ser Asn Val Leu Arg Ile Phe Asp His Thr Asp
    215                 220                 225
ttc agg aac gtg ttg aag gcg agc ata gag gtt ccc ggg agc ggg gaa      895
Phe Arg Asn Val Leu Lys Ala Ser Ile Glu Val Pro Gly Ser Gly Glu
230                 235                 240                 245
gtg gag ttt cac tgc act cat ctg gat cac ttg gac gag aag tgg aga      943
Val Glu Phe His Cys Thr His Leu Asp His Leu Asp Glu Lys Trp Arg
                250                 255                 260
atg aag caa gtc gat gcc att atc caa tcc acc aac gta ccg cac ata      991
Met Lys Gln Val Asp Ala Ile Ile Gln Ser Thr Asn Val Pro His Ile
            265                 270                 275
ctc gct ggt gct ctc aat tct ctc gac gaa tcc gat tat tct cct gag     1039
Leu Ala Gly Ala Leu Asn Ser Leu Asp Glu Ser Asp Tyr Ser Pro Glu
        280                 285                 290
aga tgg acc gac atc gtc aag tat tac gaa gag atg ggt aag cca ata     1087
Arg Trp Thr Asp Ile Val Lys Tyr Tyr Glu Glu Met Gly Lys Pro Ile
    295                 300                 305
cca aaa gca caa gtg atg aga ttc tta aag agc aaa gaa tac act gac     1135
Pro Lys Ala Gln Val Met Arg Phe Leu Lys Ser Lys Glu Tyr Thr Asp
310                 315                 320                 325
gct aag gac ttt gct gga gaa tgc gaa tct gtg gtt gtt gtc gcc aaa     1183
Ala Lys Asp Phe Ala Gly Glu Cys Glu Ser Val Val Val Val Ala Lys
                330                 335                 340
ggc caa agt gtg cag ggg aca tgc aag tac ggg aca cgc gtg gac tac     1231
Gly Gln Ser Val Gln Gly Thr Cys Lys Tyr Gly Thr Arg Val Asp Tyr
            345                 350                 355
ata ctt gct tcc tcc gat tca ccg tac cgg ttc gtg cca ggg tcg tat     1279
Ile Leu Ala Ser Ser Asp Ser Pro Tyr Arg Phe Val Pro Gly Ser Tyr
        360                 365                 370
tcc gtg ttg tct tcc aaa gga acc tcg gac cat cac ata gtc aaa gtc     1327
Ser Val Leu Ser Ser Lys Gly Thr Ser Asp His His Ile Val Lys Val
    375                 380                 385
```

```
gat gtt gta aaa gct aca tcg atc aac gtc aac gag cag gag cag cga      1375
Asp Val Val Lys Ala Thr Ser Ile Asn Val Asn Glu Gln Glu Gln Arg
390                 395                 400                 405
ccg ata aga tca cat aag ctg cag aga att aca gcg act acg tat aat      1423
Pro Ile Arg Ser His Lys Leu Gln Arg Ile Thr Ala Thr Thr Tyr Asn
                410                 415                 420
aat aac tcg tct ctc aca aag gcc tcg tgg agg aca cac tac tat aaa      1471
Asn Asn Ser Ser Leu Thr Lys Ala Ser Trp Arg Thr His Tyr Tyr Lys
            425                 430                 435
gca tga acaatcaaca tatatattac aaaatgttac aggttttttt tcgactgttg       1527
Ala

gttgattaac acaatttgag atctgtgtat agttaataat cgtgctagtc tctgtgttga   1587
acgtgaagtc tgatatactt ttgcctagat ttccattttt tctacatcct aattttggtc   1647
tgcttttaat gttaattaga caaggtggct tctttaaccg aaacctgtag tattcatgag   1707
tgagtaagta ctactgtact cttgtttttt tttctgtttg tagtttctca aaatgagatt   1767
aattagcttt cttgaaa                                                  1784

<210>  79
<211>  438
<212>  PRT
<213>  Arabidopsis thaliana

<400>  79
Met Leu Asn Leu Ile Ala Phe Leu Arg Arg Arg Leu Arg Arg Pro Arg
1               5                   10                  15

Lys Ala Arg Ile Ser Val Asn His His His Leu Ser Val Asp Ser Ser
            20                  25                  30

Pro Glu Thr His His His Gln Asn Gly Phe Ser Ser Ala Ala Ala Ile
        35                  40                  45

His Pro Asn Pro Asp Lys Thr Ile Thr Val Ala Thr Phe Asn Ala Ala
    50                  55                  60

Met Phe Ser Met Ala Pro Ala Val Pro Ser Asn Lys Gly Leu Pro Phe
65                  70                  75                  80

Arg Ser Lys Ser Thr Val Asp Arg Pro Lys Ser Ile Leu Lys Pro Met
                85                  90                  95

Asn Ala Ala Ala Ser Pro Thr His Asp Ser Arg Lys Gln Gln Arg Phe
            100                 105                 110
```

```
Ala Lys Ser Arg Pro Arg Arg Val Ser Ile Asn Leu Pro Asp Asn Glu
        115                 120                 125

Ile Ser Arg Gln Leu Ser Phe Arg Glu Asp Pro Gln His Ser Pro Leu
    130                 135                 140

Arg Pro Gly Glu Ile Gly Leu Arg Ser Thr Arg Thr Ala Leu Glu Val
145                 150                 155                 160

Leu Ser Glu Leu Asp Ala Asp Val Leu Ala Leu Gln Asp Val Lys Ala
                165                 170                 175

Asp Glu Ala Asp Gln Met Arg Pro Leu Ser Asp Leu Ala Ala Ala Leu
            180                 185                 190

Gly Met Asn Tyr Val Phe Ala Glu Ser Trp Ala Pro Glu Tyr Gly Asn
        195                 200                 205

Ala Ile Leu Ser Lys Trp Pro Ile Lys Ser Ser Asn Val Leu Arg Ile
    210                 215                 220

Phe Asp His Thr Asp Phe Arg Asn Val Leu Lys Ala Ser Ile Glu Val
225                 230                 235                 240

Pro Gly Ser Gly Glu Val Glu Phe His Cys Thr His Leu Asp His Leu
                245                 250                 255

Asp Glu Lys Trp Arg Met Lys Gln Val Asp Ala Ile Ile Gln Ser Thr
            260                 265                 270

Asn Val Pro His Ile Leu Ala Gly Ala Leu Asn Ser Leu Asp Glu Ser
        275                 280                 285

Asp Tyr Ser Pro Glu Arg Trp Thr Asp Ile Val Lys Tyr Tyr Glu Glu
    290                 295                 300

Met Gly Lys Pro Ile Pro Lys Ala Gln Val Met Arg Phe Leu Lys Ser
305                 310                 315                 320

Lys Glu Tyr Thr Asp Ala Lys Asp Phe Ala Gly Glu Cys Glu Ser Val
                325                 330                 335

Val Val Val Ala Lys Gly Gln Ser Val Gln Gly Thr Cys Lys Tyr Gly
            340                 345                 350
```

```
Thr Arg Val Asp Tyr Ile Leu Ala Ser Ser Asp Ser Pro Tyr Arg Phe
        355                 360                 365

Val Pro Gly Ser Tyr Ser Val Leu Ser Ser Lys Gly Thr Ser Asp His
    370                 375                 380

His Ile Val Lys Val Asp Val Val Lys Ala Thr Ser Ile Asn Val Asn
385                 390                 395                 400

Glu Gln Glu Gln Arg Pro Ile Arg Ser His Lys Leu Gln Arg Ile Thr
                405                 410                 415

Ala Thr Thr Tyr Asn Asn Asn Ser Ser Leu Thr Lys Ala Ser Trp Arg
            420                 425                 430

Thr His Tyr Tyr Lys Ala
        435

<210>  80
<211>  524
<212>  DNA
<213>  Arabidopsis thaliana

<220>
<221>  promoter
<222>  (1)..(524)
<223>  transcription regulating sequence from gene At2g38590

<400>  80
agtttttgta aaactcaaca atacattgga ggcagagaag atgcatcatc aaatctatat      60
gtctccctct ctcttttata atttgaaggg acctgagaat cattgattag ctttgcacaa     120
tatttagagt acacaaatgt ttattgtaat tttttgatag gataacttgg tgatgacaat     180
gatgttgatt actgttgttt tgttctccat ctttttcaga aaatcttgaa agttgaaaca     240
taaatcttaa gcatctgagc aaagacaaga ctgatgtatg caacaaactc aattatgtct     300
tacataagct ggtccagttt atttttttctt caaagtagga ccgtaaaagt gaagccataa    360
aacaaacata acatcattaa aagcctaact agattctaaa gcccacgatt agcccagctt     420
tgtttagggt ttttgtgtat cttcaattgg tgtgttggtg tatactctca tatttccctt     480
tgccgcagag aaagaaaaaa aaaaatcatt gttgcaattt gaaa                      524

<210>  81
<211>  539
<212>  DNA
<213>  Arabidopsis thaliana

<220>
```

<221> promoter
<222> (1)..(539)
<223> transcription regulating sequence from gene At2g38590

<400> 81
cggcaaagaa atagtttttg taaaactcaa caatacattg gaggcagaga agatgcatca 60
tcaaatctat atgtctccct ctctctttta taatttgaag ggacctgaga atcattgatt 120
agctttgcac aatatttaga gtacacaaat gtttattgta attttttgat aggataactt 180
ggtgatgaca atgatgttga ttactgttgt tttgttctcc atctttttca gaaaatcttg 240
aaagttgaaa cataaatctt aagcatctga gcaaagacaa gactgatgta tgcaacaaac 300
tcaattatgt cttacataag ctggtccagt ttattttttc ttcaaagtag gaccgtaaaa 360
gtgaagccat aaaacaaaca taacatcatt aaaagcctaa ctagattcta aagcccacga 420
ttagcccagc tttgtttagg gtttttgtgt atcttcaatt ggtgtgttgg tgtatactct 480
cataatttcc ctttgccgca gagaaagaaa aaaaaaaatc attgttgcaa tttgaaagt 539

<210> 82
<211> 1288
<212> DNA
<213> Arabidopsis thaliana

<220>
<221> CDS
<222> (1)..(1275)
<223> encoding F-box family protein

<400> 82
atg acg acg atg atc tct aat ctt cca agg gtt ttg ata gag gag att 48
Met Thr Thr Met Ile Ser Asn Leu Pro Arg Val Leu Ile Glu Glu Ile
1 5 10 15
ttt ttt agg gtt cct ttg aaa tct ttg aga gca gtg aga tta act tgc 96
Phe Phe Arg Val Pro Leu Lys Ser Leu Arg Ala Val Arg Leu Thr Cys
20 25 30
aaa agt tgg aac act cta tcc aaa agt agg agc ttt agg aag ttg tac 144
Lys Ser Trp Asn Thr Leu Ser Lys Ser Arg Ser Phe Arg Lys Leu Tyr
35 40 45
att agt aaa aga gca aca aga gaa gag gag tcc atg atg atc gct atg 192
Ile Ser Lys Arg Ala Thr Arg Glu Glu Glu Ser Met Met Ile Ala Met
50 55 60
atg aat ttc gat ctt tat tca atg agg gtc gta gtt gac gac gac gtt 240
Met Asn Phe Asp Leu Tyr Ser Met Arg Val Val Val Asp Asp Asp Val
65 70 75 80
gat cca tct aaa gcg ttt aaa aaa aaa agg aat aaa aaa tct ata gcg 288
Asp Pro Ser Lys Ala Phe Lys Lys Lys Arg Asn Lys Lys Ser Ile Ala
85 90 95
ttt aaa cgt caa cct att ttc ctt gat gaa caa gtc aag ata tct caa 336

```
Phe Lys Arg Gln Pro Ile Phe Leu Asp Glu Gln Val Lys Ile Ser Gln
            100                 105                 110
gtt ttt cac tgt gaa ggt tta ttg tta tgc ttc tta aaa gaa gac gat      384
Val Phe His Cys Glu Gly Leu Leu Leu Cys Phe Leu Lys Glu Asp Asp
        115                 120                 125
aca agg gtt gtc gtt tgg aat ccg tat tgc gga caa aca agg tgg atc      432
Thr Arg Val Val Val Trp Asn Pro Tyr Cys Gly Gln Thr Arg Trp Ile
    130                 135                 140
caa ctc aga tat tct cac cgt cca cac aaa gac agg ttc att tac gct      480
Gln Leu Arg Tyr Ser His Arg Pro His Lys Asp Arg Phe Ile Tyr Ala
145                 150                 155                 160
cta gga tac aag gat aag gaa tct cgt ggt agc ttc caa tta ttg agg      528
Leu Gly Tyr Lys Asp Lys Glu Ser Arg Gly Ser Phe Gln Leu Leu Arg
                165                 170                 175
ttt gta gat tat ttc cta gga gca ccc aaa aat caa tat ttt tgg tat      576
Phe Val Asp Tyr Phe Leu Gly Ala Pro Lys Asn Gln Tyr Phe Trp Tyr
            180                 185                 190
gaa att tac gat ttt aac tct gat tca tgg aca act ctt gat gtc act      624
Glu Ile Tyr Asp Phe Asn Ser Asp Ser Trp Thr Thr Leu Asp Val Thr
        195                 200                 205
cca cac tgg tgt ata tac tgt tgt gac cgt ggt gtt tct ctc aac gga      672
Pro His Trp Cys Ile Tyr Cys Cys Asp Arg Gly Val Ser Leu Asn Gly
    210                 215                 220
aac act tac tgg tgt gct aaa gaa agg aaa gca gaa gac gat att gtc      720
Asn Thr Tyr Trp Cys Ala Lys Glu Arg Lys Ala Glu Asp Asp Ile Val
225                 230                 235                 240
gat cac atc ata tct ttt gat ttt aca aac gag aga ttt ggg ccg ctt      768
Asp His Ile Ile Ser Phe Asp Phe Thr Asn Glu Arg Phe Gly Pro Leu
                245                 250                 255
ttg cct ttg ccg tct aag gtt atg gaa cat gaa tat gaa att gta act      816
Leu Pro Leu Pro Ser Lys Val Met Glu His Glu Tyr Glu Ile Val Thr
            260                 265                 270
tta tct tat gtt aaa gaa gag aag ctt gca gct tta ttt cag cat tac      864
Leu Ser Tyr Val Lys Glu Glu Lys Leu Ala Ala Leu Phe Gln His Tyr
        275                 280                 285
gaa gca gat tgg aat gag ttt gat ata tgg att acg act aag att gat      912
Glu Ala Asp Trp Asn Glu Phe Asp Ile Trp Ile Thr Thr Lys Ile Asp
    290                 295                 300
gct gaa gtg gtg tcg tgg agc atg ttc ttg aga atg gat acg gga cct      960
Ala Glu Val Val Ser Trp Ser Met Phe Leu Arg Met Asp Thr Gly Pro
305                 310                 315                 320
agg ata gag gtt cca cat ata tgt gaa ggt ttc ttc att gat gag gag     1008
Arg Ile Glu Val Pro His Ile Cys Glu Gly Phe Phe Ile Asp Glu Glu
                325                 330                 335
aag aaa gtc gcc atg ggt ttt gaa gaa gac ttc gat cgc aaa aca ttt     1056
```

Lys Lys Val Ala Met Gly Phe Glu Glu Asp Phe Asp Arg Lys Thr Phe
340 345 350

atc atc att gga gaa gct gga tac gta aga aaa ttg gat atc aaa gca 1104
Ile Ile Ile Gly Glu Ala Gly Tyr Val Arg Lys Leu Asp Ile Lys Ala
355 360 365

cat gta gac aga aaa tgt cgg cca act gtg tgt tct tat gtt cca agt 1152
His Val Asp Arg Lys Cys Arg Pro Thr Val Cys Ser Tyr Val Pro Ser
370 375 380

ttg gtc caa atc aag aaa cct gca cga ggc aaa agg aaa aga caa agc 1200
Leu Val Gln Ile Lys Lys Pro Ala Arg Gly Lys Arg Lys Arg Gln Ser
385 390 395 400

agc tta gaa aag cgt cta ttt gat caa aac atg ttg aga ctt gaa gca 1248
Ser Leu Glu Lys Arg Leu Phe Asp Gln Asn Met Leu Arg Leu Glu Ala
405 410 415

ttt aaa aag cta ggc ggc tac ttc tag aagcaatatg cag 1288
Phe Lys Lys Leu Gly Gly Tyr Phe
420

<210> 83
<211> 424
<212> PRT
<213> Arabidopsis thaliana

<400> 83

Met Thr Thr Met Ile Ser Asn Leu Pro Arg Val Leu Ile Glu Glu Ile
1 5 10 15

Phe Phe Arg Val Pro Leu Lys Ser Leu Arg Ala Val Arg Leu Thr Cys
20 25 30

Lys Ser Trp Asn Thr Leu Ser Lys Ser Arg Ser Phe Arg Lys Leu Tyr
35 40 45

Ile Ser Lys Arg Ala Thr Arg Glu Glu Glu Ser Met Met Ile Ala Met
50 55 60

Met Asn Phe Asp Leu Tyr Ser Met Arg Val Val Val Asp Asp Asp Val
65 70 75 80

Asp Pro Ser Lys Ala Phe Lys Lys Lys Arg Asn Lys Lys Ser Ile Ala
85 90 95

Phe Lys Arg Gln Pro Ile Phe Leu Asp Glu Gln Val Lys Ile Ser Gln
100 105 110

Val Phe His Cys Glu Gly Leu Leu Leu Cys Phe Leu Lys Glu Asp Asp
115 120 125

Thr Arg Val Val Val Trp Asn Pro Tyr Cys Gly Gln Thr Arg Trp Ile
130 135 140

Gln Leu Arg Tyr Ser His Arg Pro His Lys Asp Arg Phe Ile Tyr Ala
145 150 155 160

Leu Gly Tyr Lys Asp Lys Glu Ser Arg Gly Ser Phe Gln Leu Leu Arg
165 170 175

Phe Val Asp Tyr Phe Leu Gly Ala Pro Lys Asn Gln Tyr Phe Trp Tyr
180 185 190

Glu Ile Tyr Asp Phe Asn Ser Asp Ser Trp Thr Thr Leu Asp Val Thr
195 200 205

Pro His Trp Cys Ile Tyr Cys Cys Asp Arg Gly Val Ser Leu Asn Gly
210 215 220

Asn Thr Tyr Trp Cys Ala Lys Glu Arg Lys Ala Glu Asp Asp Ile Val
225 230 235 240

Asp His Ile Ile Ser Phe Asp Phe Thr Asn Glu Arg Phe Gly Pro Leu
245 250 255

Leu Pro Leu Pro Ser Lys Val Met Glu His Glu Tyr Glu Ile Val Thr
260 265 270

Leu Ser Tyr Val Lys Glu Glu Lys Leu Ala Ala Leu Phe Gln His Tyr
275 280 285

Glu Ala Asp Trp Asn Glu Phe Asp Ile Trp Ile Thr Thr Lys Ile Asp
290 295 300

Ala Glu Val Val Ser Trp Ser Met Phe Leu Arg Met Asp Thr Gly Pro
305 310 315 320

Arg Ile Glu Val Pro His Ile Cys Glu Gly Phe Phe Ile Asp Glu Glu
325 330 335

Lys Lys Val Ala Met Gly Phe Glu Glu Asp Phe Asp Arg Lys Thr Phe
340 345 350

```
Ile Ile Ile Gly Glu Ala Gly Tyr Val Arg Lys Leu Asp Ile Lys Ala
        355                 360                 365

His Val Asp Arg Lys Cys Arg Pro Thr Val Cys Ser Tyr Val Pro Ser
    370                 375                 380

Leu Val Gln Ile Lys Lys Pro Ala Arg Gly Lys Arg Lys Arg Gln Ser
385                 390                 395                 400

Ser Leu Glu Lys Arg Leu Phe Asp Gln Asn Met Leu Arg Leu Glu Ala
                405                 410                 415

Phe Lys Lys Leu Gly Gly Tyr Phe
            420
```

<210> 84
<211> 1988
<212> DNA
<213> Arabidopsis thaliana

<220>
<221> promoter
<222> (1)..(1988)
<223> transcription regulating sequence from gene At1g23000

<400> 84

```
atttaattat agtgatgaga aaaggactaa atacattttt tttttggttg tcaacaagaa      60
catatgatat aaagctctaa caaaaaaaga agaactaaat atgaaaatat gatcagagga     120
caggtaagta cattgtcggg tgatgacatg actaattatt aatttcgacc ccaattttaa     180
aacatacata ttctaaaatt ctattatggc atgtgaaatt gtgaaaagac acaaaactaa     240
tggaccgatg tggagtaaca cagaaaacgc gtggcgtaga gaggacttga taattaggag     300
tcaaggttgc gttgatgctg tgaggtccag ggttttgtag cttttcttct attgtcacgt     360
gtacggtgtt tgggaaatga acggctaaat cattcaactt gaaacactaa tcacggcatt     420
tcacgaaata agaggataat attggtatat gcagtttaca tcaagagtta aataccataa     480
tgtaatgttg gctacggatc taacaaatca tgtgagcaat acgagttttg gtaactataa     540
ttttcgtgaa aggaacatat attggactat tggaggtagc tggcaactct caaatcttaa     600
agtgaagtat atttttttgta tgcaaaatca agatgatagt atgtatgatg atcatgtata     660
tatttattgt ggacagttaa attcgtttga tgtagataaa tgcactgatt attgatttgt     720
atgcggtcaa aactatatag taatcgagcc atcgaggtgg tgtggtttat tggtgttatt     780
cgagtggtag aaatttttct atcaaggggg aaagtcctac tatctacgac cattattgct     840
cttttatttt gaccagcata ctcattttaa ctgacaagac tagactgcat gacatttcat     900
agactttggg attagtatgt gttttagctc atgatctagg ttaaaaaaga gaatgtgatt     960
attagtaaat atattttaat agctaaccaa atataatgta atcacgtaat ctttgatcat    1020
atgtatgatt tgtaaactca cattagatga agttgaaaat ccatcgaggg tttgcgattt    1080
ttttaatgtt tttttattta tgtgttatat agatttggaa agaaattaag tttaagtggg    1140
```

ggaatcaaag ggccaacatt gaacagccag actaaaaaac attaaacaga ttaatgggct 1200
taatgcgtaa tgcgcctctt tttttgttta aggcccataa tagttttatt taaaaggccg 1260
aaatgtaagg caacagtagc actacactaa caaggtcagt cagaatgtgt cactggccac 1320
tgctcataaa tcaagaaatc aaaatattat atgtaatttt tttttctta aaaccaaaaa 1380
cgtagtatag tgaaaatatt cttaaaaaca catttttatt ttttctaaaa gaaattagta 1440
ttattctaaa aacaaaaaga agaaaaaagt tgggggaaag agaggggaga agctcgagaa 1500
gagtgtggga gaaggcaaac cccaaacact gactagacac agaccgttgg gcccgactct 1560
tcatgcatgc actcttctga caacattttc aatttctcca tatctctctc tattgttttt 1620
tcatttcttg gtttcattaa tatcatagtt tatgaataat actacaagta ctatgtaaaa 1680
tatgccttaa ataaagatat cgtacaattc aaaatgcttt tgttggaatg caatttaaac 1740
tccaaaattc agaaatttaa tgaggaagtg tggtcaggtc gctgttactt ttaagtgtca 1800
gtcttcactc actcactcac tcaacatcgt ttcaaatttc agagcaaaag tctctaaacc 1860
caaagcctct ccacttaatt gcctattctt caaaaggcga gtttccttta ccaagcttgc 1920
aaagttatct aaactccaaa caatatctaa aacgcgtgta ctcatttctc ttattctttc 1980
ttcttaag 1988

<210> 85
<211> 2010
<212> DNA
<213> Arabidopsis thaliana

<220>
<221> promoter
<222> (1)..(2010)
<223> transcription regulating sequence from gene At1g23000

<400> 85
acattaggaa caatttaatt atagtgatga gaaaaggact aaatacatta ttttttggt 60
tgtcaacaag aacatatgat ataaagctct aacaaaaaaa gaagaactaa atatgaaaat 120
atgatcagag gacaggtaag tacattgtcg ggtgatgaca tgactaatta ttaatttcga 180
ccccaattta aaaacataca tattctaaaa ttctattatg gcatgtgaaa ttgtgaaaag 240
acacaaaact aatggaccga tgtggagtaa cacagaaaac gcgtggcgta gagaggactt 300
gataattagg agtcaaggtt gcgttgatgc tgtgaggtcc agggttttgt agcttttctt 360
ctattgtcac gtgtacggtg tttgggaaat gaacggctaa atcattcaac ttgaaacact 420
aatcacggca tttcacgaaa taagaggata atattggtat atgcagttta catcaagagt 480
taaataccat aatgtaatgt tggctacgga tctaacaaat catgtgagca atacgagttt 540
tggtaactat aattttcgtg aaaggaacat atattggact attggaggta gctggcaact 600
ctcaaatctt aaagtgaagt atattttttg tatgcaaaat caagatgata gtatgtatga 660
tgatcatgta tatatttatt gtggacagtt aaattcgttt gatgtagata aatgcactga 720
ttattgattt gtatgcggtc aaaactatat agtaatcgag ccatcgaggt ggtgtggttt 780
attggtgtta ttcgagtggt agaaattttt ctatcaaggg ggaaagtcct actatctacg 840
accattattg ctcttttctt ttgaccagca tactcatttt aactgacaag actagactgc 900
atgacatttc atagactttg ggattagtat gtgttttagc tcatgatcta ggttaaaaaa 960
gagaatgtga ttattagtaa acatatttta aaagctaacc aaatataatg taatcacgta 1020
atctttgatc atatgtatga tttgtaaact cccattagat caagttgaaa atccatcgag 1080

```
ggtttgcgat ttttttaatg tttttttatt tatgtgttat atagatttgg aaagaaatta   1140
agtttaagtg ggggaatcaa agggccaaca ttgaacagcc agactaaaaa aacattaaac   1200
agattaatgg gcttaatgcg taatgcgtct cttttttgt ttaaggccca taatagtttt   1260
attaaaaagg ccgaaatgta aggcaacact agcactacac taacaaggtc agtcagaatg   1320
tgctcactgg ccactgctca taaatcaaga aatcaaaata ttgcatgcaa tttttttctt   1380
aaaaccaaaa acgtagtata gtgaaaatat tctaaaaata atatgtatgt cagctcattt   1440
ttactttttc taaaagaaat tagtattatt ctataaacaa aaagaagaaa aaagttgggg   1500
gaaagagagg ggagaagctc gagaagagtg tgggagaagg caaaccccaa acactgacta   1560
gacacagacc gttgggcccg actcttcatg catgcactct tctgacaaca ttttcaattt   1620
ctccatatct ctctctattg ttttttcatt tcttagtttc attaatatca tagtttatga   1680
ataatactac aagtactatg taaaatatgc cttaaataaa gatatcgtac aattcaaaat   1740
gcttttgttg gaatgcaatt taaactccaa aattcagaat ttaatgagga agtgtggtca   1800
ggtcgctgtt actttaagt gtcagtcttc actcactcac tcaacatcgt ttcaaatttc   1860
agagcaaaag tctctaaacc caaagcctct ccacttaatt gcctattctt caaaaggcga   1920
gtttccttta ccaagcttgc aaagttatct aaactccaaa cgatatctaa aacgcgtgta   1980
ctcatttctc ttattctttc ttcttaagcc                                    2010
```

<210> 86
<211> 1776
<212> DNA
<213> Arabidopsis thaliana

<220>
<221> promoter
<222> (1)..(1776)
<223> transcription regulating sequence from gene At1g23000

<400> 86

```
atttaattat agtgatgaga aaaggactaa atacattttt tttttggttg tcaacaagaa     60
catatgatat aaagctctaa caaaaaaaga agaactaaat atgaaaatat gatcagagga    120
caggtaagta cattgtcggg tgatgacatg actaattatt aatttcgacc ccaattttaa    180
aacatacata ttctaaaatt ctattatggc atgtgaaatt gtgaaaagac acaaaactaa    240
tggaccgatg tggagtaaca cagaaaacgc gtggcgtaga gaggacttga taattaggag    300
tcaaggttgc gttgatgctg tgaggtccag ggttttgtag cttttcttct attgtcacgt    360
gtacggtgtt tgggaaatga acggctaaat cattcaactt gaaacactaa tcacggcatt    420
tcacgaaata agaggataat attggtatat gcagtttaca tcaagagtta aataccataa    480
tgtaatgttg gctacggatc taacaaatca tgtgagcaat acgagttttg gtaactataa    540
ttttcgtgaa aggaacatat attggactat tggaggtagc tggcaactct caaatcttaa    600
agtgaagtat attttttgta tgcaaaatca agatgatagt atgtatgatg atcatgtata    660
tatttattgt ggacagttaa attcgtttga tgtagataaa tgcactgatt attgatttgt    720
atgcggtcaa aactatatag taatcgagcc atcgaggtgg tgtggtttat tggtgttatt    780
cgagtggtag aaatttttct atcaaggggg aaagtcctac tatctacgac cattattgct    840
cttttatttt gaccagcata ctcattttaa ctgacaagac tagactgcat gacatttcat    900
agactttggg attagtatgt gttttagctc atgatctagg ttaaaaaaga gaatgtgatt    960
attagtaaat atattttaat agctaaccaa atataatgta atcacgtaat ctttgatcat   1020
```

```
atgtatgatt tgtaaactca cattagatga agttgaaaat ccatcgaggg tttgcgattt   1080
ttttaatgtt tttttattta tgtgttatat agatttggaa agaaattaag tttaagtggg   1140
ggaatcaaag ggccaacatt gaacagccag actaaaaaac attaaacaga ttaatgggct   1200
taatgcgtaa tgcgcctctt tttttgttta aggcccataa tagttttatt taaaaggccg   1260
aaatgtaagg caacagtagc actacactaa caaggtcagt cagaatgtgt cactggccac   1320
tgctcataaa tcaagaaatc aaaatattat atgtaatttt ttttttctta aaaccaaaaa   1380
cgtagtatag tgaaaatatt cttaaaaaca catttttatt ttttctaaaa gaaattagta   1440
ttattctaaa aacaaaaaga agaaaaaagt tgggggaaag agaggggaga agctcgagaa   1500
gagtgtggga gaaggcaaac cccaaacact gactagacac agaccgttgg gcccgactct   1560
tcatgcatgc actcttctga caacattttc aatttctcca tatctctctc tattgttttt   1620
tcatttcttg gtttcattaa tatcatagtt tatgaataat actacaagta ctatgtaaaa   1680
tatgccttaa ataaagatat cgtacaattc aaaatgcttt tgttggaatg caatttaaac   1740
tccaaaattc agaaatttaa tgaggaagtg tggtca                             1776
```

```
<210>  87
<211>  1800
<212>  DNA
<213>  Arabidopsis thaliana

<220>
<221>  promoter
<222>  (1)..(1800)
<223>  transcription regulating sequence from gene At1g23000

<400>  87
acattaggaa caatttaatt atagtgatga gaaaaggact aaatacatta tttttttggt     60
tgtcaacaag aacatatgat ataaagctct aacaaaaaaa gaagaactaa atatgaaaat    120
atgatcagag gacaggtaag tacattgtcg ggtgatgaca tgactaatta ttaatttcga    180
ccccaattta aaaacataca tattctaaaa ttctattatg gcatgtgaaa ttgtgaaaag    240
acacaaaact aatggaccga tgtggagtaa cacagaaaac gcgtggcgta gagaggactt    300
gataattagg agtcaaggtt gcgttgatgc tgtgaggtcc agggttttgt agcttttctt    360
ctattgtcac gtgtacggtg tttgggaaat gaacggctaa atcattcaac ttgaaacact    420
aatcacggca tttcacgaaa taagaggata atattggtat atgcagttta catcaagagt    480
taaataccat aatgtaatgt tggctacgga tctaacaaat catgtgagca atacgagttt    540
tggtaactat aattttcgtg aaaggaacat atattggact attggaggta gctggcaact    600
ctcaaatctt aaagtgaagt atattttttg tatgcaaaat caagatgata gtatgtatga    660
tgatcatgta tatatttatt gtggacagtt aaattcgttt gatgtagata aatgcactga    720
ttattgattt gtatgcggtc aaaactatat agtaatcgag ccatcgaggt ggtgtggttt    780
attggtgtta ttcgagtggt agaaattttt ctatcaaggg ggaaagtcct actatctacg    840
accattattg ctcttttctt ttgaccagca tactcatttt aactgacaag actagactgc    900
atgacatttc atagactttg ggattagtat gtgttttagc tcatgatcta ggttaaaaaa    960
gagaatgtga ttattagtaa acatatttta aaagctaacc aaatataatg taatcacgta   1020
atctttgatc atatgtatga tttgtaaact cccattagat caagttgaaa atccatcgag   1080
ggtttgcgat ttttttaatg ttttttttatt tatgtgttat atagatttgg aaagaaatta   1140
agtttaagtg ggggaatcaa agggccaaca ttgaacagcc agactaaaaa aacattaaac   1200
```

```
agattaatgg gcttaatgcg taatgcgtct cttttttgt ttaaggccca taatagtttt  1260
attaaaaagg ccgaaatgta aggcaacact agcactacac taacaaggtc agtcagaatg  1320
tgctcactgg ccactgctca taaatcaaga aatcaaaata ttgcatgcaa tttttttctt  1380
aaaaccaaaa acgtagtata gtgaaaatat tctaaaaata atatgtatgt cagctcattt  1440
ttactttttc taaaagaaat tagtattatt ctataaacaa aaagaagaaa aaagttgggg  1500
gaaagagagg ggagaagctc gagaagagtg tgggagaagg caaaccccaa acactgacta  1560
gacacagacc gttgggcccg actcttcatg catgcactct tctgacaaca ttttcaattt  1620
ctccatatct ctctctattg tttttcatt tcttagtttc attaatatca tagtttatga  1680
ataatactac aagtactatg taaaatatgc cttaaataaa gatatcgtac aattcaaaat  1740
gcttttgttg gaatgcaatt taaactccaa aattcagaat ttaatgagga agtgtggtca  1800
```

```
<210>  88
<211>  1492
<212>  DNA
<213>  Arabidopsis thaliana

<220>
<221>  CDS
<222>  (211)..(1287)
<223>  encoding heavy-metal-associated domain-containing protein

<400>  88
ggtcgctgtt acttttaagt gtcagtcttc actcactcac tcaacatcgt ttcaaatttc    60
agagcaaaag tctctaaacc caaagcctct ccacttaatt gcctattctt caaaaggcga   120
gtttccttta ccaagcttgc aaagttatct aaactccaaa cgatatctaa aacgcgtgta   180
ctcatttctc ttattctttc ttcttaagcc atg act aaa gat gaa gac ttt aag    234
                                 Met Thr Lys Asp Glu Asp Phe Lys
                                 1               5
ctc cta aag atc cag acg ttt tca ctc aga gtc aac att cac tgc gag    282
Leu Leu Lys Ile Gln Thr Phe Ser Leu Arg Val Asn Ile His Cys Glu
    10                  15                  20
ggc tgt aac aag aaa gtc aag aaa ctt ctt cag agg atc gaa gga gtt    330
Gly Cys Asn Lys Lys Val Lys Lys Leu Leu Gln Arg Ile Glu Gly Val
25                  30                  35                  40
tgc cac gtg aag ata gaa gca gaa cat caa aag gtg act gtt tca ggg    378
Cys His Val Lys Ile Glu Ala Glu His Gln Lys Val Thr Val Ser Gly
                45                  50                  55
tca gtt gac tca gcc aca ctc atc aac aag ctt gtt aaa gcc ggg aaa    426
Ser Val Asp Ser Ala Thr Leu Ile Asn Lys Leu Val Lys Ala Gly Lys
            60                  65                  70
cac gcc gag ctt tgg tct cct aac cca aac caa aac cag cct caa aag    474
His Ala Glu Leu Trp Ser Pro Asn Pro Asn Gln Asn Gln Pro Gln Lys
        75                  80                  85
ccc aag act aac gac ttc atc aag aac gtt aat caa aag ggt cag aag    522
Pro Lys Thr Asn Asp Phe Ile Lys Asn Val Asn Gln Lys Gly Gln Lys
```

```
    90                  95                  100
cag ggg tct gcc aaa agt ggt att gaa gcc tgt aaa ccc aag aac ggc      570
Gln Gly Ser Ala Lys Ser Gly Ile Glu Ala Cys Lys Pro Lys Asn Gly
105                 110                 115                 120
cct aaa ggt gca gcc ttt gta gct gag gaa gat gga gat ggc agt gaa      618
Pro Lys Gly Ala Ala Phe Val Ala Glu Glu Asp Gly Asp Gly Ser Glu
                125                 130                 135
gag gaa gac gga gat gtc cag ttt cct aaa ccg gcg aat cag cag cag      666
Glu Glu Asp Gly Asp Val Gln Phe Pro Lys Pro Ala Asn Gln Gln Gln
            140                 145                 150
caa caa aac gtc gtc aac gcc aag aaa aac agt gga gga gct gcg atg      714
Gln Gln Asn Val Val Asn Ala Lys Lys Asn Ser Gly Gly Ala Ala Met
        155                 160                 165
aac aat gga aac aac gga gtc aac gca gca tca aag aaa gtc aac caa      762
Asn Asn Gly Asn Asn Gly Val Asn Ala Ala Ser Lys Lys Val Asn Gln
    170                 175                 180
aaa cag agc aac cat aac cag aac act caa caa gta atg gcg gct atg      810
Lys Gln Ser Asn His Asn Gln Asn Thr Gln Gln Val Met Ala Ala Met
185                 190                 195                 200
aga atg aga acc gcc ggg aaa atg agc act ggt gtt gaa gcc aac gag      858
Arg Met Arg Thr Ala Gly Lys Met Ser Thr Gly Val Glu Ala Asn Glu
                205                 210                 215
att gga gct ctc atg ggt ctt gct ggc ttc aac ggc gca acc aac gca      906
Ile Gly Ala Leu Met Gly Leu Ala Gly Phe Asn Gly Ala Thr Asn Ala
            220                 225                 230
gtg aat cac cct cca aat ggg att caa caa cag ctt caa gct cca ccg      954
Val Asn His Pro Pro Asn Gly Ile Gln Gln Gln Leu Gln Ala Pro Pro
        235                 240                 245
tta aac aac gtc aac ggc gtc act aat cac aac ctc acc aat agt aat     1002
Leu Asn Asn Val Asn Gly Val Thr Asn His Asn Leu Thr Asn Ser Asn
    250                 255                 260
gga ggc atg atg atg aac atg aat ggg tac aat aat cat cat cca atg     1050
Gly Gly Met Met Met Asn Met Asn Gly Tyr Asn Asn His His Pro Met
265                 270                 275                 280
aac atg cag agt agg caa atg atg cat cag cct cag caa atg atg tac     1098
Asn Met Gln Ser Arg Gln Met Met His Gln Pro Gln Gln Met Met Tyr
                285                 290                 295
caa aga tca tct ttc gtt cca gca tca agc aat ggg tat tat tac aat     1146
Gln Arg Ser Ser Phe Val Pro Ala Ser Ser Asn Gly Tyr Tyr Tyr Asn
            300                 305                 310
tat acc cct agt ccc tac agt tat tat cct tat tac cct tac gca agt     1194
Tyr Thr Pro Ser Pro Tyr Ser Tyr Tyr Pro Tyr Tyr Pro Tyr Ala Ser
        315                 320                 325
gat cag tac caa caa caa agt agt cat tca cat gca aca aac atg tct     1242
Asp Gln Tyr Gln Gln Gln Ser Ser His Ser His Ala Thr Asn Met Ser
```

330 335 340
agt gaa gaa gac gct ggt aat aac aat agc tgc aac atc atg taa 1287
Ser Glu Glu Asp Ala Gly Asn Asn Asn Ser Cys Asn Ile Met
345 350 355

agtgtggatg cttgctaagg aagatatcta tcttttgctt ttggaaacaa gacttaaaag 1347
tttaatcttt tctggagtgt gatatattag tgtttggttt ttctttctct ttttttttgg 1407
tgtgtgtcat gtagctgttt tggactgaca tgaagttgtg ttggttgata tataaaatcc 1467
tagaatgtta ttgatgtttc ttgtg 1492

<210> 89
<211> 358
<212> PRT
<213> Arabidopsis thaliana

<400> 89
Met Thr Lys Asp Glu Asp Phe Lys Leu Leu Lys Ile Gln Thr Phe Ser
1 5 10 15

Leu Arg Val Asn Ile His Cys Glu Gly Cys Asn Lys Lys Val Lys Lys
20 25 30

Leu Leu Gln Arg Ile Glu Gly Val Cys His Val Lys Ile Glu Ala Glu
35 40 45

His Gln Lys Val Thr Val Ser Gly Ser Val Asp Ser Ala Thr Leu Ile
50 55 60

Asn Lys Leu Val Lys Ala Gly Lys His Ala Glu Leu Trp Ser Pro Asn
65 70 75 80

Pro Asn Gln Asn Gln Pro Gln Lys Pro Lys Thr Asn Asp Phe Ile Lys
85 90 95

Asn Val Asn Gln Lys Gly Gln Lys Gln Gly Ser Ala Lys Ser Gly Ile
100 105 110

Glu Ala Cys Lys Pro Lys Asn Gly Pro Lys Gly Ala Ala Phe Val Ala
115 120 125

Glu Glu Asp Gly Asp Gly Ser Glu Glu Glu Asp Gly Asp Val Gln Phe
130 135 140

Pro Lys Pro Ala Asn Gln Gln Gln Gln Gln Asn Val Val Asn Ala Lys
145 150 155 160

```
Lys Asn Ser Gly Gly Ala Ala Met Asn Asn Gly Asn Asn Gly Val Asn
                165                 170                 175

Ala Ala Ser Lys Lys Val Asn Gln Lys Gln Ser Asn His Asn Gln Asn
            180                 185                 190

Thr Gln Gln Val Met Ala Ala Met Arg Met Arg Thr Ala Gly Lys Met
        195                 200                 205

Ser Thr Gly Val Glu Ala Asn Glu Ile Gly Ala Leu Met Gly Leu Ala
    210                 215                 220

Gly Phe Asn Gly Ala Thr Asn Ala Val Asn His Pro Pro Asn Gly Ile
225                 230                 235                 240

Gln Gln Gln Leu Gln Ala Pro Pro Leu Asn Asn Val Asn Gly Val Thr
                245                 250                 255

Asn His Asn Leu Thr Asn Ser Asn Gly Gly Met Met Met Asn Met Asn
            260                 265                 270

Gly Tyr Asn Asn His His Pro Met Asn Met Gln Ser Arg Gln Met Met
        275                 280                 285

His Gln Pro Gln Gln Met Met Tyr Gln Arg Ser Ser Phe Val Pro Ala
    290                 295                 300

Ser Ser Asn Gly Tyr Tyr Tyr Asn Tyr Thr Pro Ser Pro Tyr Ser Tyr
305                 310                 315                 320

Tyr Pro Tyr Tyr Pro Tyr Ala Ser Asp Gln Tyr Gln Gln Gln Ser Ser
                325                 330                 335

His Ser His Ala Thr Asn Met Ser Ser Glu Glu Asp Ala Gly Asn Asn
            340                 345                 350

Asn Ser Cys Asn Ile Met
        355

<210>  90
<211>  27
<212>  DNA
<213>  Artificial

<220>
```

<223> Oligonucleotide primer

<400> 90
aacttgggat ccttttatct gtataat 27

<210> 91
<211> 23
<212> DNA
<213> Artificial

<220>
<223> Oligonucleotide primer

<400> 91
accaaaccat ggtgattatt gac 23

<210> 92
<211> 28
<212> DNA
<213> Artificial

<220>
<223> Oligonucleotide primer

<400> 92
gattttccat ggttctataa agaaaatg 28

<210> 93
<211> 27
<212> DNA
<213> Artificial

<220>
<223> Oligonucleotide primer

<400> 93
tactttggat ccctcccttt ttatgtc 27

<210> 94
<211> 23
<212> DNA
<213> Artificial

<220>
<223> Oligonucleotide primer

<400> 94
accaaaccat ggtgattatt gac 23

<210> 95
<211> 28
<212> DNA
<213> Artificial

<220>
<223> Oligonucleotide primer

<400> 95
gattttccat ggttctataa agaaaatg 28

<210> 96
<211> 28
<212> DNA
<213> Artificial

<220>
<223> Oligonucleotide primer

<400> 96
ataattggat ccactagtca tctatttc 28

<210> 97
<211> 23
<212> DNA
<213> Artificial

<220>
<223> Oligonucleotide primer

<400> 97
accaaaccat ggtgattatt gac 23

<210> 98
<211> 28
<212> DNA
<213> Artificial

<220>
<223> Oligonucleotide primer

<400> 98
gattttccat ggttctataa agaaaatg 28

<210> 99
<211> 24
<212> DNA
<213> Artificial

<220>
<223> Oligonucleotide primer

<400> 99
taaaagctcg agctcgtcat ggac 24

<210> 100
<211> 27
<212> DNA
<213> Artificial

<220>
<223> Oligonucleotide primer

<400> 100
gatgtgggat cccagaaaga tcagtcc 27

<210> 101
<211> 30
<212> DNA
<213> Artificial

<220>
<223> Oligonucleotide primer

<400> 101
agggtaaagc ttctgattga gaaaggacac 30

<210> 102
<211> 23
<212> DNA
<213> Artificial

<220>
<223> Oligonucleotide primer

<400> 102

caataagaat tcggttagtt ttc 23

<210> 103
<211> 27
<212> DNA
<213> Artificial

<220>
<223> Oligonucleotide primer

<400> 103
gatgtggaat tccagaaaga tcagtcc 27

<210> 104
<211> 30
<212> DNA
<213> Artificial

<220>
<223> oligonucleotide primer

<400> 104
agggtaaagc ttctgattga gaaaggacac 30

<210> 105
<211> 24
<212> DNA
<213> Artificial

<220>
<223> Oligonucleotide primer

<400> 105
taaaagctcg agctcgtcat ggac 24

<210> 106
<211> 25
<212> DNA
<213> Artificial

<220>
<223> Oligonucleotide primer

<400> 106
tcttacccat gggaggttag gtagg 25

<210> 107
<211> 28
<212> DNA
<213> Artificial

<220>
<223> Oligonucleotide primer

<400> 107
ggaaggccat ggaagggaga cttctgac 28

<210> 108
<211> 30
<212> DNA
<213> Artificial

<220>
<223> Oligonucleotide primer

<400> 108
agggtaaagc ttctgattga gaaaggacac 30

<210> 109
<211> 25
<212> DNA
<213> Artificial

<220>
<223> oligonucleotide primer

<400> 109
tcttacccat gggaggttag gtagg 25

<210> 110
<211> 28
<212> DNA
<213> Artificial

<220>
<223> Oligonucleotide primer

<400> 110
ggaaggccat ggaagggaga cttctgac 28

<210> 111
<211> 27
<212> DNA
<213> Artificial

<220>
<223> Oligonucleotide primer

<400> 111
taagctttct ttttgttgtg gaggata 27

<210> 112
<211> 28
<212> DNA
<213> Artificial

<220>
<223> Oligonucleotide primer

<400> 112
atctagatag aattctttaa cttgtgct 28

<210> 113
<211> 30
<212> DNA
<213> Artificial

<220>
<223> Oligonucleotide primer

<400> 113
taatctggat ccttgctttc aactccaaaa 30

<210> 114
<211> 30
<212> DNA
<213> Artificial

<220>
<223> Oligonucleotide primer

<400> 114
gcttttccat ggattgcttg aaaattgatg 30

<210> 115

<211> 28
<212> DNA
<213> Artificial

<220>
<223> Oligonucleotide primer

<400> 115
tgtcgacttt atctaatgga taaccacc 28

<210> 116
<211> 25
<212> DNA
<213> Artificial

<220>
<223> Oligonucleotide primer

<400> 116
tcccgggtta attcatatat acaac 25

<210> 117
<211> 30
<212> DNA
<213> Artificial

<220>
<223> Oligonucleotide primer

<400> 117
tttaactctc gagtgccgtt tcagttttgt 30

<210> 118
<211> 28
<212> DNA
<213> Artificial

<220>
<223> Oligonucleotide primer

<400> 118
tgtcgacttt atctaatgga taaccacc 28

<210> 119
<211> 28

<212> DNA
<213> Artificial

<220>
<223> Oligonucleotide primer

<400> 119
tcccgggaaa aatctcagct ggctctcg 28

<210> 120
<211> 30
<212> DNA
<213> Artificial

<220>
<223> Oligonucleotide primer

<400> 120
tttaactctc gagtgccgtt tcagttttgt 30

<210> 121
<211> 24
<212> DNA
<213> Artificial

<220>
<223> Oligonucleotide primer

<400> 121
aggatcctgt tattttagct aaag 24

<210> 122
<211> 25
<212> DNA
<213> Artificial

<220>
<223> Oligonucleotide primer

<400> 122
tccatggtta attcatatat acaac 25

<210> 123
<211> 29
<212> DNA

<213> Artificial

<220>
<223> Oligonucleotide primer

<400> 123
tttaactctc gagtgccgtt tcagttttg 29

<210> 124
<211> 25
<212> DNA
<213> Artificial

<220>
<223> Oligonucleotide primer

<400> 124
agagatggat ccggtgagtg ctacg 25

<210> 125
<211> 27
<212> DNA
<213> Artificial

<220>
<223> Oligonucleotide primer

<400> 125
ggttgtccat ggtgtctctt atatggc 27

<210> 126
<211> 28
<212> DNA
<213> Artificial

<220>
<223> Oligonucleotide primer

<400> 126
tttttcccat ggttcatgag aagttgtg 28

<210> 127
<211> 28
<212> DNA
<213> Artificial

<220>
<223> Oligonucleotide primer

<400> 127
gagttaggat ccctgaatac gatcgatc 28

<210> 128
<211> 27
<212> DNA
<213> Artificial

<220>
<223> Oligonucleotide primer

<400> 128
ggttgtccat ggtgtctctt atatggc 27

<210> 129
<211> 28
<212> DNA
<213> Artificial

<220>
<223> Oligonucleotide primer

<400> 129
tttttcccat ggttcatgag aagttgtg 28

<210> 130
<211> 24
<212> DNA
<213> Artificial

<220>
<223> Oligonucleotide primer

<400> 130
cttgtgggat ccatcaattc actc 24

<210> 131
<211> 28
<212> DNA
<213> Artificial

<220>
<223> Oligonucleotide primer

<400> 131
agacatccat ggcaatgaat atgaaatg 28

<210> 132
<211> 31
<212> DNA
<213> Artificial

<220>
<223> Oligonucleotide primer

<400> 132
tagccatggg aagcagcaac cctcattaat g 31

<210> 133
<211> 27
<212> DNA
<213> Artificial

<220>
<223> Oligonucleotide primer

<400> 133
taaaaaggat ccaaaaagag agtcaac 27

<210> 134
<211> 28
<212> DNA
<213> Artificial

<220>
<223> Oligonucleotide primer

<400> 134
agacatccat ggcaatgaat atgaaatg 28

<210> 135
<211> 31
<212> DNA
<213> Artificial

<220>

<223> Oligonucleotide primer

<400> 135
tagccatggg aagcagcaac cctcattaat g 31

<210> 136
<211> 24
<212> DNA
<213> Artificial

<220>
<223> Oligonucleotide primer

<400> 136
aactaaggat ccagaaaaag aatc 24

<210> 137
<211> 28
<212> DNA
<213> Artificial

<220>
<223> Oligonucleotide primer

<400> 137
agacatccat ggcaatgaat atgaaatg 28

<210> 138
<211> 31
<212> DNA
<213> Artificial

<220>
<223> Oligonucleotide primer

<400> 138
tagccatggg aagcagcaac cctcattaat g 31

<210> 139
<211> 25
<212> DNA
<213> Artificial

<220>
<223> Oligonucleotide primer

<400> 139
tgcttaggat ccaaaccagt ctctc 25

<210> 140
<211> 25
<212> DNA
<213> Artificial

<220>
<223> Oligonucleotide primer

<400> 140
ggttcaccat ggtaaaaaaa gggcg 25

<210> 141
<211> 27
<212> DNA
<213> Artificial

<220>
<223> Oligonucleotide primer

<400> 141
ggtcatccat ggttcttggg aaaatgg 27

<210> 142
<211> 28
<212> DNA
<213> Artificial

<220>
<223> Oligonucleotide primer

<400> 142
cggcaaggat ccagtttttg taaaactc 28

<210> 143
<211> 26
<212> DNA
<213> Artificial

<220>
<223> Oligonucleotide primer

<400> 143
tcgtcgccat ggtttcaaat tgcaac 26

<210> 144
<211> 26
<212> DNA
<213> Artificial

<220>
<223> Oligonucleotide primer

<400> 144
acattaggat ccatttaatt atagtg 26

<210> 145
<211> 25
<212> DNA
<213> Artificial

<220>
<223> Oligonucleotide primer

<400> 145
ctttagccat ggcttaagaa gaaag 25

<210> 146
<211> 30
<212> DNA
<213> Artificial

<220>
<223> Oligonucleotide primer

<400> 146
tgaccatggt tcctcattaa attctgaatt 30

<210> 147
<211> 8986
<212> DNA
<213> Artificial

<220>
<223> binary vector pSUN0301

<400> 147

cgttgtaaaa cgacggccag tgaattcgag ctcggtacct cgagcccggg cgatatcgga 60
tccactagtc tagagtcgat cgaccatggt acgtcctgta gaaaccccaa cccgtgaaat 120
caaaaaactc gacggcctgt gggcattcag tctggatcgc gaaaactgtg gaattggtca 180
gcgttggtgg gaaagcgcgt tacaagaaag ccgggcaatt gctgtgccag gcagttttaa 240
cgatcagttc gccgatgcag atattcgtaa ttatgcgggc aacgtctggt atcagcgcga 300
agtctttata ccgaaaggtt gggcaggcca gcgtatcgtg ctgcgtttcg atgcggtcac 360
tcattacggc aaagtgtggg tcaataatca ggaagtgatg gagcatcagg gcggctatac 420
gccatttgaa gccgatgtca cgccgtatgt tattgccggg aaaagtgtac gtaagtttct 480
gcttctacct ttgatatata tataataatt atcattaatt agtagtaata taatatttca 540
aatatttttt tcaaaataaa agaatgtagt atatagcaat tgcttttctg tagtttataa 600
gtgtgtatat tttaatttat aacttttcta atatatgacc aaaatttgtt gatgtgcagg 660
tatcaccgtt tgtgtgaaca acgaactgaa ctggcagact atcccgccgg gaatggtgat 720
taccgacgaa aacggcaaga aaaagcagtc ttacttccat gatttcttta actatgccgg 780
aatccatcgc agcgtaatgc tctacaccac gccgaacacc tgggtggacg atatcaccgt 840
ggtgacgcat gtcgcgcaag actgtaacca cgcgtctgtt gactggcagg tggtggccaa 900
tggtgatgtc agcgttgaac tgcgtgatgc ggatcaacag gtggttgcaa ctggacaagg 960
cactagcggg actttgcaag tggtgaatcc gcacctctgg caaccgggtg aaggttatct 1020
ctatgaactg tgcgtcacag ccaaaagcca gacagagtgt gatatctacc cgcttcgcgt 1080
cggcatccgg tcagtggcag tgaagggcga acagttcctg attaaccaca aaccgttcta 1140
ctttactggc tttggtcgtc atgaagatgc ggacttacgt ggcaaaggat tcgataacgt 1200
gctgatggtg cacgaccacg cattaatgga ctggattggg gccaactcct accgtacctc 1260
gcattaccct tacgctgaag agatgctcga ctgggcagat gaacatggca tcgtggtgat 1320
tgatgaaact gctgctgtcg gctttaacct ctctttaggc attggtttcg aagcgggcaa 1380
caagccgaaa gaactgtaca gcgaagaggc agtcaacggg gaaactcagc aagcgcactt 1440
acaggcgatt aaagagctga tagcgcgtga caaaaaccac ccaagcgtgg tgatgtggag 1500
tattgccaac gaaccggata cccgtccgca agtgcacggg aatatttcgc cactggcgga 1560
agcaacgcgt aaactcgacc cgacgcgtcc gatcacctgc gtcaatgtaa tgttctgcga 1620
cgctcacacc gataccatca gcgatctctt tgatgtgctg tgcctgaacc gttattacgg 1680
atggtatgtc caaagcggcg atttggaaac ggcagagaag gtactggaaa aagaacttct 1740
ggcctggcag gagaaactgc atcagccgat tatcatcacc gaatacggcg tggatacgtt 1800
agccgggctg cactcaatgt acaccgacat gtggagtgaa gagtatcagt gtgcatggct 1860
ggatatgtat caccgcgtct ttgatcgcgt cagcgccgtc gtcggtgaac aggtatggaa 1920
tttcgccgat tttgcgacct cgcaaggcat attgcgcgtt ggcggtaaca agaaagggat 1980
cttcactcgc gaccgcaaac cgaagtcggc ggcttttctg ctgcaaaaac gctggactgg 2040
catgaacttc ggtgaaaaac cgcagcaggg aggcaaacaa tgaatcaaca actctcctgg 2100
cgcaccatcg tcggctacag cctcgggaat tgctaccgag ctcggtaccc ggcgcaaaaa 2160
tcaccagtct ctctctacaa atctatctct ctctattttt ctccagaata atgtgtgagt 2220
agttcccaga taagggaatt agggttctta tagggtttcg ctcatgtgtt gagcatataa 2280
gaaaccctta gtatgtattt gtatttgtaa aatacttcta tcaataaaat ttctaattcc 2340
taaaaccaaa atccagtgac cgggtaccga gctcgaattt cgacctgcag gcatgcaagc 2400
ttggcgtaat catggtcata gctgtttcct actagatctg attgtcgttt cccgccttca 2460
gtttaaacta tcagtgtttg acaggatata ttggcgggta aacctaagag aaaagagcgt 2520
ttattagaat aatcggatat ttaaaagggc gtgaaaaggt ttatccgttc gtccatttgt 2580
atgtccatga taagtcgcgc tgtatgtgtt tgtttgaata ttcatggaac gcagtggcgg 2640
ttttcatggc ttgttatgac tgtttttttg gggtacagtc tatgcctcgg gcatccaagc 2700

```
agcaagcgcg ttacgccgtg ggtcgatgtt tgatgttatg gagcagcaac gatgttacgc   2760
agcagggcag tcgccctaaa acaaagttaa acatcatggg ggaagcggtg atcgccgaag   2820
tatcgactca actatcagag gtagttggcg tcatcgagcg ccatctcgaa ccgacgttgc   2880
tggccgtaca tttgtacggc tccgcagtgg atggcggcct gaagccacac agtgatattg   2940
atttgctggt tacggtgacc gtaaggcttg atgaaacaac gcggcgagct ttgatcaacg   3000
acctttttgga aacttcggct tcccctggag agagcgagat tctccgcgct gtagaagtca   3060
ccattgttgt gcacgacgac atcattccgt ggcgttatcc agctaagcgc gaactgcaat   3120
ttggagaatg gcagcgcaat gacattcttg caggtatctt cgagccagcc acgatcgaca   3180
ttgatctggc tatcttgctg acaaaagcaa gagaacatag cgttgccttg gtaggtccag   3240
cggcggagga actctttgat ccggttcctg aacaggatct atttgaggcg ctaaatgaaa   3300
ccttaacgct atggaactcg ccgcccgact gggctggcga tgagcgaaat gtagtgctta   3360
cgttgtcccg catttggtac agcgcagtaa ccggcaaaat cgcgccgaag gatgtcgctg   3420
ccgactgggc aatggagcgc ctgccggccc agtatcagcc cgtcatactt gaagctagac   3480
aggcttatct tggacaagaa gaagatcgct tggcctcgcg cgcagatcag ttggaagaat   3540
ttgtccacta cgtgaaaggc gagatcacca aggtagtcgg caaataatgt ctagctagaa   3600
attcgttcaa gccgacgccg cttcgcggcg cggcttaact caagcgttag atgcactaag   3660
cacataattg ctcacagcca aactatcagg tcaagtctgc ttttattatt tttaagcgtg   3720
cataataagc cctacacaaa ttgggagata tatcatgcat gaccaaaatc ccttaacgtg   3780
agttttcgtt ccactgagcg tcagaccccg tagaaaagat caaaggatct tcttgagatc   3840
cttttttct gcgcgtaatc tgctgcttgc aaacaaaaaa accaccgcta ccagcggtgg   3900
tttgtttgcc ggatcaagag ctaccaactc tttttccgaa ggtaactggc ttcagcagag   3960
cgcagatacc aaatactgtc cttctagtgt agccgtagtt aggccaccac ttcaagaact   4020
ctgtagcacc gcctacatac ctcgctctgc taatcctgtt accagtggct gctgccagtg   4080
gcgataagtc gtgtcttacc gggttggact caagacgata gttaccggat aaggcgcagc   4140
ggtcgggctg aacggggggt tcgtgcacac agcccagctt ggagcgaacg acctacaccg   4200
aactgagata cctacagcgt gagctatgag aaagcgccac gcttcccgaa gggagaaagg   4260
cggacaggta tccggtaagc ggcagggtcg gaacaggaga gcgcacgagg gagcttccag   4320
ggggaaacgc ctggtatctt tatagtcctg tcgggtttcg ccacctctga cttgagcgtc   4380
gatttttgtg atgctcgtca ggggggcgga gcctatggaa aaacgccagc aacgcggcct   4440
ttttacggtt cctggccttt tgctggcctt ttgctcacat gttctttcct gcgttatccc   4500
ctgattctgt ggataaccgt attaccgcct ttgagtgagc tgataccgct cgccgcagcc   4560
gaacgaccga gcgcagcgag tcagtgagcg aggaagcgga agagcgcctg atgcggtatt   4620
ttctccttac gcatctgtgc ggtatttcac accgcatagg ccgcgatagg ccgacgcgaa   4680
gcggcggggc gtagggagcg cagcgaccga agggtaggcg ctttttgcag ctcttcggct   4740
gtgcgctggc cagacagtta tgcacaggcc aggcgggttt taagagtttt aataagtttt   4800
aaagagtttt aggcggaaaa atcgcctttt ttctctttta tatcagtcac ttacatgtgt   4860
gaccggttcc caatgtacgg ctttgggttc ccaatgtacg ggttccggtt cccaatgtac   4920
ggctttgggt tcccaatgta cgtgctatcc acaggaaaga gaccttttcg accttttcc   4980
cctgctaggg caatttgccc tagcatctgc tccgtacatt aggaaccggc ggatgcttcg   5040
ccctcgatca ggttgcggta gcgcatgact aggatcgggc cagcctgccc cgcctcctcc   5100
ttcaaatcgt actccggcag gtcatttgac ccgatcagct tgcgcacggt gaaacagaac   5160
ttcttgaact ctccggcgct gccactgcgt tcgtagatcg tcttgaacaa ccatctggct   5220
tctgccttgc ctgcggcgcg gcgtgccagg cggtagagaa aacggccgat gccgggatcg   5280
atcaaaaagt aatcggggtg aaccgtcagc acgtccgggt tcttgccttc tgtgatctcg   5340
cggtacatcc aatcagctag ctcgatctcg atgtactccg gccgcccggt ttcgctcttt   5400
```

```
acgatcttgt agcggctaat caaggcttca ccctcggata ccgtcaccag gcggccgttc   5460
ttggccttct tcgtacgctg catggcaacg tgcgtggtgt ttaaccgaat gcaggtttct   5520
accaggtcgt ctttctgctt tccgccatcg gctcgccggc agaacttgag tacgtccgca   5580
acgtgtggac ggaacacgcg gccgggcttg tctcccttcc cttcccggta tcggttcatg   5640
gattcggtta gatgggaaac cgccatcagt accaggtcgt aatcccacac actggccatg   5700
ccggccggcc ctgcggaaac ctctacgtgc ccgtctggaa gctcgtagcg gatcacctcg   5760
ccagctcgtc ggtcacgctt cgacagacgg aaaacggcca cgtccatgat gctgcgacta   5820
tcgcgggtgc ccacgtcata gagcatcgga acgaaaaaat ctggttgctc gtcgcccttg   5880
ggcggcttcc taatcgacgg cgcaccggct gccggcggtt gccgggattc tttgcggatt   5940
cgatcagcgg ccccttgcca cgattcaccg gggcgtgctt ctgcctcgat gcgttgccgc   6000
tgggcggcct gcgcggcctt caacttctcc accaggtcat cacccagcgc cgcgccgatt   6060
tgtaccgggc cggatggttt gcgaccgctc acgccgattc ctcgggcttg gggttccag    6120
tgccattgca gggccggcag acaacccagc cgcttacgcc tggccaaccg cccgttcctc   6180
cacacatggg gcattccacg gcgtcggtgc ctggttgttc ttgattttcc atgccgcctc   6240
ctttagccgc taaaattcat ctactcattt attcatttgc tcatttactc tggtagctgc   6300
gcgatgtatt cagatagcag ctcggtaatg gtcttgcctt ggcgtaccgc gtacatcttc   6360
agcttggtgt gatcctccgc cggcaactga aagttgaccc gcttcatggc tggcgtgtct   6420
gccaggctgg ccaacgttgc agccttgctg ctgcgtgcgc tcggacggcc ggcacttagc   6480
gtgtttgtgc ttttgctcat ttctctttta cctcattaac tcaaatgagt tttgatttaa   6540
tttcagcggc cagcgcctgg acctcgcggg cagcgtcgcc ctcgggttct gattcaagaa   6600
cggttgtgcc ggcggcggca gtgcctgggt agctcacgcg ctgcgtgata cgggactcaa   6660
gaatgggcag ctcgtacccg gccagcgcct cggcaacctc accgccgatg cgcgtgcctt   6720
tgatcgcccg cgacacgaca aaggccgctt gtagccttcc atccgtgacc tcaatgcgct   6780
gcttaaccag ctccaccagg tcggcggtgg cccatatgtc gtaagggctt ggctgcaccg   6840
gaatcagcac gaagtcggct gccttgatcg cggacacagc caagtccgcc gcctggggcg   6900
ctccgtcgat cactacgaag tcgcgccggc cgatggcctt cacgtcgcgg tcaatcgtcg   6960
ggcggtcgat gccgacaacg gttagcggtt gatcttcccg cacggccgcc caatcgcggg   7020
cactgccctg gggatcggaa tcgactaaca gaacatcggc cccggcgagt tgcagggcgc   7080
gggctagatg ggttgcgatg gtcgtcttgc ctgacccgcc tttctggtta agtacagcga   7140
taaccttcat gcgttcccct tgcgtatttg tttatttact catcgcatca tatacgcagc   7200
gaccgcatga cgcaagctgt tttactcaaa tacacatcac ctttttagac gcgtggtgat   7260
tttgtgccga gctgccggtc ggggagctgt tggctggctg gtggcaggat atattgtggt   7320
gtaaacaaat tgacgcttag acaacttaat aacacattgc ggacgtcttt aatgtactga   7380
attaacatcc gtttgatact tgtctaaaat tggctgattt cgagtgcatc tatgcataaa   7440
aacaatctaa tgacaattat taccaagcag tgatcctgtc aaacactgat agtttaaact   7500
gaaggcggga aacgacaatc tgatcatgag cggagaatta agggagtcac gttatgaccc   7560
ccgccgatga cgcgggacaa gccgttttac gtttggaact gacagaaccg caacgttgaa   7620
ggagccactc agccgcgggt ttctggagtt taatgagcta agcacatacg tcagaaacca   7680
ttattgcgcg ttcaaaagtc gcctaaggtc actatcagct agcaaatatt tcttgtcaaa   7740
aatgctccac tgacgttcca taaattcccc tcggtatcca attagagtct catattcact   7800
ctcaatccaa ataatctgca ccggatctgg atcgtttcgc atgattgaac aagatggatt   7860
gcacgcaggt tctccggccg cttgggtgga gaggctattc ggctatgact gggcacaaca   7920
gacaatcggc tgctctgatg ccgccgtgtt ccggctgtca gcgcaggggc gcccggttct   7980
ttttgtcaag accgacctgt ccggtgccct gaatgaactg caggacgagg cagcgcggct   8040
atcgtggctg gccacgacgg gcgttccttg cgcagctgtg ctcgacgttg tcactgaagc   8100
```

gggaagggac tggctgctat tgggcgaagt gccggggcag gatctcctgt catctcacct 8160
tgctcctgcc gagaaagtat ccatcatggc tgatgcaatg cggcggctgc atacgcttga 8220
tccggctacc tgcccattcg accaccaagc gaaacatcgc atcgagcgag cacgtactcg 8280
gatggaagcc ggtcttgtcg atcaggatga tctggacgaa gagcatcagg ggctcgcgcc 8340
agccgaactg ttcgccaggc tcaaggcgcg catgcccgac ggcgaggatc tcgtcgtgac 8400
acatggcgat gcctgcttgc cgaatatcat ggtggaaaat ggccgctttt ctggattcat 8460
cgactgtggc cggctgggtg tggcggaccg ctatcaggac atagcgttgg ctacccgtga 8520
tattgctgaa gagcttggcg gcgaatgggc tgaccgcttc ctcgtgcttt acggtatcgc 8580
cgctcccgat tcgcagcgca tcgccttcta tcgccttctt gacgagttct tctgagcggg 8640
acccaagctc tagatcttgc tgcgttcgga tattttcgtg gagttcccgc cacagacccg 8700
gatgatcccc gatcgttcaa acatttggca ataaagtttc ttaagattga atcctgttgc 8760
cggtcttgcg atgattatca tataatttct gttgaattac gttaagcatg taataattaa 8820
catgtaatgc atgacgttat ttatgagatg ggtttttatg attagagtcc cgcaattata 8880
catttaatac gcgatagaaa acaaaatata gcgcgcaaac taggataaat tatcgcgcgc 8940
ggtgtcatct atgttactag atcgggcctc ctgtcaagct ctgagt 8986

<210> 148
<211> 1106
<212> DNA
<213> Arabidopsis thaliana

<220>
<221> promoter
<222> (1)..(1106)
<223> transcription regulating sequence from Arabidopsis thaliana gene At3g15510

<400> 148
agcgagtatt tgattttatg tgttagtgta aatggcgaaa gcgatatata tcttgaaatt 60
atgtacaaat tttttgtttt gttaagatat atcgttttcg acgttaattc cagcctcctc 120
tccattgtcg tagttcgtcc gtgtcgcgag gctatacata aaagtcataa acgcagagaa 180
agaaaattga tatttaattc aatggttaat tgtagctatt atgtaattag caatgactaa 240
tctaacctta tgcagtttgg ttaatttagt aattatacac agactcgaca tgattagtca 300
ggctttgtat atgtgtcgca ttgtcgccac attaagatcc tttagtgcaa ccaaaacaca 360
ctaaactcgg ctgaaatatg gggcatcttg taccttttaa ttatcgctga ttactgattc 420
acatcaaata tatggttcca aattatttta ttaactcaag tgtatagtaa tgtttgactc 480
tttttctgc aaatgtttcg aagacaaagt gctattaagt tttatcaaaa ctatgatgtt 540
ttgtagtttt agatgattat ataacgtttg tcaatctgtc ttttaattta cttaaatcat 600
tagggcaggt aactatattt aaataaaaaa cagagattgt gtatgagttt gaaataaaaa 660
cgtgttaaaa tggaaaaaat aaaccatgcc aagtatttac tttactacaa aggaaaaagt 720
atacagaatt tttacatgat gtcgtatgaa ttacatcatg taataatagt aacttagata 780
ttctgtaaga aaattagatt tgcataatta cgttaatatc cgagggtgta tataaataag 840
aaagaaagaa cagaggatta aaacccacaa attggcgttt tatacatccc catttaaaaa 900
gaatggtaac gccgactctg tgaagcattt ttcagcactc ctttcctttc ccccacaaac 960
taaacaaaaa accattaaca aaaaaaccat tcacaaaaaa aatagagaga atcatctgag 1020

agagtagtag tgatgatatg atcgcttctt ctcctacaat ctcagaaacc tccgatcacg 1080
gttttagata tcttctacaa cggata 1106

<210> 149
<211> 1115
<212> DNA
<213> Arabidopsis thaliana

<220>
<221> promoter
<222> (1)..(1115)
<223> transcription regulating sequence from Arabidopsis thaliana gene At3g15510

<400> 149
aagatatagc gagtatttga ttttatgtgt tagtgtaaat ggcgaaagcg atatatatct 60
tgaaattatg tacaaatttt ttgttttgtt aagatatatc gttttcgacg ttaattccag 120
cctcctctcc attgtcgtag ttcgtccgtg tcgcgaggct atacataaaa gtcataaacg 180
cagagaaaga aaattgatat ttaattcaat ggttaattgt agctattatg taattagcaa 240
tgactaatct aaccttatgc agtttggtta atttagtaat tatacacaga ctcgacatga 300
ttagtcaggc tttgtatatg tgtcgcattg tcgccacatt aagatccttt agtgcaacca 360
aaacacacta aactcggctg aaatatgggg catcttgtac cttttaatta tcgctgatta 420
ctgattcaca tcaaatatat ggttccaaat tattttatta actcaagtgt atagtaatgt 480
ttgactcttt tttctgcaaa tgtttcgaag acaaagtgct attaagtttt atcaaaacta 540
tgatgttttg tagttttaga tgattatata acgtttgtca atctgtcttt taatttactt 600
aaatcattag ggcaggtaac tatatttaaa taaaaaacag agattgtgta tgagtttgaa 660
ataaaaacgt gttaaaatgg aaaaaataaa ccatgccaag tatttacttt actacaaagg 720
aaaaagtata cagaattttt acatgatgtc gtatgaatta catcatgtaa taatagtaac 780
ttagatattc tgtaagaaaa ttagatttgc ataattacgt taatatccga gggtgtatat 840
aaataagaaa gaaagaacag aggattaaaa cccacaaatt ggcgttttat acatccccat 900
ttaaaaagaa tggtaacgcc gactctgtga agcatttttc agcactcctt tcctttcccc 960
cacaaactaa acaaaaaacc attaacaaaa aaaccattca caaaaaaaat agagagaatc 1020
atctgagaga gtagtagtga tgatatgatc gcttcttctc ctacaatctc agaaacctcc 1080
gatcacggtt ttagatatct tctacaacgg ataca 1115

<210> 150
<211> 923
<212> DNA
<213> Arabidopsis thaliana

<220>
<221> promoter
<222> (1)..(923)
<223> transcription regulating sequence from Arabidopsis thaliana gene At3g15510

<400> 150

```
agcgagtatt tgattttatg tgttagtgta aatggcgaaa gcgatatata tcttgaaatt     60
atgtacaaat tttttgtttt gttaagatat atcgttttcg acgttaattc cagcctcctc    120
tccattgtcg tagttcgtcc gtgtcgcgag gctatacata aaagtcataa acgcagagaa    180
agaaaattga tatttaattc aatggttaat tgtagctatt atgtaattag caatgactaa    240
tctaacctta tgcagtttgg ttaatttagt aattatacac agactcgaca tgattagtca    300
ggctttgtat atgtgtcgca ttgtcgccac attaagatcc tttagtgcaa ccaaaacaca    360
ctaaactcgg ctgaaatatg gggcatcttg taccttttaa ttatcgctga ttactgattc    420
acatcaaata tatggttcca aattatttta ttaactcaag tgtatagtaa tgtttgactc    480
tttttctgc aaatgtttcg aagacaaagt gctattaagt tttatcaaaa ctatgatgtt    540
ttgtagtttt agatgattat ataacgtttg tcaatctgtc ttttaattta cttaaatcat    600
tagggcaggt aactatattt aaataaaaaa cagagattgt gtatgagttt gaaataaaaa    660
cgtgttaaaa tggaaaaaat aaaccatgcc aagtatttac tttactacaa aggaaaaagt    720
atacagaatt tttacatgat gtcgtatgaa ttacatcatg taataatagt aacttagata    780
ttctgtaaga aaattagatt tgcataatta cgttaatatc cgagggtgta tataaataag    840
aaagaaagaa cagaggatta aaacccacaa attggcgttt tatacatccc catttaaaaa    900
gaatggtaac gccgactctg tga                                             923
```

<210> 151
<211> 930
<212> DNA
<213> Arabidopsis thaliana

<220>
<221> promoter
<222> (1)..(930)
<223> transcription regulating sequence from Arabidopsis thaliana gene At3g15510

<400> 151

```
aagatatagc gagtatttga ttttatgtgt tagtgtaaat ggcgaaagcg atatatatct     60
tgaaattatg tacaaatttt ttgttttgtt aagatatatc gttttcgacg ttaattccag    120
cctcctctcc attgtcgtag ttcgtccgtg tcgcgaggct atacataaaa gtcataaacg    180
cagagaaaga aaattgatat ttaattcaat ggttaattgt agctattatg taattagcaa    240
tgactaatct aaccttatgc agtttggtta atttagtaat tatacacaga ctcgacatga    300
ttagtcaggc tttgtatatg tgtcgcattg tcgccacatt aagatccttt agtgcaacca    360
aaacacacta aactcggctg aaatatgggg catcttgtac cttttaatta tcgctgatta    420
ctgattcaca tcaaatatat ggttccaaat tattttatta actcaagtgt atagtaatgt    480
ttgactcttt ttctgcaaa tgtttcgaag acaaagtgct attaagtttt atcaaaacta    540
tgatgttttg tagttttaga tgattatata acgtttgtca atctgtcttt taatttactt    600
aaatcattag ggcaggtaac tatatttaaa taaaaaacag agattgtgta tgagtttgaa    660
ataaaaacgt gttaaaatgg aaaaaataaa ccatgccaag tatttacttt actacaaagg    720
aaaaagtata cagaattttt acatgatgtc gtatgaatta catcatgtaa taatagtaac    780
ttagatattc tgtaagaaaa ttagatttgc ataattacgt taatatccga gggtgtatat    840
```

aaataagaaa gaaagaacag aggattaaaa cccacaaatt ggcgttttat acatccccat 900
ttaaaaagaa tggtaacgcc gactctgtga 930

<210> 152
<211> 1845
<212> DNA
<213> Arabidopsis thaliana

<220>
<221> promoter
<222> (1)..(1845)
<223> transcription regulating sequence from Arabidopsis thaliana gene At3g15510

<400> 152
aagtgctcta aactgacaaa actggagaaa tagtaggctg tatttgattt tgtgaaccaa 60
ccaaaaaacc tttgtatttg atttataata ttgtgtgttt taaacataaa gaaaaagata 120
tgagataagg aatctcatgg tcagaagtca aaactagaaa gtcataaaat aaaccactcc 180
taagaatctg ataagtaaac aaactatata tttgcacact aaacgtaatt tagtggtcga 240
aaaatatgca tgggttataa tcatcttatg acgttggaaa aaaaacaaga ataaatcata 300
taataatgaa aatataaaag taatcgaaac ttaaaaatga catgaaattg gtcgatttgt 360
ttgtttccat aatatttatt atctaaaatt caaaaaatat tctggtggtg cgattatctc 420
aataacaaaa gcgtgacatg tgacatctga aacgtaaaac tttttttttt ttcccacacc 480
cacccggcca tctttttcca cgtcatcaat ttgttacctt ctctggcgat ttctgtgggc 540
cctactgccc cgattgccac gtatgaatat atcgtccata ggtatcacac gtgtcgatta 600
ttcattggat atttaacaag aacagtaacc acacaaaatt aataaaatcg tattacagca 660
tacaagatga aattagaaaa tttcttcaac tttttttgt tttgcgatat tgtcttgata 720
atgtattata gtaagatata gcgagtattt gattttatgt gttagtgtaa atggcgaaag 780
cgatatatat cttgaaatta tgtacaaatt ttttgttttg ttaagatata tcgttttcga 840
cgttaattcc agcctcctct ccattgtcgt agttcgtccg tgtcgcgagg ctatacataa 900
aagtcataaa cgcagagaaa gaaaattgat atttaattca atggttaatt gtagctatta 960
tgtaattagc aatgactaat ctaaccttat gcagtttggt taatttagta attatacaca 1020
gactcgacat gattagtcag gctttgtata tgtgtcgcat tgtcgccaca ttaagatcct 1080
ttagtgcaac caaaacacac taaactcggc tgaaatatgg ggcatcttgt accttttaat 1140
tatcgctgat tactgattca catcaaatat atggttccaa attattttat taactcaagt 1200
gtatagtaat gtttgactct tttttctgca aatgtttcga agacaaagtg ctattaagtt 1260
ttatcaaaac tatgatgttt tgtagtttta gatgattata taacgtttgt caatctgtct 1320
tttaatttac ttaaatcatt agggcaggta actatattta aataaaaaac agagattgtg 1380
tatgagtttg aaataaaaac gtgttaaaat ggaaaaaata aaccatgcca agtatttact 1440
ttactacaaa ggaaaaagta tacagaattt ttacatgatg tcgtatgaat tacatcatgt 1500
aataatagta acttagatat tctgtaagaa aattagattt gcataattac gttaatatcc 1560
gagggtgtat ataaataaga aagaaagaac agaggattaa aacccacaaa ttggcgtttt 1620
atacatcccc atttaaaaag aatggtaacg ccgactctgt gaagcatttt tcagcactcc 1680
tttcctttcc cccacaaact aaacaaaaaa ccattaacaa aaaaaccatt cacaaaaaaa 1740
atagagagaa tcatctgaga gagtagtagt gatgatatga tcgcttcttc tcctacaatc 1800

tcagaaacct ccgatcacgg ttttagatat cttctacaac ggata 1845

<210> 153
<211> 1854
<212> DNA
<213> Arabidopsis thaliana

<220>
<221> promoter
<222> (1)..(1854)
<223> transcription regulating sequence from Arabidopsis thaliana gene At3g15510

<400> 153
tcaaacaaag tgctctaaac tgacaaaact ggagaaatag taggctgtat ttgattttgt 60
gaaccaacca aaaaaccttt gtatttgatt tataatattg tgtgttttaa acataaagaa 120
aaagatatga gataaggaat ctcatggtca gaagtcaaaa ctagaaagtc ataaaataaa 180
ccactcctaa gaatctgata agtaaacaaa ctatatattt gcacactaaa cgtaatttag 240
tggtcgaaaa atatgcatgg gttataatca tcttatgacg ttggaaaaaa aacaagaata 300
aatcatataa taatgaaaat ataaaagtaa tcgaaactta aaaatgacat gaaattggtc 360
gatttgtttg tttccataat atttattatc taaaattcaa aaaatattct ggtggtgcga 420
ttatctcaat aacaaaagcg tgacatgtga catctgaaac gtaaaacttt tttttttttc 480
ccacacccac ccggccatct ttttccacgt catcaatttg ttaccttctc tggcgatttc 540
tgtgggccct actgccccga ttgccacgta tgaatatatc gtccataggt atcacacgtg 600
tcgattattc attggatatt taacaagaac agtaaccaca caaaattaat aaaatcgtat 660
tacagcatac aagatgaaat tagaaaattt cttcaacttt tttttgtttt gcgatattgt 720
cttgataatg tattatagta agatatagcg agtatttgat tttatgtgtt agtgtaaatg 780
gcgaaagcga tatatatctt gaaattatgt acaaattttt tgttttgtta agatatatcg 840
ttttcgacgt taattccagc ctcctctcca ttgtcgtagt tcgtccgtgt cgcgaggcta 900
tacataaaag tcataaacgc agagaaagaa aattgatatt taattcaatg gttaattgta 960
gctattatgt aattagcaat gactaatcta accttatgca gtttggttaa tttagtaatt 1020
atacacagac tcgacatgat tagtcaggct ttgtatatgt gtcgcattgt cgccacatta 1080
agatccttta gtgcaaccaa aacacactaa actcggctga aatatggggc atcttgtacc 1140
ttttaattat cgctgattac tgattcacat caaatatatg gttccaaatt attttattaa 1200
ctcaagtgta tagtaatgtt tgactctttt ttctgcaaat gtttcgaaga caaagtgcta 1260
ttaagtttta tcaaaactat gatgttttgt agttttagat gattatataa cgtttgtcaa 1320
tctgtctttt aatttactta aatcattagg gcaggtaact atatttaaat aaaaaacaga 1380
gattgtgtat gagtttgaaa taaaaacgtg ttaaaatgga aaaaataaac catgccaagt 1440
atttacttta ctacaaagga aaaagtatac agaattttta catgatgtcg tatgaattac 1500
atcatgtaat aatagtaact tagatattct gtaagaaaat tagatttgca taattacgtt 1560
aatatccgag ggtgtatata aataagaaag aaagaacaga ggattaaaac ccacaaattg 1620
gcgttttata catccccatt taaaaagaat ggtaacgccg actctgtgaa gcatttttca 1680
gcactccttt cctttccccc acaaactaaa caaaaaacca ttaacaaaaa aaccattcac 1740
aaaaaaaata gagagaatca tctgagagag tagtagtgat gatatgatcg cttcttctcc 1800
tacaatctca gaaacctccg atcacggttt tagatatctt ctacaacgga taca 1854

<210> 154
<211> 1662
<212> DNA
<213> Arabidopsis thaliana

<220>
<221> promoter
<222> (1)..(1662)
<223> transcription regulating sequence from Arabidopsis thaliana gene At3g15510

<400> 154
aagtgctcta aactgacaaa actggagaaa tagtaggctg tatttgattt tgtgaaccaa 60
ccaaaaaacc tttgtatttg atttataata ttgtgtgttt taaacataaa gaaaaagata 120
tgagataagg aatctcatgg tcagaagtca aaactagaaa gtcataaaat aaaccactcc 180
taagaatctg ataagtaaac aaactatata tttgcacact aaacgtaatt tagtggtcga 240
aaaatatgca tgggttataa tcatcttatg acgttggaaa aaaaacaaga ataaatcata 300
taataatgaa aatataaaag taatcgaaac ttaaaaatga catgaaattg gtcgatttgt 360
ttgtttccat aatatttatt atctaaaatt caaaaaatat tctggtggtg cgattatctc 420
aataacaaaa gcgtgacatg tgacatctga aacgtaaaac tttttttttt ttcccacacc 480
cacccggcca tctttttcca cgtcatcaat ttgttacctt ctctggcgat ttctgtgggc 540
cctactgccc cgattgccac gtatgaatat atcgtccata ggtatcacac gtgtcgatta 600
ttcattggat atttaacaag aacagtaacc acacaaaatt aataaaatcg tattacagca 660
tacaagatga aattagaaaa tttcttcaac ttttttttgt tttgcgatat tgtcttgata 720
atgtattata gtaagatata gcgagtattt gattttatgt gttagtgtaa atggcgaaag 780
cgatatatat cttgaaatta tgtacaaatt ttttgttttg ttaagatata tcgttttcga 840
cgttaattcc agcctcctct ccattgtcgt agttcgtccg tgtcgcgagg ctatacataa 900
aagtcataaa cgcagagaaa gaaaattgat atttaattca atggttaatt gtagctatta 960
tgtaattagc aatgactaat ctaaccttat gcagtttggt taatttagta attatacaca 1020
gactcgacat gattagtcag gctttgtata tgtgtcgcat tgtcgccaca ttaagatcct 1080
ttagtgcaac caaaacacac taaactcggc tgaaatatgg ggcatcttgt accttttaat 1140
tatcgctgat tactgattca catcaaatat atggttccaa attattttat taactcaagt 1200
gtatagtaat gtttgactct tttttctgca aatgtttcga agacaaagtg ctattaagtt 1260
ttatcaaaac tatgatgttt tgtagtttta gatgattata taacgtttgt caatctgtct 1320
tttaatttac ttaaatcatt agggcaggta actatattta aataaaaaac agagattgtg 1380
tatgagtttg aaataaaaac gtgttaaaat ggaaaaaata aaccatgcca agtatttact 1440
ttactacaaa ggaaaaagta tacagaattt ttacatgatg tcgtatgaat tacatcatgt 1500
aataatagta acttagatat tctgtaagaa aattagattt gcataattac gttaatatcc 1560
gagggtgtat ataaataaga aagaaagaac agaggattaa aacccacaaa ttggcgtttt 1620
atacatcccc atttaaaaag aatggtaacg ccgactctgt ga 1662

<210> 155
<211> 1669
<212> DNA

<213> Arabidopsis thaliana

<220>
<221> promoter
<222> (1)..(1669)
<223> transcription regulating sequence from Arabidopsis thaliana gene At3g15510

<400> 155
```
tcaaacaaag tgctctaaac tgacaaaact ggagaaatag taggctgtat ttgattttgt     60
gaaccaacca aaaaaccttt gtatttgatt tataatattg tgtgttttaa acataaagaa    120
aaagatatga gataaggaat ctcatggtca gaagtcaaaa ctagaaagtc ataaaataaa    180
ccactcctaa gaatctgata agtaaacaaa ctatatattt gcacactaaa cgtaatttag    240
tggtcgaaaa atatgcatgg gttataatca tcttatgacg ttggaaaaaa aacaagaata    300
aatcatataa taatgaaaat ataaaagtaa tcgaaactta aaaatgacat gaaattggtc    360
gatttgtttg tttccataat atttattatc taaaattcaa aaaatattct ggtggtgcga    420
ttatctcaat aacaaaagcg tgacatgtga catctgaaac gtaaaacttt tttttttttc    480
ccacacccac ccggccatct ttttccacgt catcaatttg ttaccttctc tggcgatttc    540
tgtgggccct actgccccga ttgccacgta tgaatatatc gtccataggt atcacacgtg    600
tcgattattc attggatatt taacaagaac agtaaccaca caaaattaat aaaatcgtat    660
tacagcatac aagatgaaat tagaaaattt cttcaacttt tttttgtttt gcgatattgt    720
cttgataatg tattatagta agatatagcg agtatttgat tttatgtgtt agtgtaaatg    780
gcgaaagcga tatatatctt gaaattatgt acaaattttt tgttttgtta agatatatcg    840
ttttcgacgt taattccagc ctcctctcca ttgtcgtagt tcgtccgtgt cgcgaggcta    900
tacataaaag tcataaacgc agagaaagaa aattgatatt taattcaatg gttaattgta    960
gctattatgt aattagcaat gactaatcta accttatgca gtttggttaa tttagtaatt   1020
atacacagac tcgacatgat tagtcaggct ttgtatatgt gtcgcattgt cgccacatta   1080
agatccttta gtgcaaccaa aacacactaa actcggctga aatatggggc atcttgtacc   1140
ttttaattat cgctgattac tgattcacat caaatatatg gttccaaatt attttattaa   1200
ctcaagtgta tagtaatgtt tgactctttt ttctgcaaat gtttcgaaga caaagtgcta   1260
ttaagtttta tcaaaactat gatgttttgt agttttagat gattatataa cgtttgtcaa   1320
tctgtctttt aatttactta aatcattagg gcaggtaact atatttaaat aaaaaacaga   1380
gattgtgtat gagtttgaaa taaaaacgtg ttaaaatgga aaaaataaac catgccaagt   1440
atttacttta ctacaaagga aaaagtatac agaattttta catgatgtcg tatgaattac   1500
atcatgtaat aatagtaact tagatattct gtaagaaaat tagatttgca taattacgtt   1560
aatatccgag ggtgtatata aataagaaag aaagaacaga ggattaaaac ccacaaattg   1620
gcgttttata catccccatt taaaaagaat ggtaacgccg actctgtga              1669
```

<210> 156
<211> 1625
<212> DNA
<213> Arabidopsis thaliana

<220>
<221> CDS

<222> (186)..(1280)
<223> encoding Arabidopsis thaliana no apical meristem (NAM) family protein (NAC2)

<400> 156
agcatttttc agcactcctt tcctttcccc cacaaactaa acaaaaaacc attaacaaaa 60
aaaccattca caaaaaaaat agagagaatc atctgagaga gtagtagtga tgatatgatc 120
gcttcttctc ctacaatctc agaaacctcc gatcacggtt ttagatatct tctacaacgg 180
ataca atg gag agc acc gat tct tcc ggt ggt cca cca ccg cca caa cct 230
Met Glu Ser Thr Asp Ser Ser Gly Gly Pro Pro Pro Pro Gln Pro
1 5 10 15
aac ctt cct cca ggc ttc cgg ttt cac cct acc gac gaa gag ctt gtt 278
Asn Leu Pro Pro Gly Phe Arg Phe His Pro Thr Asp Glu Glu Leu Val
20 25 30
gtt cac tac ctc aaa cgc aaa gca gcc tct gct cct tta cct gtc gcc 326
Val His Tyr Leu Lys Arg Lys Ala Ala Ser Ala Pro Leu Pro Val Ala
35 40 45
atc atc gcc gaa gtc gat ctc tat aaa ttt gat cca tgg gaa ctt ccc 374
Ile Ile Ala Glu Val Asp Leu Tyr Lys Phe Asp Pro Trp Glu Leu Pro
50 55 60
gct aaa gca tcg ttt gga gaa caa gaa tgg tac ttc ttt agt cca cga 422
Ala Lys Ala Ser Phe Gly Glu Gln Glu Trp Tyr Phe Phe Ser Pro Arg
65 70 75
gat cgg aag tat cca aac gga gca aga cca aac aga gcg gcg act tca 470
Asp Arg Lys Tyr Pro Asn Gly Ala Arg Pro Asn Arg Ala Ala Thr Ser
80 85 90 95
ggt tat tgg aaa gcg acc ggt aca gat aaa ccg gta ctt gct tcc gac 518
Gly Tyr Trp Lys Ala Thr Gly Thr Asp Lys Pro Val Leu Ala Ser Asp
100 105 110
ggt aac caa aag gtg ggc gtg aag aag gca cta gtc ttc tac agt ggt 566
Gly Asn Gln Lys Val Gly Val Lys Lys Ala Leu Val Phe Tyr Ser Gly
115 120 125
aaa cca cca aaa ggc gtt aaa agt gat tgg atc atg cat gag tat cgt 614
Lys Pro Pro Lys Gly Val Lys Ser Asp Trp Ile Met His Glu Tyr Arg
130 135 140
ctc atc gaa aac aaa cca aac aat cga cct cct ggc tgt gat ttc ggc 662
Leu Ile Glu Asn Lys Pro Asn Asn Arg Pro Pro Gly Cys Asp Phe Gly
145 150 155
aac aaa aaa aac tca ctc aga ctt gat gat tgg gtg tta tgt aga atc 710
Asn Lys Lys Asn Ser Leu Arg Leu Asp Asp Trp Val Leu Cys Arg Ile
160 165 170 175
tac aag aag aac aac gca agt cga cat gtt gat aac gat aag gat cat 758
Tyr Lys Lys Asn Asn Ala Ser Arg His Val Asp Asn Asp Lys Asp His
180 185 190
gat atg atc gat tac att ttc agg aag att cct ccg tct tta tca atg 806

Asp Met Ile Asp Tyr Ile Phe Arg Lys Ile Pro Pro Ser Leu Ser Met
195 200 205

gcg gct gct tct aca gga ctt cac caa cat cat cat aat gtc tca aga 854
Ala Ala Ala Ser Thr Gly Leu His Gln His His His Asn Val Ser Arg
210 215 220

tca atg aat ttc ttc cct ggc aaa ttc tcc ggt ggt ggt tac ggg att 902
Ser Met Asn Phe Phe Pro Gly Lys Phe Ser Gly Gly Gly Tyr Gly Ile
225 230 235

ttc tct gac ggt ggt aac acg agt ata tac gac ggc ggt ggc atg atc 950
Phe Ser Asp Gly Gly Asn Thr Ser Ile Tyr Asp Gly Gly Gly Met Ile
240 245 250 255

aac aat att ggt act gac tca gta gat cac gac aat aac gct gac gtc 998
Asn Asn Ile Gly Thr Asp Ser Val Asp His Asp Asn Asn Ala Asp Val
260 265 270

gtt ggt tta aat cat gct tcg tcg tca ggt cct atg atg atg gcg aat 1046
Val Gly Leu Asn His Ala Ser Ser Ser Gly Pro Met Met Met Ala Asn
275 280 285

ttg aaa cga act ctc ccg gtg ccg tat tgg cct gta gca gat gag gag 1094
Leu Lys Arg Thr Leu Pro Val Pro Tyr Trp Pro Val Ala Asp Glu Glu
290 295 300

caa gat gca tct ccg agc aaa cgg ttt cac ggt gta gga gga gga gga 1142
Gln Asp Ala Ser Pro Ser Lys Arg Phe His Gly Val Gly Gly Gly Gly
305 310 315

gga gat tgt tcg aac atg tct tcc tcc atg atg gaa gag act cca cca 1190
Gly Asp Cys Ser Asn Met Ser Ser Ser Met Met Glu Glu Thr Pro Pro
320 325 330 335

ttg atg caa caa caa ggt ggt gtg tta gga gat gga tta ttc aga acg 1238
Leu Met Gln Gln Gln Gly Gly Val Leu Gly Asp Gly Leu Phe Arg Thr
340 345 350

aca tcg tac caa tta ccc ggt tta aat tgg tac tct tct taa 1280
Thr Ser Tyr Gln Leu Pro Gly Leu Asn Trp Tyr Ser Ser
355 360

tcaaatgtgt ttcgccgccg gtgtgaagaa ttttccggtg acagtgaaga tttttttccg 1340
attggtgggg tcatttgcat gcattatata atttgagatt tgtgtatatg ttttgggtta 1400
attaattggt cacaggggtt agaggaagaa gattatttag agagcggaga agaagtgagg 1460
caacatatag aaaacatagg tttaaaaaat attactgttt gttaagttta atgattaaat 1520
gtataaatct tatacttaga tgtttatata tagtttgtat agatgcatat agtttctctc 1580
aatgctattt tgagattgta ttttcttgta aaggaaatgt ttttt 1625

<210> 157
<211> 364
<212> PRT
<213> Arabidopsis thaliana

<400> 157

```
Met Glu Ser Thr Asp Ser Ser Gly Gly Pro Pro Pro Pro Gln Pro Asn
1               5                   10                  15

Leu Pro Pro Gly Phe Arg Phe His Pro Thr Asp Glu Glu Leu Val Val
            20                  25                  30

His Tyr Leu Lys Arg Lys Ala Ala Ser Ala Pro Leu Pro Val Ala Ile
        35                  40                  45

Ile Ala Glu Val Asp Leu Tyr Lys Phe Asp Pro Trp Glu Leu Pro Ala
    50                  55                  60

Lys Ala Ser Phe Gly Glu Gln Glu Trp Tyr Phe Phe Ser Pro Arg Asp
65                  70                  75                  80

Arg Lys Tyr Pro Asn Gly Ala Arg Pro Asn Arg Ala Ala Thr Ser Gly
                85                  90                  95

Tyr Trp Lys Ala Thr Gly Thr Asp Lys Pro Val Leu Ala Ser Asp Gly
            100                 105                 110

Asn Gln Lys Val Gly Val Lys Lys Ala Leu Val Phe Tyr Ser Gly Lys
        115                 120                 125

Pro Pro Lys Gly Val Lys Ser Asp Trp Ile Met His Glu Tyr Arg Leu
    130                 135                 140

Ile Glu Asn Lys Pro Asn Asn Arg Pro Pro Gly Cys Asp Phe Gly Asn
145                 150                 155                 160

Lys Lys Asn Ser Leu Arg Leu Asp Asp Trp Val Leu Cys Arg Ile Tyr
                165                 170                 175

Lys Lys Asn Asn Ala Ser Arg His Val Asp Asn Asp Lys Asp His Asp
            180                 185                 190

Met Ile Asp Tyr Ile Phe Arg Lys Ile Pro Pro Ser Leu Ser Met Ala
        195                 200                 205

Ala Ala Ser Thr Gly Leu His Gln His His His Asn Val Ser Arg Ser
    210                 215                 220

Met Asn Phe Phe Pro Gly Lys Phe Ser Gly Gly Gly Tyr Gly Ile Phe
225                 230                 235                 240
```

```
Ser Asp Gly Gly Asn Thr Ser Ile Tyr Asp Gly Gly Gly Met Ile Asn
                245                 250                 255

Asn Ile Gly Thr Asp Ser Val Asp His Asp Asn Asn Ala Asp Val Val
            260                 265                 270

Gly Leu Asn His Ala Ser Ser Ser Gly Pro Met Met Met Ala Asn Leu
        275                 280                 285

Lys Arg Thr Leu Pro Val Pro Tyr Trp Pro Val Ala Asp Glu Glu Gln
    290                 295                 300

Asp Ala Ser Pro Ser Lys Arg Phe His Gly Val Gly Gly Gly Gly Gly
305                 310                 315                 320

Asp Cys Ser Asn Met Ser Ser Ser Met Met Glu Glu Thr Pro Pro Leu
                325                 330                 335

Met Gln Gln Gln Gly Gly Val Leu Gly Asp Gly Leu Phe Arg Thr Thr
            340                 345                 350

Ser Tyr Gln Leu Pro Gly Leu Asn Trp Tyr Ser Ser
        355                 360
```

```
<210>  158
<211>  1017
<212>  DNA
<213>  Arabidopsis thaliana

<220>
<221>  promoter
<222>  (1)..(1017)
<223>  transcription regulating sequence from Arabidopsis thaliana gene
       At3g50870

<400>  158
ttcttcttct tctacgtttt ttattttctg ttgaattttc ctccacaaaa tcgataattt      60
caatttttc atacaatatc atagttctag taaagtaaac attcgtgtgt gagtgacata      120
ataattgctt caaaaagaaa taacgaatta ataagagttg tgtgcttgat ggtccaaaag     180
tcgtatgttt ttcaccatca caataagatt taccatatct acttaaacca aacttcaatc     240
tatgatattt ggaagctagc aatgaatgat tttgttttag catatgttac cgagcctatt     300
catctttaga ttgacattcg cattttaaag ttttgtgtcc aacaactagc aaacccttcg     360
ctttttaaac caagatcagt ttttctacat agtttcgact ttttgttatt ttacttccca     420
tcatattttg ttttatttac tactgacatt ttcataatgt acggtacatt ttcatatatt     480
agcatcctta tctaaaagct aatcaactcc ataacgtact tgaaataggg atattttagt     540
```

```
tgagttaatt attcaaaaca atcacacagt gacacagact ctatgtagac caagcatgtg    600
ttatgtaatt agaaaaaata aactgtgatt aaaatatctt ttaacttgtt aacttggctg    660
cctaaagaaa cttgatactg tatcatcgtt catcgtattt tgtaattaat aaaacataaa    720
aaagcaagga aaacccattt gagtaaatga tatattaata ttacttcgta aaaagctgcg    780
tccccacata ttctattgat tttaaatact attcgtatta atataaacct gcatataagc    840
gtacatatac atttctgatt ctatatatag gtgtgagacc ttcttctcca ttctacactt    900
cactccaacg tgaaacctgc aaactgaact tatctcttca tattttcttt agcttctctt    960
aattttacac ttagggtttt caaaccagta gagagagaga gaagtgtgta agagcga      1017
```

<210> 159
<211> 1008
<212> DNA
<213> Arabidopsis thaliana

<220>
<221> promoter
<222> (1)..(1008)
<223> transcription regulating sequence from Arabidopsis thaliana gene At3g50870

<400> 159

```
cactctctcc ttcttcttct tctacgtttt ttattttctg ttaaatttcc tccacaaaat     60
cgataatttc aattttttca tacaatatca tagttctagt aaagtaaaca ttcgtgtgtg    120
agtgacataa taattgcttc aaaaagaaat aacgaattaa taagagttgt gtgcttgatg    180
gtccaaaagt cgtatgtttt tcaccatcac aataagattt accatatcta cttaaaccaa    240
acttcaatct atgatatttg gaagctagca atgaatgatt ttgttttagc atatgttacc    300
gagcctattc atctttagat tgaaagtttt gtgtccaaca actagcaaac ccttcgcttt    360
ttaaaccaag atcagttttt ctacatagtt tcgacttttt gttattttac ttcccatcat    420
atttgtttt atttactact gacattttca taatgtacgg tacattttca tatattagca    480
tccttatcta aaagctaatc aactccataa cgtacttgaa atagggatat tttagttgaa    540
ttattcaaaa caatcacaca gtgacacaga ctctatgtag accaagcatg tgttatgtaa    600
ttagaaaaaa taaactgtga ttaaaatatc ttttaacttg ttaacttggc tgcctaaaga    660
aacttgatac tgtatcatcg ttcatcgtat tttgtaatta ataaaacata aaaaagcaag    720
gaaaacccat ttgagtaaat gatatattaa tattacttcg taaaaagctg cgtccccaca    780
tattctattg attttaaata ctattcgtat taatataaac ctgcatataa gcgtacatat    840
acatttctga ttctatatat aggtgtgaga ccttcttctc cattctacac ttcactccaa    900
cgtgaaacct gcaaactgaa cttatctctt catattttct ttagcttctc ttaattttac    960
acttagggtt ttcaaaccag tagagagaga gagaagtgtg taagagcg                1008
```

<210> 160
<211> 894
<212> DNA
<213> Arabidopsis thaliana

<220>

<221> promoter
<222> (1)..(894)
<223> transcription regulating sequence from Arabidopsis thaliana gene At3g50870

<400> 160
```
ttcttcttct tctacgtttt ttattttctg ttgaattttc ctccacaaaa tcgataattt     60
caatttttc atacaatatc atagttctag taaagtaaac attcgtgtgt gagtgacata    120
ataattgctt caaaaagaaa taacgaatta ataagagttg tgtgcttgat ggtccaaaag    180
tcgtatgttt ttcaccatca caataagatt taccatatct acttaaacca aacttcaatc    240
tatgatattt ggaagctagc aatgaatgat tttgttttag catatgttac cgagcctatt    300
catctttaga ttgacattcg cattttaaag ttttgtgtcc aacaactagc aaacccttcg    360
ctttttaaac caagatcagt ttttctacat agtttcgact ttttgttatt ttacttccca    420
tcatattttg ttttatttac tactgacatt ttcataatgt acggtacatt ttcatatatt    480
agcatcctta tctaaaagct aatcaactcc ataacgtact tgaaataggg atattttagt    540
tgagttaatt attcaaaaca atcacacagt gacacagact ctatgtagac caagcatgtg    600
ttatgtaatt agaaaaaata aactgtgatt aaaatatctt ttaacttgtt aacttggctg    660
cctaaagaaa cttgatactg tatcatcgtt catcgtattt tgtaattaat aaaacataaa    720
aaagcaagga aaacccattt gagtaaatga tatattaata ttacttcgta aaaagctgcg    780
tccccacata ttctattgat tttaaatact attcgtatta atataaacct gcatataagc    840
gtacatatac atttctgatt ctatatatag gtgtgagacc ttcttctcca ttct          894
```

<210> 161
<211> 886
<212> DNA
<213> Arabidopsis thaliana

<220>
<221> promoter
<222> (1)..(886)
<223> transcription regulating sequence from Arabidopsis thaliana gene At3g50870

<400> 161
```
cactctctcc ttcttcttct tctacgtttt ttattttctg ttaaatttcc tccacaaaat     60
cgataatttc aatttttca tacaatatca tagttctagt aaagtaaaca ttcgtgtgtg    120
agtgacataa taattgcttc aaaaagaaat aacgaattaa taagagttgt gtgcttgatg    180
gtccaaaagt cgtatgtttt tcaccatcac aataagattt accatatcta cttaaaccaa    240
acttcaatct atgatatttg gaagctagca atgaatgatt ttgttttagc atatgttacc    300
gagcctattc atctttagat tgaaagtttt gtgtccaaca actagcaaac ccttcgcttt    360
ttaaaccaag atcagttttt ctacatagtt tcgacttttt gttattttac ttcccatcat    420
attttgtttt atttactact gacattttca taatgtacgg tacattttca tatattagca    480
tccttatcta aaagctaatc aactccataa cgtacttgaa atagggatat tttagttgaa    540
ttattcaaaa caatcacaca gtgacacaga ctctatgtag accaagcatg tgttatgtaa    600
ttagaaaaaa taaactgtga ttaaaatatc ttttaacttg ttaacttggc tgcctaaaga    660
```

aacttgatac tgtatcatcg ttcatcgtat tttgtaatta ataaaacata aaaaagcaag 720
gaaaacccat ttgagtaaat gatatattaa tattacttcg taaaaagctg cgtccccaca 780
tattctattg attttaaata ctattcgtat taatataaac ctgcatataa gcgtacatat 840
acatttctga ttctatatat aggtgtgaga ccttcttctc cattct 886

<210> 162
<211> 2017
<212> DNA
<213> Arabidopsis thaliana

<220>
<221> promoter
<222> (1)..(2017)
<223> transcription regulating sequence from Arabidopsis thaliana gene At3g50870

<400> 162
atcaatagaa gagtaattaa tcgttccatc ggaacatccg atgaaattgg aacgagacct 60
atacagaaga aaacaaaaga gcaactaatc atcttgatca catgcccaat atgcatttaa 120
gtaaattgga gagaatgata tagaaaaaca agacattatc tacaactaca agatatcaaa 180
gcactcggtt aaccgcgtcc ctgtgaagtg ttagttatca gtacatgaat tgtgatttca 240
gcatcttggt cctttttttt ttggacaacc gtcatcttta ttcttgatcg gtttttataa 300
cgtacaaaca tatactattt caacagtttt cttttaattt ttcttttttt ttgaatatac 360
actttctttc ttttattttt ttcattacaa aattttcaaa aaatagtgtt cggcaaaaaa 420
aaaaattaga tcggccaatg cattatatcc cctagcgtaa gctctttttc aaccaatcga 480
gagtccatgt tttcaataaa aatgggtttt attagtaaca gtagttaaag catttaacat 540
acgtaatccc aaaaaaataa aataattctg tatagggacg aagactagta ttcgttgacg 600
acaagagtag agtagactct agctagctgc aagttttgaa gaaatctaat aaccaaatct 660
aagtcatttt acttacatca tcatggatca tcattaacca catgaggctg agcagatatc 720
gaacgtgtga ttgcgatgtg gagaagtaaa ggacgaggcc acgcgcaact gagcaggagc 780
gcgtgagatt cagggtttta gggcagagat acggaaagag acagaggttt tatagcatta 840
tattgaaaga gaacaaataa aaataaaaat atgagcctcg tgatcggaga tttttgcagt 900
gatgactgta ctcttcttcc tcttctctcc cccttgtcac gtgggctaaa ttttgaccac 960
ccttcttcaa tgaccatcaa catcacatca cactctctcc ttcttcttct tctacgtttt 1020
ttattttctg ttgaattttc ctccacaaaa tcgataattt caatttttc atacaatatc 1080
atagttctag taaagtaaac attcgtgtgt gagtgacata ataattgctt caaaaagaaa 1140
taacgaatta ataagagttg tgtgcttgat ggtccaaaag tcgtatgttt ttcaccatca 1200
caataagatt taccatatct acttaaacca aacttcaatc tatgatattt ggaagctagc 1260
aatgaatgat tttgttttag catatgttac cgagcctatt catctttaga ttgacattcg 1320
catttaaaag ttttgtgtcc aacaactagc aaacccttcg ctttttaaac caagatcagt 1380
ttttctacat agtttcgact ttttgttatt ttacttccca tcatattttg ttttatttac 1440
tactgacatt ttcataatgt acggtacatt ttcatatatt agcatcctta tctaaaagct 1500
aatcaactcc ataacgtact tgaaataggg atattttagt tgagttaatt attcaaaaca 1560
atcacacagt gacacagact ctatgtagac caagcatgtg ttatgtaatt agaaaaaata 1620
aactgtgatt aaaatatctt ttaacttgtt aacttggctg cctaaagaaa cttgatactg 1680

tatcatcgtt catcgtattt tgtaattaat aaaacataaa aaagcaagga aaacccattt 1740
gagtaaatga tatattaata ttacttcgta aaaagctgcg tccccacata ttctattgat 1800
tttaaatact attcgtatta atataaacct gcatataagc gtacatatac atttctgatt 1860
ctatatatag gtgtgagacc ttcttctcca ttctacactt cactccaacg tgaaacctgc 1920
aaactgaact tatctcttca tattttcttt agcttctctt aattttacac ttagggtttt 1980
caaaccagta gagagagaga gaagtgtgta agagcga 2017

<210> 163
<211> 2004
<212> DNA
<213> Arabidopsis thaliana

<220>
<221> promoter
<222> (1)..(2004)
<223> *transcription regulating sequence from* Arabidopsis thaliana *gene* At3g50870

<400> 163
tataaacatc aatagaagag taattaatcg ttccatcgga acatccgatg aaattggaac 60
gagacctata cagaagaaaa caaaagagca actaatcatc ttgatcacat gcccaatatg 120
catttaagta aattggagag aatgatatag aaaaacaaga cattatctac aactacaaga 180
tatcaaagca ctcggttaac cgcgtccctg tgaagtgtta gttatcagta catgaattgt 240
gatttcagca tcttggtcct tttttttttg gacaaccgtc atctttttct tgatcggttt 300
ttataacgta caaacatata ctatttcaac agttttcttt taattttttt tttttttgaa 360
tatacacttt ctttctttta tttgtttgat tacaaaattt tcaaaaaata gtgttcggca 420
aaaaaaaaaa ttagatcggc caatgcatta tatcccctag cgtaagctct ttttcaacca 480
atcgagagtc catgttttca ataaaaatag ggttttatta gtaacagtag ttaaagcatt 540
taacatacgt aatcccaaaa aaataaaata attctgtata gggacgaaga ctagtattcg 600
ttgacgacaa gagtagagta gactctagct agctgcaagt tttgaagaaa tctaataacc 660
aaatctaagt cattttactt acatcatcat ggatcatcat taaccacatg aggctgagca 720
gatatcgaac gtgtgattgc gatgtggaga agtaaaggac gaggccacgc gcaactgagc 780
aggagcgcgt gagattcagg gttttagggc agagatacgg aaagagacag aggttttata 840
gcattatatt gaaagagaac aaataaaaat aaaaatatga gcctcgtgat cggagatttt 900
tgcagtgatg actgtactct tcttcctctt ctctccccct tgtcacgtgg gctaaatttt 960
gaccacccttt cttcaatgac catcaacatc gcatcacact ctctccttct tcttcttcta 1020
cgttttttat tttctgttaa atttcctcca caaaatcgat aatttcaatt ttttcataca 1080
atatcatagt tctagtaaag taaacattcg tgtgtgagtg acataataat tgcttcaaaa 1140
agaaataacg aattaataag agttgtgtgc ttgatggtcc aaaagtcgta tgtttttcac 1200
catcacaata agatttacca tatctactta aaccaaactt caatctatga tatttggaag 1260
ctagcaatga atgattttgt tttagcatat gttaccgagc ctattcatct ttagattgaa 1320
agttttgtgt ccaacaacta gcaaaccctt cgctttttaa accaagatca gtttttctac 1380
atagtttcga ctttttgtta ttttacttcc catcatattt tgttttattt actactgaca 1440
ttttcataat gtacggtaca ttttcatata ttagcatcct tatctaaaag ctaatcaact 1500
ccataacgta cttgaaatag ggatatttta gttgaattat tcaaaacaat cacacagtga 1560

cacagactct atgtagacca agcatgtgtt atgtaattag aaaaaataaa ctgtgattaa 1620
aatatctttt aacttgttaa cttggctgcc taaagaaact tgatactgta tcatcgttca 1680
tcgtattttg taattaataa aacataaaaa agcaaggaaa acccatttga gtaaatgata 1740
tattaatatt acttcgtaaa aagctgcgtc cccacatatt ctattgattt taaatactat 1800
tcgtattaat ataaacctgc atataagcgt acatatacat ttctgattct atatataggt 1860
gtgagacctt cttctccatt ctacacttca ctccaacgtg aaacctgcaa actgaactta 1920
tctcttcata ttttctttag cttctcttaa ttttacactt agggttttca aaccagtaga 1980
gagagagaga agtgtgtaag agcg 2004

<210> 164
<211> 1894
<212> DNA
<213> Arabidopsis thaliana

<220>
<221> promoter
<222> (1)..(1894)
<223> transcription regulating sequence from Arabidopsis thaliana gene At3g50870

<400> 164
atcaatagaa gagtaattaa tcgttccatc ggaacatccg atgaaattgg aacgagacct 60
atacagaaga aaacaaaaga gcaactaatc atcttgatca catgcccaat atgcatttaa 120
gtaaattgga gagaatgata tagaaaaaca agacattatc tacaactaca agatatcaaa 180
gcactcggtt aaccgcgtcc ctgtgaagtg ttagttatca gtacatgaat tgtgatttca 240
gcatcttggt cctttttttt ttggacaacc gtcatcttta ttcttgatcg gtttttataa 300
cgtacaaaca tatactattt caacagtttt cttttaattt ttcttttttt ttgaatatac 360
actttctttc ttttattttt ttcattacaa aattttcaaa aaatagtgtt cggcaaaaaa 420
aaaaattaga tcggccaatg cattatatcc cctagcgtaa gctctttttc aaccaatcga 480
gagtccatgt tttcaataaa aatgggtttt attagtaaca gtagttaaag catttaacat 540
acgtaatccc aaaaaaataa aataattctg tatagggacg aagactagta ttcgttgacg 600
acaagagtag agtagactct agctagctgc aagttttgaa gaaatctaat aaccaaatct 660
aagtcatttt acttacatca tcatggatca tcattaacca catgaggctg agcagatatc 720
gaacgtgtga ttgcgatgtg gagaagtaaa ggacgaggcc acgcgcaact gagcaggagc 780
gcgtgagatt cagggtttta gggcagagat acggaaagag acagaggttt tatagcatta 840
tattgaaaga gaacaaataa aaataaaaat atgagcctcg tgatcggaga tttttgcagt 900
gatgactgta ctcttcttcc tcttctctcc cccttgtcac gtgggctaaa ttttgaccac 960
ccttcttcaa tgaccatcaa catcacatca cactctctcc ttcttcttct tctacgtttt 1020
ttattttctg ttgaattttc ctccacaaaa tcgataattt caattttttc ataccaatatc 1080
atagttctag taaagtaaac attcgtgtgt gagtgacata ataattgctt caaaaagaaa 1140
taacgaatta ataagagttg tgtgcttgat ggtccaaaag tcgtatgttt ttcaccatca 1200
caataagatt taccatatct acttaaacca aacttcaatc tatgatattt ggaagctagc 1260
aatgaatgat tttgttttag catatgttac cgagcctatt catctttaga ttgacattcg 1320
cattttaaag ttttgtgtcc aacaactagc aaacccttcg ctttttaaac caagatcagt 1380
ttttctacat agtttcgact ttttgttatt ttacttccca tcatattttg ttttatttac 1440

tactgacatt ttcataatgt acggtacatt ttcatatatt agcatcctta tctaaaagct 1500
aatcaactcc ataacgtact tgaaataggg atattttagt tgagttaatt attcaaaaca 1560
atcacacagt gacacagact ctatgtagac caagcatgtg ttatgtaatt agaaaaaata 1620
aactgtgatt aaaatatctt ttaacttgtt aacttggctg cctaaagaaa cttgatactg 1680
tatcatcgtt catcgtattt tgtaattaat aaaacataaa aaagcaagga aaacccattt 1740
gagtaaatga tatattaata ttacttcgta aaaagctgcg tccccacata ttctattgat 1800
tttaaatact attcgtatta atataaacct gcatataagc gtacatatac atttctgatt 1860
ctatatatag gtgtgagacc ttcttctcca ttct 1894

<210> 165
<211> 1882
<212> DNA
<213> Arabidopsis thaliana

<220>
<221> promoter
<222> (1)..(1882)
<223> transcription regulating sequence from Arabidopsis thaliana gene At3g50870

<400> 165
tataaacatc aatagaagag taattaatcg ttccatcgga acatccgatg aaattggaac 60
gagacctata cagaagaaaa caaaagagca actaatcatc ttgatcacat gcccaatatg 120
catttaagta aattggagag aatgatatag aaaaacaaga cattatctac aactacaaga 180
tatcaaagca ctcggttaac cgcgtccctg tgaagtgtta gttatcagta catgaattgt 240
gatttcagca tcttggtcct tttttttttg gacaaccgtc atctttttct tgatcggttt 300
ttataacgta caaacatata ctatttcaac agtttctttt taattttttt ttttttgaa 360
tatacacttt ctttctttta tttgtttgat tacaaaattt tcaaaaaata gtgttcggca 420
aaaaaaaaaa ttagatcggc caatgcatta tatcccctag cgtaagctct ttttcaacca 480
atcgagagtc catgttttca ataaaaatag ggtttatta gtaacagtag ttaaagcatt 540
taacatacgt aatcccaaaa aaataaaata attctgtata gggacgaaga ctagtattcg 600
ttgacgacaa gagtagagta gactctagct agctgcaagt tttgaagaaa tctaataacc 660
aaatctaagt cattttactt acatcatcat ggatcatcat taaccacatg aggctgagca 720
gatatcgaac gtgtgattgc gatgtggaga agtaaaggac gaggccacgc gcaactgagc 780
aggagcgcgt gagattcagg gttttagggc agagatacgg aaagagacag aggttttata 840
gcattatatt gaaagagaac aaataaaaat aaaaatatga gcctcgtgat cggagatttt 900
tgcagtgatg actgtactct tcttcctctt ctctccccct tgtcacgtgg gctaaatttt 960
gaccaccctt cttcaatgac catcaacatc gcatcacact ctctccttct tcttcttcta 1020
cgtttttttat tttctgttaa atttcctcca caaaatcgat aatttcaatt ttttcataca 1080
atatcatagt tctagtaaag taaacattcg tgtgtgagtg acataataat tgcttcaaaa 1140
agaaataacg aattaataag agttgtgtgc ttgatggtcc aaaagtcgta tgtttttcac 1200
catcacaata agatttacca tatctactta aaccaaactt caatctatga tatttggaag 1260
ctagcaatga atgattttgt tttagcatat gttaccgagc ctattcatct ttagattgaa 1320
agttttgtgt ccaacaacta gcaaaccctt cgctttttaa accaagatca gtttttctac 1380
atagtttcga ctttttgtta ttttacttcc catcatattt tgttttattt actactgaca 1440

```
ttttcataat gtacggtaca ttttcatata ttagcatcct tatctaaaag ctaatcaact   1500
ccataacgta cttgaaatag ggatatttta gttgaattat tcaaaacaat cacacagtga   1560
cacagactct atgtagacca agcatgtgtt atgtaattag aaaaaataaa ctgtgattaa   1620
aatatctttt aacttgttaa cttggctgcc taaagaaact tgatactgta tcatcgttca   1680
tcgtattttg taattaataa aacataaaaa agcaaggaaa acccatttga gtaaatgata   1740
tattaatatt acttcgtaaa aagctgcgtc cccacatatt ctattgattt taaatactat   1800
tcgtattaat ataaacctgc atataagcgt acatatacat ttctgattct atatataggt   1860
gtgagacctt cttctccatt ct                                            1882

<210>  166
<211>  1249
<212>  DNA
<213>  Arabidopsis thaliana

<220>
<221>  CDS
<222>  (123)..(1010)
<223>  encoding Arabidopsis thaliana zinc finger (GATA type) family
       protein

<400>  166
acacttcact ccaacgtgaa acctgcaaac tgaacttatc tcttcatatt ttctttagct     60
tctcttaatt ttacacttag ggttttcaaa ccagtagaga gagagagaag tgtgtaagag    120
cg atg atg cag act ccg tac act act tca acg cag ggg caa tat tgt      167
   Met Met Gln Thr Pro Tyr Thr Thr Ser Thr Gln Gly Gln Tyr Cys
   1               5                   10                  15
cat tct tgt gga atg ttc cac cac cat agc caa agc tgc tgc tac aac      215
His Ser Cys Gly Met Phe His His His Ser Gln Ser Cys Cys Tyr Asn
                20                  25                  30
aac aac aac aac tcc aac gcc ggt tct tac tcg atg gtc ttc tcc atg      263
Asn Asn Asn Asn Ser Asn Ala Gly Ser Tyr Ser Met Val Phe Ser Met
            35                  40                  45
caa aac ggt ggc gtt ttc gag cag aac ggt gag gac tat cat cac tct      311
Gln Asn Gly Gly Val Phe Glu Gln Asn Gly Glu Asp Tyr His His Ser
        50                  55                  60
tcc tcc ctc gtt gac tgc act ctc tct ctt gga act cct tct acg agg      359
Ser Ser Leu Val Asp Cys Thr Leu Ser Leu Gly Thr Pro Ser Thr Arg
    65                  70                  75
ctt tgt gag gaa gat gag aaa cgt aga cgc tct act tca tct ggt gct      407
Leu Cys Glu Glu Asp Glu Lys Arg Arg Arg Ser Thr Ser Ser Gly Ala
80                  85                  90                  95
tct tct tgc atc tcc aac ttt tgg gac ttg att cac acc aaa aac aac      455
Ser Ser Cys Ile Ser Asn Phe Trp Asp Leu Ile His Thr Lys Asn Asn
                100                 105                 110
aac tcc aaa acg gca ccg tac aat aac gtt cct tct ttc tcc gct aac      503
```

```
Asn Ser Lys Thr Ala Pro Tyr Asn Asn Val Pro Ser Phe Ser Ala Asn
            115                 120                 125
aag cca agt cgc ggt tgt tcc ggt ggt ggt ggt ggc gga gga ggc ggt      551
Lys Pro Ser Arg Gly Cys Ser Gly Gly Gly Gly Gly Gly Gly Gly Gly
        130                 135                 140
ggc gga ggt gac tct ctt ctc gct aga cgc tgt gcc aac tgt gac act      599
Gly Gly Gly Asp Ser Leu Leu Ala Arg Arg Cys Ala Asn Cys Asp Thr
    145                 150                 155
act tct act cca cta tgg agg aat ggt cct aga ggc cct aag tcc cta      647
Thr Ser Thr Pro Leu Trp Arg Asn Gly Pro Arg Gly Pro Lys Ser Leu
160                 165                 170                 175
tgc aac gca tgc ggc att cgt ttc aag aag gaa gag aga aga act act      695
Cys Asn Ala Cys Gly Ile Arg Phe Lys Lys Glu Glu Arg Arg Thr Thr
                180                 185                 190
gcg gct aca gga aac acc gtc gtc gga gct gca ccg gtt caa acc gac      743
Ala Ala Thr Gly Asn Thr Val Val Gly Ala Ala Pro Val Gln Thr Asp
            195                 200                 205
cag tac ggg cat cac aac tct ggc tac aat aat tac cat gct gcc act      791
Gln Tyr Gly His His Asn Ser Gly Tyr Asn Asn Tyr His Ala Ala Thr
        210                 215                 220
aat aac aac aat aat aat ggt act ccg tgg gct cat cac cac tcg acg      839
Asn Asn Asn Asn Asn Asn Gly Thr Pro Trp Ala His His His Ser Thr
    225                 230                 235
cag agg gtt ccg tgt aat tat ccg gca aat gag atc agg ttc atg gat      887
Gln Arg Val Pro Cys Asn Tyr Pro Ala Asn Glu Ile Arg Phe Met Asp
240                 245                 250                 255
gat tac ggc agt gga gta gca aac aac gtt gaa tcc gac ggt gct cac      935
Asp Tyr Gly Ser Gly Val Ala Asn Asn Val Glu Ser Asp Gly Ala His
                260                 265                 270
ggc ggt gtt ccg ttc ctt tct tgg agg ctt aat gta gcg gat agg gca      983
Gly Gly Val Pro Phe Leu Ser Trp Arg Leu Asn Val Ala Asp Arg Ala
            275                 280                 285
agt ctt gtc cat gac ttt acc aga tga aaggagaata gaaaacgacg           1030
Ser Leu Val His Asp Phe Thr Arg
        290                 295
ttagattctc ttaaaatttc catgtggttt aattatgtct aattaaaaaa aaaccaagaa   1090
aatatttctg aaaaaaatag aagtgtatat cgagagacga tgttgatgtt tattgatagt   1150
gacggtctaa cttatggttt atctataaag taccgttctg gtcaattttt aaaatcattt   1210
tctaatattt attttaaaaa tataaccttc tttttatgg                          1249
```

<210> 167
<211> 295
<212> PRT
<213> Arabidopsis thaliana

<400> 167

Met Met Gln Thr Pro Tyr Thr Thr Ser Thr Gln Gly Gln Tyr Cys His
1 5 10 15

Ser Cys Gly Met Phe His His His Ser Gln Ser Cys Cys Tyr Asn Asn
20 25 30

Asn Asn Asn Ser Asn Ala Gly Ser Tyr Ser Met Val Phe Ser Met Gln
35 40 45

Asn Gly Gly Val Phe Glu Gln Asn Gly Glu Asp Tyr His His Ser Ser
50 55 60

Ser Leu Val Asp Cys Thr Leu Ser Leu Gly Thr Pro Ser Thr Arg Leu
65 70 75 80

Cys Glu Glu Asp Glu Lys Arg Arg Arg Ser Thr Ser Ser Gly Ala Ser
85 90 95

Ser Cys Ile Ser Asn Phe Trp Asp Leu Ile His Thr Lys Asn Asn Asn
100 105 110

Ser Lys Thr Ala Pro Tyr Asn Asn Val Pro Ser Phe Ser Ala Asn Lys
115 120 125

Pro Ser Arg Gly Cys Ser Gly Gly Gly Gly Gly Gly Gly Gly Gly Gly
130 135 140

Gly Gly Asp Ser Leu Leu Ala Arg Arg Cys Ala Asn Cys Asp Thr Thr
145 150 155 160

Ser Thr Pro Leu Trp Arg Asn Gly Pro Arg Gly Pro Lys Ser Leu Cys
165 170 175

Asn Ala Cys Gly Ile Arg Phe Lys Lys Glu Glu Arg Arg Thr Thr Ala
180 185 190

Ala Thr Gly Asn Thr Val Val Gly Ala Ala Pro Val Gln Thr Asp Gln
195 200 205

Tyr Gly His His Asn Ser Gly Tyr Asn Asn Tyr His Ala Ala Thr Asn
210 215 220

Asn Asn Asn Asn Asn Gly Thr Pro Trp Ala His His His Ser Thr Gln
225 230 235 240

Arg Val Pro Cys Asn Tyr Pro Ala Asn Glu Ile Arg Phe Met Asp Asp
245 250 255

Tyr Gly Ser Gly Val Ala Asn Asn Val Glu Ser Asp Gly Ala His Gly
260 265 270

Gly Val Pro Phe Leu Ser Trp Arg Leu Asn Val Ala Asp Arg Ala Ser
275 280 285

Leu Val His Asp Phe Thr Arg
290 295

<210> 168
<211> 1300
<212> DNA
<213> Arabidopsis thaliana

<220>
<221> promoter
<222> (1)..(1300)
<223> transcription regulating sequence from Arabidopsis thaliana gene At4g00220

<400> 168
atccgcactg actagactat acattacata tatcttgtaa tttcggaaag ttcaaatatt 60
tgtgaaatat atgcactagt tggagtttcc ttatttgtac cttaatcgaa tattgagtaa 120
acatgattcc ctgacccaaa tatagattgc gtacatcaac ttattaatac cgtcaataaa 180
tacgagtttt tgcatataat cattacatgg tcatatgtaa taaatctagt tgttttggaa 240
attgcatatt ctctccctcc ctctttatca attttacata tatgtagcat cagcaatata 300
tgtgaatttg ttacattagt agtttagttt gtattttaat ttttttggat aaattatgaa 360
ttctattacc aaaattgaac attgtttttt acaaatactt tggggcagtg aatttctaaa 420
accaaaaaaa aaacagttag taatttgtat aggtattatc gccgtggtat acacttaatt 480
caaatatcat aatatacttc gtgtcatata cgacaaaagt aaaattaaag tttgcaaaaa 540
aaattttatt caaagaatct aacggaaaaa aaccgcttaa attttctaga agatattgcc 600
aaatgttgat aaaatatctg attaaccaaa aagaaaataa cgcatagcgg tgagccgtta 660
catttcgact acaaagttat cacattaaaa taccgccaaa aattagtaat taattagatt 720
atatatatat atttaagctc attttataca tttatttgtt tgtaaaatta atttctcata 780
gtattagatt gaatgttcat atattacaat tggtatatac cataagatag ttgtgtggtt 840
cgatgtaaca aaaataaaat taaaccagaa gtagaaaaag tgaaggaaag ggaataacaa 900
tgaagaagtt gaaagatatg gaatgttttg cttcacgcaa agagcttaac acgagacaag 960
tacgctccat cgcgttgctt tgaagtcttt tttttcttct tcttcttctt gtcttaacct 1020
cttttttttt ttttagtctt ggtaataaca agactaatta aattattatt ttactttttt 1080
tattatctac caacaaccaa accctagttt tgcttacgtc cgatctcctc cttctctctt 1140
ttcttctctc ccaaacgttg cctttctttt ttttctctaa tcacgaacca aaaagcatca 1200

taatctcgat gagttaaatc catgtatctc aaaaccctaa tcaccactta gctagtaaaa 1260
ggaaaggaaa ataaaaagga cttgtgtggt agaaaaggac 1300

<210> 169
<211> 1337
<212> DNA
<213> Arabidopsis thaliana

<220>
<221> promoter
<222> (1)..(1337)
<223> transcription regulating sequence from Arabidopsis thaliana gene At4g00220

<400> 169
aatatattgg agatccgcac tgactagact atacattaca tagatcttgt aatttcggaa 60
agttcaaata tttgtgaaat atatgcacta gttggagttt ccttatttgt accttaatcg 120
aatattgagt aaacatgatt ccctgaccca aatatagatt gcgtacatca actcattaat 180
accgtcaata aatacgagtt tttgcatata atcattacat ggtcatatgt aataaatcta 240
gttgttttgg aaattgcata ttctctccct ccctctttat caattttaca tatatgtagc 300
atcagcaata tatgtgaatt tgttacatta gtagtttagt ttgtatttta attttttgga 360
taaattatga attctattac caaaattgaa cattgttttt tacaaatact ttggggcagt 420
gaatttctaa aaccaaaaaa aaaaagttag taatttgtat aggtattatc gccgtggtat 480
acacttaatt caaatatcat aatatacatg ttcgtgtcat atacgacaaa agtaaaatta 540
aagtttgcaa aaaaaatttt atttaaagaa tctaacggaa aaaaaccgct taaattttct 600
agaaaatgtt gccaaatgtt gataaaatat ccgattaacc aaaaagaaaa taacgcatag 660
cggtgaaccg ttacatttcg actacaaagt tatcacatta aaatcaccgc caaaaattag 720
taattaatta gattatatat atatatatat atatatatat atatatattt aagctcattt 780
tatacattta tttgtttgta aaattaattt ctcatagtat tagattgaat gttcatatat 840
tacaattggt atataccata agatagttgt gtggttcgat gtaacaaaaa taaaattaaa 900
ccagaagtag aaaaagtgaa ggaaagggaa taacaatgaa gaagttgaaa gatatggaat 960
gttttgcttc acgcaaagag cttaacacga gacaagtacg ctccatcgcg ttgctttgaa 1020
gtcttttttt tcttcttctt cttcttgtct taacctcttt tttatttttt agtcttggta 1080
ataacaagac taattaaatt attattttac ttttttttatt atctaccaac aaccaaaccc 1140
tagttttgct tacgtccgat ctcctccttc tctcttttct tctctcccaa acgttccctt 1200
tctttttttt ctctaatcac gaaccaaaaa gcatcataat ctcgatgagt taaatccatg 1260
tatctcaaaa ccctaatcac cacttagcta gtaaaaggaa aggaaaataa aaaggacttg 1320
tgtggtagaa aaggaag 1337

<210> 170
<211> 1190
<212> DNA
<213> Arabidopsis thaliana

<220>

<221> promoter
<222> (1)..(1190)
<223> transcription regulating sequence from Arabidopsis thaliana gene At4g00220

<400> 170
```
atccgcactg actagactat acattacata tatcttgtaa tttcggaaag ttcaaatatt     60
tgtgaaatat atgcactagt tggagtttcc ttatttgtac cttaatcgaa tattgagtaa    120
acatgattcc ctgacccaaa tatagattgc gtacatcaac ttattaatac cgtcaataaa    180
tacgagtttt tgcatataat cattacatgg tcatatgtaa taaatctagt tgttttggaa    240
attgcatatt ctctccctcc ctctttatca attttacata tatgtagcat cagcaatata    300
tgtgaatttg ttacattagt agtttagttt gtattttaat ttttttggat aaattatgaa    360
ttctattacc aaaattgaac attgtttttt acaaatactt tggggcagtg aatttctaaa    420
accaaaaaaa aaacagttag taatttgtat aggtattatc gccgtggtat acacttaatt    480
caaatatcat aatatacttc gtgtcatata cgacaaaagt aaaattaaag tttgcaaaaa    540
aaattttatt caaagaatct aacggaaaaa aaccgcttaa attttctaga agatattgcc    600
aaatgttgat aaaatatctg attaaccaaa aagaaaataa cgcatagcgg tgagccgtta    660
catttcgact acaaagttat cacattaaaa taccgccaaa aattagtaat taattagatt    720
atatatatat atttaagctc attttataca tttatttgtt tgtaaaatta atttctcata    780
gtattagatt gaatgttcat atattacaat tggtatatac cataagatag ttgtgtggtt    840
cgatgtaaca aaaataaaat taaaccagaa gtagaaaaag tgaaggaaag ggaataacaa    900
tgaagaagtt gaaagatatg gaatgttttg cttcacgcaa agagcttaac acgagacaag    960
tacgctccat cgcgttgctt tgaagtcttt tttttcttct tcttcttctt gtcttaacct   1020
cttttttttt ttttagtctt ggtaataaca agactaatta aattattatt ttactttttt   1080
tattatctac caacaaccaa accctagttt tgcttacgtc cgatctcctc cttctctctt   1140
ttcttctctc ccaaacgttg cctttctttt ttttctctaa tcacgaacca               1190
```

<210> 171
<211> 1226
<212> DNA
<213> Arabidopsis thaliana

<220>
<221> promoter
<222> (1)..(1226)
<223> transcription regulating sequence from Arabidopsis thaliana gene At4g00220

<400> 171
```
aatatattgg agatccgcac tgactagact atacattaca tagatcttgt aatttcggaa     60
agttcaaata tttgtgaaat atatgcacta gttggagttt ccttatttgt accttaatcg    120
aatattgagt aaacatgatt ccctgaccca aatatagatt gcgtacatca actcattaat    180
accgtcaata aatacgagtt tttgcatata atcattacat ggtcatatgt aataaatcta    240
gttgttttgg aaattgcata ttctctccct ccctctttat caattttaca tatatgtagc    300
atcagcaata tatgtgaatt tgttacatta gtagtttagt ttgtatttta atttttttgga    360
```

```
taaattatga attctattac caaaattgaa cattgttttt tacaaatact ttggggcagt     420
gaatttctaa aaccaaaaaa aaaaagttag taatttgtat aggtattatc gccgtggtat     480
acacttaatt caaatatcat aatatacatg ttcgtgtcat atacgacaaa agtaaaatta     540
aagtttgcaa aaaaaatttt atttaaagaa tctaacggaa aaaaaccgct taaattttct     600
agaaaatgtt gccaaatgtt gataaaatat ccgattaacc aaaaagaaaa taacgcatag     660
cggtgaaccg ttacatttcg actacaaagt tatcacatta aaatcaccgc caaaaattag     720
taattaatta gattatatat atatatatat atatatatat atatatattt aagctcattt     780
tatacattta tttgtttgta aaattaattt ctcatagtat tagattgaat gttcatatat     840
tacaattggt atataccata agatagttgt gtggttcgat gtaacaaaaa taaaattaaa     900
ccagaagtag aaaaagtgaa ggaaagggaa taacaatgaa gaagttgaaa gatatggaat     960
gttttgcttc acgcaaagag cttaacacga gacaagtacg ctccatcgcg ttgctttgaa    1020
gtcttttttt tcttcttctt cttcttgtct taacctcttt tttatttttt agtcttggta    1080
ataacaagac taattaaatt attattttac ttttttttatt atctaccaac aaccaaaccc    1140
tagttttgct tacgtccgat ctcctccttc tctcttttct tctctcccaa acgttccctt    1200
tctttttttt ctctaatcac gaacca                                         1226
```

<210> 172
<211> 3232
<212> DNA
<213> Arabidopsis thaliana

<220>
<221> promoter
<222> (1)..(3232)
<223> transcription regulating sequence from Arabidopsis thaliana gene At4g00220

<400> 172

```
ttttatgtca aagacgtaac gttttgtttg ggggaagttg tgtcgtcgtc gaccacagtt      60
attatatatc caatccaaag agatgtttaa gagagagaga tggatgggtt tgtttatgaa     120
taaatgtttt taagttgatg tggagcatag aggatcgtgt caggcaagaa aatcatgtct     180
tgcttttggt ggtgggtcat ttataatcat tacatttttc tataacttga tcatcttttc     240
ttttgtatgt ccatattttt gttttttgtt ttccttggtg tgtttcactt ttctgtacat     300
agttgaatca cacactcaag aaacaagcta ttttcaaaca agtgaaagaa ttatctattt     360
atggaatcct aaaatcaatg aaagcatata acacaaggag ggataacata tacttgtaaa     420
ttgaaaagta gacaaatcaa caatatatat catctaaatt agcaatcata ggtttaagac     480
ataaaaagat tttgtatatg ccactgagaa tctagaacta actatcagtc tatcacacaa     540
gcaaggccta gaaagtagtg aacaagtaag gatttagaat tttagatagt gagtaatgaa     600
gatatatata ggctggtggg ttcagttaac aaggtactaa aatcggatta taacgaggga     660
tcaagtggtg gggattatga attattatag taatgagaat gaatgtacta gtactaatta     720
gaatgagtaa agcttggaag caacggcaga gcagatttgt gatatataca aaggacaaac     780
aaacacaaat gctttgcgtg aagcaaaaca caattagctg cttgctgacg tatttctctc     840
tcaccactca gccgcaggtt tccctgtcac acacacgtgc gatccctcac cacacgtgtg     900
taattctttt atatatgtta tatactgtat ataatttttt gtcaaactag ttctctatgg     960
acagctttcg tgatagaatt atagagagaa aatcgagatg tatgttgctt cttgtttcta    1020
```

```
caaatcgtat agtcagaaac agacatgaca ctgatcactg tttgcttccc ccgcaagtac    1080
aacctcagtc agtcagttac tgtcatgtct ctctttctct ctgtttgaca agatccctga    1140
cccttgttca cttccctaat ttaaagcctt ctccttaatg ggccgggcct tgtatctgtt    1200
tcggatcctt ctttatagcg aaatgattaa tagtagtgat tacacctaaa atgtacgtat    1260
atgacaaaaa gaatagtttt tttcttctgt ttctcttgta ctattaggcc tagtgaatgt    1320
attaaataca aacagtgtac attagcgtac gcaagcggtg aggaaaggag cagaagaggg    1380
cgtgatacga ggacacgtgt ctcatgttag tacaaacaaa gattggacaa gagccggacg    1440
gcacggtgac ctctgtcaac aactgttacc gtttcttcta atctatttgt taatctccta    1500
agtaatgtaa ttaattagtt ttcactgttc ataattaatc aagtaataat aacataagaa    1560
atgaaatggg agcgttactt gtataccata tgcactcgca aacgagccat tggttgcgtc    1620
agaacggtta cgttcttcat aaaagtcaca ttcgacacat ggcgatcata tatacgtgcc    1680
aatttttccg gttctctcaa atccaatccc ggtttctttc ttttatgttt tgatcaccgg    1740
ttcttcttct aaaccaactt agctttactc atttggtgat tattattgga ccgtcgtaat    1800
cgtcactctt ttcttttttg aatcaatgat gtgagatatg tatattatac acgcacacgt    1860
acacacgtgt atttgatcat tcttgccgtc accaaatttt aaactcattt atcgtgtaat    1920
aatatattgg agatccgcac tgactagact atacattaca tatatcttgt aatttcggaa    1980
agttcaaata tttgtgaaat atatgcacta gttggagttt ccttatttgt accttaatcg    2040
aatattgagt aaacatgatt ccctgaccca aatatagatt gcgtacatca acttattaat    2100
accgtcaata aatacgagtt tttgcatata atcattacat ggtcatatgt aataaatcta    2160
gttgttttgg aaattgcata ttctctccct ccctctttat caattttaca tatatgtagc    2220
atcagcaata tatgtgaatt tgttacatta gtagtttagt ttgtatttta atttttttgg    2280
ataaattatg aattctatta ccaaaattga acattgtttt ttacaaatac tttggggcag    2340
tgaatttcta aaaccaaaaa aaaaacagtt agtaatttgt ataggtatta tcgccgtggt    2400
atacacttaa ttcaaatatc ataatatact tcgtgtcata tacgacaaaa gtaaaattaa    2460
agtttgcaaa aaaaatttta ttcaaagaat ctaacggaaa aaaaccgctt aaattttcta    2520
gaagatattg ccaaatgttg ataaaatatc tgattaacca aaaagaaaat aacgcatagc    2580
ggtgagccgt tacatttcga ctacaaagtt atcacattaa aatcaccgcc aaaaattagt    2640
aattaattag attatatata tatatttaag ctcattttat acatttattt gtttgtaaaa    2700
ttaatttctc atagtattag attgaatgtt catatattac aattggtata taccataaga    2760
tagttgtgtg gttcgatgta acaaaaataa aattaaacca gaagtagaaa aagtgaagga    2820
aagggaataa caatgaagaa gttgaaagat atggaatgtt ttgcttcacg caaagagctt    2880
aacacgagac aagtacgctc catcgcgttg ctttgaagtc ttttttttct tcttcttctt    2940
cttgtcttaa cctctttttt tttttttagt cttggtaata acaagactaa ttaaattatt    3000
atttactttt ttttattatc taccaacaac caaaccctag ttttgcttac gtccgatctc    3060
ctccttctct cttttcttct ctcccaaacg ttgcctttct tttttttctc taatcacgaa    3120
ccaaaaagca tcataatctc gatgagttaa atccatgtat ctcaaaaccc taatcaccac    3180
ttagctagta aaaggaaagg aaaataaaaa ggacttgtgt ggtagaaaag ga            3232
```

<210> 173
<211> 3269
<212> DNA
<213> Arabidopsis thaliana

<220>
<221> promoter

<222> (1)..(3269)
<223> transcription regulating sequence from Arabidopsis thaliana gene At4g00220

<400> 173

```
cttccgctca tcttttatgt caaagacgta acgttttgtt tgggggaagt tgtgtcgtcg     60
tcgaccacag ttattatata tccaatccaa agagatgttt aagagagaga gatggatggg    120
tttgtttatg aataaatgtt tttaagttga tgtggagcat agaggatcgt gtcaggcaag    180
aaaatcatgt cttgcttttg gtggtgggtc atttataatc attacatttt tctataactt    240
gatcatcttt tcttttgtat gtccatattt ttgtttttg ttttccttgg tgtgtttcac    300
ttttctgtac atagttgaat cacacactca agaaacaagc tattttcaaa caagtgaaag    360
aattatctat ttatggactc ctaaaatcaa tgaaagcata taacacaagg agggataaca    420
tatacttgta aattgaaaag tagacaaatc aacaatatat atcatctaaa ttagcaatca    480
taggtttaag acataaaaag attttgtata tgccactgag aatctagaac taactatcag    540
tctatcacac aagcaaggcc tagaaagtag tgaacaagta aggatttaga attttagata    600
gtgagtaatg aagatatata taggctggtg ggttcagtta acaaggtact aaaatcggat    660
tataacgagg gatcaagtgg tggggattat gaattattat agtaatgaga atgaatgtac    720
tagtactaat tagaatgagt aaagcttgga agcaacggca gagcagattt gtgatatata    780
caaaggacaa acaaacacaa atgctttgcg tgaagcaaaa cacaattagc tgcttgctga    840
cgtatttctc tctcaccact cagccgcagg tttccctgtc acacacacgt gcgatccctc    900
accacacgtg tgtaattctt ttatatatgt tatatactgt atataatttt ttgtcaaact    960
agttctctat ggacagcttt cgtgatagaa ttatagagag aaaatcgaga tgtatgttgc   1020
ttcttgtttc tacaaatcgt atagtcagaa acagacatga cactgatcac tgtttgcttc   1080
ccccgcaagt acaacctcag tcagtcagtt actgtcatgt ctctctttct ctctgtttga   1140
caagatccct gacccttgtt cacttcccta atttaaagcc ttctccttaa tgggccgggc   1200
cttgtatctg tttcggatcc ttctttatag cgaaatgatt aatagtagtg attacaccta   1260
aaatgtacgt atatgacaaa aagaatagtt tttttcttct gtttctcttg tactattagg   1320
cctagtgaat gtattaaata caaacagtgt acattagcgt acgcaagcgg tgaggaaagg   1380
agcagaagag ggcgtgatac gaggacacgt gtctcatgtt agtacaaaca aagattggac   1440
aagagccgga cggcacggtg acctctgtca acaactgtta ccgtttcttc taatctattt   1500
gttaatctcc taagtaatgt aattaattag ttttcactgt tcataattaa tcaagtaata   1560
ataacataag aaatgaaatg ggagcgttac ttgtatacca tatgcactcg caaacgagcc   1620
attggttgcg tcagaacggt tacgttcttc ataaaagtca cattcgacac atggcgatca   1680
tatatacgtg ccaatttttc cggttctctc aaatccaatc ccggtttctt tcttttatgt   1740
tttgatcacc ggttcttctt ctaaaccaac ttagctttac tcatttggtg attattattg   1800
gaccgtcgta atcgtcactc ttttcttttt tgaatcaatg atgtgagata tgtatattat   1860
acacgcacac gtacacacgt gtatttgatc attcttgccg tcaccaaatt ttaaactcat   1920
ttatcgtgta ataatatatt ggagatccgc actgactaga ctatacatta catagatctt   1980
gtaatttcgg aaagttcaaa tatttgtgaa atatatgcac tagttggagt ttccttattt   2040
gtaccttaat cgaatattga gtaaacatga ttccctgacc caaatataga ttgcgtacat   2100
caactcatta ataccgtcaa taaatacgag tttttgcata taatcattac atggtcatat   2160
gtaataaatc tagttgtttt ggaaattgca tattctctcc ctccctcttt atcaatttta   2220
catatatgta gcatcagcaa tatatgtgaa tttgttacat tagtagttta gtttgtattt   2280
taatttttg gataaattat gaattctatt accaaaattg aacattgttt tttacaaata   2340
ctttggggca gtgaatttct aaaaccaaaa aaaaaaagtt agtaatttgt ataggtatta   2400
```

tcgccgtggt atacacttaa ttcaaatatc ataatataca tgttcgtgtc atatacgaca 2460
aaagtaaaat taaagtttgc aaaaaaaatt ttatttaaag aatctaacgg aaaaaaaccg 2520
cttaaatttt ctagaaaatg ttgccaaatg ttgataaaat atccgattaa ccaaaaagaa 2580
aataacgcat agcggtgaac cgttacattt cgactacaaa gttatcacat taaaatcacc 2640
gccaaaaatt agtaattaat tagattatat atatatatat atatatatat atatatatat 2700
ttaagctcat tttatacatt tatttgtttg taaaattaat ttctcatagt attagattga 2760
atgttcatat attacaattg gtatatacca taagatagtt gtgtggttcg atgtaacaaa 2820
aataaaatta aaccagaagt agaaaaagtg aaggaaaggg aataacaatg aagaagttga 2880
aagatatgga atgttttgct tcacgcaaag agcttaacac gagacaagta cgctccatcg 2940
cgttgctttg aagtcttttt tttcttcttc ttcttcttgt cttaacctct tttttatttt 3000
ttagtcttgg taataacaag actaattaaa ttattatttt actttttttta ttatctacca 3060
acaaccaaac cctagttttg cttacgtccg atctcctcct tctctctttt cttctctccc 3120
aaacgttccc tttctttttt ttctctaatc acgaaccaaa aagcatcata atctcgatga 3180
gttaaatcca tgtatctcaa aaccctaatc accacttagc tagtaaaagg aaaggaaaat 3240
aaaaaggact tgtgtggtag aaaaggaag 3269

<210> 174
<211> 3123
<212> DNA
<213> Arabidopsis thaliana

<220>
<221> promoter
<222> (1)..(3123)
<223> transcription regulating sequence from Arabidopsis thaliana gene At4g00220

<400> 174
ttttatgtca aagacgtaac gttttgtttg ggggaagttg tgtcgtcgtc gaccacagtt 60
attatatatc caatccaaag agatgtttaa gagagagaga tggatgggtt tgtttatgaa 120
taaatgtttt taagttgatg tggagcatag aggatcgtgt caggcaagaa aatcatgtct 180
tgcttttggt ggtgggtcat ttataatcat tacatttttc tataacttga tcatcttttc 240
ttttgtatgt ccatattttt gtttttttgtt ttccttggtg tgtttcactt ttctgtacat 300
agttgaatca cacactcaag aaacaagcta ttttcaaaca agtgaaagaa ttatctattt 360
atggaatcct aaaatcaatg aaagcatata acacaaggag ggataacata tacttgtaaa 420
ttgaaaagta gacaaatcaa caatatatat catctaaatt agcaatcata ggtttaagac 480
ataaaaagat tttgtatatg ccactgagaa tctagaacta actatcagtc tatcacacaa 540
gcaaggccta gaaagtagtg aacaagtaag gatttagaat tttagatagt gagtaatgaa 600
gatatatata ggctggtggg ttcagttaac aaggtactaa aatcggatta taacgaggga 660
tcaagtggtg gggattatga attattatag taatgagaat gaatgtacta gtactaatta 720
gaatgagtaa agcttggaag caacggcaga gcagatttgt gatatataca aaggacaaac 780
aaacacaaat gctttgcgtg aagcaaaaca caattagctg cttgctgacg tatttctctc 840
tcaccactca gccgcaggtt tccctgtcac acacacgtgc gatccctcac cacacgtgtg 900
taattctttt atatatgtta tatactgtat ataatttttt gtcaaactag ttctctatgg 960
acagctttcg tgatagaatt atagagagaa aatcgagatg tatgttgctt cttgtttcta 1020

caaatcgtat agtcagaaac agacatgaca ctgatcactg tttgcttccc ccgcaagtac 1080
aacctcagtc agtcagttac tgtcatgtct ctctttctct ctgtttgaca agatccctga 1140
cccttgttca cttccctaat ttaaagcctt ctccttaatg ggccgggcct tgtatctgtt 1200
tcggatcctt ctttatagcg aaatgattaa tagtagtgat tacacctaaa atgtacgtat 1260
atgacaaaaa gaatagtttt tttcttctgt ttctcttgta ctattaggcc tagtgaatgt 1320
attaaataca aacagtgtac attagcgtac gcaagcggtg aggaaaggag cagaagaggg 1380
cgtgatacga ggacacgtgt ctcatgttag tacaaacaaa gattggacaa gagccggacg 1440
gcacggtgac ctctgtcaac aactgttacc gtttcttcta atctatttgt taatctccta 1500
agtaatgtaa ttaattagtt ttcactgttc ataattaatc aagtaataat aacataagaa 1560
atgaaatggg agcgttactt gtataccata tgcactcgca aacgagccat tggttgcgtc 1620
agaacggtta cgttcttcat aaaagtcaca ttcgacacat ggcgatcata tatacgtgcc 1680
aatttttccg gttctctcaa atccaatccc ggtttctttc ttttatgttt tgatcaccgg 1740
ttcttcttct aaaccaactt agctttactc atttggtgat tattattgga ccgtcgtaat 1800
cgtcactctt ttcttttttg aatcaatgat gtgagatatg tatattatac acgcacacgt 1860
acacacgtgt atttgatcat tcttgccgtc accaaatttt aaactcattt atcgtgtaat 1920
aatatattgg agatccgcac tgactagact atacattaca tatatcttgt aatttcggaa 1980
agttcaaata tttgtgaaat atatgcacta gttggagttt ccttatttgt accttaatcg 2040
aatattgagt aaacatgatt ccctgaccca aatatagatt gcgtacatca acttattaat 2100
accgtcaata aatacgagtt tttgcatata atcattacat ggtcatatgt aataaatcta 2160
gttgttttgg aaattgcata ttctctccct ccctctttat caattttaca tatatgtagc 2220
atcagcaata tatgtgaatt tgttacatta gtagtttagt ttgtatttta attttttttgg 2280
ataaattatg aattctatta ccaaaattga acattgtttt ttacaaatac tttggggcag 2340
tgaatttcta aaaccaaaaa aaaaacagtt agtaatttgt ataggtatta tcgccgtggt 2400
atacacttaa ttcaaatatc ataatatact tcgtgtcata tacgacaaaa gtaaaattaa 2460
agtttgcaaa aaaaatttta ttcaaagaat ctaacggaaa aaaaccgctt aaattttcta 2520
gaagatattg ccaaatgttg ataaaatatc tgattaacca aaaagaaaat aacgcatagc 2580
ggtgagccgt tacatttcga ctacaaagtt atcacattaa aatcaccgcc aaaaattagt 2640
aattaattag attatatata tatatttaag ctcattttat acatttattt gtttgtaaaa 2700
ttaatttctc atagtattag attgaatgtt catatattac aattggtata taccataaga 2760
tagttgtgtg gttcgatgta acaaaaataa aattaaacca gaagtagaaa aagtgaagga 2820
aagggaataa caatgaagaa gttgaaagat atggaatgtt ttgcttcacg caaagagctt 2880
aacacgagac aagtacgctc catcgcgttg ctttgaagtc tttttttttct tcttcttctt 2940
cttgtcttaa cctctttttt tttttttagt cttggtaata acaagactaa ttaaattatt 3000
attttacttt ttttattatc taccaacaac caaaccctag ttttgcttac gtccgatctc 3060
ctccttctct cttttcttct ctcccaaacg ttgcctttct ttttttttctc taatcacgaa 3120
cca 3123

<210> 175
<211> 3158
<212> DNA
<213> Arabidopsis thaliana

<220>
<221> promoter
<222> (1)..(3158)

<223> transcription regulating sequence from Arabidopsis thaliana gene At4g00220

<400> 175

```
cttccgctca tcttttatgt caaagacgta acgttttgtt tgggggaagt tgtgtcgtcg     60
tcgaccacag ttattatata tccaatccaa agagatgttt aagagagaga gatggatggg    120
tttgtttatg aataaatgtt tttaagttga tgtggagcat agaggatcgt gtcaggcaag    180
aaaatcatgt cttgcttttg gtggtgggtc atttataatc attacatttt tctataactt    240
gatcatcttt tcttttgtat gtccatattt ttgtttttttg ttttccttgg tgtgtttcac    300
ttttctgtac atagttgaat cacacactca agaaacaagc tattttcaaa caagtgaaag    360
aattatctat ttatggactc ctaaaatcaa tgaaagcata taacacaagg agggataaca    420
tatacttgta aattgaaaag tagacaaatc aacaatatat atcatctaaa ttagcaatca    480
taggtttaag acataaaaag atttgtata tgccactgag aatctagaac taactatcag    540
tctatcacac aagcaaggcc tagaaagtag tgaacaagta aggatttaga attttagata    600
gtgagtaatg aagatatata taggctggtg ggttcagtta acaaggtact aaaatcggat    660
tataacgagg gatcaagtgg tggggattat gaattattat agtaatgaga atgaatgtac    720
tagtactaat tagaatgagt aaagcttgga agcaacggca gagcagattt gtgatatata    780
caaaggacaa acaaacacaa atgctttgcg tgaagcaaaa cacaattagc tgcttgctga    840
cgtatttctc tctcaccact cagccgcagg tttccctgtc acacacacgt gcgatccctc    900
accacacgtg tgtaattctt ttatatatgt tatatactgt atataatttt ttgtcaaact    960
agttctctat ggacagcttt cgtgatagaa ttatagagag aaaatcgaga tgtatgttgc   1020
ttcttgtttc tacaaatcgt atagtcagaa acagacatga cactgatcac tgtttgcttc   1080
ccccgcaagt acaacctcag tcagtcagtt actgtcatgt ctctctttct ctctgtttga   1140
caagatccct gacccttgtt cacttcccta atttaaagcc ttctccttaa tgggccgggc   1200
cttgtatctg tttcggatcc ttctttatag cgaaatgatt aatagtagtg attacaccta   1260
aaatgtacgt atatgacaaa aagaatagtt tttttcttct gtttctcttg tactattagg   1320
cctagtgaat gtattaaata caaacagtgt acattagcgt acgcaagcgg tgaggaaagg   1380
agcagaagag ggcgtgatac gaggacacgt gtctcatgtt agtacaaaca aagattggac   1440
aagagccgga cggcacggtg acctctgtca acaactgtta ccgtttcttc taatctattt   1500
gttaatctcc taagtaatgt aattaattag ttttcactgt tcataattaa tcaagtaata   1560
ataacataag aaatgaaatg ggagcgttac ttgtatacca tatgcactcg caaacgagcc   1620
attggttgcg tcagaacggt tacgttcttc ataaaagtca cattcgacac atggcgatca   1680
tatatacgtg ccaatttttc cggttctctc aaatccaatc ccggtttctt tcttttatgt   1740
tttgatcacc ggttcttctt ctaaaccaac ttagctttac tcatttggtg attattattg   1800
gaccgtcgta atcgtcactc ttttcttttt tgaatcaatg atgtgagata tgtatattat   1860
acacgcacac gtacacacgt gtatttgatc attcttgccg tcaccaaatt ttaaactcat   1920
ttatcgtgta ataatatatt ggagatccgc actgactaga ctatacatta catagatctt   1980
gtaatttcgg aaagttcaaa tatttgtgaa atatatgcac tagttggagt ttccttattt   2040
gtaccttaat cgaatattga gtaaacatga ttccctgacc caaatataga ttgcgtacat   2100
caactcatta ataccgtcaa taaatacgag tttttgcata taatcattac atggtcatat   2160
gtaataaatc tagttgtttt ggaaattgca tattctctcc ctccctcttt atcaatttta   2220
catatatgta gcatcagcaa tatatgtgaa tttgttacat tagtagttta gtttgtattt   2280
taattttttg gataaattat gaattctatt accaaaattg aacattgttt tttacaaata   2340
ctttggggca gtgaatttct aaaaccaaaa aaaaaaagtt agtaatttgt ataggtatta   2400
tcgccgtggt atacacttaa ttcaaatatc ataatataca tgttcgtgtc atatacgaca   2460
```

```
aaagtaaaat taaagtttgc aaaaaaaatt ttatttaaag aatctaacgg aaaaaaaccg    2520
cttaaatttt ctagaaaatg ttgccaaatg ttgataaaat atccgattaa ccaaaaagaa    2580
aataacgcat agcggtgaac cgttacattt cgactacaaa gttatcacat taaaatcacc    2640
gccaaaaatt agtaattaat tagattatat atatatatat atatatatat atatatatat    2700
ttaagctcat tttatacatt tatttgtttg taaaattaat ttctcatagt attagattga    2760
atgttcatat attacaattg gtatatacca taagatagtt gtgtggttcg atgtaacaaa    2820
aataaaatta aaccagaagt agaaaaagtg aaggaaaggg aataacaatg aagaagttga    2880
aagatatgga atgttttgct tcacgcaaag agcttaacac gagacaagta cgctccatcg    2940
cgttgctttg aagtcttttt tttcttcttc ttcttcttgt cttaacctct tttttatttt    3000
ttagtcttgg taataacaag actaattaaa ttattatttt actttttta ttatctacca     3060
acaaccaaac cctagttttg cttacgtccg atctcctcct tctctctttt cttctctccc    3120
aaacgttccc tttctttttt ttctctaatc acgaacca                            3158
```

<210> 176
<211> 948
<212> DNA
<213> Arabidopsis thaliana

<220>
<221> CDS
<222> (112)..(798)
<223> encoding Arabidopsis thaliana LOB domain protein 30 / lateral organ boundaries domain protein 30 (LBD30)

<400> 176

```
aaaagcatca taatctcgat gagttaaatc catgtatctc aaaaccctaa tcaccactta      60
gctagtaaaa ggaaaggaaa ataaaaagga cttgtgtggt agaaaaggaa g atg agc      117
                                                         Met Ser
                                                         1

agt agc gga aac cct agc agc agc agc ggt gga gga gga gga ccg tgc        165
Ser Ser Gly Asn Pro Ser Ser Ser Ser Gly Gly Gly Gly Gly Pro Cys
        5                   10                  15

ggc gcg tgc aag ttc ttg aga agg aag tgt gtc gct ggc tgc atc ttc        213
Gly Ala Cys Lys Phe Leu Arg Arg Lys Cys Val Ala Gly Cys Ile Phe
    20                  25                  30

gct cct tac ttt gac tcc gag caa gga gcg gcg cac ttc gcg gcg gtt        261
Ala Pro Tyr Phe Asp Ser Glu Gln Gly Ala Ala His Phe Ala Ala Val
35                  40                  45                  50

cac aag gtg ttc gga gcg agc aac gtc tcc aaa ctc ctc cac cat gtt        309
His Lys Val Phe Gly Ala Ser Asn Val Ser Lys Leu Leu His His Val
                55                  60                  65

ccc gag cat aaa cga cca gac gcc gtc gtt tca atc tgc ttt gag gct        357
Pro Glu His Lys Arg Pro Asp Ala Val Val Ser Ile Cys Phe Glu Ala
            70                  75                  80

caa gct cgt ctc aga gac cct atc tac ggc tgc gtc tct cac atc gtc        405
```

```
Gln Ala Arg Leu Arg Asp Pro Ile Tyr Gly Cys Val Ser His Ile Val
        85                  90                  95
tct ctt cag caa cag gtg gta agt ttg caa act gag ctt tca tat cta      453
Ser Leu Gln Gln Gln Val Val Ser Leu Gln Thr Glu Leu Ser Tyr Leu
    100                 105                 110
caa gca cac tta gca act cta gag ctg cca caa cct caa cca cca cag      501
Gln Ala His Leu Ala Thr Leu Glu Leu Pro Gln Pro Gln Pro Pro Gln
115                 120                 125                 130
gtt ccg gtg tca tct tca gga tct cta cag gct ctt tcc ata acg gac      549
Val Pro Val Ser Ser Ser Gly Ser Leu Gln Ala Leu Ser Ile Thr Asp
                135                 140                 145
ctc cca aca ata tca ccg tcg gtg tac gac ctc tcc tcc atc ttc gag      597
Leu Pro Thr Ile Ser Pro Ser Val Tyr Asp Leu Ser Ser Ile Phe Glu
            150                 155                 160
ccg gtc atg tcc tcc act tgg gcc atg cag caa caa cca cga ccg tct      645
Pro Val Met Ser Ser Thr Trp Ala Met Gln Gln Gln Pro Arg Pro Ser
        165                 170                 175
gat cat ctc ttc ggc gtc ccg tca tcg tcc aat atg ggc gga ggc ggt      693
Asp His Leu Phe Gly Val Pro Ser Ser Ser Asn Met Gly Gly Gly Gly
    180                 185                 190
gag ctc caa gca ctg gcg cgt gag ttt att cac ggt ggg caa atg cca      741
Glu Leu Gln Ala Leu Ala Arg Glu Phe Ile His Gly Gly Gln Met Pro
195                 200                 205                 210
gct cag cca tcg ccg gga acc agt ggt tct gcc tcg tca gtg ata aaa      789
Ala Gln Pro Ser Pro Gly Thr Ser Gly Ser Ala Ser Ser Val Ile Lys
                215                 220                 225
cga gaa tga agttattttt atcgtatgtt tgtttatttg gaccgtaaga             838
Arg Glu

gatctatagt tagccatatg tttctccttg taatatatat acttggtcga attcgtattt    898
aaattgttgt ctagaatgtt aatataagaa ctatatgatt tacattgatt               948

<210>  177
<211>  228
<212>  PRT
<213>  Arabidopsis thaliana

<400>  177
Met Ser Ser Ser Gly Asn Pro Ser Ser Ser Ser Gly Gly Gly Gly Gly
1               5                   10                  15

Pro Cys Gly Ala Cys Lys Phe Leu Arg Arg Lys Cys Val Ala Gly Cys
            20                  25                  30

Ile Phe Ala Pro Tyr Phe Asp Ser Glu Gln Gly Ala Ala His Phe Ala
```

```
        35                  40                  45

Ala Val His Lys Val Phe Gly Ala Ser Asn Val Ser Lys Leu Leu His
    50                  55                  60

His Val Pro Glu His Lys Arg Pro Asp Ala Val Val Ser Ile Cys Phe
65                  70                  75                  80

Glu Ala Gln Ala Arg Leu Arg Asp Pro Ile Tyr Gly Cys Val Ser His
                85                  90                  95

Ile Val Ser Leu Gln Gln Gln Val Val Ser Leu Gln Thr Glu Leu Ser
            100                 105                 110

Tyr Leu Gln Ala His Leu Ala Thr Leu Glu Leu Pro Gln Pro Gln Pro
        115                 120                 125

Pro Gln Val Pro Val Ser Ser Ser Gly Ser Leu Gln Ala Leu Ser Ile
    130                 135                 140

Thr Asp Leu Pro Thr Ile Ser Pro Ser Val Tyr Asp Leu Ser Ser Ile
145                 150                 155                 160

Phe Glu Pro Val Met Ser Ser Thr Trp Ala Met Gln Gln Gln Pro Arg
                165                 170                 175

Pro Ser Asp His Leu Phe Gly Val Pro Ser Ser Ser Asn Met Gly Gly
            180                 185                 190

Gly Gly Glu Leu Gln Ala Leu Ala Arg Glu Phe Ile His Gly Gly Gln
        195                 200                 205

Met Pro Ala Gln Pro Ser Pro Gly Thr Ser Gly Ser Ala Ser Ser Val
    210                 215                 220

Ile Lys Arg Glu
225

<210>  178
<211>  1504
<212>  DNA
<213>  Arabidopsis thaliana

<220>
<221>  promoter
```

<222> (1)..(1504)
<223> transcription regulating sequence from Arabidopsis thaliana gene At4g26320

<400> 178
tcattgtaaa gtttttcatt ttttttcctt ttgtaaaatt cctctgtttt ctctctgttc 60
ttcatgtagt tcttgcgatt tgccgaaaat tgaatctcgt ttcttgatta cagtttgtat 120
ccatgtgaat atgtctagat tttttttttt tttttttgaa gtatagtcat tgatgattag 180
accatcattt cggttttacc tcgagatgtt taagacaaat gttgctgtct tagatttgtt 240
ttgtgtccgt tcataacttt tggggaaaaa aatgagtaaa gatcaaaagg tttttgagtt 300
aatcatccgg tttaggtggt attgaaagta gctacctcgg tattggtgtt aaaatgtctg 360
tactccttga gctctctgcg agagacagat tttgtttaat gatcaatcaa acatttatca 420
aatctctgag cttagtatat cttcattttt catttaatag caagtctcaa tcgttatggc 480
tttgtatcca gatataatca actctaagat caaggacggt gtttgttttt aagctaactt 540
tcttgggtct ctaggtttgg tgtggtgagt ttttggtttc ttgtcttcca agaaaaaaag 600
gctagtgtaa atgggagagt ttcacggttc ttcatttctt tatttctttt ggtttgaatg 660
aaagtcttgt atggccagta ctgtgtatgg caataggcaa taacatgagc ttgtacctgc 720
taatcagaga aatataatgg aactaagttt gatagcgtga cgaaactatt tataattaaa 780
ttgtagtact tcgtagttct aaaacttgaa gaataacaga taaatttcaa aatgtagaat 840
acaaaaaatg ataaatactt ttgttagaaa atacctaatt catctttatg gagactcacg 900
tttataagtt cttgtttcat cgaatattga gcaataattg ggggagaaat ggaccgagcc 960
aattatatac cagagagcac atggaaccat caatcaaatt ccaaacagct cattgcttct 1020
cctatctatt ttctattcat ttcacagaaa aaaacaaaca gtttcctatt tctttcactc 1080
ttattatcag agagaaaacg ttcctactat gaactgtatt agttaaaaaa tacaaagaac 1140
taaaacatat tatattcatt tctacaataa aaaacaaaag gcgataacat aactaaaatc 1200
agctgcagta ctctttccaa ctgtttattt ttttcttcct ttttcaacta agatctcagt 1260
tggctttaca gagattgatg tgctaatata gttttttttc ctcaccaaat acaaagaatt 1320
aaaacaattc gaaaatgatt aactgtgttg atgttcatca aactgtaaag tgataattac 1380
acagttggcc ttacatgcat ctatgtgtat atataaacca caagtcacaa cacatcagaa 1440
cacaccacaa aacactaaac atataatctc ttcgtgcttt ctcaagaacc acctagagat 1500
aacc 1504

<210> 179
<211> 1492
<212> DNA
<213> Arabidopsis thaliana

<220>
<221> promoter
<222> (1)..(1492)
<223> transcription regulating sequence from Arabidopsis thaliana gene At4g26320

<400> 179
ataatgttat cttcattgta aagtttttca tttttttttc cttttgtaaa attcctcttt 60

```
tttctctctg ttcttaatgt agttcttgcg atttgccgaa aattgaatct cgtttcttga    120
ttacagtttg tatccatgtg aatatgtcta gatttttttt tttttttttg aagtatagtc    180
attgatgatt agaccatcat ttcggtttta cctcgagatg tttaagacaa atgttgctgt    240
cttagatttg ttttgtgttc gttcataact tctggggaaa aaaatgagta aagatcaaga    300
ggttttgagt taatcatccg gtttaagtgg tattgaaagt agctacctcg gtattggtgt    360
taaaatgtct gtactccttg agcgctctgc gagagacaga tatcttcatt tttcatttaa    420
tagcaagtct caatcgttat ggatttgtat ccagatataa tcaactctaa gatcaaggac    480
ggtgtttgtt tttaagctaa ctttcttggg tctctaggtt tggtgtggtg agtttttggt    540
ttcttgtctt ccaagaaaaa aggctagtgt aaatgggaga gtttcacggt tcttcatttc    600
tttatttctt ttggtttgaa tgaaagtctt gtatggccag tactgtgtat gggcaatagg    660
caataacatg agcttgtacc tgctaatcag agaaatatag tggaactaag tttgatagcg    720
tgacgaaact atttatgatt aaatgatagc acttcgtagt tctaaaactt aaagaataac    780
agataaattt caaaatgtag aagtacaaaa aatgataaat acttttgtta gaaaatacct    840
aattcatctt tatgttctct tataaaatgg agacttacgt ttataagttc ttgtttcatc    900
gaatattgag caataattgg gggagaaatg gattgagcca attatatacc agagagcaca    960
tggaaccatc aatcaaattc caaacagctc attgcttctc ttatctattt tctattcatt   1020
tcacgaccgt cacagaaaaa aaaaaacagt ttcctatttc tttcactctt attatcagag   1080
agaaaacgtt cctactatga actgtattag ttaaaaaata caaagaacta aaacatatta   1140
tattcatttc tacaataaaa aacaaaaggc gatgacataa ctaaaatcag ctgcagtact   1200
ctttccaact gtttattttt tctttctttt tcaactaaga tctcagttgg ctttacagag   1260
attgatgtgc taataaagtt tttgttcacc aaatacaaag aattaaaaca attcgaaaat   1320
gattaattgt gttgatgtgt taatcaaact gtaaagtgat aattacacag ttggccttac   1380
atgcatctat gtgtatatat aaaccacaag tcacaacaca tcaaaacaca ccacaaaaca   1440
ctaaacatat agtctcttcg tgctttctca agaaccacct agagataacc ca           1492
```

<210> 180
<211> 1450
<212> DNA
<213> Arabidopsis thaliana

<220>
<221> promoter
<222> (1)..(1450)
<223> transcription regulating sequence from Arabidopsis thaliana gene At4g26320

<400> 180

```
tcattgtaaa gtttttcatt ttttttcctt ttgtaaaatt cctctgtttt ctctctgttc     60
ttcatgtagt tcttgcgatt tgccgaaaat tgaatctcgt ttcttgatta cagtttgtat    120
ccatgtgaat atgtctagat tttttttttt tttttttgaa gtatagtcat tgatgattag    180
accatcattt cggttttacc tcgagatgtt taagacaaat gttgctgtct tagatttgtt    240
ttgtgtccgt tcataacttt tggggaaaaa aatgagtaaa gatcaaaagg tttttgagtt    300
aatcatccgg tttaggtggt attgaaagta gctacctcgg tattggtgtt aaaatgtctg    360
tactccttga gctctctgcg agagacagat tttgtttaat gatcaatcaa acatttatca    420
aatctctgag cttagtatat cttcattttt catttaatag caagtctcaa tcgttatggc    480
```

```
tttgtatcca gatataatca actctaagat caaggacggt gtttgttttt aagctaactt     540
tcttgggtct ctaggtttgg tgtggtgagt ttttggtttc ttgtcttcca agaaaaaaag     600
gctagtgtaa atgggagagt ttcacggttc ttcatttctt tatttctttt ggtttgaatg     660
aaagtcttgt atggccagta ctgtgtatgg caataggcaa taacatgagc ttgtacctgc     720
taatcagaga aatataatgg aactaagttt gatagcgtga cgaaactatt tataattaaa     780
ttgtagtact tcgtagttct aaaacttgaa gaataacaga taaatttcaa aatgtagaat     840
acaaaaaatg ataaatactt ttgttagaaa atacctaatt catctttatg gagactcacg     900
tttataagtt cttgtttcat cgaatattga gcaataattg ggggagaaat ggaccgagcc     960
aattatatac cagagagcac atggaaccat caatcaaatt ccaaacagct cattgcttct    1020
cctatctatt ttctattcat ttcacagaaa aaaacaaaca gtttcctatt tctttcactc    1080
ttattatcag agagaaaacg ttcctactat gaactgtatt agttaaaaaa tacaaagaac    1140
taaaacatat tatattcatt tctacaataa aaaacaaaag gcgataacat aactaaaatc    1200
agctgcagta ctctttccaa ctgtttattt ttttcttcct ttttcaacta agatctcagt    1260
tggctttaca gagattgatg tgctaatata gtttttttc ctcaccaaat acaaagaatt    1320
aaaacaattc gaaaatgatt aactgtgttg atgttcatca aactgtaaag tgataattac    1380
acagttggcc ttacatgcat ctatgtgtat atataaacca caagtcacaa cacatcagaa    1440
cacaccacaa                                                           1450

<210>  181
<211>  1436
<212>  DNA
<213>  Arabidopsis thaliana

<220>
<221>  promoter
<222>  (1)..(1436)
<223>  transcription regulating sequence from Arabidopsis thaliana gene
       At4g26320

<400>  181
ataatgttat cttcattgta aagtttttca tttttttttc cttttgtaaa attcctcttt      60
tttctctctg ttcttaatgt agttcttgcg atttgccgaa aattgaatct cgtttcttga     120
ttacagtttg tatccatgtg aatatgtcta gatttttttt tttttttttg aagtatagtc     180
attgatgatt agaccatcat ttcggtttta cctcgagatg tttaagacaa atgttgctgt     240
cttagatttg ttttgtgttc gttcataact tctggggaaa aaaatgagta aagatcaaga     300
ggttttgagt taatcatccg gtttaagtgg tattgaaagt agctacctcg gtattggtgt     360
taaaatgtct gtactccttg agcgctctgc gagagacaga tatcttcatt tttcatttaa     420
tagcaagtct caatcgttat ggatttgtat ccagatataa tcaactctaa gatcaaggac     480
ggtgtttgtt tttaagctaa ctttcttggg tctctaggtt tggtgtggtg agtttttggt     540
ttcttgtctt ccaagaaaaa aggctagtgt aaatgggaga gtttcacggt tcttcatttc     600
tttatttctt ttggtttgaa tgaaagtctt gtatggccag tactgtgtat gggcaatagg     660
caataacatg agcttgtacc tgctaatcag agaaatatag tggaactaag tttgatagcg     720
tgacgaaact atttatgatt aaatgatagc acttcgtagt tctaaaactt aaagaataac     780
agataaattt caaaatgtag aagtacaaaa aatgataaat acttttgtta gaaaatacct     840
aattcatctt tatgttctct tataaaatgg agacttacgt ttataagttc ttgtttcatc     900
```

gaatattgag caataattgg gggagaaatg gattgagcca attatatacc agagagcaca 960
tggaaccatc aatcaaattc caaacagctc attgcttctc ttatctattt tctattcatt 1020
tcacgaccgt cacagaaaaa aaaaaacagt ttcctatttc tttcactctt attatcagag 1080
agaaaacgtt cctactatga actgtattag ttaaaaaata caaagaacta aaacatatta 1140
tattcatttc tacaataaaa aacaaaaggc gatgacataa ctaaaatcag ctgcagtact 1200
ctttccaact gtttattttt tctttctttt tcaactaaga tctcagttgg ctttacagag 1260
attgatgtgc taataaagtt tttgttcacc aaatacaaag aattaaaaca attcgaaaat 1320
gattaattgt gttgatgtgt taatcaaact gtaaagtgat aattacacag ttggccttac 1380
atgcatctat gtgtatatat aaaccacaag tcacaacaca tcaaaacaca ccacaa 1436

<210> 182
<211> 3019
<212> DNA
<213> Arabidopsis thaliana

<220>
<221> promoter
<222> (1)..(3019)
<223> transcription regulating sequence from Arabidopsis thaliana gene At4g26320

<400> 182
ttgactgaca agaaaaaaag gactgtagaa gagatgagtt ttgaagaagg taaccttgtg 60
aagactttag aaagaagatg aaggtggtgg cttgaagcca aagcagcgtt ttttagccgg 120
ttagcaccta aatatacaac gtaaacctgg aagacatgcc aaatccaaac ataaagaaat 180
tctgcaaatg aaaaggtata ttcatgcaca ggattaatga ttagtgattt acatgagaaa 240
ttgatgttgt tgcttgaatt gatgagtgaa gtagtaatgt aagagcaaga aagatccaac 300
caagaagtcg ttccattgat gttatacggc cggagaaaga tctagtttga cacatattgc 360
tgctacaaat cactcacact cttataacta attcaaactc aagcttatat taataatata 420
taatcaatca ttgtgacaaa ctcaacaaga cataggctcg cgagccacat cattctcact 480
tttaggtcta aacttgttaa tagctagatc gtctaattcc tctaacagta ggcctgtttt 540
gaaagatgtc tccatttgtt atgtatagct taacttttat catcaacgct ataacaaatt 600
gttaggatca ataagtagct taggaatgtt cctttgtaga gttttactta gaacaaagag 660
tggtaacttg caatttaggt gaaacctata gagaatgtag ctcaatgggt taaggtgaat 720
tctatagaca cattagcttg cgagtctcat cattctcact tcaactagaa gttaacataa 780
tatttcatca attcactaaa gaacataacc caaaaataca ttaaaaagga actaatatta 840
acagatctcc ggtaggttta cttctaataa tttgcaagaa ttcttattgt tgaagaatcg 900
aaacgaaata tagtgtagaa tcctttaatt ttgttcaaat actaactcaa aaaaggcttc 960
atatttgatt atttcctagt tgtttaatca ataaaatact ttaaaaaatg gattaggaac 1020
tattcgatca atgatgatga ctaaacatgg aagtcaacgt ggcaagatct agaaaagccc 1080
ccgacctctg tacattgtat acacatacac agacaaattg aacgaaaatc acggaatcat 1140
cattcagtta cagttttttt ttctgtgtgt gtatgaacat ggtgttcaac ggccagacgg 1200
ttatgtctgt agcccatttg tcggctgaga tttggcagcg tttacgattg atcccaccgt 1260
ccgatcgtat aagcagccga gagatgcttg agctagtctg cttctttccg ctccagcaat 1320
taggtcgatt ggctttatgt ctcttgactt gtctatgtct tccttctcca ggtttgctct 1380

atcctgaaac tgcggaagac gatcgtgatc acgtttatgt ttatggttcc tcttcttctt 1440
ccatcgctac atatcagcat cactttcgtc tgcattttga gtgatcaaaa tcatcaaact 1500
cgataatgtc atcttcattg taaagttttt catttttttt cctttgtaa aattcctctg 1560
ttttctctct gttcttcatg tagttcttgc gatttgccga aaattgaatc tcgtttcttg 1620
attacagttt gtatccatgt gaatatgtct agattttttt ttttttttt ttgaagtata 1680
gtcattgatg attagaccat catttcggtt ttacctcgag atgtttaaga caaatgttgc 1740
tgtcttagat ttgttttgtg tccgttcata actttgggg aaaaaaatga gtaaagatca 1800
aaaggttttt gagttaatca tccggtttag gtggtattga aagtagctac ctcggtattg 1860
gtgttaaaat gtctgtactc cttgagctct ctgcgagaga cagattttgt ttaatgatca 1920
atcaaacatt tatcaaatct ctgagcttag tatatcttca tttttcattt aatagcaagt 1980
ctcaatcgtt atggctttgt atccagatat aatcaactct aagatcaagg acggtgtttg 2040
tttttaagct aactttcttg ggtctctagg tttggtgtgg tgagttttg gtttcttgtc 2100
ttccaagaaa aaaaggctag tgtaaatggg agagtttcac ggttcttcat ttctttattt 2160
cttttggttt gaatgaaagt cttgtatggc cagtactgtg tatggcaata ggcaataaca 2220
tgagcttgta cctgctaatc agagaaatat aatggaacta agtttgatag cgtgacgaaa 2280
ctatttataa ttaaattgta gtacttcgta gttctaaaac ttgaagaata acagataaat 2340
ttcaaaatgt agaatacaaa aaatgataaa tacttttgtt agaaaatacc taattcatct 2400
ttatggagac tcacgtttat aagttcttgt ttcatcgaat attgagcaat aattggggga 2460
gaaatggacc gagccaatta tataccagag agcacatgga accatcaatc aaattccaaa 2520
cagctcattg cttctcctat ctattttcta ttcatttcac agaaaaaaac aaacagtttc 2580
ctatttcttt cactcttatt atcagagaga aaacgttcct actatgaact gtattagtta 2640
aaaaatacaa agaactaaaa catattatat tcatttctac aataaaaaac aaaaggcgat 2700
aacataacta aaatcagctg cagtactctt tccaactgtt tattttttc ttcctttttc 2760
aactaagatc tcagttggct ttacagagat tgatgtgcta atatagtttt ttttcctcac 2820
caaatacaaa gaattaaaac aattcgaaaa tgattaactg tgttgatgtt catcaaactg 2880
taaagtgata attacacagt tggccttaca tgcatctatg tgtatatata aaccacaagt 2940
cacaacacat cagaacacac cacaaaacac taaacatata atctcttcgt gctttctcaa 3000
gaaccaccta gagataacc 3019

<210> 183
<211> 3006
<212> DNA
<213> Arabidopsis thaliana

<220>
<221> promoter
<222> (1)..(3006)
<223> transcription regulating sequence from Arabidopsis thaliana gene At4g26320

<400> 183
tggacgagat cattgactga caagaaaaaa aggactgtag aagagatgag ttttgaagaa 60
ggtaaccttg tgaagacttt agaaagaaga tgaaggtggt ggcttgaagc caaagcagcg 120
ttttttagcc ggttagcacc taaatataca acgtaaacct ggaagacatg ccaaatccaa 180
acataaagaa attctgcaaa tgaaaaggta tattcatgca caggattaat gattagtgat 240

```
ttacatgaga aattgatgtt gttgcttgaa ttgatgagtg aagtagtaat gtaagagcaa     300
gaaagatcca accaagaagt cgttccattg atgttatacg gccggagaaa gatctagttt     360
gacacatatt gctgctacaa atcactcaca ctcttataac taattcaaac tcaagcttat     420
attaataata tataatcaat cattgtgaca aactcaacaa gacataggct cgcgagccac     480
atcattctca cttttaggtc taaacttgtt aatagctaga tcgtctaatt cctctaacag     540
taggcctgtt ttgaaagatg tctccatttg ttatgtatag cttaactttt atcatcaacg     600
ctataacaaa ttgttaggat caataagtag cttaggaatg ttcctttgta gagttttact     660
tagaacaaag agtggtaact tgcaatttag gtgaaaccta tagagaatgt agctcaatgg     720
gttaaggtga attctataga cacattagct tgcgagtctc atcattctca cttcaactag     780
aagttaacat aatatttcat caattcacta aagaacataa cccaaaaata cattaaaaag     840
gaactaatat taacagatct ccggtaggtt tacttctaat aatttgcaag aattcttatt     900
gttgaagaat cgaaacgaaa tatagtgtag aatcctttaa ttttgttcaa atactaactc     960
aaaaaaggct tcatatttga ttatttccta gttgtttaat caataaaata cttttaaaaa    1020
tggattagga actattcgat caatgatgat gactaaacat ggaagtcaac gtggcaagat    1080
ctagaaaagc ccccgacctc tgtacattgt atacacatac acagacaaat tgaacgaaaa    1140
tcacggaatc atcattcagt tacagttttt ttttctgtgt gtgtatgaac atggtgttca    1200
acggccagac ggttatgtct gtagcccatt tgtcggctga gatttggcag cgtttacgat    1260
tgatcccacc gtccgatcgt ataagcagcc gagagatgct tgagctagtc tgcttctttc    1320
cgctccagca attaggtcga ttggctttat gtctcttgac ttgtctatgt cttccttctc    1380
caggtttgct ctatcctgaa actgcggaag acgatcgtga tcacgtttat gtttatggtt    1440
cctcttcttc ttccatcgct acatatcagc atcactttcg tctgcatttt gagtgatcaa    1500
aatcatcaaa ctcgataatg ttatcttcat tgtaaagttt ttcatttttt tttccttttg    1560
taaaattcct ctttttctc tctgttctta atgtagttct tgcgatttgc cgaaaattga    1620
atctcgtttc ttgattacag tttgtatcca tgtgaatatg tctagatttt tttttttttt    1680
tttgaagtat agtcattgat gattagacca tcatttcggt tttacctcga gatgtttaag    1740
acaaatgttg ctgtcttaga tttgttttgt gttcgttcat aacttctggg gaaaaaaatg    1800
agtaaagatc aagaggtttt gagttaatca tccggtttaa gtggtattga aagtagctac    1860
ctcggtattg gtgttaaaat gtctgtactc cttgagcgct ctgcgagaga cagatatctt    1920
catttttcat ttaatagcaa gtctcaatcg ttatggattt gtatccagat ataatcaact    1980
ctaagatcaa ggacggtgtt tgtttttaag ctaactttct tgggtctcta ggtttggtgt    2040
ggtgagtttt tggtttcttg tcttccaaga aaaaaggcta gtgtaaatgg gagagtttca    2100
cggttcttca tttctttatt tcttttggtt tgaatgaaag tcttgtatgg ccagtactgt    2160
gtatgggcaa taggcaataa catgagcttg tacctgctaa tcagagaaat atagtggaac    2220
taagtttgat agcgtgacga aactatttat gattaaatga tagcacttcg tagttctaaa    2280
acttaaagaa taacagataa atttcaaaat gtagaagtac aaaaaatgat aaatactttt    2340
gttagaaaat acctaattca tctttatgtt ctcttataaa atggagactt acgtttataa    2400
gttcttgttt catcgaatat tgagcaataa ttgggggaga aatggattga gccaattata    2460
taccagagag cacatggaac catcaatcaa attccaaaca gctcattgct tctcttatct    2520
atttctatt catttcacga ccgtcacaga aaaaaaaaa cagtttccta tttctttcac    2580
tcttattatc agagagaaaa cgttcctact atgaactgta ttagttaaaa aatacaaaga    2640
actaaaacat attatattca tttctacaat aaaaaacaaa aggcgatgac ataactaaaa    2700
tcagctgcag tactctttcc aactgtttat tttttctttc tttttcaact aagatctcag    2760
ttggctttac agagattgat gtgctaataa agtttttgtt caccaaatac aaagaattaa    2820
aacaattcga aaatgattaa ttgtgttgat gtgttaatca aactgtaaag tgataattac    2880
acagttggcc ttacatgcat ctatgtgtat atataaacca caagtcacaa cacatcaaaa    2940
```

cacaccacaa aacactaaac atatagtctc ttcgtgcttt ctcaagaacc acctagagat 3000
aaccca 3006

<210> 184
<211> 2965
<212> DNA
<213> Arabidopsis thaliana

<220>
<221> promoter
<222> (1)..(2965)
<223> transcription regulating sequence from Arabidopsis thaliana gene At4g26320

<400> 184
ttgactgaca agaaaaaaag gactgtagaa gagatgagtt ttgaagaagg taaccttgtg 60
aagactttag aaagaagatg aaggtggtgg cttgaagcca aagcagcgtt ttttagccgg 120
ttagcaccta aatatacaac gtaaacctgg aagacatgcc aaatccaaac ataaagaaat 180
tctgcaaatg aaaaggtata ttcatgcaca ggattaatga ttagtgattt acatgagaaa 240
ttgatgttgt tgcttgaatt gatgagtgaa gtagtaatgt aagagcaaga aagatccaac 300
caagaagtcg ttccattgat gttatacggc cggagaaaga tctagtttga cacatattgc 360
tgctacaaat cactcacact cttataacta attcaaactc aagcttatat taataatata 420
taatcaatca ttgtgacaaa ctcaacaaga cataggctcg cgagccacat cattctcact 480
tttaggtcta aacttgttaa tagctagatc gtctaattcc tctaacagta ggcctgtttt 540
gaaagatgtc tccatttgtt atgtatagct taacttttat catcaacgct ataacaaatt 600
gttaggatca ataagtagct taggaatgtt cctttgtaga gttttactta gaacaaagag 660
tggtaacttg caatttaggt gaaacctata gagaatgtag ctcaatgggt taaggtgaat 720
tctatagaca cattagcttg cgagtctcat cattctcact tcaactagaa gttaacataa 780
tatttcatca attcactaaa gaacataacc caaaaataca ttaaaaagga actaatatta 840
acagatctcc ggtaggttta cttctaataa tttgcaagaa ttcttattgt tgaagaatcg 900
aaacgaaata tagtgtagaa tcctttaatt ttgttcaaat actaactcaa aaaaggcttc 960
atatttgatt atttcctagt tgtttaatca ataaaatact ttaaaaaatg gattaggaac 1020
tattcgatca atgatgatga ctaaacatgg aagtcaacgt ggcaagatct agaaaagccc 1080
ccgacctctg tacattgtat acacatacac agacaaattg aacgaaaatc acggaatcat 1140
cattcagtta cagttttttt ttctgtgtgt gtatgaacat ggtgttcaac ggccagacgg 1200
ttatgtctgt agcccatttg tcggctgaga tttggcagcg tttacgattg atcccaccgt 1260
ccgatcgtat aagcagccga gagatgcttg agctagtctg cttctttccg ctccagcaat 1320
taggtcgatt ggctttatgt ctcttgactt gtctatgtct tccttctcca ggtttgctct 1380
atcctgaaac tgcggaagac gatcgtgatc acgtttatgt ttatggttcc tcttcttctt 1440
ccatcgctac atatcagcat cactttcgtc tgcattttga gtgatcaaaa tcatcaaact 1500
cgataatgtc atcttcattg taaagttttt catttttttt ccttttgtaa aattcctctg 1560
ttttctctct gttcttcatg tagttcttgc gatttgccga aaattgaatc tcgtttcttg 1620
attacagttt gtatccatgt gaatatgtct agattttttt ttttttttt ttgaagtata 1680
gtcattgatg attagaccat catttcggtt ttacctcgag atgtttaaga caaatgttgc 1740
tgtcttagat ttgttttgtg tccgttcata acttttgggg aaaaaaatga gtaaagatca 1800

aaaggttttt gagttaatca tccggtttag gtggtattga aagtagctac ctcggtattg 1860
gtgttaaaat gtctgtactc cttgagctct ctgcgagaga cagattttgt ttaatgatca 1920
atcaaacatt tatcaaatct ctgagcttag tatatcttca tttttcattt aatagcaagt 1980
ctcaatcgtt atggctttgt atccagatat aatcaactct aagatcaagg acggtgtttg 2040
tttttaagct aactttcttg ggtctctagg tttggtgtgg tgagtttttg gtttcttgtc 2100
ttccaagaaa aaaaggctag tgtaaatggg agagtttcac ggttcttcat ttctttattt 2160
cttttggttt gaatgaaagt cttgtatggc cagtactgtg tatggcaata ggcaataaca 2220
tgagcttgta cctgctaatc agagaaatat aatggaacta agtttgatag cgtgacgaaa 2280
ctatttataa ttaaattgta gtacttcgta gttctaaaac ttgaagaata acagataaat 2340
ttcaaaatgt agaatacaaa aaatgataaa tacttttgtt agaaaatacc taattcatct 2400
ttatggagac tcacgtttat aagttcttgt ttcatcgaat attgagcaat aattggggga 2460
gaaatggacc gagccaatta tataccagag agcacatgga accatcaatc aaattccaaa 2520
cagctcattg cttctcctat ctatttcta ttcatttcac agaaaaaaac aaacagtttc 2580
ctatttcttt cactcttatt atcagagaga aaacgttcct actatgaact gtattagtta 2640
aaaaatacaa agaactaaaa catattatat tcatttctac aataaaaaac aaaaggcgat 2700
aacataacta aaatcagctg cagtactctt tccaactgtt tattttttc ttcctttttc 2760
aactaagatc tcagttggct ttacagagat tgatgtgcta atatagtttt ttttcctcac 2820
caaatacaaa gaattaaaac aattcgaaaa tgattaactg tgttgatgtt catcaaactg 2880
taaagtgata attacacagt tggccttaca tgcatctatg tgtatatata aaccacaagt 2940
cacaacacat cagaacacac cacaa 2965

<210> 185
<211> 2950
<212> DNA
<213> Arabidopsis thaliana

<220>
<221> promoter
<222> (1)..(2950)
<223> transcription regulating sequence from Arabidopsis thaliana gene At4g26320

<400> 185
tggacgagat cattgactga caagaaaaaa aggactgtag aagagatgag ttttgaagaa 60
ggtaaccttg tgaagacttt agaaagaaga tgaaggtggt ggcttgaagc caaagcagcg 120
ttttttagcc ggttagcacc taaatataca acgtaaacct ggaagacatg ccaaatccaa 180
acataaagaa attctgcaaa tgaaaaggta tattcatgca caggattaat gattagtgat 240
ttacatgaga aattgatgtt gttgcttgaa ttgatgagtg aagtagtaat gtaagagcaa 300
gaaagatcca accaagaagt cgttccattg atgttatacg gccggagaaa gatctagttt 360
gacacatatt gctgctacaa atcactcaca ctcttataac taattcaaac tcaagcttat 420
attaataata tataatcaat cattgtgaca aactcaacaa gacataggct cgcgagccac 480
atcattctca cttttaggtc taaacttgtt aatagctaga tcgtctaatt cctctaacag 540
taggcctgtt ttgaaagatg tctccatttg ttatgtatag cttaactttt atcatcaacg 600
ctataacaaa ttgttaggat caataagtag cttaggaatg ttcctttgta gagttttact 660
tagaacaaag agtggtaact tgcaatttag gtgaaaccta tagagaatgt agctcaatgg 720

```
gttaaggtga attctataga cacattagct tgcgagtctc atcattctca cttcaactag    780
aagttaacat aatatttcat caattcacta aagaacataa cccaaaaata cattaaaaag    840
gaactaatat taacagatct ccggtaggtt tacttctaat aatttgcaag aattcttatt    900
gttgaagaat cgaaacgaaa tatagtgtag aatcctttaa ttttgttcaa atactaactc    960
aaaaaaggct tcatatttga ttatttccta gttgtttaat caataaaata cttttaaaaa   1020
tggattagga actattcgat caatgatgat gactaaacat ggaagtcaac gtggcaagat   1080
ctagaaaagc ccccgacctc tgtacattgt atacacatac acagacaaat tgaacgaaaa   1140
tcacggaatc atcattcagt tacagttttt ttttctgtgt gtgtatgaac atggtgttca   1200
acggccagac ggttatgtct gtagcccatt tgtcggctga gatttggcag cgtttacgat   1260
tgatcccacc gtccgatcgt ataagcagcc gagagatgct tgagctagtc tgcttctttc   1320
cgctccagca attaggtcga ttggctttat gtctcttgac ttgtctatgt cttccttctc   1380
caggtttgct ctatcctgaa actgcggaag acgatcgtga tcacgtttat gtttatggtt   1440
cctcttcttc ttccatcgct acatatcagc atcactttcg tctgcatttt gagtgatcaa   1500
aatcatcaaa ctcgataatg ttatcttcat tgtaaagttt ttcatttttt tttcctttttg   1560
taaaattcct cttttttctc tctgttctta atgtagttct tgcgatttgc cgaaaattga   1620
atctcgtttc ttgattacag tttgtatcca tgtgaatatg tctagatttt tttttttttt   1680
tttgaagtat agtcattgat gattagacca tcatttcggt tttacctcga gatgtttaag   1740
acaaatgttg ctgtcttaga tttgttttgt gttcgttcat aacttctggg gaaaaaaatg   1800
agtaaagatc aagaggtttt gagttaatca tccggtttaa gtggtattga aagtagctac   1860
ctcggtattg gtgttaaaat gtctgtactc cttgagcgct ctgcgagaga cagatatctt   1920
catttttcat ttaatagcaa gtctcaatcg ttatggattt gtatccagat ataatcaact   1980
ctaagatcaa ggacggtgtt tgtttttaag ctaactttct tgggtctcta ggtttggtgt   2040
ggtgagtttt tggtttcttg tcttccaaga aaaaaggcta gtgtaaatgg gagagtttca   2100
cggttcttca tttctttatt tcttttggtt tgaatgaaag tcttgtatgg ccagtactgt   2160
gtatgggcaa taggcaataa catgagcttg tacctgctaa tcagagaaat atagtggaac   2220
taagtttgat agcgtgacga aactatttat gattaaatga tagcacttcg tagttctaaa   2280
acttaaagaa taacagataa atttcaaaat gtagaagtac aaaaaatgat aaatactttt   2340
gttagaaaat acctaattca tctttatgtt ctcttataaa atggagactt acgtttataa   2400
gttcttgttt catcgaatat tgagcaataa ttgggggaga aatggattga gccaattata   2460
taccagagag cacatggaac catcaatcaa attccaaaca gctcattgct tctcttatct   2520
attttctatt catttcacga ccgtcacaga aaaaaaaaaa cagtttccta tttctttcac   2580
tcttattatc agagagaaaa cgttcctact atgaactgta ttagttaaaa aatacaaaga   2640
actaaaacat attatattca tttctacaat aaaaaacaaa aggcgatgac ataactaaaa   2700
tcagctgcag tactctttcc aactgtttat tttttctttc tttttcaact aagatctcag   2760
ttggctttac agagattgat gtgctaataa agtttttgtt caccaaatac aaagaattaa   2820
aacaattcga aaatgattaa ttgtgttgat gtgttaatca aactgtaaag tgataattac   2880
acagttggcc ttacatgcat ctatgtgtat atataaacca caagtcacaa cacatcaaaa   2940
cacaccacaa                                                          2950
```

<210> 186
<211> 371
<212> DNA
<213> Arabidopsis thaliana

<220>

```
<221>  CDS
<222>  (57)..(236)
<223>  encoding Arabidopsis thaliana arabinogalactan-protein (AGP13)

<400>  186
aacactaaac atatagtctc ttcgtgcttt ctcaagaacc acctagagat aaccca atg     59
                                                              Met
                                                              1
gag gca atg aag atg aga ctc ttt gtg gcg gtt ttg gtg gca gcg atg      107
Glu Ala Met Lys Met Arg Leu Phe Val Ala Val Leu Val Ala Ala Met
            5                   10                  15
gct ttc tct gcg gtg caa cag gct gcc gcg gtg gag gct cca gct ccg      155
Ala Phe Ser Ala Val Gln Gln Ala Ala Ala Val Glu Ala Pro Ala Pro
        20                  25                  30
agt cct acc tcc gat gct tcc ttg gcc atc ccc gct ttc ttc gcc tcg      203
Ser Pro Thr Ser Asp Ala Ser Leu Ala Ile Pro Ala Phe Phe Ala Ser
    35                  40                  45
gta gcc act ttg gcc ttt ggg ttt ctc ttc tag acaaatttgt ttttgtcttt    256
Val Ala Thr Leu Ala Phe Gly Phe Leu Phe
50                  55
ttaatgttat aaaaatcata ttcttgtttt tttatttcgg ttttcattct tatgtactgt    316
tctaagattt tccacatttg aatcaataaa attatctcgg tcactttttt tttat         371

<210>  187
<211>  59
<212>  PRT
<213>  Arabidopsis thaliana

<400>  187
Met Glu Ala Met Lys Met Arg Leu Phe Val Ala Val Leu Val Ala Ala
1               5                   10                  15

Met Ala Phe Ser Ala Val Gln Gln Ala Ala Ala Val Glu Ala Pro Ala
            20                  25                  30

Pro Ser Pro Thr Ser Asp Ala Ser Leu Ala Ile Pro Ala Phe Phe Ala
        35                  40                  45

Ser Val Ala Thr Leu Ala Phe Gly Phe Leu Phe
    50                  55

<210>  188
<211>  29
<212>  DNA
<213>  Artificial
```

<220>
<223> oligonucleotide primer

<400> 188
aggatccagc gagtatttga ttttatgtg 29

<210> 189
<211> 27
<212> DNA
<213> Artificial

<220>
<223> oligonucleotide primer

<400> 189
tccatggtat ccgttgtaga agatatc 27

<210> 190
<211> 33
<212> DNA
<213> Artificial

<220>
<223> oligonucleotide primer

<400> 190
tcacagccat ggcgttacca ttctttttaa atg 33

<210> 191
<211> 29
<212> DNA
<213> Artificial

<220>
<223> oligonucleotide primer

<400> 191
tggatccaag tgctctaaac tgacaaaac 29

<210> 192
<211> 27
<212> DNA
<213> Artificial

<220>
<223> oligonucleotide primer

<400> 192
tccatggtat ccgttgtaga agatatc 27

<210> 193
<211> 33
<212> DNA
<213> Artificial

<220>
<223> oligonucleotide primer

<400> 193
tcacagccat ggcgttacca ttctttttaa atg 33

<210> 194
<211> 26
<212> DNA
<213> Artificial

<220>
<223> oligonucleotide primer

<400> 194
cactggatcc ttcttcttct tctacg 26

<210> 195
<211> 26
<212> DNA
<213> Artificial

<220>
<223> oligonucleotide primer

<400> 195
tccatggttg ttgttgttgt agcagc 26

<210> 196
<211> 30
<212> DNA
<213> Artificial

<220>

<223> oligonucleotide primer

<400> 196
agaatgccat ggaaggtctc acacctatat 30

<210> 197
<211> 29
<212> DNA
<213> Artificial

<220>
<223> oligonucleotide primer

<400> 197
tggatccatc aatagaagag taattaatc 29

<210> 198
<211> 26
<212> DNA
<213> Artificial

<220>
<223> oligonucleotide primer

<400> 198
tccatggttg ttgttgttgt agcagc 26

<210> 199
<211> 30
<212> DNA
<213> Artificial

<220>
<223> oligonucleotide primer

<400> 199
agaatgccat ggaaggtctc acacctatat 30

<210> 200
<211> 29
<212> DNA
<213> Artificial

<220>
<223> oligonucleotide primer

<400> 200
aatatactcg agatccgcac tgactagac 29

<210> 201
<211> 28
<212> DNA
<213> Artificial

<220>
<223> oligonucleotide primer

<400> 201
tactgcccat ggtccttttc taccacac 28

<210> 202
<211> 29
<212> DNA
<213> Artificial

<220>
<223> oligonucleotide primer

<400> 202
tggttcccat ggagagaaaa aaaagaaag 29

<210> 203
<211> 28
<212> DNA
<213> Artificial

<220>
<223> oligonucleotide primer

<400> 203
cttccgctcg agttttatgt caaagacg 28

<210> 204
<211> 28
<212> DNA
<213> Artificial

<220>
<223> oligonucleotide primer

<400> 204
tactgcccat ggtccttttc taccacac 28

<210> 205
<211> 29
<212> DNA
<213> Artificial

<220>
<223> oligonucleotide primer

<400> 205
tggttcccat ggagagaaaa aaaagaaag 29

<210> 206
<211> 29
<212> DNA
<213> Artificial

<220>
<223> oligonucleotide primer

<400> 206
ataatgggat cctcattgta aagtttttc 29

<210> 207
<211> 25
<212> DNA
<213> Artificial

<220>
<223> oligonucleotide primer

<400> 207
ttgcctccat ggggttatct ctagg 25

<210> 208
<211> 30
<212> DNA
<213> Artificial

<220>
<223> oligonucleotide primer

<400> 208

ttgtggccat ggttgatgtg ttgtgacttg 30

<210> 209
<211> 24
<212> DNA
<213> Artificial

<220>
<223> oligonucleotide primer

<400> 209
tggacgggat ccttgactga caag 24

<210> 210
<211> 25
<212> DNA
<213> Artificial

<220>
<223> oligonucleotide primer

<400> 210
ttgcctccat ggggttatct ctagg 25

<210> 211
<211> 30
<212> DNA
<213> Artificial

<220>
<223> oligonucleotide primer

<400> 211
ttgtggccat ggttgatgtg ttgtgacttg 30

SEQUENCE LISTING

<110> SunGene GmbH&co.KGaA

<120> Expression cassettes for root preferential expression in plants

<130> AE20040857 / PF 56073-2 EP

<160> 69

<170> PatentIn version 3.3

<210> 1
<211> 1656
<212> DNA
<213> Arabidopsis thaliana

<220>
<221> promoter
<222> (1)..(1656)
<223> transcription regulating sequence from Arabidopsis thaliana gene At1g66280

<400> 1
```
gtttggccta aatgtgtaat gctgttactt cttttgactc aaagacatgt ttttgctcat      60
tagtaattca gacttcgagt agaaaaatag ataatggatt acaaaaacga aaaataaaat     120
aaagatgctt caaatttaac ctaaaatagt attagatctt tgtaagacac taagacccaa     180
ggattaaaca ttctaggaac taaattataa ttaaactctt ggaaaattaa gatctatttg     240
tttaggagct tttaagatat acatgtctag gctttaatga gctttacgac cgaagaaaat     300
tagagagctt gtgattttga tcatgatctg agtgagtctc aaaaactaaa aggaggacaa     360
taatgaagat tcgaattgaa ttctttaaac tagttaacta ggttaattgt tttatgggcc     420
tgttatgtta agttaataat ttttggggcc ttttatcttc aacatataaa gggctcttct     480
attgtttttt tccaactcca acaactaaaa tacaaacaaa ttttggacct tttattgtgg     540
accgtaggca gatgcccatg tttccatggg tgagagacgg acctgaggtg gtcgcaactc     600
gcaaccgacg ttcagaggga gaagcggagg tggtggtgga agcaaagaga gttagatgat     660
ggctgtggtg aggactatgt aaagtgtaaa catgtgtcac ttatctttgc ttattttaca     720
gttggaggaa atgccgaaat tgtatgtacc aagaaaatat acatgtatct gtttttatac     780
ttgttttaat gtctacagtg ttcgataaag ataccctgat caacttcata atgtaacaaa     840
aatgaagtca tgtatcaccg tagtggagtt aggtttgatg aattttaaaa ataaaactaa     900
ttcaactagc ttaaaattaa ttatcgccaa ataagtacca aacgtagccc gggctaaacc     960
ttagtatttt aaatatgcta aaaatctatt tacagataga tggtgcatcg ccatcaagta    1020
gcaaaataag cgtcagcgca atactatcaa cgaaaaaaga tggtatgtta aaaaaaggtt    1080
```